# EUROPÄISCHE PATENTSCHRIFT

(11) **EP 3 135 110 B1**
(45) Veröffentlichungstag und Bekanntmachung des Hinweises auf die Patenterteilung: **10.07.2019**
(21) Anmeldenummer: 15182597.3
(22) Anmeldetag: 26.08.2015
(51) Int. Cl.: A01N 35/02, A01N 47/44, A01N 55/02, A01N 55/08, A01N 57/34, C07F 9/54, C07C 279/08, C07C 217/08, C07C 217/18, C07C 217/20

(54) **1,7-DIARYL-1,6-HEPTADIEN-3,5-DION-DERIVATE, VERFAHREN ZUR HERSTELLUNG UND VERWENDUNG DERSELBEN**
1,7-DIARYL-1,6-HEPTADIEN-3,5-DION DERIVATIVES, METHODS OF MAKING AND USING THE SAME
DERIVES DE 1,7-DIARYL-1,6-HEPTADIENE -3,5-DIONE ET SON PROCEDE DE FABRICATION ET UTILISATION

(43) Veröffentlichungstag der Anmeldung: 01.03.2017
(73) Patentinhaber: TriOptoTec GmbH, 93053 Regensburg (DE)
(72) Erfinder: SPÄTH, Andreas, 93073 Neutraubling (DE); PLÄTZER, Kristjan, 5023 Salzburg (AT); MAISCH, Tim, 90425 Nürnberg (DE); EICHNER, Anja, 93051 Regensburg (DE)
(74) Vertreter: Louis Pöhlau Lohrentz

(56) Entgegenhaltungen:
- CN-A- 101 570 496
- FERNANDO LUIS ESTEBAN FLOREZ ET AL: "Viability study of antimicrobial photodynamic therapy using curcumin, hypericin and photogem photosensitizers in planktonic cells of Streptococcus mutans", SCIENTIFIC JOURNAL OF DENTISTRY, Bd. 2, 1. Januar 2015 (2015-01-01), Seiten 22-27, XP055242379, ISSN: 2394-7144, DOI: 10.15713/ins.sjod.18
- WAINWRIGHT M: "PHOTODYNAMIC ANTIMICROBIAL CHEMOTHERAPY (PACT)", JOURNAL OF ANTIMICROBIAL CHEMOTHERAPY, OXFORD UNIVERSITY PRESS, GB, Bd. 42, 1. Januar 1998 (1998-01-01), Seiten 13-28, XP002949235, ISSN: 0305-7453, DOI: 10.1093/JAC/42.1.13
- Tyler G St Denis ET AL: "An Introduction to Photoantimicrobials: Photodynamic Therapy as a Novel Method of Microbial Pathogen Eradication", Science against microbial pathogens: communicating current research and technological advances, 1. Januar 2011 (2011-01-01), Seiten 675-683, XP055242748, Gefunden im Internet: URL:http://www.formatex.info/microbiology3 /book/675-683.pdf [gefunden am 2016-01-19]
- LUDMILA M. BALTAZAR ET AL: "Antimicrobial photodynamic therapy: an effective alternative approach to control fungal infections", FRONTIERS IN MICROBIOLOGY, Bd. 6, 13. März 2015 (2015-03-13), Seite 202, XP055242757, DOI: 10.3389/fmicb.2015.00202
- J.R. MANJUNATHA ET AL: "Synthesis of amino acid conjugates of tetrahydrocurcumin and evaluation of their antibacterial and anti-mutagenic properties", FOOD CHEMISTRY, Bd. 139, Nr. 1-4, 1. August 2013 (2013-08-01), Seiten 332-338, XP055242653, NL ISSN: 0308-8146, DOI: 10.1016/j.foodchem.2013.01.081
- ABDELLAH FELOUAT ET AL: "Synthesis and Photophysical Properties of Difluoroboron Complexes of Curcuminoid Derivatives Bearing Different Terminal Aromatic Units and a meso-Aryl Ring", THE JOURNAL OF ORGANIC CHEMISTRY, Bd. 78, Nr. 9, 3. Mai 2013 (2013-05-03), Seiten 4446-4455, XP055242587, US ISSN: 0022-3263, DOI: 10.1021/jo400389h
- FANG XUBIN ET AL: "Design and synthesis of dimethylaminomethyl-substituted curcumin derivatives/analogues: Potent antitumor and antioxidant activity, improved stability and aqueous solubility compared with curcumin", BIOORGANIC & MEDICINAL CHEMISTRY LETTERS, PERGAMON, AMSTERDAM, NL, Bd. 23, Nr. 5, 9. Januar 2013 (2013-01-09) , Seiten 1297-1301, XP028976439, ISSN: 0960-894X, DOI: 10.1016/J.BMCL.2012.12.098
- JAMES A. LENHART ET AL: ""Clicked" Bivalent Ligands Containing Curcumin and Cholesterol As Multifunctional A[beta] Oligomerization Inhibitors: Design, Synthesis, and Biological Characterization", JOURNAL OF MEDICINAL CHEMISTRY, Bd. 53, Nr. 16, 26. August 2010 (2010-08-26), Seiten 6198-6209, XP55135432, ISSN: 0022-2623, DOI: 10.1021/jm100601q
- RAGHAVAN SAIHARISH ET AL: "Synthesis and anticancer activity of novel curcumin-quinolone hyb", BIOORGANIC & MEDICINAL CHEMISTRY LETTERS, Bd. 25, Nr. 17, 26. Juni 2015 (2015-06-26) , Seiten 3601-3605, XP029249453, ISSN: 0960-894X, DOI: 10.1016/J.BMCL.2015.06.068
- XUELI ZHANG ET AL: "Near-infrared fluorescence molecular imaging of amyloid beta species and monitoring therapy in animal models of Alzheimer's disease", PROCEEDINGS OF THE NATIONAL ACADEMY OF SCIENCES, Bd. 112, Nr. 31, 21. Juli 2015 (2015-07-21), Seiten 9734-9739, XP55242549, US ISSN: 0027-8424, DOI: 10.1073/pnas.1505420112
- LEI FANG ET AL: "Design, synthesis and anti-Alzheimer properties of dimethylaminomethyl-substituted curcumin derivatives", BIOORGANIC & MEDICINAL CHEMISTRY LETTERS, Bd. 24, Nr. 1, 1. Januar 2014 (2014-01-01) , Seiten 40-43, XP055242615, AMSTERDAM, NL ISSN: 0960-894X, DOI: 10.1016/j.bmcl.2013.12.011
- CHERUKU APOORVA REDDY ET AL: "Mitochondrial-Targeted Curcuminoids: A Strategy to Enhance Bioavailability and Anticancer Efficacy of Curcumin", PLOS ONE, Bd. 9, Nr. 3, 12. März 2014 (2014-03-12), Seite e89351, XP55243282, DOI: 10.1371/journal.pone.0089351

## Beschreibung

Die vorliegende Erfindung betrifft 1,7-Diaryl-1,6-heptadien-3,5-dion-Derivate, deren Herstellung und Verwendung.
Durch das Auftreten immer neuer multiresistenter Bakterien-Isolate ist die Therapie bakterieller Erkrankungen schwieriger geworden. Zunehmend strengere Hygienestandards und eine weltweite Ausbreitung an nosokomialen Infektionen haben das Interesse für neue Präparate, Methoden und Anwendungen, die eine Ausbreitung von multiresistenten Keimen verhindern könnten, geweckt.

Die Forschung nach Alternativen zur Antibiotikatherapie ist von enormer Bedeutung für die Behandlung von Infektionen, die beispielsweise durch Bakterien verursacht werden, insbesondere infolge der Identifizierung und dem vermehrtem Auftreten vacomycinresistenter Bakterienstämme (VRSA) bereits im Jahr 2002 in Japan und den USA. In Europa wurde 2013 das ersten VRSA Patientenisolat in Portugal isoliert.
Auch die Zunahme von Resistenzen bei Pilzinfektionen gegenüber Antipilzpräparaten verschärft zusehends die Problematik bei der Behandlung von oberflächlichen Infektionen. Die klinische Konsequenz der Resistenz gegenüber Antipilzpräparaten zeigt sich durch ein Versagen der Behandlung, ganz besonders bei immunsupprimierten Patienten.
Neue Ansätze zur Bekämpfung von resistenten bzw. multiresistenten Krankheitskeimen sind daher einerseits die Suche nach neuen Gegenmitteln, beispielsweise Antibiotika oder Antimikotika, und andererseits die Suche nach alternativen Inaktivierungsmöglichkeiten.

Als alternatives Verfahren hat sich die photodynamische Inaktivierung von Mikroorganismen bewährt. Bei der photodynamischen Inaktivierung von Mikroorganismen spielen zwei photooxidative Prozesse eine entscheidende Rolle.

Ein Photosensibilisator wird durch Licht einer bestimmten Wellenlänge angeregt. Der angeregte Photosensibilisator kann die Bildung von reaktiven Sauerstoffspezies (ROS) bewirken, wobei einerseits Radikale, beispielsweise Superoxidanionen, Wasserstoffperoxid oder Hydroxylradikale, und/oder andererseits angeregter molekularer Sauerstoff, beispielsweise Singulett-Sauerstoff, gebildet werden können.

Bei beiden Reaktionen steht die Photooxidation von spezifischen Biomolekülen, die sich in direkter Nachbarschaft zu den reaktiven Sauerstoffspezies (ROS) befindet, im Vordergrund. Dabei findet insbesondere eine Oxidation von Lipiden und Proteinen statt, die beispielsweise als Bestandteile der Zellmembran von Mikroorganismen vorkommen. Durch die Zerstörung der Zellmembran wiederum kommt es zur Inaktivierung der betreffenden Mikroorganismen. Für Viren und Pilze wird ein ähnlicher Eliminationsprozess angenommen.

Beispielsweise werden durch Singulett-Sauerstoff vorzugsweise oxidationsempfindliche Moleküle angegriffen. Oxidationsempfindliche Moleküle sind beispielsweise Moleküle, die Doppelbindungen oder oxidationsempfindliche Gruppen wie Phenole, Sulfide oder Thiole enthalten. Besonders anfällig für eine Schädigung sind allerdings ungesättigte Fettsäuren in den Membranen von Bakterien.

Aus dem Stand der Technik sind zahlreiche Photosensibilisatoren bekannt, die beispielsweise aus der Gruppe der Porphyrine und deren Derivate oder Phthalocyanine und deren Derivate oder Fullerene und deren Derivate oder Derivate der Phenothiazinium-Struktur, wie beispielsweise Methylenblau oder Toluidinblau, oder Vertreter der Phenoxanzinium-Reihe, wie beispielsweise Nile blue, stammen. Die Photodynamik von Methylenblau bzw. Toluidinblau gegenüber Bakterien wird beispielsweise bereits in der Zahnheilkunde eingesetzt.

Bei den aus dem Stand der Technik bekannten Photosensibilisatoren handelt es sich zumeist um Substanzen mit einer relativ komplexen Molekülstruktur und daher aufwendigen Herstellungsverfahren.

Curcumin (1,7-Bis-(4-hydroxy-3-methoxyphenyl)-1,6-heptadien-3,5-dion ist eines der wichtigsten Curcuminoide in Kurkuma. Kurkuma (*Curcuma longa*) ist eine Pflanzenart innerhalb der Familie der Ingwergewächse. Das Rhizom ist intensiv gelb gefärbt. Die gelbe Färbung entsteht hauptsächlich durch Curcumin, Desmethoxycurcumin und Bisdesmethoxycurcumin.

Natürliches Curcumin weist eine geringe Photostabilität auf. Das Ausbleichen einer Curcumin-Lösung erfolgt bei Tageslicht beispielsweise innerhalb von 30 min.

Natürliches Curcumin weist weiterhin eine geringe Wasserlöslichkeit auf. In vielen Fällen ist die Verwendung von Löslichkeitsvermittlern wie DMSO oder anderen, nicht biokompatiblen Stoffen erforderlich, um eine Anwendung in wässrigem Milieu zu ermöglichen. Auch ist die Wirksamkeit gegenüber gram-negative Bakterien begrenzt, weil die Zellwandstruktur dieser Mikroorganismen eine effiziente Aufnahme des Curcumins verhindert.

Die Nicht-Patentliteratur Florez, L.E.F et al. (Sci. J. Dent. 2015, 2, Seiten 22 bis 27; DOI: 10.15713/ins.sjod.18) beschreibt die Verwendung von Curcumin, Hypericin und Photogem bei der antimikrobiellen photodynamischen Therapie. Die Nicht-Patentliteratur Baltazar, L.M et al. (Front. Microbiol. 2015, 6, Seiten 202; DOI: 10.3389/fmicb.2015.00202) beschreibt die Verwendung der antimikrobiellen photodynamischen Therapie zur Kontrolle von Pilzinfektionen. Die Nicht-Patentliteratur Manjunatha, J.R. et al. (Food Chem. 2013,136(2), Seiten 650 bis 658; DOI: 10.1016/j.foodchem.2012.08.052) beschreibt die Synthese von Aminosäure-Konjugaten von Tetrahydrocurcumin und eine Evaluierung ihrer antibakteriellen Eigenschaften. Die Nicht-Patentliteratur Felouat, A. et al. (J. Org. Chem. 2013, 78 (9), Seiten 4446 bis 4455; DOI: 10.1021/jo400389h) beschreibt die Synthese und photophysikalischen Eigenschaften von Difluoroboronkomplexen von Curcuminoid-Derivaten mit unterschiedlichen terminalen aromatischen Resten und einem meso-Aryl Ring. Die Nicht-Patentliteratur Raghavan, S. et al. (Bioorg. Med. Chem. Lett. 2015, 25(17), Seiten 3601 bis 3605; DOI: 10.1016/j.bmcl.2015.06.068) beschreibt die Synthese und Antikrebs-Aktivität von neuen Curcumin-Quinolon-Hybriden.
Eine Aufgabe der vorliegenden Erfindung ist es daher, neue Photosensibilisatoren bereitzustellen, die Mikroorganismen effizienter inaktivieren.
Eine weitere Aufgabe der vorliegenden Erfindung ist es, neue Photosensibilisatoren bereitzustellen, die eine photodynamische Entkeimung von Gegenständen und/oder Flüssigkeiten und/oder Patienten bei der Therapie und/oder Prophylaxe ermöglicht.

Die Aufgabe der vorliegenden Erfindung wird durch die Bereitstellung eines nichttherapeutischen Verfahrens zur Inaktivierung von Mikroorganismen, die vorzugsweise Viren, Archäeen, Bakterien, Bakteriensporen, Pilzen, Pilzsporen, Protozoen, Algen, blutübertragbaren Parasiten oder Kombinationen davon umfassen, gelöst, wobei das Verfahren folgende Schritte umfasst:
(A) in Kontakt bringen der Mikroorganismen mit wenigstens einem Photosensibilisator, wobei der Photosensibilisator wenigstens ein 1,7-Diaryl-1,6-heptadien-3,5-dion-Derivat der Formel (100): 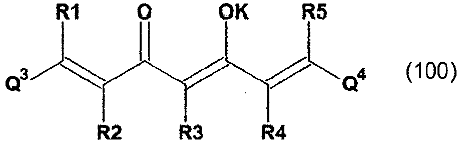 und/oder wenigstens ein 1,7-Diaryl-1,6-heptadien-3,5-dion-Derivat der Formel (101): 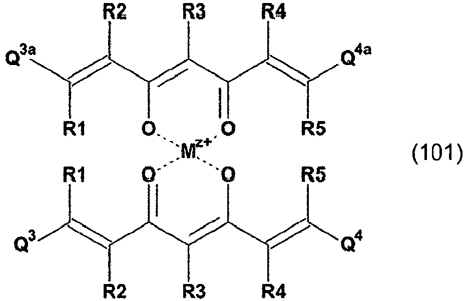 oder jeweils ein pharmakologisch verträgliches Salz und/oder Ester und/oder Komplex davon ist,
   wobei die Reste Q³, Q^{3a}, Q⁴ und Q^{4a} jeweils unabhängig voneinander 1 substituierter oder unsubstituierter monozyklischer oder polyzyklischer, aromatischer Rest oder 1 substituierter oder unsubstituierter monozyklischer oder polyzyklischer, heteroaromatischer Rest bedeuten,
   wobei die Verbindung mit der Formel (100) keine OH-Gruppe, die direkt an den organischen Rest Q³ oder Q⁴ gebunden ist, enthält, und
   wobei die Verbindung mit der Formel (101) keine OH-Gruppe, die direkt an den organischen Rest Q³, Q^{3a}, Q⁴, oder Q^{4a} gebunden ist, enthält, und
   wobei K Wasserstoff oder ein Kation bedeutet, und
   wobei M^{z+} ein Kation eines Metalls bedeutet, wobei z die formale Oxidationszahl des Metalls M ist und z eine ganze Zahl von 1 bis 7, vorzugsweise von 2 bis 5, bedeutet, und wobei
   (a1) wenigstens einer der Reste Q³, Q^{3a}, Q⁴ und Q^{4a} jeweils unabhängig voneinander ein unsubstituierter monozyklischer oder polyzyklischer, heteroaromatischer Rest ist, der wenigstens 5 Ringatome aufweist, wobei die Ringatome mindestens 1 Kohlenstoffatom und wenigstens 1, vorzugsweise protonierbares, Stickstoffatom enthalten, oder
   (a2) wenigstens einer der Reste Q³, Q^{3a}, Q⁴ und Q^{4a}, vorzugsweise jeder der Reste Q³ und Q⁴, vorzugsweise jeder der Reste Q³ und Q^{3a}, vorzugsweise jeder der Reste Q³, Q^{3a} und Q⁴, vorzugsweise jeder der Reste Q³, Q^{3a}, Q⁴ und Q^{4a}, jeweils unabhängig voneinander mit mindestens einem, vorzugsweise 1 bis 9, weiter bevorzugt 1 bis 7, weiter bevorzugt 1 bis 5, weiter bevorzugt 1 bis 4, weiter bevorzugt 2 bis 3, organischen Rest(en) W1, der die allgemeine Formel (4), (5), (6), (7), (8), oder (9), vorzugsweise (5), (7), oder (9), aufweist:

      -(C(D)(E))ₕ-X, (4)

      -A-(C(D)(E))ₕ-X, (5)

      -(C(D)(E))ₖ-Aryl-(C(D)(E))ₗ-X, (6)

      -A-(C(D)(E))ₖ-Aryl-(C(D)(E))ₗ-X, (7)

      -((C(D)(E))ₘ-A)ₚ-(C(D)(E))ₙ-X, (8),

      -A-((C(D)(E))ₘ-A)ₚ-(C(D)(E))ₙ-X, (9),

      substituiert ist,
      wobei h eine ganze Zahl von 1 bis 20, vorzugsweise von 2 bis 8, bedeutet, wobei k eine ganze Zahl von 0 bis 10, vorzugsweise von 1 bis 8, vorzugsweise von 2 bis 6, bedeutet, wobei I eine ganze Zahl von 0 bis 10, vorzugsweise von 1 bis 8, vorzugsweise von 2 bis 6, bedeutet, und wobei m, n und p jeweils unabhängig voneinander eine ganze Zahl von 1 bis 6, vorzugsweise von 2 bis 4, bedeuten, und
      wobei A jeweils unabhängig voneinander Sauerstoff oder Schwefel, vorzugsweise Sauerstoff, bedeutet,
      wobei D und E jeweils unabhängig voneinander Wasserstoff, Halogen, G-R^{(I)}, oder G-C(=G)-R^{(II)}, bedeuten, wobei G jeweils unabhängig voneinander Sauerstoff oder Schwefel, vorzugsweise Sauerstoff, bedeutet, und wobei die Reste R^{(I)} und R^{(II)} jeweils unabhängig voneinander Wasserstoff, Methyl, Ethyl, n-Propyl, n-Butyl, n-Pentyl, Phenyl oder Benzyl bedeutet, wobei Phenyl und Benzyl unsubstituiert oder substituiert sein können,
      wobei Aryl einen substituierten oder unsubstituierten Aromaten oder einen substituierten oder unsubstituierten Heteroaromaten, der kein Stickstoffatom enthält, bedeutet,
      wobei X jeweils unabhängig voneinander ein organischer Rest ist, der (i) wenigstens ein neutrales, protonierbares Stickstoffatom, oder (ii) wenigstens ein positiv geladenes, vorzugsweise quartäres, Stickstoffatom oder (iii) wenigstens ein positiv geladenes, vorzugsweise quartäres, Phosphoratom enthält, und
      wobei die Reste R1, R2, R3, R4 und R5 jeweils unabhängig voneinander Wasserstoff, Halogen, Alkyl mit 1 bis 12 C-Atomen, Cycloalkyl mit 1 bis 12 C-Atomen, Alkylaryl mit 1 bis 12 C-Atomen, Aryl mit 5 bis 20 C-Atomen, Ether mit 2 bis 12 C-Atomen oder Glykol mit 2 bis 12 C-Atomen bedeuten,
      oder wobei
   (b) der Rest R3 ein organischer Rest W2 ist, der die allgemeine Formel (4), (5), (6), (7), (8), (9), oder (10), vorzugsweise (4), aufweist:

      -(C(D)(E))ₕ-X, (4)

      -A-(C(D)(E))ₕ-X, (5)

      -(C(D)(E))ₖ-Aryl-(C(D)(E))ₗ-X, (6)

      -A-(C(D)(E))ₖ-Aryl-(C(D)(E))ᵢ-X, (7)

      -[(C(D)(E))m-A]p-(C(D)(E))n-X, (8)

      -A-[(C(D)(E))ₘ-A]ₚ-(C(D)(E))ₙ-X, (9)

      -(-C(D)=C(E)-)ᵣ-X, (10),

      und
      wobei, optional, wenigstens einer der Reste Q³, Q^{3a}, Q⁴ und Q^{4a}, vorzugsweise jeder der Reste Q³ und Q⁴, vorzugsweise jeder der Reste Q³ und Q^{3a}, vorzugsweise jeder der Reste Q³, Q^{3a} und Q⁴, vorzugsweise jeder der Reste Q³, Q^{3a}, Q⁴ und Q^{4a}, jeweils unabhängig voneinander mit mindestens einem, vorzugsweise 1 bis 9, weiter bevorzugt 1 bis 7, weiter bevorzugt 1 bis 5, weiter bevorzugt 1 bis 4, weiter bevorzugt 2 bis 3, organischen Rest(en) W1, der die allgemeine Formel (4), (5), (6), (7), (8), oder (9), vorzugsweise (5), (7), oder (9), aufweist, substituiert ist,
      wobei h eine ganze Zahl von 1 bis 20, vorzugsweise von 2 bis 8, bedeutet, wobei k eine ganze Zahl von 0 bis 10, vorzugsweise von 1 bis 8, vorzugsweise von 2 bis 6, bedeutet, wobei I eine ganze Zahl von 0 bis 10, vorzugsweise von 1 bis 8, vorzugsweise von 2 bis 6, bedeutet, und wobei m, n, p, und r jeweils unabhängig voneinander eine ganze Zahl von 1 bis 6, vorzugsweise von 2 bis 4, bedeuten, und
      wobei A jeweils unabhängig voneinander Sauerstoff oder Schwefel, vorzugsweise Sauerstoff, bedeutet,
      wobei D und E jeweils unabhängig voneinander Wasserstoff, Halogen, G-R^{(I)}, oder G-C(=G)-R^{(II)}, bedeuten, wobei G jeweils unabhängig voneinander Sauerstoff oder Schwefel, vorzugsweise Sauerstoff, bedeutet, und wobei die Reste R^{(I)} und R^{(II)} jeweils unabhängig voneinander Wasserstoff, Methyl, Ethyl, n-Propyl, n-Butyl, n-Pentyl, Phenyl oder Benzyl bedeutet, wobei Phenyl und Benzyl unsubstituiert oder substituiert sein können,
      wobei Aryl einen substituierten oder unsubstituierten Aromaten oder einen substituierten oder unsubstituierten Heteroaromaten, der kein Stickstoffatom enthält, bedeutet,
      wobei X jeweils unabhängig voneinander ein organischer Rest ist, der (i) wenigstens ein neutrales, protonierbares Stickstoffatom, (ii) wenigstens ein positiv geladenes, vorzugsweise quartäres, Stickstoffatom oder (iii) wenigstens ein positiv geladenes, vorzugsweise quartäres, Phosphoratom enthält, und
      wobei die Reste R1, R2, R4 und R5 jeweils unabhängig voneinander Wasserstoff, Halogen, Alkyl mit 1 bis 12 C-Atomen, Alkylaryl mit 1 bis 12 C-Atomen, Aryl mit 5 bis 20 C-Atomen, Ether mit 2 bis 12 C-Atomen oder Glykol mit 2 bis 12 C-Atomen bedeuten, und
(B) Bestrahlen der Mikroorganismen und des wenigstens einen Photosensibilisators mit elektromagnetischer Strahlung geeigneter Wellenlänge und Energiedichte.

Vorzugsweise wird das erfindungsgemäße Verfahren zur Inaktivierung von Mikroorganismen bei der
photodynamischen Entkeimung einer Oberfläche
eines Gegenstandes oder einer Flüssigkeit, durchgeführt.
Die Aufgabe der vorliegenden Erfindung wird ebenfalls gelöst durch die Bereitstellung wenigstens eines 1,7-Diaryl-1,6-heptadien-3,5-dion-Derivats der Formel (100) 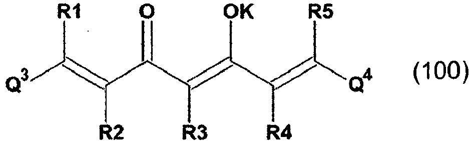 und/oder wenigstens eines 1,7-Diaryl-1,6-heptadien-3,5-dion-Derivats der Formel (101): 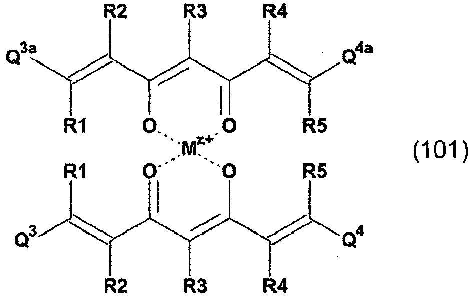 oder jeweils ein pharmakologisch verträgliches Salz und/oder Ester und/oder Komplex davon zur Verwendung als Photosensibilisator bei der medizinischen Behandlung zur Inaktivierung von Mikroorganismen, die vorzugsweise aus der Gruppe, die aus Viren, Archäeen, Bakterien, Bakteriensporen, Pilzen, Pilzsporen, Protozoen, Algen und blutübertragbaren Parasiten besteht, ausgewählt werden,
wobei Q³, Q^{3a}, Q⁴ und Q^{4a} jeweils unabhängig voneinander 1 substituierter oder unsubstituierter monozyklischer oder polyzyklischer, aromatischer Rest oder 1 substituierter oder unsubstituierter monozyklischer oder polyzyklischer, heteroaromatischer Rest bedeuten,
wobei die Verbindung mit der Formel (100) keine OH-Gruppe, die direkt an den organischen Rest Q³ oder Q⁴ gebunden ist, enthält, und
wobei die Verbindung mit der Formel (101) keine OH-Gruppe, die direkt an den organischen Rest Q³, Q^{3a}, Q⁴, oder Q^{4a} gebunden ist, enthält, und
wobei K Wasserstoff oder ein Kation bedeutet, und
wobei M^{z+} ein Kation eines Metalls bedeutet, wobei z die formale Oxidationszahl des Metalls M ist und z eine ganze Zahl von 1 bis 7, vorzugsweise von 2 bis 5, bedeutet, und wobei
(a1) wenigstens einer der Reste Q³, Q^{3a}, Q⁴ und Q^{4a} jeweils unabhängig voneinander ein unsubstituierter monozyklischer oder polyzyklischer, heteroaromatischer Rest ist, der wenigstens 5 Ringatome aufweist, wobei die Ringatome mindestens 1 Kohlenstoffatom und wenigstens 1, vorzugsweise protonierbares, Stickstoffatom enthalten, oder
(a2) wenigstens einer der Reste Q³, Q^{3a}, Q⁴ und Q^{4a}, vorzugsweise jeder der Reste Q³ und Q⁴, vorzugsweise jeder der Reste Q³ und Q^{3a}, vorzugsweise jeder der Reste Q³, Q^{3a} und Q⁴, vorzugsweise jeder der Reste Q³, Q^{3a}, Q⁴ und Q^{4a}, jeweils unabhängig voneinander mit mindestens einem, vorzugsweise 1 bis 9, weiter bevorzugt 1 bis 7, weiter bevorzugt 1 bis 5, weiter bevorzugt 1 bis 4, weiter bevorzugt 2 bis 3, organischen Rest(en) W1 der allgemeinen Formel (4), (5), (6), (7), (8), oder (9); vorzugsweise (5), (7), oder (9),:

   -(C(D)(E))ₕ-X, (4)

   -A-(C(D)(E))ₕ-X, (5)

   -(C(D)(E))ₖ-Aryl-(C(D)(E))ₗ-X, (6)

   -A-(C(D)(E))ₖ-Aryl-(C(D)(E))ᵢ-X, (7)

   -((C(D)(E))ₘ-A)ₚ-(C(D)(E))ₙ-X, (8)

   -A-((C(D)(E))ₘ-A)ₚ-(C(D)(E))ₙ-X, (9),

   substituiert ist,
   wobei h eine ganze Zahl von 1 bis 20, vorzugsweise von 2 bis 8, bedeutet, wobei k eine ganze Zahl von 0 bis 10, vorzugsweise von 1 bis 8, vorzugsweise von 2 bis 6, bedeutet, wobei I eine ganze Zahl von 0 bis 10, vorzugsweise von 1 bis 8, vorzugsweise von 2 bis 6, bedeutet, und wobei m, n und p jeweils unabhängig voneinander eine ganze Zahl von 1 bis 6, vorzugsweise von 2 bis 4, bedeuten, und
   wobei A jeweils unabhängig voneinander Sauerstoff oder Schwefel, vorzugsweise Sauerstoff, bedeutet,
   wobei D und E jeweils unabhängig voneinander Wasserstoff, Halogen, G-R^{(I)}, oder G-C(=G)-R^{(II)}, bedeuten, wobei G jeweils unabhängig voneinander Sauerstoff oder Schwefel, vorzugsweise Sauerstoff, bedeutet, und wobei die Reste R^{(I)} und R^{(II)} jeweils unabhängig voneinander Wasserstoff, Methyl, Ethyl, n-Propyl, n-Butyl, n-Pentyl, Phenyl oder Benzyl bedeutet, wobei Phenyl und Benzyl unsubstituiert oder substituiert sein können,
   wobei Aryl einen substituierten oder unsubstituierten Aromaten oder einen substituierten oder unsubstituierten Heteroaromaten, der kein Stickstoffatom enthält, bedeutet,
   wobei X jeweils unabhängig voneinander ein organischer Rest ist, der (i) wenigstens ein neutrales, protonierbares Stickstoffatom, oder (ii) wenigstens ein positiv geladenes, vorzugsweise quartäres, Stickstoffatom oder (iii) wenigstens ein positiv geladenes, vorzugsweise quartäres, Phosphoratom enthält, und
   wobei die Reste R1, R2, R3, R4 und R5 jeweils unabhängig voneinander Wasserstoff, Halogen, Alkyl mit 1 bis 12 C-Atomen, Cycloalkyl mit 1 bis 12 C-Atomen, Alkylaryl mit 1 bis 12 C-Atomen, Aryl mit 5 bis 20 C-Atomen, Ether mit 2 bis 12 C-Atomen oder Glykol mit 2 bis 12 C-Atomen bedeuten,
   oder wobei
(b) der Rest R3 ein organischer Rest W2 ist, wobei der eine organische Rest W2 die allgemeine Formel (4), (5), (6), (7), (8), (9), oder (10), vorzugsweise (4):

   -(C(D)(E))ₕ-X, (4)

   -A-(C(D)(E))ₕ-X, (5)

   -(C(D)(E))ₖ-Aryl-(C(D)(E))ᵢ-X, (6)

   -A-(C(D)(E))ₖ-Aryl-(C(D)(E))ₗ-X, (7)

   -[(C(D)(E))m-A]p-(C(D)(E))n-X, (8)

   -A-((C(D)(E))ₘ-A)ₚ-(C(D)(E))ₙ-X, (9)

   -(-C(D)=C(E)-)ᵣ-X, (10),

   aufweist, und
   wobei, optional, wenigstens einer der Reste Q³, Q^{3a}, Q⁴ und Q^{4a}, vorzugsweise jeder der Reste Q³ und Q⁴, vorzugsweise jeder der Reste Q³ und Q^{3a}, vorzugsweise jeder der Reste Q³, Q^{3a} und Q⁴, vorzugsweise jeder der Reste Q³, Q^{3a}, Q⁴ und Q^{4a}, jeweils unabhängig voneinander mit mindestens einem, vorzugsweise 1 bis 9, weiter bevorzugt 1 bis 7, weiter bevorzugt 1 bis 5, weiter bevorzugt 1 bis 4, weiter bevorzugt 2 bis 3, organischen Rest(en) W1 der allgemeinen Formel (4), (5), (6), (7), (8), oder (9), vorzugsweise (5), (7), oder (9), substituiert ist,
   wobei h eine ganze Zahl von 1 bis 20, vorzugsweise von 2 bis 8, bedeutet, wobei k eine ganze Zahl von 0 bis 10, vorzugsweise von 1 bis 8, vorzugsweise von 2 bis 6, bedeutet, und wobei I eine ganze Zahl von 0 bis 10, vorzugsweise von 1 bis 8, vorzugsweise von 2 bis 6, bedeutet, und wobei m, n, p, und r jeweils unabhängig voneinander eine ganze Zahl von 1 bis 6, vorzugsweise von 2 bis 4, bedeuten, und
   wobei A jeweils unabhängig voneinander Sauerstoff oder Schwefel, vorzugsweise Sauerstoff, bedeutet,
   wobei D und E jeweils unabhängig voneinander Wasserstoff, Halogen, G-R^{(I)}, oder G-C(=G)-R^{(II)}, bedeuten, wobei G jeweils unabhängig voneinander Sauerstoff oder Schwefel, vorzugsweise Sauerstoff, bedeutet, und wobei die Reste R^{(I)} und R^{(II)} jeweils unabhängig voneinander Wasserstoff, Methyl, Ethyl, n-Propyl,,n-Butyl, n-Pentyl, Phenyl oder Benzyl bedeutet, wobei Phenyl und Benzyl unsubstituiert oder substituiert sein können,
   wobei Aryl einen substituierten oder unsubstituierten Aromaten oder einen substituierten oder unsubstituierten Heteroaromaten, der kein Stickstoffatom enthält, bedeutet,
   wobei X jeweils unabhängig voneinander ein organischer Rest ist, der (i) wenigstens ein neutrales, protonierbares Stickstoffatom, (ii) wenigstens ein positiv geladenes, vorzugsweise quartäres, Stickstoffatom oder (iii) wenigstens ein positiv geladenes, vorzugsweise quartäres, Phosphoratom enthält, und
   wobei die Reste R1, R2, R4 und R5 jeweils unabhängig voneinander Wasserstoff, Halogen, Alkyl mit 1 bis 12 C-Atomen, Alkylaryl mit 1 bis 12 C-Atomen, Aryl mit 5 bis 20 C-Atomen, Ether mit 2 bis 12 C-Atomen oder Glykol mit 2 bis 12 C-Atomen bedeuten.

Vorzugsweise werden die Mikroorganismen und der wenigstens eine Photosensibilisator mit elektromagnetischer Strahlung geeigneter Wellenlänge und Energiedichte bestrahlt.

Bei einer bevorzugten Ausführungsform der Erfindung ist in der Verbindung mit der Formel (100) K ein Kation M^{z+} eines Metalls M, wobei z die formale Oxidationszahl des Metalls M ist und wobei z eine ganze Zahl von 1 bis 7, vorzugsweise von 2 bis 3, bedeutet, und wobei die Verbindung die Formel (102) aufweist: 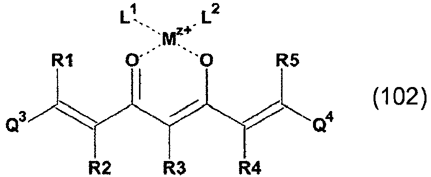 wobei L¹ und L² jeweils unabhängig voneinander Wasser, Fluorid, Chlorid, Bromid, lodid, Phosphat, Hydrogenphosphat, Dihydrogenphosphat, Sulfat, Hydrogensulfat, Tosylat, Mesylat oder wenigstens ein Carboxylation einer Carbonsäure mit 1 bis 15 Kohlenstoffatomen und/oder Mischungen davon bedeuten. Vorzugsweise ist ein Carboxylation einer Carbonsäure mit 1 bis 15 Kohlenstoffatomen Formiat, Acetat, n-Propionat, Lactat, Oxalat, Fumarat, Maleinat, Tartrat, Succinylat, Benzoat, Salicylat, Citrat, und/oder Mischungen davon.

Geeignete 1,7-Diaryl-1,6-heptadien-3,5-dion-Derivate der Formel (100) und deren Herstellung werden beispielsweise in der CA 2 888 140 A1 beschrieben.
Vorzugsweise ist ein geeignetes 1,7-Diaryl-1,6-heptadien-3,5-dion-Derivat der Formel (100) wenigstens
eine Verbindung der Formel (110) bis (141):

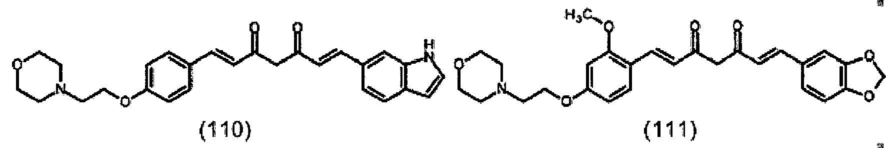

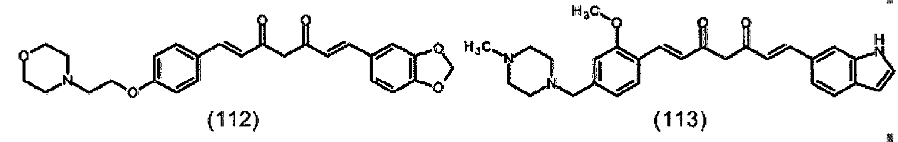

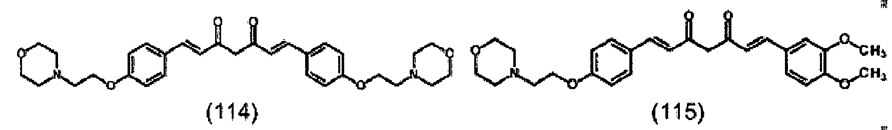

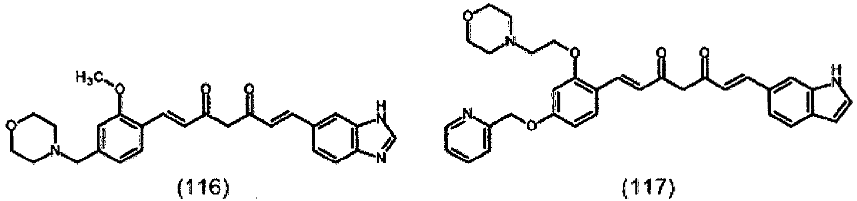

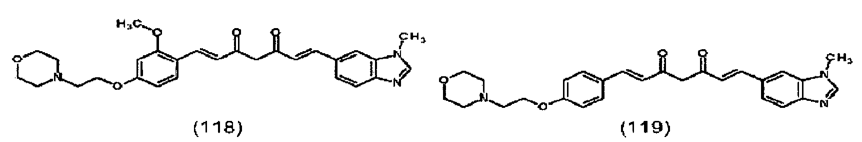

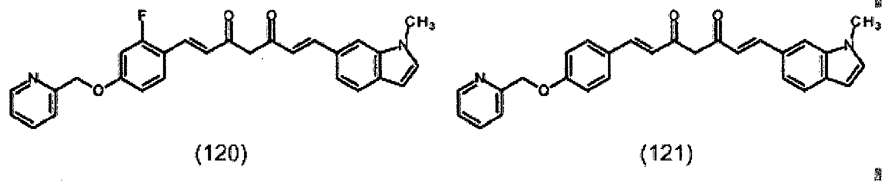

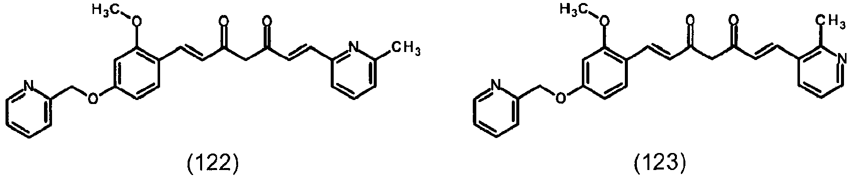

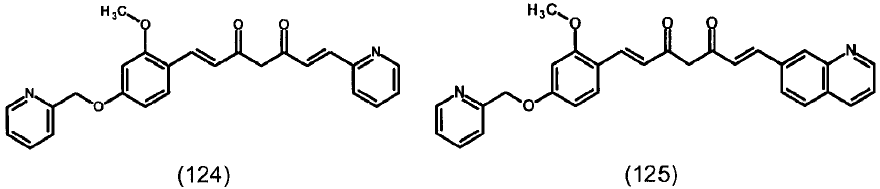

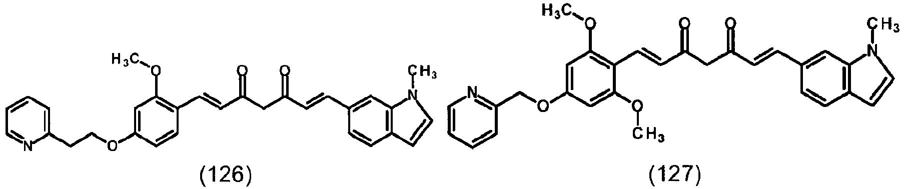

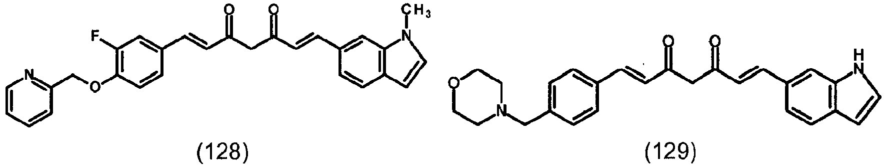

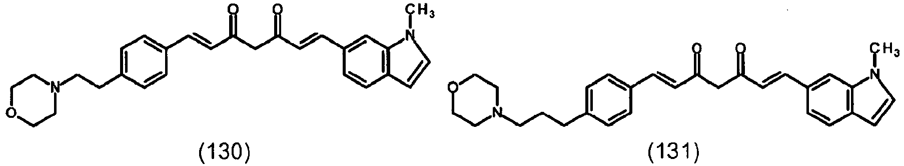

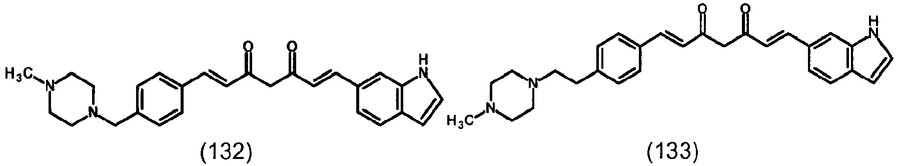

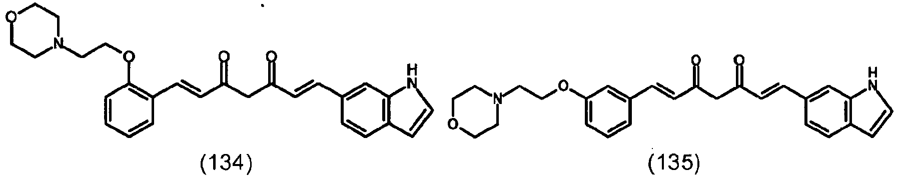

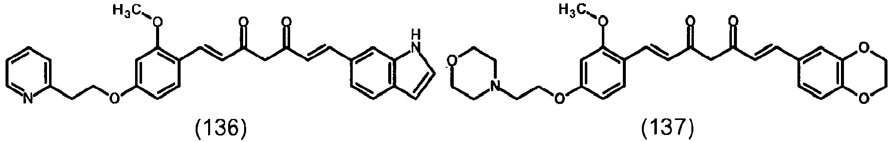

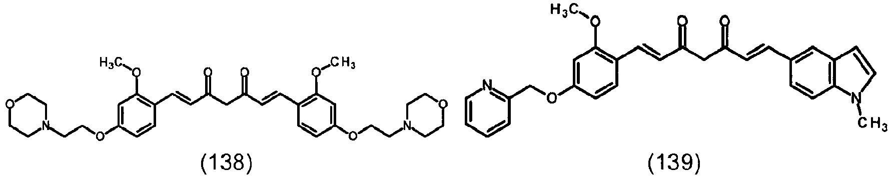

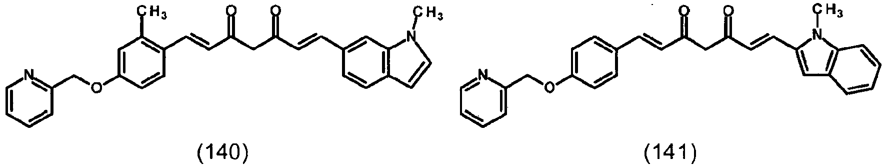

Geeignete 1,7-Diaryl-1,6-heptadien-3,5-dion-Derivate der Formel (100) und deren Herstellung werden ebenfalls in der EP 2 698 368 A1 beschrieben.

Vorzugsweise ist ein geeignetes 1,7-Diaryl-1,6-heptadien-3,5-dion-Derivat der Formel (100) wenigstens eine Verbindung der Formel (150) bis (227), vorzugsweise wenigstens eine Verbindung der Formel (150) bis (211): 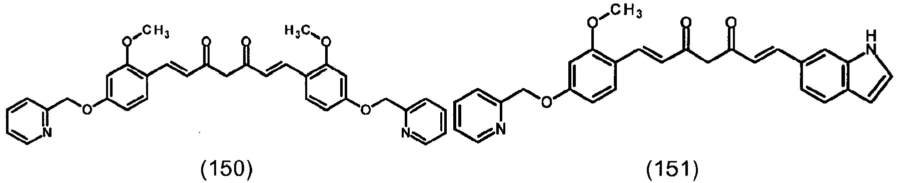 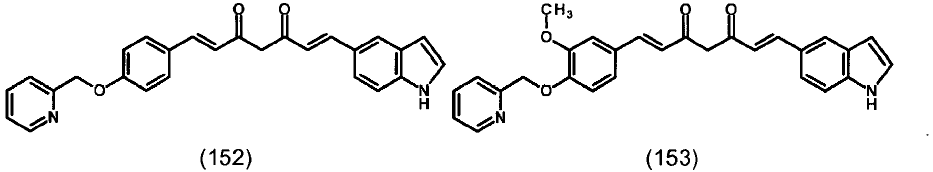 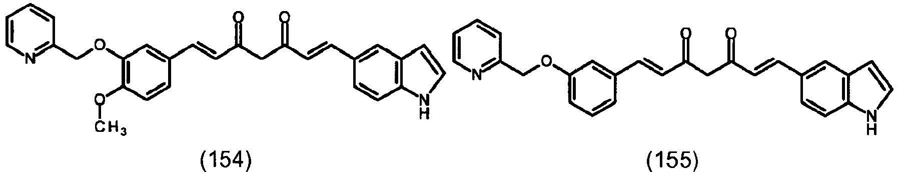 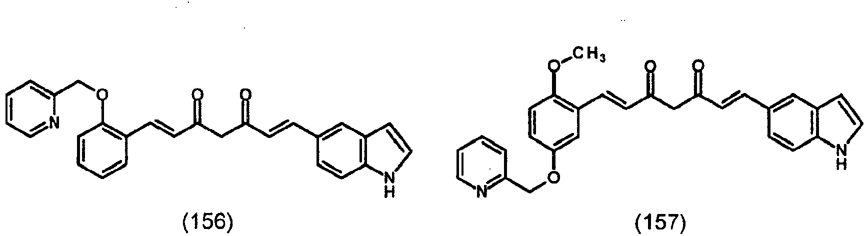 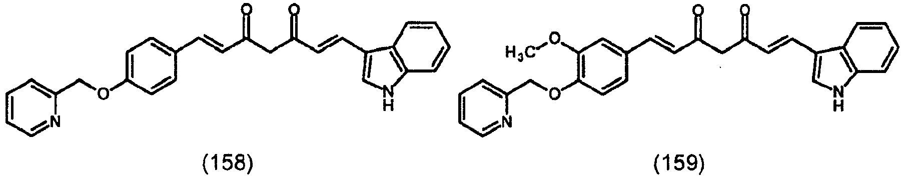 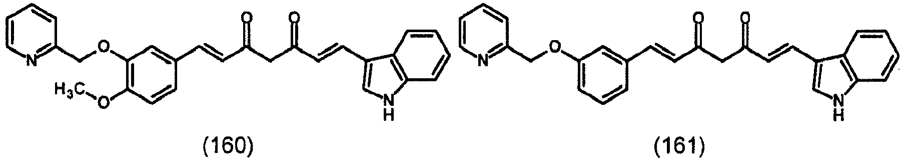 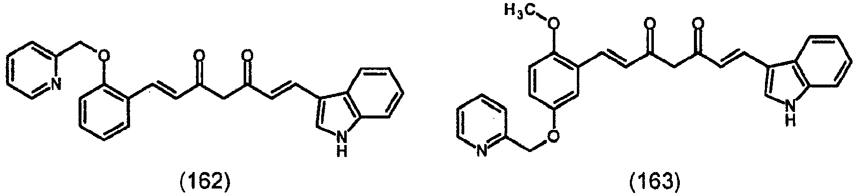 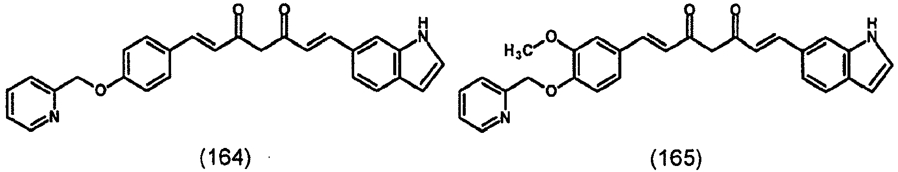 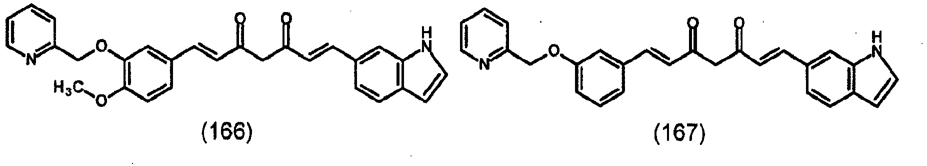 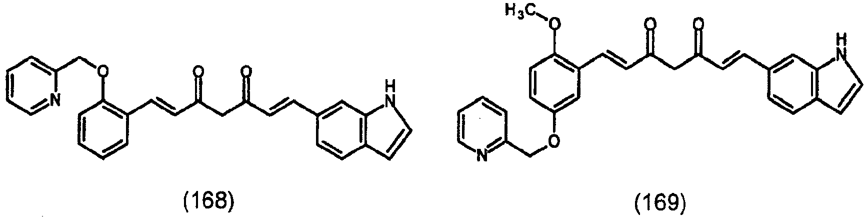 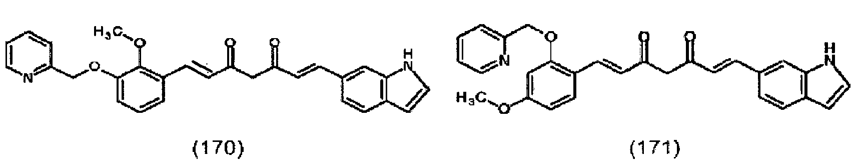 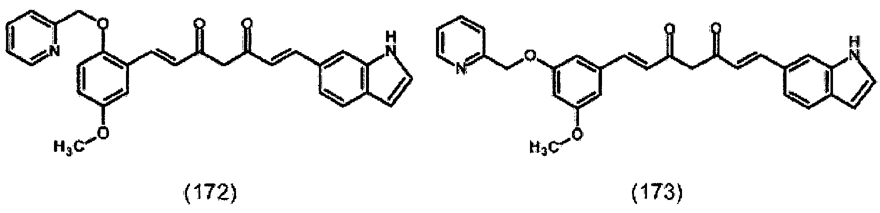 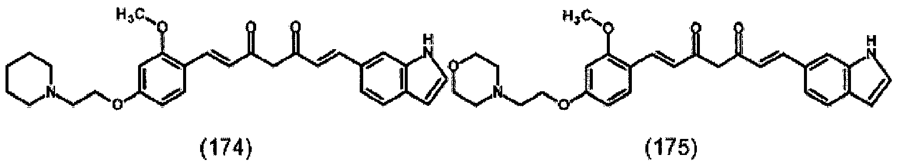 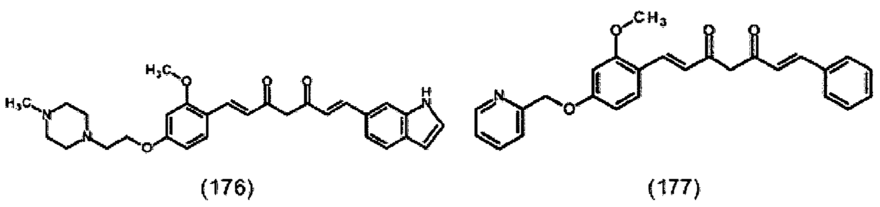 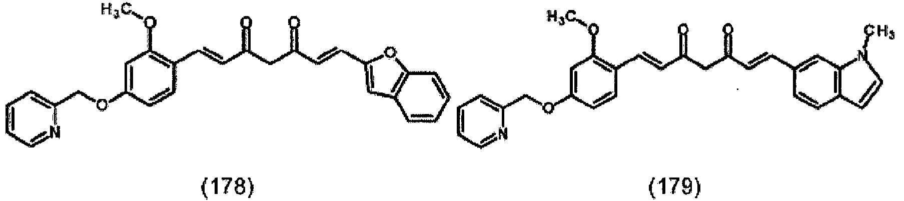 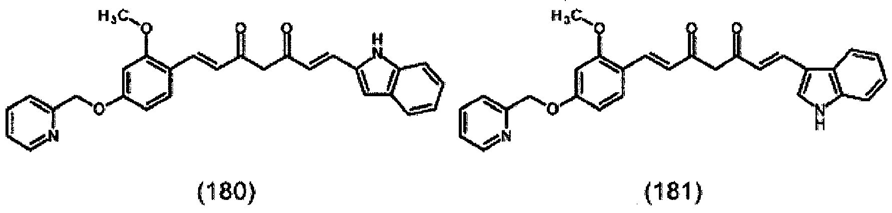 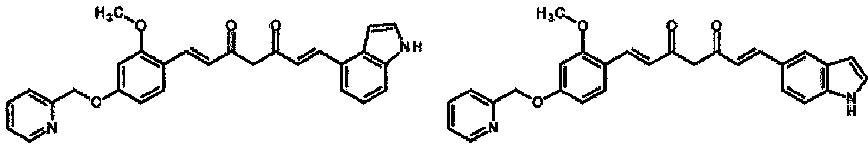 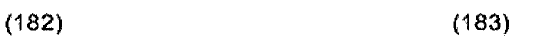 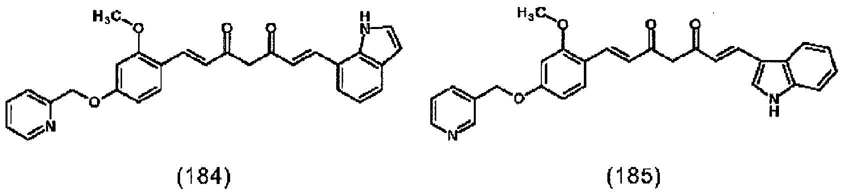 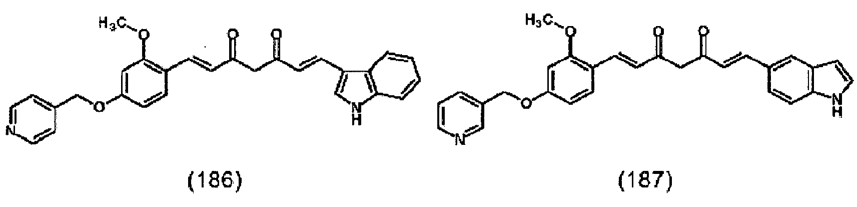 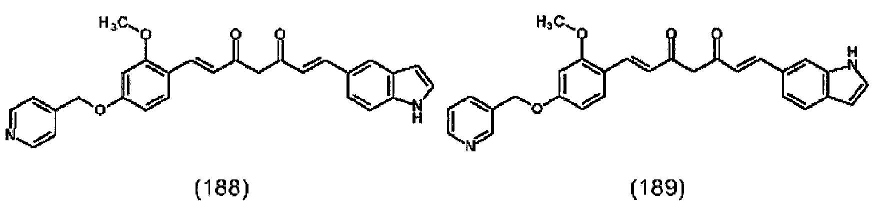 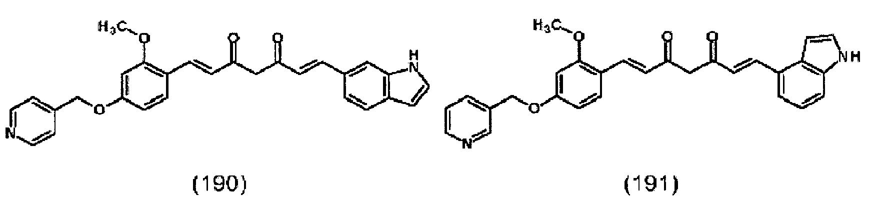 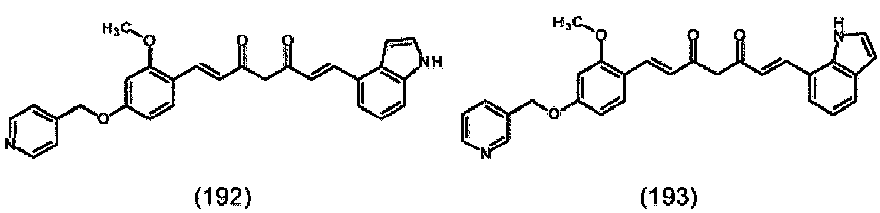 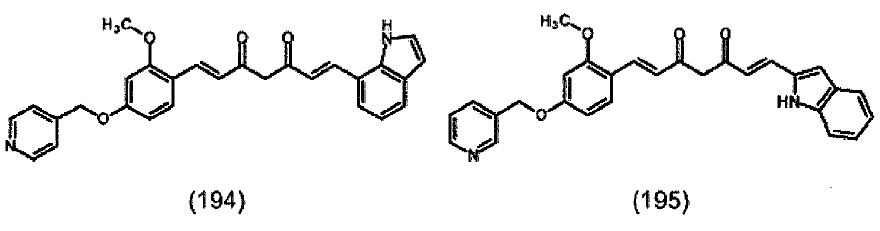 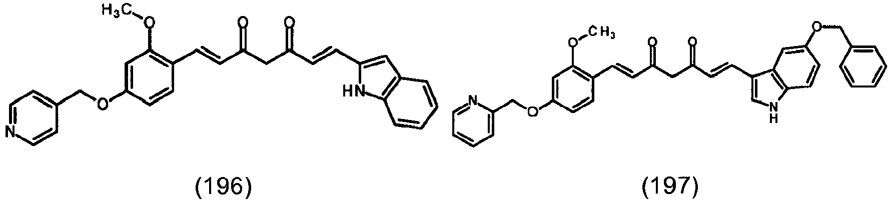 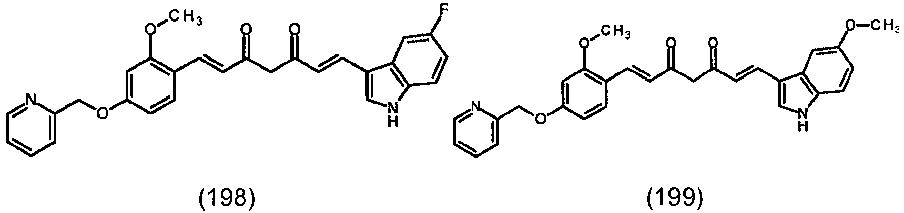 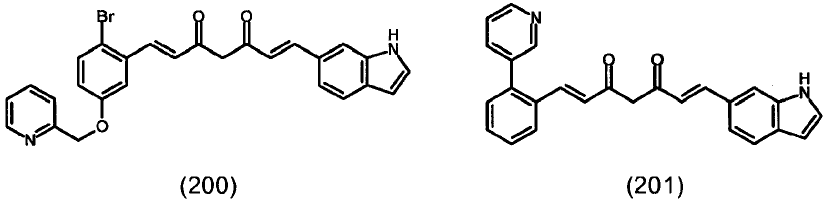 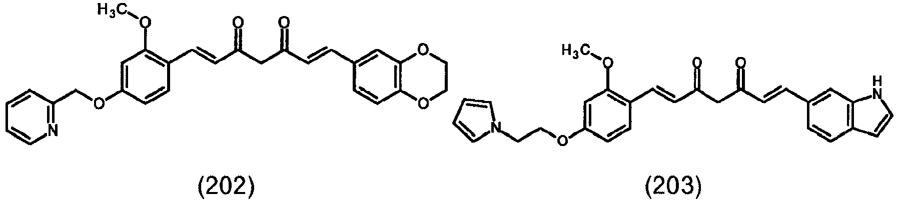 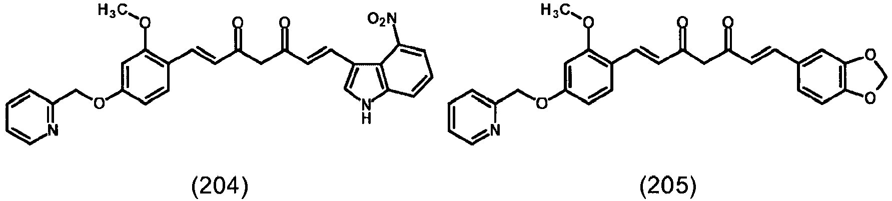 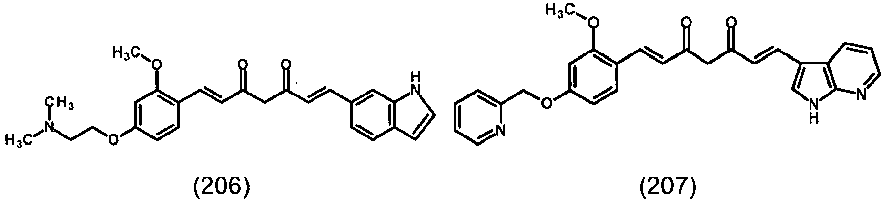 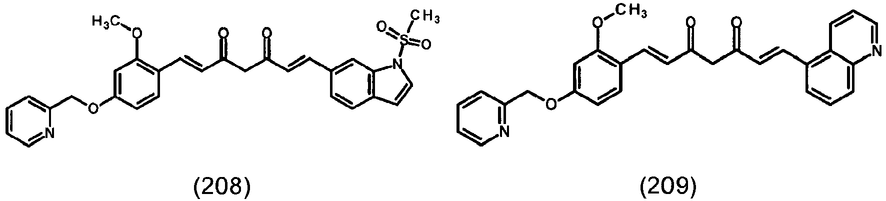 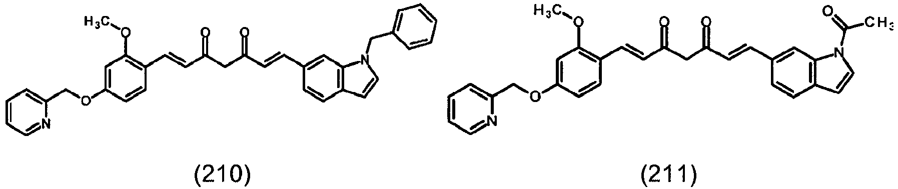 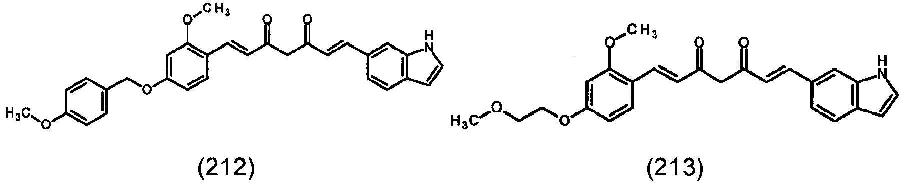 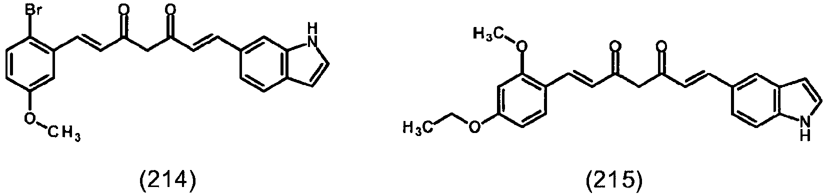 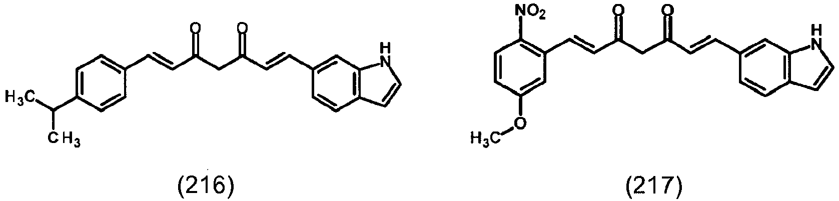 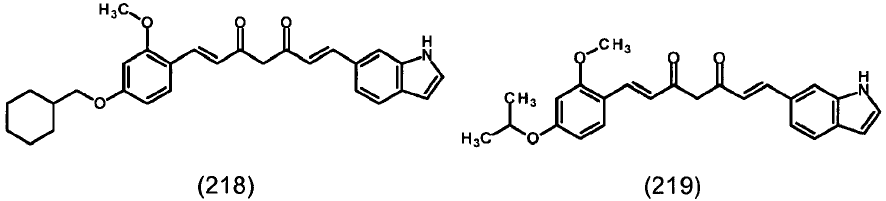 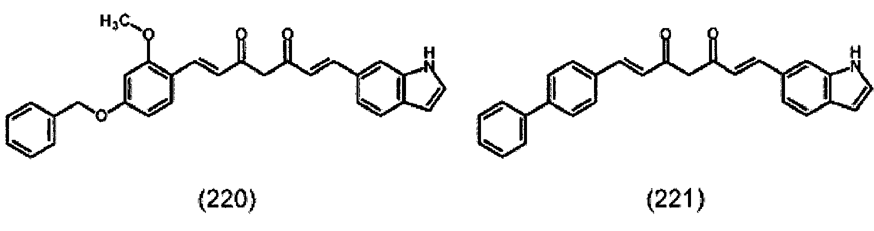 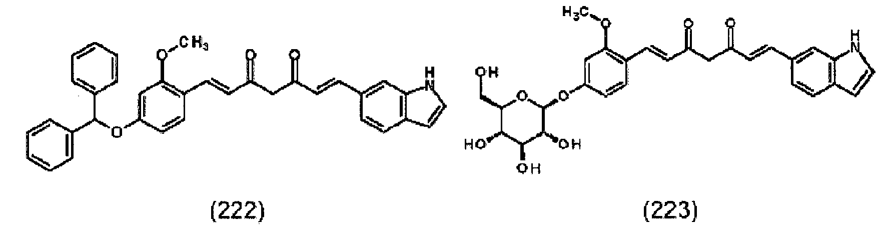 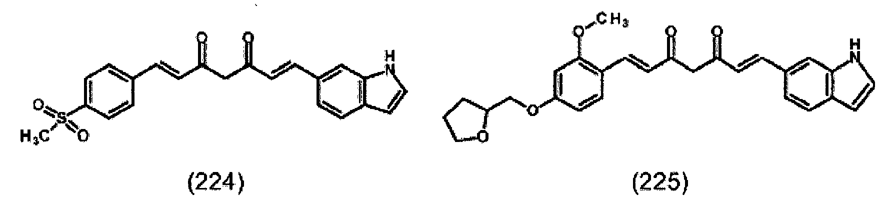 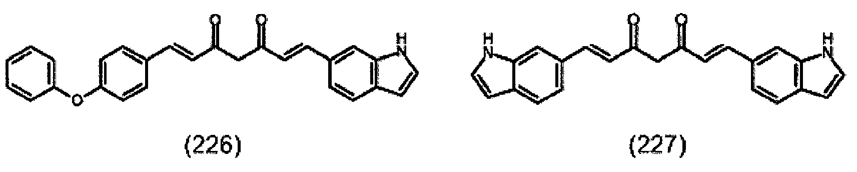 Ein geeignetes 1,7-Diaryl-1,6-heptadien-3,5-dion-Derivat der Formel (100) und dessen Herstellung wird ebenfalls in Taka et al. (Bioorg. Med. Chem. Lett. 24, 2014, Seiten 5242 bis 5246) beschrieben. Vorzugsweise ist ein geeignetes 1,7-Diaryl-1,6-heptadien-3,5-dion-Derivat der Formel
(100) eine Verbindung der Formel (228):

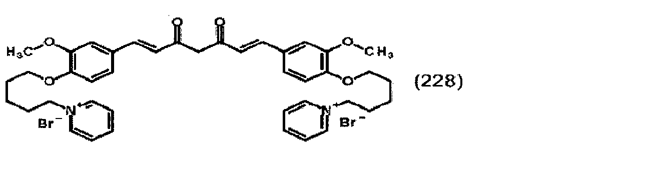

Ein geeignetes 1,7-Diaryl-1,6-heptadien-3,5-dion-Derivat der Formel (100) und dessen Herstellung wird ebenfalls in CN103952008 A1 beschrieben.

Vorzugsweise ist ein geeignetes 1,7-Diaryl-1,6-heptadien-3,5-dion-Derivat der Formel (100) eine Verbindung der Formel (229):

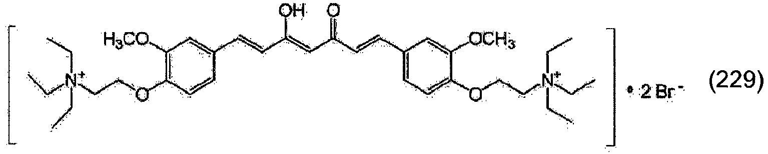

Die Aufgabe der vorliegenden Erfindung wird weiterhin gelöst durch die Bereitstellung eines 1,7-Diaryl-1,6-heptadien-3,5-dion-Derivates der Formel (1): 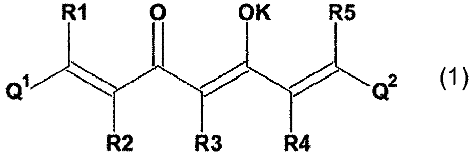 wobei die Reste Q¹ und Q² jeweils unabhängig voneinander ein (Zahl = 1) substituierter oder unsubstituierter monozyklischer oder polyzyklischer, aromatischer Rest bedeuten,
wobei die Verbindung mit der Formel (1) keine OH-Gruppe, die direkt an den organischen Rest Q¹ oder Q² gebunden ist, enthält, und
wobei K Wasserstoff oder ein Kation bedeutet, und wobei
(a) wenigstens einer der Reste Q¹ und Q², vorzugsweise jeder der Reste Q¹ und Q², jeweils unabhängig voneinander mit mindestens einem, vorzugsweise 1 bis 9, weiter bevorzugt 1 bis 7, weiter bevorzugt 1 bis 5, weiter bevorzugt 1 bis 4, weiter bevorzugt 2 bis 3, organischen Rest(en) W1a der allgemeinen Formel (5a), (6a), (7a), (8a), oder (9a), vorzugsweise (5a), (7a), oder (9a),:

   -A-(C(D)(E))ₕ-X^{a}, (5a)

   -(C(D)(E))ₖ-Aryl-(C(D)(E))ₗ-X^{a}, (6a)

   -A-(C(D)(E))ₖ-Aryl-(C(D)(E))ₗ-X^{a}, (7a)

   -((C(D)(E))ₘ-A)ₚ-(C(D)(E))ₙ-X^{a}, (8a)

   -A-((C(D)(E))ₘ-A)ₚ-(C(D)(E))ₙ-X^{a}, (9a)

   substituiert ist,
   wobei h eine ganze Zahl von 1 bis 20, vorzugsweise von 2 bis 8, bedeutet, wobei k eine ganze Zahl von 0 bis 10, vorzugsweise von 1 bis 8, vorzugsweise von 2 bis 6, bedeutet, wobei I eine ganze Zahl von 0 bis 10, vorzugsweise von 1 bis 8, vorzugsweise von 2 bis 6, bedeutet, und wobei m, n und p jeweils unabhängig voneinander eine ganze Zahl von 1 bis 6, vorzugsweise von 2 bis 4, bedeuten, und
   wobei A jeweils unabhängig voneinander Sauerstoff oder Schwefel, vorzugsweise Sauerstoff, bedeutet,
   wobei D und E jeweils unabhängig voneinander Wasserstoff, Halogen, G-R^{(I)}, oder G-C(=G)-R^{(II)}, bedeuten, wobei G jeweils unabhängig voneinander Sauerstoff oder Schwefel, vorzugsweise Sauerstoff, bedeutet, und wobei die Reste R^{(I)} und R^{(II)} jeweils unabhängig voneinander Wasserstoff, Methyl, Ethyl, n-Propyl, n-Butyl, n-Pentyl, Phenyl oder Benzyl bedeutet, wobei Phenyl und Benzyl unsubstituiert oder substituiert sein können,
   wobei Aryl einen substituierten oder unsubstituierten Aromaten oder einen substituierten oder unsubstituierten Heteroaromaten, der kein Stickstoffatom enthält, bedeutet,
   wobei X^{a} jeweils unabhängig voneinander ein Rest der Formel (20c), (20d), oder (21) bedeutet: 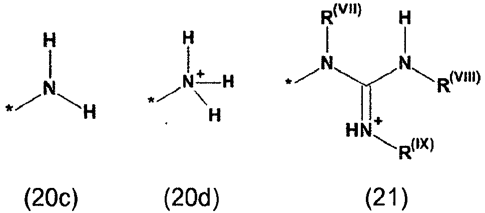 wobei jeder der Reste R^{(VII)}, R^{(VIII)}, und R^{(IX)} jeweils unabhängig voneinander Wasserstoff, ein Arylrest mit 5 bis 12 C-Atomen, ein Alkylarylrest mit 5 bis 12 C-Atomen, ein Alkylrest, der geradkettig oder verzweigt sein kann, mit 1 bis 8 C-Atomen, oder ein Etherrest, der geradkettig oder verzweigt sein kann, mit 1 bis 8 C-Atomen, vorzugsweise Wasserstoff, bedeutet, und
   wobei die Reste R1, R2, R3, R4 und R5 jeweils unabhängig voneinander Wasserstoff, Halogen, Alkyl mit 1 bis 12 C-Atomen, Cycloalkyl mit 1 bis 12 C-Atomen, Alkylaryl mit 1 bis 12 C-Atomen, Aryl mit 5 bis 20 C-Atomen, Ether mit 2 bis 12 C-Atomen oder Glykol mit 2 bis 12 C-Atomen bedeuten.

Bei einer bevorzugten Ausführungsform der Erfindung ist in der Verbindung mit der Formel (1) K ein Kation M^{z+} eines Metalls M, wobei z die formale Oxidationszahl des Metalls M ist und wobei z eine ganze Zahl von 1 bis 7, vorzugsweise von 2 bis 3, bedeutet, und wobei die Verbindung die Formel (2) aufweist: 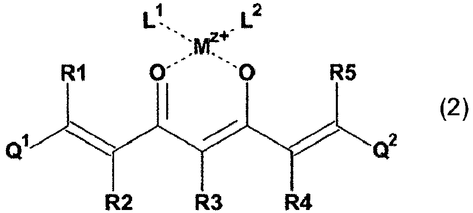 wobei L¹ und L² jeweils unabhängig voneinander Wasser, Fluorid, Chlorid, Bromid, lodid, Phosphat, Hydrogenphosphat, Dihydrogenphosphat, Sulfat, Hydrogensulfat, Tosylat, Mesylat oder wenigstens ein Carboxylation einer Carbonsäure mit 1 bis 15 Kohlenstoffatomen und/oder Mischungen davon bedeuten. Vorzugsweise ist ein Carboxylation einer Carbonsäure mit 1 bis 15 Kohlenstoffatomen Formiat, Acetat, n-Propionat, Lactat, Oxalat, Fumarat, Maleinat, Tartrat, Succinylat, Benzoat, Salicylat, Citrat, und/oder Mischungen davon.

Die Aufgabe der vorliegenden Erfindung wird ebenfalls gelöst durch die Bereitstellung einer Verbindung mit der Formel (3): 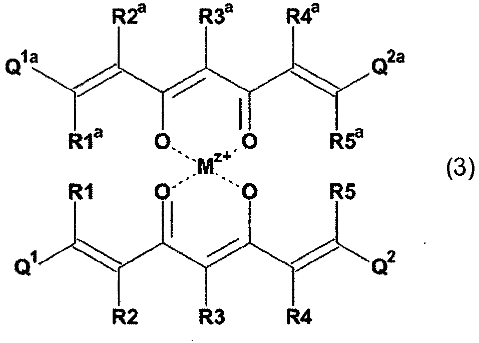 wobei M^{z+} ein Kation eines Metalls bedeutet, wobei z die formale Oxidationszahl des Metalls M ist und wobei z eine ganze Zahl von 1 bis 7, vorzugsweise von 2 bis 5, bedeutet, und
wobei die Reste Q¹ und Q² jeweils unabhängig voneinander 1 substituierter oder unsubstituierter monozyklischer oder polyzyklischer, aromatischer Rest bedeuten, und
wobei die Verbindung mit der Formel (3) keine OH-Gruppe, die direkt an den organischen Rest Q¹, Q^{1a}, Q², oder Q^{2a} gebunden ist, enthält,
wobei die Reste Q^{1a} und Q^{2a} jeweils unabhängig voneinander 1 substituierter oder unsubstituierter monozyklischer oder polyzyklischer, aromatischer Rest oder 1 substituierter oder unsubstituierter monozyklischer oder polyzyklischer, heteroaromatischer Rest, vorzugsweise 1 substituierter oder unsubstituierter monozyklischer oder polyzyklischer, aromatischer Rest, bedeuten,
und wobei
(a) wenigstens einer der Reste Q¹ und Q², vorzugsweise jeder der Reste Q¹ und Q², jeweils unabhängig voneinander mit mindestens einem, vorzugsweise 1 bis 9, weiter bevorzugt 1 bis 7, weiter bevorzugt 1 bis 5, weiter bevorzugt 1 bis 4, weiter bevorzugt 2 bis 3, organischen Rest(en) W1a der allgemeinen Formel (5a), (6a), (7a), (8a), oder (9a), vorzugsweise (5a), (7a), oder (9a),:

   -A-(C(D)(E))ₕ-X^{a}, (5a)

   -(C(D)(E))ₖ-Aryl-(C(D)(E))ₗ-X^{a}, (6a)

   -A-(C(D)(E))ₖ-Aryl-(C(D)(E))ₗ-X^{a}, (7a)

   -((C(D)(E))ₘ-A)ₚ-(C(D)(E))ₙ-X^{a}, (8a)

   -A-((C(D)(E))ₘ-A)ₚ-(C(D)(E))ₙ-X^{a}, (9a)

   substituiert ist,
   wobei h eine ganze Zahl von 1 bis 20, vorzugsweise von 2 bis 8, bedeutet, wobei k eine ganze Zahl von 0 bis 10, vorzugsweise von 1 bis 8, vorzugsweise von 2 bis 6, bedeutet, wobei I eine ganze Zahl von 0 bis 10, vorzugsweise von 1 bis 8, vorzugsweise von 2 bis 6, bedeutet, und wobei m, n und p jeweils unabhängig voneinander eine ganze Zahl von 1 bis 6, vorzugsweise von 2 bis 4, bedeuten, und
   wobei A jeweils unabhängig voneinander Sauerstoff oder Schwefel, vorzugsweise Sauerstoff, bedeutet,
   wobei D und E jeweils unabhängig voneinander Wasserstoff, Halogen, G-R^{(I)}, oder G-C(=G)-R^{(II)}, bedeuten, wobei G jeweils unabhängig voneinander Sauerstoff oder Schwefel, vorzugsweise Sauerstoff, bedeutet, und wobei die Reste R^{(I)} und R^{(II)} jeweils unabhängig voneinander Wasserstoff, Methyl, Ethyl, n-Propyl, n-Butyl, n-Pentyl, Phenyl oder Benzyl bedeutet, wobei Phenyl und Benzyl unsubstituiert oder substituiert sein können,
   wobei Aryl einen substituierten oder unsubstituierten Aromaten oder einen substituierten oder unsubstituierten Heteroaromaten, der kein Stickstoffatom enthält, bedeutet,
   wobei X^{a} jeweils unabhängig voneinander ein Rest der Formel (20c), (20d), oder (21) bedeutet: 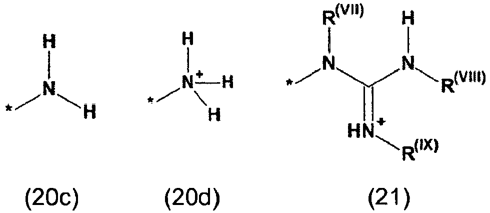 wobei jeder der Reste R^{(VII)}, R^{(VIII)}, und R^{(IX)} jeweils unabhängig voneinander Wasserstoff, ein Arylrest mit 5 bis 12 C-Atomen, ein Alkylarylrest mit 5 bis 12 C-Atomen, ein Alkylrest, der geradkettig oder verzweigt sein kann, mit 1 bis 8 C-Atomen, oder ein Etherrest, der geradkettig oder verzweigt sein kann, mit 1 bis 8 C-Atomen, vorzugsweise Wasserstoff, bedeutet, und
   wobei wenigstens einer der Reste Q^{1a} und Q^{2a}, vorzugsweise jeder der Reste Q^{1a} und Q^{2a}, jeweils unabhängig voneinander mit mindestens einem, vorzugsweise 1 bis 9, weiter bevorzugt 1 bis 7, weiter bevorzugt 1 bis 5, weiter bevorzugt 1 bis 4, weiter bevorzugt 2 bis 3, organischen Rest(en) W1c der allgemeinen Formel (5c), (6c), (7c), (8c), oder (9c), vorzugsweise (5c), (7c), oder (9c),:

   -A-(C(D)(E))ₕ-X^{c}, (5c)

   -(C(D)(E))ₖ-Aryl-(C(D)(E))ₗ-X^{c}, (6c)

   -A-(C(D)(E))ₖ-Aryl-(C(D)(E))ₗ-X^{c}, (7c)

   -((C(D)(E))ₘ-A)ₚ-(C(D)(E))ₙ-X^{c}, (8c)

   -A-((C(D)(E))ₘ-A)ₚ-(C(D)(E))ₙ-X^{c}, (9c)

   substituiert ist,
   wobei h eine ganze Zahl von 1 bis 20, vorzugsweise von 2 bis 8, bedeutet, wobei k eine ganze Zahl von 0 bis 10, vorzugsweise von 1 bis 8, vorzugsweise von 2 bis 6, bedeutet, wobei I eine ganze Zahl von 0 bis 10, vorzugsweise von 1 bis 8, vorzugsweise von 2 bis 6, bedeutet, und wobei m, n und p jeweils unabhängig voneinander eine ganze Zahl von 1 bis 6, vorzugsweise von 2 bis 4, bedeuten, und
   wobei A jeweils unabhängig voneinander Sauerstoff oder Schwefel, vorzugsweise Sauerstoff, bedeutet,
   wobei D und E jeweils unabhängig voneinander Wasserstoff, Halogen, G-R^{(I)}, oder G-C(=G)-R^{(II)}, bedeuten, wobei G jeweils unabhängig voneinander Sauerstoff oder Schwefel, vorzugsweise Sauerstoff, bedeutet, und wobei die Reste R^{(I)} und R^{(II)} jeweils unabhängig voneinander Wasserstoff, Methyl, Ethyl, n-Propyl, n-Butyl, n-Pentyl, Phenyl oder Benzyl bedeutet, wobei Phenyl und Benzyl unsubstituiert oder substituiert sein können,
   wobei Aryl einen substituierten oder unsubstituierten Aromaten oder einen substituierten oder unsubstituierten Heteroaromaten, der kein Stickstoffatom enthält, bedeutet,
   wobei X^{c} jeweils unabhängig voneinander ein organischer Rest ist, der (i) wenigstens ein neutrales, protonierbares Stickstoffatom, oder (ii) wenigstens ein positiv geladenes, vorzugsweise quartäres, Stickstoffatom, oder (iii) wenigstens ein positiv geladenes, vorzugsweise quartäres, Phosphoratom enthält, wobei vorzugsweise X^{c} jeweils unabhängig voneinander ein Rest der Formel (20c), (20d), (21), oder (24), weiter bevorzugt ein Rest der Formel (20c), (20d), oder (21), weiter bevorzugt ein Rest der Formel (24), bedeutet: 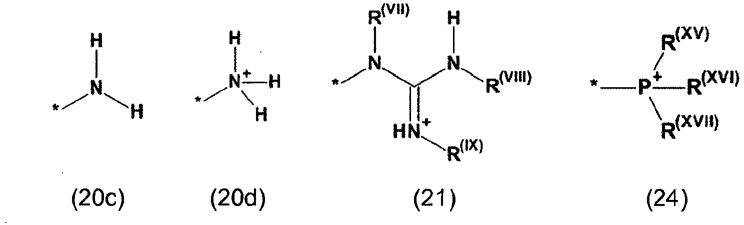 wobei jeder der Reste R^{(VII)}, R^{(VIII)}, und R^{(IX)} jeweils unabhängig voneinander Wasserstoff, ein Arylrest mit 5 bis 12 C-Atomen, ein Alkylarylrest mit 5 bis 12 C-Atomen, ein Alkylrest, der geradkettig oder verzweigt sein kann, mit 1 bis 8 C-Atomen, oder ein Etherrest, der geradkettig oder verzweigt sein kann, mit 1 bis 8 C-Atomen, vorzugsweise Wasserstoff, bedeutet und wobei jeder der Reste R^{(XV)}, R^{(XVI)}, und R^{(XVII)} jeweils unabhängig voneinander ein Arylrest mit 5 bis 12 C-Atomen, ein Alkylarylrest mit 5 bis 12 C-Atomen, ein Alkylrest, der geradkettig oder verzweigt sein kann, mit 1 bis 8 C-Atomen, oder ein Etherrest, der geradkettig oder verzweigt sein kann, mit 1 bis 8 C-Atomen, vorzugsweise ein Arylrest mit 5 bis 12 C-Atomen, ein Alkylarylrest mit 5 bis 12 C-Atomen, oder ein Alkylrest, der geradkettig oder verzweigt sein kann, mit 1 bis 8 C-Atomen, bedeutet,
   wobei die Reste R1, R1^{a}, R2, R2^{a}, R3, R3^{a}, R4, R4^{a}, R5 und R5^{a} jeweils unabhängig voneinander Wasserstoff, Halogen, Alkyl mit 1 bis 12 C-Atomen, Cycloalkyl mit 1 bis 12 C-Atomen, Alkylaryl mit 1 bis 12 C-Atomen, Aryl mit 5 bis 20 C-Atomen, Ether mit 2 bis 12 C-Atomen oder Glykol mit 2 bis 12 C-Atomen bedeuten.

Vorzugsweise bedeuten bei der Verbindung mit der Formel (3) die Reste Q¹, Q^{1a}, Q² und Q^{2a} jeweils unabhängig voneinander 1 substituierter oder unsubstituierter monozyklischer oder polyzyklischer, aromatischer Rest, und
(a) wenigstens einer der Reste Q¹, Q^{1a}, Q² und Q^{2a}, vorzugsweise jeder der Reste Q¹ und Q², vorzugsweise jeder der Reste Q¹ und Q^{1a}, vorzugsweise jeder der Reste Q¹, Q^{1a} und Q², vorzugsweise jeder der Reste Q¹, Q^{1a}, Q² und Q^{2a}, ist jeweils unabhängig voneinander mit mindestens einem, vorzugsweise 1 bis 9, weiter bevorzugt 1 bis 7, weiter bevorzugt 1 bis 5, weiter bevorzugt 1 bis 4, weiter bevorzugt 2 bis 3, organischen Rest(en) W1a der allgemeinen Formel (5a), (6a), (7a), (8a), oder (9a), vorzugsweise (5a), (7a), oder (9a),:

   -A-(C(D)(E))ₕ-X^{a}, (5a)

   -(C(D)(E))ₖ-Aryl-(C(D)(E))ₗ-X^{a}, (6a)

   -A-(C(D)(E))ₖ-Aryl-(C(D)(E))ᵢ-X^{a}, (7a)

   -((C(D)(E))ₘ-A)ₚ-(C(D)(E))ₙ-X^{a}, (8a)

   -A-((C(D)(E))ₘ-A)ₚ-(C(D)(E))ₙ-X^{a}, (9a)

   substituiert,
   wobei h eine ganze Zahl von 1 bis 20, vorzugsweise von 2 bis 8, bedeutet, wobei k eine ganze Zahl von 0 bis 10, vorzugsweise von 1 bis 8, vorzugsweise von 2 bis 6, bedeutet, wobei I eine ganze Zahl von 0 bis 10, vorzugsweise von 1 bis 8, vorzugsweise von 2 bis 6, bedeutet, und wobei m, n und p jeweils unabhängig voneinander eine ganze Zahl von 1 bis 6, vorzugsweise von 2 bis 4, bedeuten, und
   wobei A jeweils unabhängig voneinander Sauerstoff oder Schwefel, vorzugsweise Sauerstoff, bedeutet,
   wobei D und E jeweils unabhängig voneinander Wasserstoff, Halogen, G-R^{(I)}, oder G-C(=G)-R^{(II)}, bedeuten, wobei G jeweils unabhängig voneinander Sauerstoff oder Schwefel, vorzugsweise Sauerstoff, bedeutet, und wobei die Reste R^{(I)} und R^{(II)} jeweils unabhängig voneinander Wasserstoff, Methyl, Ethyl, n-Propyl, n-Butyl, n-Pentyl, Phenyl oder Benzyl bedeutet, wobei Phenyl und Benzyl unsubstituiert oder substituiert sein können,
   wobei Aryl einen substituierten oder unsubstituierten Aromaten oder einen substituierten oder unsubstituierten Heteroaromaten, der kein Stickstoffatom enthält, bedeutet,
   wobei X^{a} jeweils unabhängig voneinander ein Rest der Formel (20c), (20d), oder (21) bedeutet: 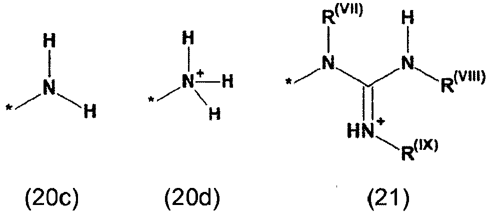 wobei jeder der Reste R^{(VII)}, R^{(VIII)}, und R^{(IX)} jeweils unabhängig voneinander Wasserstoff, ein Arylrest mit 5 bis 12 C-Atomen, ein Alkylarylrest mit 5 bis 12 C-Atomen, ein Alkylrest, der geradkettig oder verzweigt sein kann, mit 1 bis 8 C-Atomen, oder ein Etherrest, der geradkettig oder verzweigt sein kann, mit 1 bis 8 C-Atomen, vorzugsweise Wasserstoff, bedeutet, und
   wobei die Reste R1, R1^{a}, R2, R2^{a}, R3, R3^{a}, R4, R4^{a}, R5 und R5^{a} jeweils unabhängig voneinander Wasserstoff, Halogen, Alkyl mit 1 bis 12 C-Atomen, Cycloalkyl mit 1 bis 12 C-Atomen, Alkylaryl mit 1 bis 12 C-Atomen, Aryl mit 5 bis 20 C-Atomen, Ether mit 2 bis 12 C-Atomen oder Glykol mit 2 bis 12 C-Atomen bedeuten.

Bei den erfindungsgemäßen Verbindungen mit der Formel (1), mit der Formel (2) und mit der Formel (3) handelt es sich jeweils um ein 1,7-Diaryl-1,6-heptadien -3,5-dion-Derivat, das im Folgenden so bezeichnet wird.

Als Gegenion zum positiv geladenen Stickstoffatom, beispielsweise protonierten Stickstoffatom oder quartären Stickstoffatom, oder positiv geladenen Phosphoratom, beispielsweise quartären Phosphoratom, kann jedes geeignete Anion verwendet werden. Vorzugsweise werden als Gegenion zum positiv geladenen Stickstoffatom oder Phosphoratom Anionen verwendet, die die Bereitstellung eines pharmakologisch verträglichen Salzes ermöglichen.

Bei einer bevorzugten Ausführungsform der vorliegenden Erfindung ist X, X^{a}, X^{b} und/oder X^{c} in dem erfindungsgemäßen 1,7-Diaryl-1,6-heptadien -3,5-dion-Derivat der Formel (1), der Formel (2), und/oder der Formel (3) sowie dem erfindungsgemäß zu verwendenden 1,7-Diaryl-1,6-heptadien -3,5-dion-Derivat der Formel (100), der Formel (101), und/oder der Formel (102) ein organischer Rest mit wenigstens einem protonierten Stickstoffatom, oder wenigstens einem quartären Phosphoratom, vorzugsweise ein organischer Rest der allgemeinen Formel (20b), (20d), (21) oder (24), das als Gegenion Fluorid, Chlorid, Bromid, lodid, Sulfat, Hydrogensulfat, Phosphat, Hydrogenphosphat, Dihydrogenphosphat, Tosylat, Mesylat, oder wenigstens ein Carboxylation einer Carbonsäure mit 1 bis 15 Kohlenstoffatomen und/oder Mischungen davon aufweist. Vorzugsweise ist ein Carboxylation einer Carbonsäure mit 1 bis 15 Kohlenstoffatomen Formiat, Acetat, n-Propionat, Lactat, Oxalat, Fumarat, Maleinat, Tartrat, Succinylat, Benzoat, Salicylat, Citrat, und/oder Mischungen davon.

Weitere bevorzugte Ausführungsformen der vorliegenden Erfindung sind in den abhängigen Ansprüchen beschrieben.

Bei einer weiteren bevorzugten Ausführungsform enthält das erfindungsgemäße 1,7-Diaryl-1,6-heptadien -3,5-dion-Derivat der Formel (1), der Formel (2), und/oder der Formel (3) sowie das erfindungsgemäß zu verwendende 1,7-Diaryl-1,6-heptadien -3,5-dion-Derivat der Formel (100), der Formel (101), und/oder der Formel (102) kein neutrales, protonierbares Stickstoffatom, beispielsweise als Aminorest, Methylaminorest oder Dimethylaminorest, und kein positiv geladenes, vorzugsweise quartäres, Stickstoffatom, beispielsweise als Pyridin-1-ium-1-yl-Rest oder Trimethylammonio-Rest, sowie kein positiv geladenes, vorzugsweise quartäres, Phosphoratom, das direkt an den organischen Rest Q¹, Q^{1a}, Q², Q^{2a}, Q³, Q^{3a}, Q⁴, oder Q^{4a} gebunden ist.

Unter "direkt" wird dabei verstanden, dass das Stickstoffatom und/oder das Phosphoratom unmittelbar an den aromatischen Rest Q¹, Q^{1a}, Q², Q^{2a}, Q³, Q^{3a}, Q⁴, oder Q^{4a} gebunden ist.

Die Erfinder haben überraschend festgestellt, dass bei einer direkten Anordnung des Stickstoffatoms und/oder Phosphoratoms an ein aromatisches Ringsystem der Reste Q¹, Q^{1a}, Q², Q^{2a}, Q³, Q^{3a}, Q⁴, oder Q^{4a} es beispielsweise zu einer signifikanten Absenkung der Ausbeute an reaktiven Sauerstoffspezies kommt.

Eine möglichst hohe Ausbeute an reaktiven Sauerstoffspezies ist für eine antimikrobielle Wirksamkeit bei der photodynamischen Therapie oder bei der photodynamischen Reinigung von Oberflächen oder Flüssigkeiten erforderlich. Bei einer direkten Anordnung des Stickstoffatoms und/oder Phosphoratoms am aromatischen Ringsystem des Restes Q¹, Q^{1a}, Q², Q^{2a}, Q³, Q^{3a}, Q⁴, oder Q^{4a} wird die aufgenommene Energie überwiegend durch Fluoreszenzeffekte abgegeben. Dies führt zu einer signifikanten Verringerung der photodynamischen Effizienz, d.h. zu einer Verringerung der durch photodynamische Prozesse gebildeten reaktiven Sauerstoffspezies (ROS) und/oder des durch photodynamische Prozesse gebildeten angeregten molekularen Sauerstoffs.

Darüber hinaus haben die Erfinder überraschend festgestellt, dass durch die Anordnung des wenigstens einen neutralen, protonierbaren Stickstoffatoms, und/oder wenigstens einen positiv geladenen, vorzugsweise quartären, Stickstoffatoms und/oder wenigstens einen positiv geladenen, vorzugsweise quartären, Phosphoratoms über wenigstens ein Kohlenstoffatom, beispielsweise in Form einer Methylengruppe, das somit von dem aromatischen Rest Q¹, Q^{1a}, Q², Q^{2a}, Q³, Q^{3a}, Q⁴, oder Q^{4a} getrennt ist, die photophysikalischen Eigenschaften der Verbindung mit der Formel (1) und/oder der Verbindung mit der Formel (2) und/oder der Verbindung mit der Formel (3) und/oder der Verbindung mit der Formel (100) und/oder der Verbindung mit der Formel (101) und/oder der Verbindung mit der Formel (102) nicht negativ beeinflusst wird.

Eine positive Ladung an dem Stickstoffatom und/oder Phosphoratom führt weiterhin zu einer effektiven Anlagerung der erfindungsgemäßen Verbindung mit der Formel (1), der Formel (2) und/oder der Formel (3) und/oder der erfindungsgemäß zu verwendenden Verbindung mit der Formel (100), der Formel (101) und/oder der Formel (102) an negativ geladene Komponenten in der Zellwand von Mikroorganismen. Dabei wirkt das wenigstens eine Kohlenstoffatom, beispielsweise in Form einer Methylengruppe, auch als Abstandshalter, sodass die voluminöse Struktur der Verbindung mit der Formel (1) und/oder der Verbindung mit der Formel (2) und/oder der Verbindung mit der Formel (3) und/oder der Verbindung mit der Formel (100) und/oder der Verbindung mit der Formel (101) und/oder der Verbindung mit der Formel (102) effizient an der Zellwand der Mikroorganismen angeordnet werden kann.

Weiterhin haben die Erfinder überraschend festgestellt, dass durch die Anordnung des wenigstens einen neutralen, protonierbaren Stickstoffatoms, und/oder wenigstens einen positiv geladenen, vorzugsweise quartären, Stickstoffatoms und/oder wenigstens einen positiv geladenen, vorzugsweise quartären, Phosphoratoms die Löslichkeit der erfindungsgemäßen Verbindung mit der Formel (1), der Formel (2) und/oder der Formel (3) und/oder der erfindungsgemäß zu verwendenden Verbindung mit der Formel (100), der Formel (101) und/oder der Formel (102) in polaren Lösungsmitteln, beispielsweise Wasser, verbessert wird.

Das erfindungsgemäße 1,7-Diaryl-1,6-heptadien -3,5-dion-Derivat der Formel (1), der Formel (2), und/oder der Formel (3) sowie das erfindungsgemäß zu verwendende 1,7-Diaryl-1,6-heptadien-3,5-dion-Derivat der Formel (100), der Formel (101), und/oder der Formel (102) enthält keine OH-Gruppe, die direkt an den organischen Rest Q¹, Q^{1a}, Q², Q^{2a}, Q³, Q^{3a}, Q⁴, oder Q^{4a} gebunden ist.

Unter "direkt" wird dabei verstanden, dass die OH-Gruppe unmittelbar an den aromatischen Rest Q^{1,} Q^{1a}, Q², Q^{2a}, Q³, Q^{3a}, Q⁴, oder Q^{4a} gebunden ist.

Die Erfinder haben überraschend festgestellt, dass bei einer direkten Anordnung der OH-Gruppe an ein aromatisches Ringsystem der Reste Q¹, Q^{1a}, Q², Q^{2a}, Q³, Q^{3a}, Q⁴, oder Q^{4a} es zu einer signifikanten Verschlechterung der Photostabilität des Photosensibilisators kommt. Eine Verschlechterung der Photostabilität führt zu einem schnelleren Ausbleichen und somit zu einer schnelleren Inaktivierung des Photosensibilisators bei Bestrahlung mit elektromagnetischer Strahlung geeigneter Wellenlänge.

Vorzugsweise ist das wenigstens eine neutrale, protonierbare Stickstoffatom, und das wenigstens eine positiv geladene, vorzugsweise quartäre, Stickstoffatom kein Carbonsäuream id.

Bei einer weiteren bevorzugten Ausführungsform enthält das erfindungsgemäße 1,7-Diaryl-1,6-heptadien -3,5-dion-Derivat der Formel (1), der Formel (2), und/oder der Formel (3) sowie das erfindungsgemäß zu verwendende 1,7-Diaryl-1,6-heptadien-3,5-dion-Derivat der Formel (100), der Formel (101), und/oder der Formel (102) keinen Carbamat-Rest, der vorzugsweise direkt an den organischen Rest Q¹, Q^{1a}, Q², Q^{2a}, Q³, Q^{3a}, Q⁴, oder Q^{4a} gebunden ist.

Die Erfinder haben festgestellt, dass bei einer direkten Anordnung eines Carbamat-Restes an ein aromatisches Ringsystem der Reste Q¹, Q^{1a}, Q², Q^{2a}, Q³, Q^{3a}, Q⁴, oder Q^{4a} es zu einer signifikanten Verschlechterung der Photostabilität des Photosensibilisators kommt. Eine Verschlechterung der Photostabilität führt zu einem schnelleren Ausbleichen und somit zu einer schnelleren Inaktivierung des Photosensibilisators bei Bestrahlung mit elektromagnetischer Strahlung geeigneter Wellenlänge.

Als "Photosensibilisator" im Sinn der Erfindung werden Verbindungen verstanden, die elektromagnetische Strahlung, vorzugsweise sichtbares Licht, UV-Licht und/oder Infrarotlicht, absorbieren und sodann reaktive Sauerstoffspezies (ROS), vorzugsweise freie Radikale und/oder Singulett-Sauerstoff, aus Triplett-Sauerstoff erzeugen.

Unter dem Begriff "photodynamische Therapie" wird erfindungsgemäß die lichtinduzierte Inaktivierung von Zellen oder Mikroorganismen, die vorzugsweise Viren, Archäeen, Bakterien, Bakteriensporen, Pilzen, Pilzsporen, Protozoen, Algen, blutübertragbaren Parasiten oder Kombinationen davon umfassen, auf und/oder in Patienten verstanden.

Unter dem Begriff "photodynamische Entkeimung" wird erfindungsgemäß die lichtinduzierte Inaktivierung von Mikroorganismen, die vorzugsweise Viren, Archäeen, Bakterien, Bakteriensporen, Pilzen, Pilzsporen, Protozoen, Algen, blutübertragbaren Parasiten oder Kombinationen davon umfassen, auf Oberflächen von Gegenständen und/oder Lebensmitteln und/oder in Flüssigkeiten verstanden.

Unter dem Begriff "Inaktivierung" wird erfindungsgemäß die Reduzierung der Lebensfähigkeit oder die Zerstörung eines Mikroorganismus, vorzugsweise dessen Zerstörung, verstanden. Eine lichtinduzierte Inaktivierung kann beispielsweise durch Verringerung der Anzahl von Mikroorganismen nach Bestrahlung einer definierten Ausgangsmenge dieser Mikroorganismen in Gegenwart wenigstens einer erfindungsgemäßen Verbindung mit der Formel (1), der Formel (2) und/oder der Formel (3) und/oder der erfindungsgemäß zu verwendenden Verbindung mit der Formel (100), der Formel (101) und/oder der Formel (102) bestimmt werden.

Erfindungsgemäß wird unter einer Reduzierung der Lebensfähigkeit verstanden, dass die Anzahl der Mikroorganismen um wenigstens 80,0 %, vorzugsweise wenigstens 99,0 %, vorzugsweise wenigstens 99,9 %, weiter bevorzugt um wenigstens 99,99 %, weiter bevorzugt um wenigstens 99,999 %, noch weiter bevorzugt um wenigstens 99,9999 %, verringert wird. Äußerst bevorzugt wird die Anzahl der Mikroorganismen um mehr als 99,9 bis 100 %, vorzugsweise um mehr als 99,99 bis 100 % verringert wird.

Vorzugsweise wird die Reduzierung der Anzahl der Mikroorganismen gemäß Boyce, J. M. und Pittet, D. ("Guidelines for hand hygiene in healthcare settings. Recommendations of the Healthcare Infection Control Practices Advisory Committee and the HIPAC/SHEA/APIC/IDSA Hand Hygiene Task Force", Am.J.lnfect.Control 30 (8), 2002, Seite 1 - 46) als log₁₀-Reduktionsfaktor angegeben.

Erfindungsgemäß wird unter dem Begriff "log₁₀-Reduktionsfaktor" die Differenz zwischen dem dekadischen Logarithmus der Anzahl der Mikroorganismen vor und dem dekadischen Logarithmus der Anzahl der Mikroorganismen nach einer Bestrahlung dieser Mikroorganismen mit elektromagnetischer Strahlung in Gegenwart wenigstens einer erfindungsgemäßen Verbindung mit der Formel (1), der Formel (2) und/oder der Formel (3) und/oder der erfindungsgemäß zu verwendenden Verbindung mit der Formel (100), der Formel (101) und/oder der Formel (102) verstanden.

Geeignete Methoden zur Bestimmung des log₁₀-Reduktionsfaktors sind beispielsweise in der DIN EN 14885:2007-01 "Chemische Desinfektionsmittel und Antiseptika - Anwendung Europäischer Normen für chemische Desinfektionsmittel und Antiseptika" oder in Rabenau, H. F. und Schwebke, I. ("Leitlinie der Deutschen Vereinigung zur Bekämpfung der Viruskrankheiten (DVV) e. V. und der Robert Koch-Instituts (RKI) zur Prüfung von chemischen Desinfektionsmitteln auf Wirksamkeit gegen Viren in der Humanmedizin" Bundesgesundheitsblatt, Gesundheitsforschung, Gesundheitsschutz 51(8), (2008), Seiten 937 - 945) beschrieben.

Vorzugsweise beträgt der log₁₀-Reduktionsfaktor nach einer Bestrahlung von Mikroorganismen mit elektromagnetischer Strahlung in Gegenwart wenigstens einer erfindungsgemäßen Verbindung mit der Formel (1), der Formel (2) und/oder der Formel (3) und/oder der erfindungsgemäß zu verwendenden Verbindung mit der Formel (100), der Formel (101) und/oder der Formel (102) mindestens 2 log₁₀, vorzugsweise mindestens 3 log₁₀, weiter bevorzugt mindestens 4 log₁₀, weiter bevorzugt mindestens 4,5 log₁₀, weiter bevorzugt mindestens 5 log₁₀, weiter bevorzugt mindestens 6 log₁₀, noch weiter bevorzugt mindestens 7 log₁₀, noch weiter bevorzugt mindestens 7,5 log₁₀.

Beispielsweise bedeutet eine Reduktion der Anzahl der Mikroorganismen nach einer Bestrahlung dieser Mikroorganismen mit elektromagnetischer Strahlung in Gegenwart wenigstens einer erfindungsgemäßen Verbindung mit der Formel (1), der Formel (2) und/oder der Formel (3) und/oder der erfindungsgemäß zu verwendenden Verbindung mit der Formel (100), der Formel (101) und/oder der Formel (102) um 2 Zehnerpotenzen, bezogen auf die Ausgangsmenge dieser Mikroorganismen, einen log₁₀-Reduktionsfaktor von 2 log₁₀.

Weiter bevorzugt wird die Anzahl der Mikroorganismen nach einer Bestrahlung dieser Mikroorganismen mit elektromagnetischer Strahlung in Gegenwart wenigstens einer erfindungsgemäßen Verbindung mit der Formel (1), der Formel (2) und/oder der Formel (3) und/oder der erfindungsgemäß zu verwendenden Verbindung mit der Formel (100), der Formel (101) und/oder der Formel (102) um mindestens 1 Zehnerpotenz, weiter bevorzugt um mindestens 2 Zehnerpotenzen, vorzugsweise um mindestens 4 Zehnerpotenzen, weiter bevorzugt um mindestens 5 Zehnerpotenzen, weiter bevorzugt um mindestens 6 Zehnerpotenzen, noch weiter bevorzugt um mindestens 7 Zehnerpotenzen, jeweils bezogen auf die Ausgangsmenge dieser Mikroorganismen, verringert.

Unter dem Begriff "Mikroorganismen" werden im Sinne der Erfindung insbesondere Viren, Archäeen, prokaryote Mikroorganismen, wie Pilze, Protozoen, Pilzsporen, einzellige Algen, verstanden. Die Mikroorganismen können dabei einzellig oder mehrzellig, beispielsweise als Pilzmycel, auftreten.

Ein erfindungsgemäßes 1,7-Diaryl-1,6-heptadien -3,5-dion-Derivat weist die Formel (1) auf 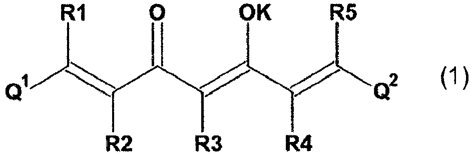 wobei die Reste Q¹ und Q² jeweils unabhängig voneinander 1 substituierter oder unsubstituierter monozyklischer oder polyzyklischer, aromatischer Rest bedeuten,
wobei die Verbindung mit der Formel (1) keine OH-Gruppe, die direkt an den organischen Rest Q¹ oder Q² gebunden ist, enthält, und
wobei K Wasserstoff oder ein Kation bedeutet.

Bei Variante (a) des erfindgungsgemäßen 1,7-Diaryl-1,6-heptadien -3,5-dion-Derivat der Formel (1) bedeuten die Reste Q¹ und Q² jeweils unabhängig voneinander ein substituierter oder unsubstituierter monozyklischer oder polyzyklischer, aromatischer Rest, K bedeutet Wasserstoff oder ein Kation, und wenigstens einer der Reste Q¹ und Q², vorzugsweise jeder der Reste Q¹ und Q², ist jeweils unabhängig voneinander mit mindestens einem, vorzugsweise 1 bis 9, weiter bevorzugt 1 bis 7, weiter bevorzugt 1 bis 5, weiter bevorzugt 1 bis 4, weiter bevorzugt 2 bis 3, organischen Rest(en) W1a der allgemeinen Formel (5a), (6a), (7a), (8a) oder (9a), vorzugsweise (5a), (7a), oder (9a),:

-A-(C(D)(E))ₕ-X^{a}, (5a)

-(C(D)(E))ₖ-Aryl-(C(D)(E))ₗ-X^{a}, (6a)

-A-(C(D)(E))ₖ-Aryl-(C(D)(E))ₗ-X^{a}, (7a)

-((C(D)(E))ₘ-A)ₚ-(C(D)(E))ₙ-X^{a}, (8a)

-A-((C(D)(E))ₘ-A)ₚ-(C(D)(E))ₙ-X^{a}, (9a)

substituiert,
wobei h eine ganze Zahl von 1 bis 20, vorzugsweise von 2 bis 8, bedeutet, wobei k eine ganze Zahl von 0 bis 10, vorzugsweise von 1 bis 8, vorzugsweise von 2 bis 6, bedeutet, wobei I eine ganze Zahl von 0 bis 10, vorzugsweise von 1 bis 8, vorzugsweise von 2 bis 6, bedeutet, und wobei m, n und p jeweils unabhängig voneinander eine ganze Zahl von 1 bis 6, vorzugsweise von 2 bis 4, bedeuten, und
wobei A jeweils unabhängig voneinander Sauerstoff oder Schwefel, vorzugsweise Sauerstoff, bedeutet,
wobei D und E jeweils unabhängig voneinander Wasserstoff, Halogen, G-R^{(I)}, oder G-C(=G)-R^{(II)}, bedeuten, wobei G jeweils unabhängig voneinander Sauerstoff oder Schwefel, vorzugsweise Sauerstoff, bedeutet, und wobei die Reste R^{(I)} und R^{(II)} jeweils unabhängig voneinander Wasserstoff, Methyl, Ethyl, n-Propyl, n-Butyl, n-Pentyl, Phenyl oder Benzyl bedeutet, wobei Phenyl und Benzyl unsubstituiert oder substituiert sein können,
wobei Aryl einen substituierten oder unsubstituierten Aromaten oder einen substituierten oder unsubstituierten Heteroaromaten, der kein Stickstoffatom enthält, bedeutet,
wobei X^{a} jeweils unabhängig voneinander ein Rest der Formel (20c), (20d), oder (21) bedeutet: 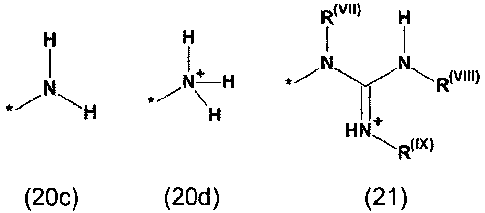 wobei jeder der Reste R^{(VII)}, R^{(VIII)}, und R^{(IX)} jeweils unabhängig voneinander Wasserstoff, ein Arylrest mit 5 bis 12 C-Atomen, ein Alkylarylrest mit 5 bis 12 C-Atomen, ein Alkylrest, der geradkettig oder verzweigt sein kann, mit 1 bis 8 C-Atomen, oder ein Etherrest, der geradkettig oder verzweigt sein kann, mit 1 bis 8 C-Atomen, vorzugsweise Wasserstoff, bedeutet, und
wobei die Reste R1, R2, R3, R4 und R5 jeweils unabhängig voneinander Wasserstoff, Halogen, Alkyl mit 1 bis 12 C-Atomen, Cycloalkyl mit 1 bis 12 C-Atomen, Alkylaryl mit 1 bis 12 C-Atomen, Aryl mit 5 bis 20 C-Atomen, Ether mit 2 bis 12 C-Atomen oder Glykol mit 2 bis 12 C-Atomen bedeuten.

Bei einer bevorzugten Ausführungsform der Verbindung mit der Formel (1) ist K ein Kation M^{z+} eines Metalls M, wobei z die formale Oxidationszahl des Metalls M ist und wobei z eine ganze Zahl von 1 bis 7, vorzugsweise von 2 bis 5, weiter bevorzugt von 2 bis 3, bedeutet, und wobei die Verbindung die Formel (2) aufweist: 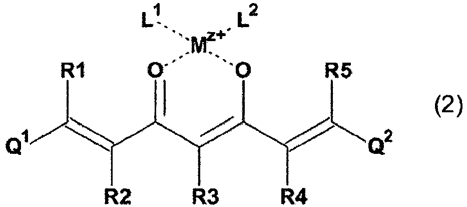 wobei L¹ und L² jeweils unabhängig voneinander Wasser, Fluorid, Chlorid, Bromid, lodid, Cyanid, Carbonyl, Thiocyanat, Phosphat, Hydrogenphosphat, Dihydrogenphosphat, Sulfat, Hydrogensulfat, Acetylacetonat, Acetessigester, Acetonitril, Tosylat, Mesylat oder wenigstens ein Carboxylation einer Carbonsäure mit 1 bis 15 Kohlenstoffatomen und/oder Kombinationen davon bedeuten.
Vorzugsweise ist ein Carboxylation einer Carbonsäure mit 1 bis 15 Kohlenstoffatomen Formiat, Acetat, n-Propionat, Lactat, Oxalat, Fumarat, Maleinat, Tartrat, Succinylat, Benzoat, Salicylat, Citrat, und/oder Kombinationen davon.

Weiter bevorzugt sind L1 und L2 gleich, beispielsweise ein mehrzähniger Ligand. Geeignete mehrzähnige Liganden weisen zwei oder mehr Koordinationststellen auf, die an das Kation M^{z+} binden können. Geeignete mehrzähnige Liganden sind beispielsweise Ethylendiamin, Nitrilotriessigsäure (NTA), Ethylendiamintetraacetat-Salze (EDTA), Acetylacetonat, Acetessigester, Citrat, Bis(2-methoxyethyl)ether (Diglyme), 8-Hydroxychinolin, 2,2'-Bipyridin, 1,10-Phenanthrolin (phen), Dimercaptobernsteinsäure, Tartrat oder Oxalat.

Ein erfindungsgemäßes 1,7-Diaryl-1,6-heptadien -3,5-dion-Derivat weist ebenfalls die Formel (3) auf.

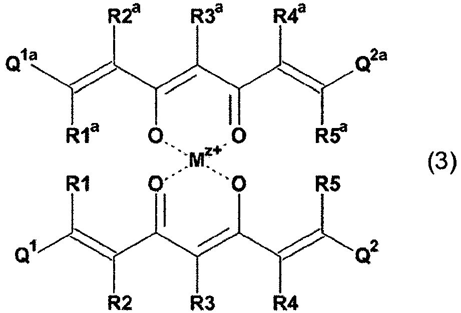

Bei Variante (a) des erfindungsgemäßen 1,7-Diaryl-1,6-heptadien -3,5-dion-Derivats der Formel (3) bedeutet M^{z+} ein Kation eines Metalls M, wobei z die formale Oxidationszahl des Metalls M ist und wobei z eine ganze Zahl von 1 bis 7, vorzugsweise von 2 bis 3, bedeutet, und
wobei die Reste Q¹ und Q² jeweils unabhängig voneinander 1 substituierter oder unsubstituierter monozyklischer oder polyzyklischer, aromatischer Rest bedeuten,
wobei die Verbindung mit der Formel (3) keine OH-Gruppe, die direkt an den organischen Rest Q¹, Q^{1a}, Q², oder Q^{2a} gebunden ist, enthält,
wobei die Reste Q^{1a} und Q^{2a} jeweils unabhängig voneinander 1 substituierter oder unsubstituierter monozyklischer oder polyzyklischer, aromatischer Rest oder 1 substituierter oder unsubstituierter monozyklischer oder polyzyklischer, heteroaromatischer Rest, vorzugsweise 1 substituierter oder unsubstituierter monozyklischer oder polyzyklischer, aromatischer Rest, bedeuten,
und wobei wenigstens einer der Reste Q¹ und Q², vorzugsweise jeder der Reste Q¹ und Q², jeweils unabhängig voneinander mit mindestens einem, vorzugsweise 1 bis 9, weiter bevorzugt 1 bis 7, weiter bevorzugt 1 bis 5, weiter bevorzugt 1 bis 4, weiter bevorzugt 2 bis 3, organischen Rest(en) W1a der allgemeinen Formel (5a), (6a), (7a), (8a), oder (9a), vorzugsweise (5b), (7b), oder (9b),:

   -A-(C(D)(E))ₕ-X^{a}, (5a)

   -(C(D)(E))ₖ-Aryl-(C(D)(E))ₗ-X^{a}, (6a)

   -A-(C(D)(E))ₖ-Aryl-(C(D)(E))ₗ-X^{a}, (7a)

   -((C(D)(E))ₘ-A)ₚ-(C(D)(E))ₙ-X^{a}, (8a)

   -A-((C(D)(E))ₘ-A)ₚ-(C(D)(E))ₙ-X^{a}, (9a),

   substituiert ist,
   wobei h eine ganze Zahl von 1 bis 20, vorzugsweise von 2 bis 8, bedeutet, wobei k eine ganze Zahl von 0 bis 10, vorzugsweise von 1 bis 8, vorzugsweise von 2 bis 6, bedeutet, wobei I eine ganze Zahl von 0 bis 10, vorzugsweise von 1 bis 8, vorzugsweise von 2 bis 6, bedeutet, und wobei m, n und p jeweils unabhängig voneinander eine ganze Zahl von 1 bis 6, vorzugsweise von 2 bis 4, bedeuten, und
   wobei A jeweils unabhängig voneinander Sauerstoff oder Schwefel, vorzugsweise Sauerstoff, bedeutet,
   wobei D und E jeweils unabhängig voneinander Wasserstoff, Halogen, G-R^{(I)}, oder G-C(=G)-R^{(II)}, bedeuten, wobei G jeweils unabhängig voneinander Sauerstoff oder Schwefel, vorzugsweise Sauerstoff, bedeutet, und wobei die Reste R^{(I)} und R^{(II)} jeweils unabhängig voneinander Wasserstoff, Methyl, Ethyl, n-Propyl, n-Butyl, n-Pentyl, Phenyl oder Benzyl bedeutet, wobei Phenyl und Benzyl unsubstituiert oder substituiert sein können,
   wobei Aryl einen substituierten oder unsubstituierten Aromaten oder einen substituierten oder unsubstituierten Heteroaromaten, der kein Stickstoffatom enthält, bedeutet,
   wobei X^{a} jeweils unabhängig voneinander ein Rest der Formel (20c), (20d), oder (21) bedeutet: 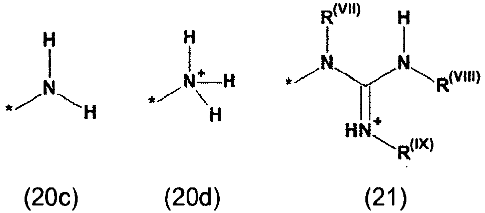 wobei jeder der Reste R^{(VII)}, R^{(VIII)}, und R^{(IX)} jeweils unabhängig voneinander Wasserstoff, ein Arylrest mit 5 bis 12 C-Atomen, ein Alkylarylrest mit 5 bis 12 C-Atomen, ein Alkylrest, der geradkettig oder verzweigt sein kann, mit 1 bis 8 C-Atomen, oder ein Etherrest, der geradkettig oder verzweigt sein kann, mit 1 bis 8 C-Atomen, vorzugsweise Wasserstoff, bedeutet, und
   wobei wenigstens einer der Reste Q^{1a} und Q^{2a}, vorzugsweise jeder der Reste Q^{1a} und Q^{2a}, jeweils unabhängig voneinander mit mindestens einem, vorzugsweise 1 bis 9, weiter bevorzugt 1 bis 7, weiter bevorzugt 1 bis 5, weiter bevorzugt 1 bis 4, weiter bevorzugt 2 bis 3, organischen Rest(en) W1c der allgemeinen Formel (5c), (6c), (7c), (8c), oder (9c), vorzugsweise (5c), (7c), oder (9c),:

   -A-(C(D)(E)ₕ-X^{c}, (5c)

   -(C(D)(E))ₖ-Aryl-(C(D)(E))ₗ-X^{c}, (6c)

   -A-(C(D)(E))ₖ-Aryl-(C(D)(E))ₗ-X^{c}, (7c)

   -((C(D)(E))ₘ-A)ₚ-(C(D)(E))ₙ-X^{c}, (8c)

   -A-((C(D)(E))ₘ-A)ₚ-(C(D)(E))ₙ-X^{c}, (9c),

   substituiert ist,
   wobei h eine ganze Zahl von 1 bis 20, vorzugsweise von 2 bis 8, bedeutet, wobei k eine ganze Zahl von 0 bis 10, vorzugsweise von 1 bis 8, vorzugsweise von 2 bis 6, bedeutet, wobei I eine ganze Zahl von 0 bis 10, vorzugsweise von 1 bis 8, vorzugsweise von 2 bis 6, bedeutet, und wobei m, n und p jeweils unabhängig voneinander eine ganze Zahl von 1 bis 6, vorzugsweise von 2 bis 4, bedeuten, und
   wobei A jeweils unabhängig voneinander Sauerstoff oder Schwefel, vorzugsweise Sauerstoff, bedeutet,
   wobei D und E jeweils unabhängig voneinander Wasserstoff, Halogen, G-R^{(I)}, oder G-C(=G)-R^{(II)}, bedeuten, wobei G jeweils unabhängig voneinander Sauerstoff oder Schwefel, vorzugsweise Sauerstoff, bedeutet, und wobei die Reste R^{(I)} und R^{(II)} jeweils unabhängig voneinander Wasserstoff, Methyl, Ethyl, n-Propyl, n-Butyl, n-Pentyl, Phenyl oder Benzyl bedeutet, wobei Phenyl und Benzyl unsubstituiert oder substituiert sein können,
   wobei Aryl einen substituierten oder unsubstituierten Aromaten oder einen substituierten oder unsubstituierten Heteroaromaten, der kein Stickstoffatom enthält, bedeutet,
   wobei X^{c} jeweils unabhängig voneinander ein organischer Rest ist, der (i) wenigstens ein neutrales, protonierbares Stickstoffatom, oder (ii) wenigstens ein positiv geladenes, vorzugsweise quartäres, Stickstoffatom, oder (iii) wenigstens ein positiv geladenes, vorzugsweise quartäres, Phosphoratom enthält, wobei vorzugsweise X^{c} jeweils unabhängig voneinander ein Rest der Formel (20c), (20d), (21), oder (24), weiter bevorzugt ein Rest der Formel (20c), (20d), oder (21), weiter bevorzugt ein Rest der Formel (24), bedeutet: 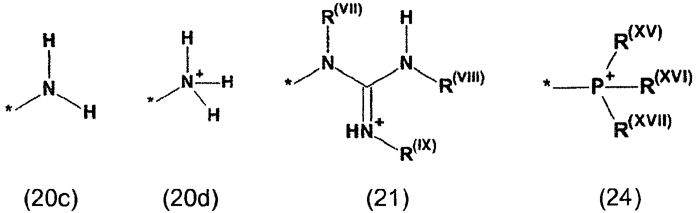 wobei jeder der Reste R^{(VII)}, R^{(VIII)}, und R^{(IX)} jeweils unabhängig voneinander Wasserstoff, ein Arylrest mit 5 bis 12 C-Atomen, ein Alkylarylrest mit 5 bis 12 C-Atomen, ein Alkylrest, der geradkettig oder verzweigt sein kann, mit 1 bis 8 C-Atomen, oder ein Etherrest, der geradkettig oder verzweigt sein kann, mit 1 bis 8 C-Atomen, vorzugsweise Wasserstoff, bedeutet und wobei jeder der Reste R^{(XV)}, R^{(XVI),} und R^{(XVII)} jeweils unabhängig voneinander ein Arylrest mit 5 bis 12 C-Atomen, ein Alkylarylrest mit 5 bis 12 C-Atomen, ein Alkylrest, der geradkettig oder verzweigt sein kann, mit 1 bis 8 C-Atomen, oder ein Etherrest, der geradkettig oder verzweigt sein kann, mit 1 bis 8 C-Atomen, vorzugsweise ein Arylrest mit 5 bis 12 C-Atomen, ein Alkylarylrest mit 5 bis 12 C-Atomen, oder ein Alkylrest, der geradkettig oder verzweigt sein kann, mit 1 bis 8 C-Atomen, bedeutet,
   wobei die Reste R1, R1^{a}, R2, R2^{a}, R3, R3^{a}, R4, R4^{a}, R5 und R5^{a} jeweils unabhängig voneinander Wasserstoff, Halogen, Alkyl mit 1 bis 12 C-Atomen, Cycloalkyl mit 1 bis 12 C-Atomen, Alkylaryl mit 1 bis 12 C-Atomen, Aryl mit 5 bis 20 C-Atomen, Ether mit 2 bis 12 C-Atomen oder Glykol mit 2 bis 12 C-Atomen bedeuten.

Bei einer bevorzugten Ausführungsform der Verbindung mit der Formel (3) weist die Verbindun die Formel (3a) auf:

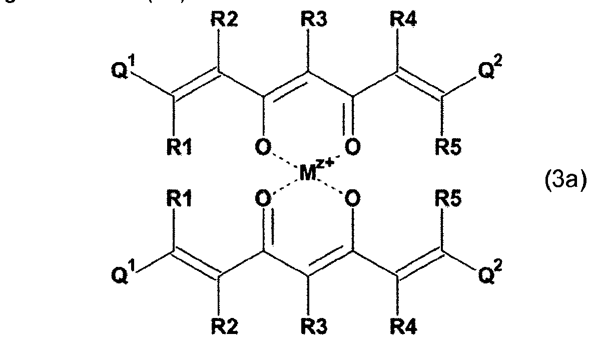

Bei Variante (a) des erfindungsgemäßen 1,7-Diaryl-1,6-heptadien -3,5-dion-Derivats der Formel (3a) bedeutet M^{z+} ein Kation eines Metalls M, wobei z die formale Oxidationszahl des Metalls M ist und wobei z eine ganze Zahl von 1 bis 7, vorzugsweise von 2 bis 3, bedeutet, und
wobei die Reste Q¹ und Q² jeweils unabhängig voneinander 1 substituierter oder unsubstituierter monozyklischer oder polyzyklischer, aromatischer Rest bedeuten,
wobei die Verbindung mit der Formel (3a) keine OH-Gruppe, die direkt an den organischen Rest Q¹ oder Q² gebunden ist, enthält, und
wobei wenigstens einer der Reste Q¹ und Q², vorzugsweise jeder der Reste Q¹ und Q2, jeweils unabhängig voneinander mit mindestens einem, vorzugsweise 1 bis 9, weiter bevorzugt 1 bis 7, weiter bevorzugt 1 bis 5, weiter bevorzugt 1 bis 4, weiter bevorzugt 2 bis 3, organischen Rest(en) W1a der allgemeinen Formel (5a), (6a), (7a), (8a), oder (9a), vorzugsweise (5a), (7a), oder (9a),:

-A-(C(D)(E))ₕ-X^{a}, (5a)

-(C(D)(E))ₖ-Aryl-(C(D)(E))ₗ-X^{a}, (6a)

-A-(C(D)(E))ₖ-Aryl-(C(D)(E))ₗ-X^{a}, (7a)

-((C(D)(E))ₘ-A)ₚ-(C(D)(E))ₙ-X^{a}, (8a)

-A-((C(D)(E))ₘ-A)ₚ-(C(D)(E))ₙ-X^{a}, (9a)

substituiert ist,
wobei h eine ganze Zahl von 1 bis 20, vorzugsweise von 2 bis 8, bedeutet, wobei k eine ganze Zahl von 0 bis 10, vorzugsweise von 1 bis 8, vorzugsweise von 2 bis 6, bedeutet, wobei I eine ganze Zahl von 0 bis 10, vorzugsweise von 1 bis 8, vorzugsweise von 2 bis 6, bedeutet, und wobei m, n und p jeweils unabhängig voneinander eine ganze Zahl von 1 bis 6, vorzugsweise von 2 bis 4, bedeuten, und
wobei A jeweils unabhängig voneinander Sauerstoff oder Schwefel, vorzugsweise Sauerstoff, bedeutet,
wobei D und E jeweils unabhängig voneinander Wasserstoff, Halogen, G-R^{(I)}, oder G-C(=G)-R^{(II)}, bedeuten, wobei G jeweils unabhängig voneinander Sauerstoff oder Schwefel, vorzugsweise Sauerstoff, bedeutet, und wobei die Reste R^{(I)} und R^{(II)} jeweils unabhängig voneinander Wasserstoff, Methyl, Ethyl, n-Propyl, n-Butyl, n-Pentyl, Phenyl oder Benzyl bedeutet, wobei Phenyl und Benzyl unsubstituiert oder substituiert sein können,
wobei Aryl einen substituierten oder unsubstituierten Aromaten oder einen substituierten oder unsubstituierten Heteroaromaten, der kein Stickstoffatom enthält, bedeutet,
wobei X^{a} jeweils unabhängig voneinander ein Rest der Formel (20c), (20d), oder (21) bedeutet: 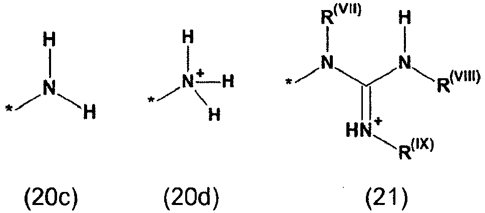 wobei jeder der Reste R^{(VII)}, R^{(VIII)}, und R^{(IX)} jeweils unabhängig voneinander Wasserstoff, ein Arylrest mit 5 bis 12 C-Atomen, ein Alkylarylrest mit 5 bis 12 C-Atomen, ein Alkylrest, der geradkettig oder verzweigt sein kann, mit 1 bis 8 C-Atomen, oder ein Etherrest, der geradkettig oder verzweigt sein kann, mit 1 bis 8 C-Atomen, vorzugsweise Wasserstoff, bedeutet, und
wobei die Reste R1, R2, R3, R4, und R5 jeweils unabhängig voneinander Wasserstoff, Halogen, Alkyl mit 1 bis 12 C-Atomen, Cycloalkyl mit 1 bis 12 C-Atomen, Alkylaryl mit 1 bis 12 C-Atomen, Aryl mit 5 bis 20 C-Atomen, Ether mit 2 bis 12 C-Atomen oder Glykol mit 2 bis 12 C-Atomen bedeuten.

Bei einer bevorzugten Ausführungsform der Verbindung mit der Formel (2), und/oder der Verbindung mit der Formel (3), und/oder der Verbindung mit der Formel (3a), und/oder der Verbindung mit der Formel (101), und/oder der Verbindung mit der Formel (102) wird das Metall M aus der Gruppe ausgewählt, die aus Alkalimetallen, Erdalkalimetallen, Übergangsmetallen, Metallen und Halbmetallen der dritten, vierten, fünften und sechsten Hauptgruppe des Periodensystems der Elemente und Kombinationen davon besteht, vorzugsweise wird das Metall M aus der Gruppe ausgewählt, die aus Be, Mg, Ca, Sr, Ba, Ti, Zr, Hf, V, Nb, Ta, Cr, Mo, W, Mn, Tc, Rh, Fe, Ru, Os, Co, Rh, Ir, Ni, Pd, Pt, Cu, Ag, Au, Zn, B, Al, Ga, In, Si, Ge, Sn, Bi, Se, Te und Kombinationen davon besteht.

Weiter bevorzugt wird das Metall M aus der Gruppe ausgewählt, die aus B, Al, Zn, Cu, Mg, Ca, Fe, Si, Ga, Sn, Rh, Co, Ti, Zr, V, Cr, Mo, Mn, Ru, Pd, Ir, Ni, und Kombinationen davon besteht. Weiter bevorzugt bedeutet M^{z+} Mg²⁺, Ca²⁺, Fe²⁺, Fe³⁺, Zn²⁺, Cu²⁺, B³⁺ oder Si⁴⁺.

Erfindungsgemäß sind bei der Verbindung mit der Formel (1) oder der Verbindung mit der Formel (2) oder der Verbindung mit der Formel (3a) die Reste Q¹ und Q² oder bei der Verbindung mit der Formel (3) die Reste Q¹, Q^{1a}, Q² und Q^{2a} jeweils unabhängig voneinander ein substituierter oder unsubstituierter monozyklischer oder polyzyklischer, aromatischer Rest, der vorzugsweise jeweils unabhängig voneinander ein Phenylrest, ein Naphthylrest, ein Azulenrest, ein Phenalenrest, ein Acenaphthylrest, ein Fluorenrest, ein Phenanthrenrest, ein Anthracenrest, ein Fluoranthenrest, ein Pyrenrest, ein Benzo[a]anthracenrest, ein Chrysenrest, ein Benzo[b]flouranthenrest, ein Benzo[h]flouranthenrest, ein Benzo[a]pyrenrest, ein Dibenzo[a,h]anthracenrest, ein Tetracenrest, ein Triphenylenrest, ein Pentacenrest, oder ein Hexacenrest, weiter bevorzugt ein Phenylrest, ein Naphthylrest, ein Phenalenrest, oder ein Anthracenrest, ist.

Erfindungsgemäß sind bei der Verbindung mit der Formel (100) oder der Verbindung mit der Formel (102) die Reste Q³ und Q⁴ oder bei der Verbindung mit der Formel (101) die Reste Q³, Q^{3a}, Q⁴ und Q^{4a} jeweils unabhängig voneinander ein substituierter oder unsubstituierter monozyklischer oder polyzyklischer, aromatischer Rest, der vorzugsweise jeweils unabhängig voneinander ein Phenylrest, ein Naphthylrest, ein Azulenrest, ein Phenalenrest, ein Acenaphthylrest, ein Fluorenrest, ein Phenanthrenrest, ein Anthracenrest, ein Fluoranthenrest, ein Pyrenrest, ein Benzo[a]anthracenrest, ein Chrysenrest, ein Benzo[b]flouranthenrest, ein Benzo[h]flouranthenrest, ein Benzo[a]pyrenrest, ein Dibenzo[a,h]anthracenrest, ein Tetracenrest, ein Triphenylenrest, ein Pentacenrest, oder ein Hexacenrest, weiter bevorzugt ein Phenylrest, ein Naphthylrest, ein Phenalenrest, oder ein Anthracenrest, ist, oder ein substituierter oder unsubstituierter monozyklischer oder polyzyklischer, heteroaromatischer Rest, der vorzugsweise wenigstens 5 Ringatome aufweist, wobei die Ringatome mindestens 1 Kohlenstoffatom und wenigstens 1, vorzugsweise protonierbares, Stickstoffatom enthalten, wobei der eine substituierte oder unsubstituierte monozyklische oder polyzyklische, heteroaromatische Rest weiter bevorzugt jeweils unabhängig voneinander ein Pyrolrest, ein Pyridinrest, ein Chinolinrest, ein Isochinolinrest, ein Indolrest, ein Isoindolrest, ein Pyrimidinrest, ein Pyrazinrest, ein Imidazolrest, oder ein Acridinrest, weiter bevorzugt ein Pyridinrest, ein Chinolinrest, ein Isochinolinrest, oder ein Indolrest, ist.

Vorzugsweise weist der eine substituierte oder unsubstituierte monozyklische oder polyzyklische, heteroaromatische Rest 5 bis 14, weiter bevorzugt 6 bis 10 Ringatome auf, wobei die Ringatome mindestens 1 Kohlenstoffatom und wenigstens 1, vorzugsweise protonierbares, Stickstoffatom, enthalten. Weiter bevorzugt enthalten die Ringatome 1 bis 3, vorzugsweise protonierbare, Stickstoffatome, die weiter bevorzugt nicht benachbart angeordnet sind.

Bei Variante (a1) der Verbindung mit der Formel (100), der Verbindung mit der Formel (101) und/oder der Verbindung mit der Formel (102) ist wenigstens einer der Reste Q³, Q^{3a}, Q⁴ und Q^{4a} jeweils unabhängig voneinander ein unsubstituierter monozyklischer oder polyzyklischer, heteroaromatischer Rest, der wenigstens 5, vorzugsweise 5 bis 14, weiter bevorzugt 6 bis 10, Ringatome aufweist, wobei die Ringatome mindestens 1 Kohlenstoffatom und wenigstens 1, vorzugsweise protonierbares, Stickstoffatom enthalten. Weiter bevorzugt enthalten die Ringatome 1 bis 3, vorzugsweise 1 oder 2, Stickstoffatome, die vorzugsweise protonierbar sind. Die Stickstoffatome sind weiter bevorzugt nicht benachbart angeordnet.

Vorzugsweise sind vorgenannte substituierte monozyklische oder polyzyklische, aromatische Reste oder substituierte monozyklische oder polyzyklische, heteroaromatische Reste, mit mindestens einem organischen Rest W1 oder einem organischen Rest W1a oder einem organischen Rest W1b oder einem organischen Rest W1c und/oder mit Halogen, Nitro, Carboxylat, Aldehyd mit 1 bis 8 C-Atomen, Keton mit 2 bis 8 C-Atomen, O-Alkyl mit 1 bis 12 C-Atomen, O-Alkenyl mit 2 bis 12 C-Atomen, O-Aryl mit 5 bis 20 C-Atomen, Ether mit 2 bis 12 C-Atomen, Carbonsäureester mit 1 bis 12 C-Atomen, Carbonsäureamid mit 1 bis 12 C-Atomen, wobei vorzugsweise das C-Atom der Carboxamidgruppe an vorgenannte substituierte monozyklische oder polyzyklische, aromatische Reste oder substituierte monozyklische oder polyzyklische, heteroaromatische Reste gebunden ist, Alkyl mit 1 bis 12 C-Atomen, Alkenyl mit 2 bis 12 C-Atomen, Cycloalkyl mit 3 bis 12 C-Atomen, Cycloalkenyl mit 3 bis 12 C-Atomen, Alkylaryl mit 1 bis 12 C-Atomen, Aryl mit 5 bis 20 C-Atomen oder Heteroaryl, das kein Stickstoffatom enthält, mit 4 bis 20 C-Atomen substituiert.

Weiter bevorzugt sind vorgenannte substituierte monozyklische oder polyzyklische, aromatische Reste oder substituierte monozyklische oder polyzyklische, heteroaromatische Reste, mit mindestens einem organischen Rest W1 oder einem organischen Rest W1a oder einem organischen Rest W1b oder einem organischen Rest W1c und/oder mit Halogen, Nitro, Aldehyd mit 1 bis 8 C-Atomen, Keton mit 2 bis 8 C-Atomen, O-Alkyl mit 1 bis 12 C-Atomen, O-Alkenyl mit 2 bis 12 C-Atomen, O-Aryl mit 5 bis 20 C-Atomen, Ether mit 2 bis 12 C-Atomen, Carbonsäureester mit 1 bis 12 C-Atomen, Alkyl mit 1 bis 12 C-Atomen, Alkenyl mit 2 bis 12 C-Atomen, Cycloalkyl mit 3 bis 12 C-Atomen, Cycloalkenyl mit 3 bis 12 C-Atomen, Alkylaryl mit 1 bis 12 C-Atomen, Aryl mit 5 bis 20 C-Atomen oder Heteroaryl, das kein Stickstoffatom enthält, mit 4 bis 20 C-Atomen substituiert.

Vorzugsweise weist der eine substituierte oder unsubstituierte monozyklische oder polyzyklische, aromatische Rest wenigstens 5 Ringatome, vorzugsweise 5 bis 16, weiter bevorzugt 6 bis 14, weiter bevorzugt 6 bis 10, die jeweils mindestens 1 Kohlenstoffatom und optional wenigstens ein, vorzugsweise 1 bis 2, Sauerstoffatom(e), die weiter bevorzugt nicht benachbart angeordnet sind, enthalten, auf.

Vorzugsweise ist bei der Verbindung mit der Formel (1), und/oder der Verbindung mit der Formel (2), und/oder der Verbindung mit der Formel (3a), der Rest Q¹ oder bei der Verbindung mit der Formel (100) und/oder der Verbindung mit der Formel (102) der Rest Q³ ein aromatischer Rest der allgemeinen Formel (11a), (12a), (13a), (14a), (15a), (16a), (17a), (18a), oder (19a): 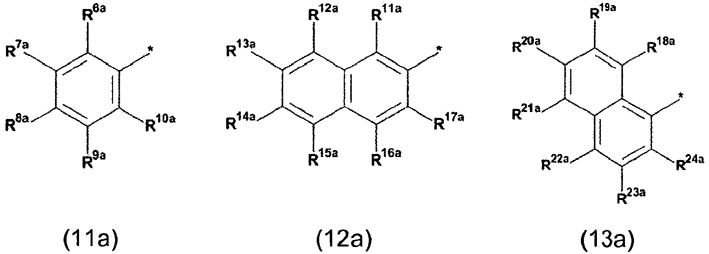 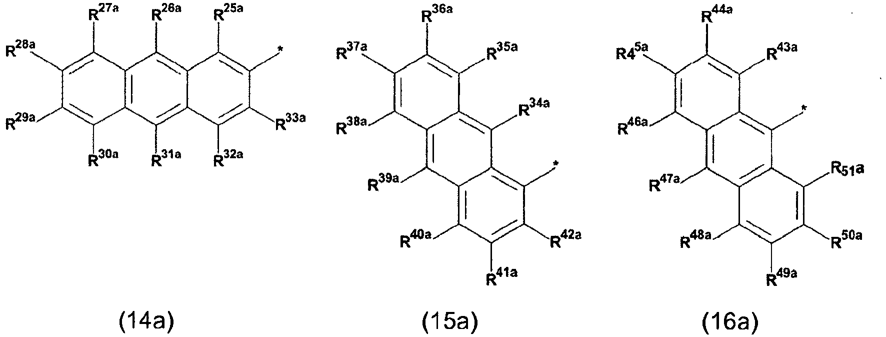 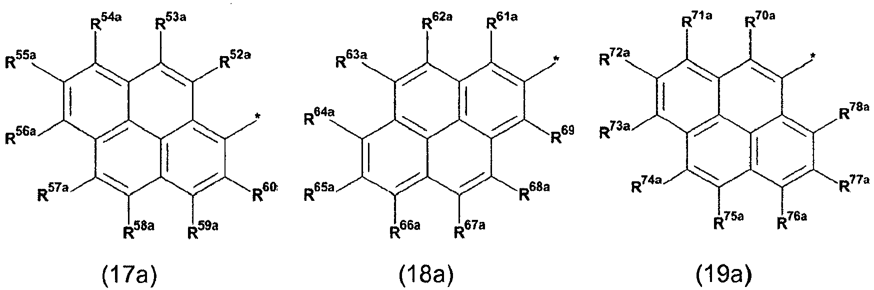 wobei bei der Verbindung mit der Formel (1), und/oder der Verbindung mit der Formel (2), und/oder der Verbindung mit der Formel (3a), der Rest Q² oder bei der Verbindung mit der Formel (100) und/oder der Verbindung mit der Formel (102) der Rest Q⁴ ein aromatischer Rest der allgemeinen Formel (11b), (12b), (13b), (14b), (15b), (16b), (17b), (18b), oder (19b) bedeutet: 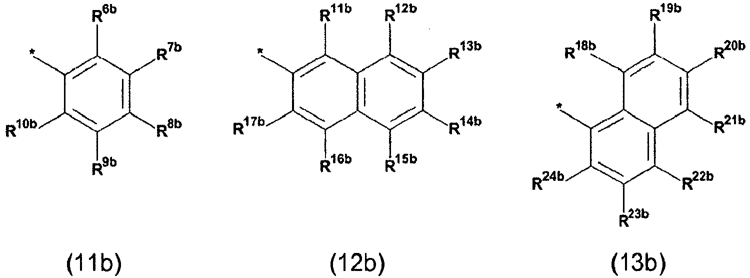 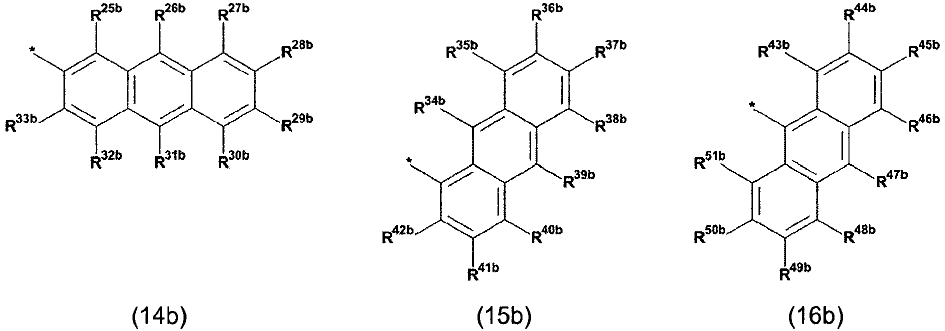 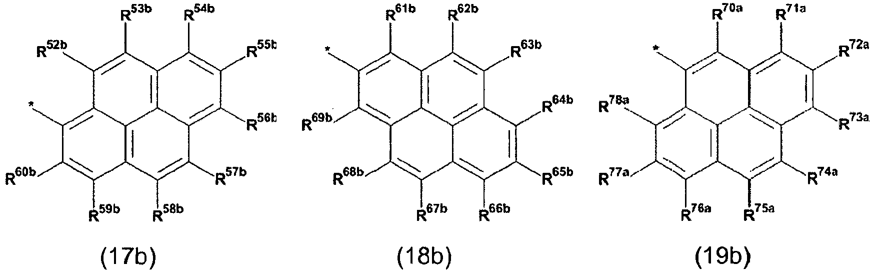 wobei jeweils mindestens 1, vorzugsweise 1 bis 9, weiter bevorzugt 1 bis 7, weiter bevorzugt 1 bis 5, weiter bevorzugt 1 bis 4, weiter bevorzugt 2 bis 3, Rest(e) R^{6a} bis R^{10a}, R^{11a} bis R^{17a}, R^{18a} bis R^{24a}, R^{25a} bis R^{33a}, R^{34a} bis R^{42a}, R^{43a} bis R^{51a}, R^{52a} bis R^{60a}, R^{61a} bis R^{69a}, R^{70a} bis R^{78a}, R^{6b} bis R^{10b}, R^{11b} bis R^{17b}, R^{18b} bis R^{24b}, R^{25b} bis R^{33b}, R^{34b} bis R^{42b}, R^{43b} bis R^{51b}, R^{52b} bis R^{60b}, R^{61b} bis R^{69b}, oder R^{70b} bis R^{78b} jeweils unabhängig voneinander ein organischer Rest W1 oder ein organischer Rest W1 a oder ein organischer Rest W1 b oder ein organischer Rest W1 c ist, und
wobei die Reste R^{6a} bis R^{10a}, R^{11a} bis R^{17a}, R^{18a} bis R^{24a}, R^{25a} bis R^{33a}, R^{34a} bis R^{42a}, R^{43a} bis R^{51a}, R^{52a} bis R^{60a}, R^{61a} bis R^{69a}, R^{70a} bis R^{78a}, R^{6b} bis R^{10b}, R^{11b} bis R^{17b}, R^{18b} bis R^{24b}, R^{25b} bis R^{33b}, R^{34b} bis R^{42b}, R^{43b} bis R^{51b}, R^{52b} bis R^{60b}, R^{61b} bis R^{69b}, oder R^{70b} bis R^{78b}, die nicht ein organischer Rest W1 oder ein organischer Rest W1 a oder ein organischer Rest W1b oder ein organischer Rest W1c sind, jeweils unabhängig voneinander gleich oder verschieden sind und Wasserstoff, Halogen, Nitro, Carboxylat, Aldehyd mit 1 bis 8 C-Atomen, Keton mit 2 bis 8 C-Atomen, O-Alkyl mit 1 bis 12 C-Atomen, O-Alkenyl mit 2 bis 12 C-Atomen, O-Aryl mit 5 bis 20 C-Atomen, Ether mit 2 bis 12 C-Atomen, Carbonsäureester mit 1 bis 12 C-Atomen, Carbonsäureamid mit 1 bis 12 C-Atomen, wobei vorzugsweise jeweils das C-Atom der Carboxamidgruppe an einen der Reste Q¹ bis Q⁴ gebunden ist, Alkyl mit 1 bis 12 C-Atomen, Alkenyl mit 2 bis 12 C-Atomen, Cycloalkyl mit 3 bis 12 C-Atomen, Cycloalkenyl mit 3 bis 12 C-Atomen, Alkylaryl mit 1 bis 12 C-Atomen, Aryl mit 5 bis 20 C-Atomen oder Heteroaryl, das kein Stickstoffatom enthält, mit 4 bis 20 C-Atomen, vorzugsweise Wasserstoff, Halogen, Nitro, Carboxylat, Aldehyd mit 1 bis 8 C-Atomen, Keton mit 2 bis 8 C-Atomen, O-Alkyl mit 1 bis 12 C-Atomen, O-Alkenyl mit 2 bis 12 C-Atomen, O-Aryl mit 5 bis 20 C-Atomen, Ether mit 2 bis 12 C-Atomen, Carbonsäureester mit 1 bis 12 C-Atomen, Alkyl mit 1 bis 12 C-Atomen, Alkenyl mit 2 bis 12 C-Atomen, Cycloalkyl mit 3 bis 12 C-Atomen, Cycloalkenyl mit 3 bis 12 C-Atomen, Alkylaryl mit 1 bis 12 C-Atomen, Aryl mit 5 bis 20 C-Atomen oder Heteroaryl, das kein Stickstoffatom enthält, mit 4 bis 20 C-Atomen, bedeuten.

Weiter bevorzugt ist der Rest Q¹ bzw. der Rest Q³ ein aromatischer Rest der allgemeinen Formel (11a), (12a) oder (13a) 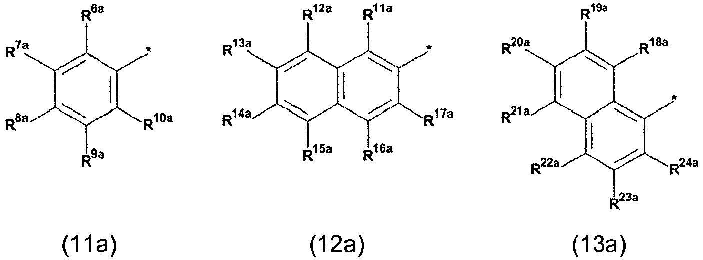 und der Rest Q² bzw. der Rest Q⁴ ist ein aromatischer Rest der allgemeinen Formel (11b), (12b) oder (13b), 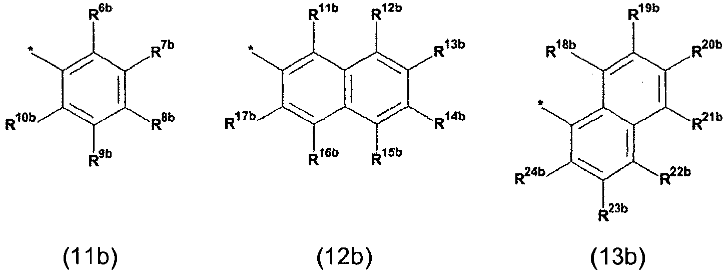 wobei jeweils mindestens 1, vorzugsweise 1 bis 9, weiter bevorzugt 1 bis 7, weiter bevorzugt 1 bis 5, weiter bevorzugt 1 bis 4, weiter bevorzugt 2 bis 3, Rest(e) R^{6a} bis R^{10a}, R^{11a} bis R^{17a}, R^{18a} bis R^{24a}, R^{6b} bis R^{10b}, R^{11b} bis R^{17b} oder R^{18b} bis R^{24b} jeweils unabhängig voneinander ein organischer Rest W1 oder ein organischer Rest W1a oder ein organischer Rest W1b oder ein organischer Rest W1c ist, und wobei die Reste R^{10a}, R^{11a} bis R^{17a}, R^{18a} bis R^{24a}, R^{6b} bis R^{10b}, R^{11b} bis R^{17b} und R^{18b} bis R^{24b}, die nicht ein organischer Rest W1 oder ein organischer Rest W1a oder ein organischer Rest W1b oder ein organischer Rest W1c sind, jeweils unabhängig voneinander gleich oder verschieden sind und Wasserstoff, Halogen, Nitro, Carboxylat, Aldehyd mit 1 bis 8 C-Atomen, Keton mit 2 bis 8 C-Atomen, O-Alkyl mit 1 bis 12 C-Atomen, O-Alkenyl mit 2 bis 12 C-Atomen, O-Aryl mit 5 bis 20 C-Atomen, Ether mit 2 bis 12 C-Atomen, Carbonsäureester mit 1 bis 12 C-Atomen, Carbonsäureamid mit 1 bis 12 C-Atomen; wobei vorzugsweise jeweils das C-Atom der Carboxamidgruppe an einen der Reste Q¹ bis Q⁴ gebunden ist, Alkyl mit 1 bis 12 C-Atomen, Alkenyl mit 2 bis 12 C-Atomen, Cycloalkyl mit 3 bis 12 C-Atomen, Cycloalkenyl mit 3 bis 12 C-Atomen, Alkylaryl mit 1 bis 12 C-Atomen, Aryl mit 5 bis 20 C-Atomen oder Heteroaryl, das kein Stickstoffatom enthält, mit 4 bis 20 C-Atomen, vorzugsweise Wasserstoff, Halogen, Nitro, Carboxylat, Aldehyd mit 1 bis 8 C-Atomen, Keton mit 2 bis 8 C-Atomen, O-Alkyl mit 1 bis 12 C-Atomen, O-Alkenyl mit 2 bis 12 C-Atomen, O-Aryl mit 5 bis 20 C-Atomen, Ether mit 2 bis 12 C-Atomen, Carbonsäureester mit 1 bis 12 C-Atomen, Alkyl mit 1 bis 12 C-Atomen, Alkenyl mit 2 bis 12 C-Atomen, Cycloalkyl mit 3 bis 12 C-Atomen, Cycloalkenyl mit 3 bis 12 C-Atomen, Alkylaryl mit 1 bis 12 C-Atomen, Aryl mit 5 bis 20 C-Atomen oder Heteroaryl, das kein Stickstoffatom enthält, mit 4 bis 20 C-Atomen, bedeuten.

Vorzugsweise sind bei der Verbindung mit der Formel (3) die Reste Q¹ und Q^{1a} oder bei der Verbindung mit der Formel (101) die Reste Q³ und Q^{3a} jeweils unabhängig voneinander ein aromatischer Rest der allgemeinen Formel (11a), (12a), (13a), (14a), (15a), (16a), (17a), (18a), oder (19a): 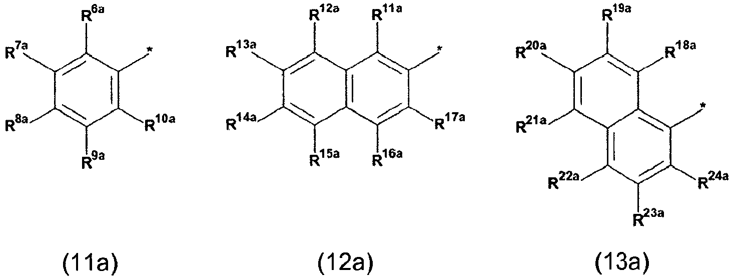 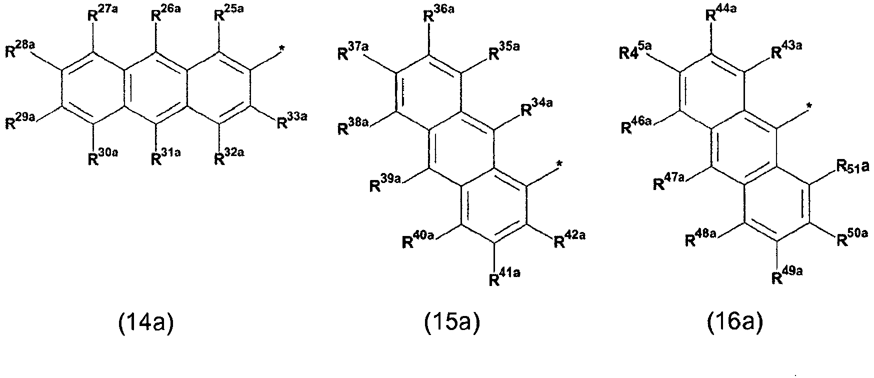 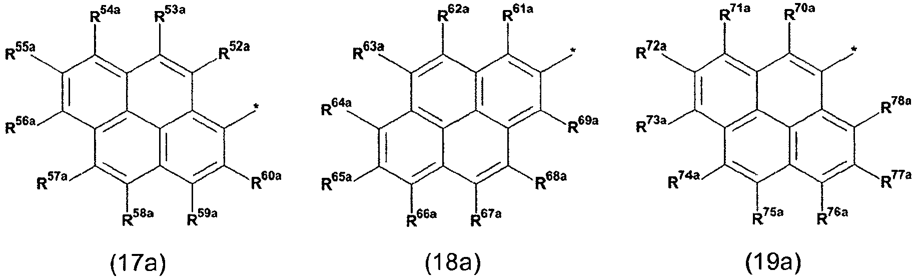 und wobei bei der Verbindung mit der Formel (3) die Reste Q² und Q^{2a} oder bei der Verbindung mit der Formel (101) die Reste Q⁴ und Q^{4a} jeweils unabhängig voneinander ein aromatischer Rest der allgemeinen Formel (11b), (12b), (13b), (14b), (15b), (16b), (17b), (18b), oder (19b) bedeuten: 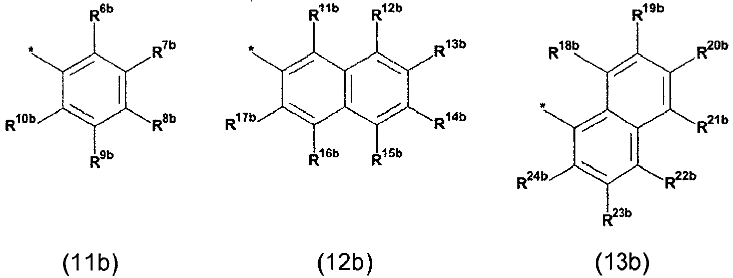 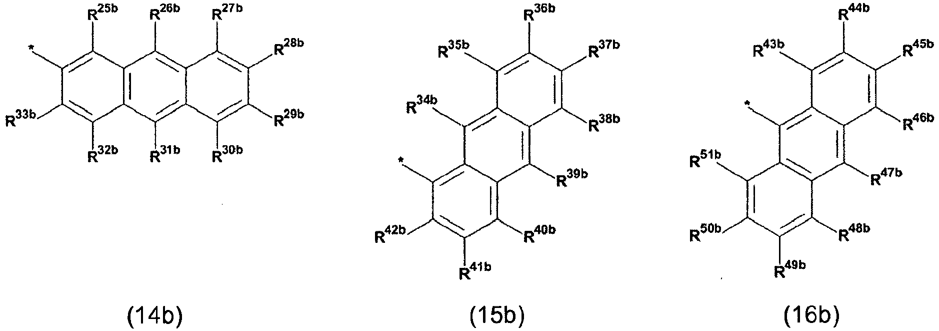 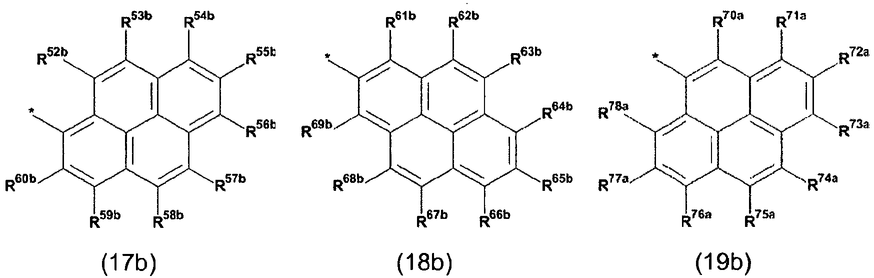 wobei jeweils mindestens 1, vorzugsweise 1 bis 9, weiter bevorzugt 1 bis 7, weiter bevorzugt 1 bis 5, weiter bevorzugt 1 bis 4, weiter bevorzugt 2 bis 3, Rest(e) R^{6a} bis R^{10a}, R^{11a} bis R^{17a}, R^{18a} bis R^{24a}, R^{25a} bis R^{33a}, R^{34a} bis R^{42a}, R^{43a} bis R^{51a}, R^{52a} bis R^{60a}, R^{61a} bis R^{69a}, R^{70a} bis R^{78a}, R^{6b} bis R^{10b}, R^{11b} bis R^{17b}, R^{18b} bis R^{24b}, R^{25b} bis R^{33b}, R^{34b} bis R^{42b}, R^{43b} bis R^{51b}, R^{52b} bis R^{60b}, R^{61b} bis R^{69b}, oder R^{70b} bis R^{78b} jeweils unabhängig voneinander ein organischer Rest W1 oder ein organischer Rest W1a oder ein organischer Rest W1b oder ein organischer Rest W1c ist, und
wobei die Reste R^{6a} bis R^{10a}, R^{11a} bis R^{17a}, R^{18a} bis R^{24a}, R^{25a} bis R^{33a}, R^{34a} bis R^{42a}, R^{43a} bis R^{51a}, R^{52a} bis R^{60a}, R^{61a} bis R^{69a}, R^{70a} bis R^{78a}, R^{6b} bis R^{10b}, R^{11b} bis R^{17b}, R^{18b} bis R^{24b}, R^{25b} bis R^{33b}, R^{34b} bis R^{42b}, R^{43b} bis R^{51b}, R^{52b} bis R^{60b}, R^{61b} bis R^{69b}, oder R^{70b} bis R^{78b}, die nicht ein organischer Rest W1 oder ein organischer Rest W1a oder ein organischer Rest W1b oder ein organischer Rest W1c sind, jeweils unabhängig voneinander gleich oder verschieden sind und Wasserstoff, Halogen, Nitro, Carboxylat, Aldehyd mit 1 bis 8 C-Atomen, Keton mit 2 bis 8 C-Atomen, O-Alkyl mit 1 bis 12 C-Atomen, O-Alkenyl mit 2 bis 12 C-Atomen, O-Aryl mit 5 bis 20 C-Atomen, Ether mit 2 bis 12 C-Atomen, , Carbonsäureester mit 1 bis 12 C-Atomen, Carbonsäureamid mit 1 bis 12 C-Atomen, wobei vorzugsweise jeweils das C-Atom der Carboxamidgruppe an einen der Reste Q¹ bis Q^{4a} gebunden ist, Alkyl mit 1 bis 12 C-Atomen, Alkenyl mit 2 bis 12 C-Atomen, Cycloalkyl mit 3 bis 12 C-Atomen, Cycloalkenyl mit 3 bis 12 C-Atomen, Alkylaryl mit 1 bis 12 C-Atomen, Aryl mit 5 bis 20 C-Atomen oder Heteroaryl, das kein Stickstoffatom enthält, mit 4 bis 20 C-Atomen, vorzugsweise Wasserstoff, Halogen, Nitro, Carboxylat, Aldehyd mit 1 bis 8 C-Atomen, Keton mit 2 bis 8 C-Atomen, O-Alkyl mit 1 bis 12 C-Atomen, O-Alkenyl mit 2 bis 12 C-Atomen, O-Aryl mit 5 bis 20 C-Atomen, Ether mit 2 bis 12 C-Atomen, Carbonsäureester mit 1 bis 12 C-Atomen, Alkyl mit 1 bis 12 C-Atomen, Alkenyl mit 2 bis 12 C-Atomen, Cycloalkyl mit 3 bis 12 C-Atomen, Cycloalkenyl mit 3 bis 12 C-Atomen, Alkylaryl mit 1 bis 12 C-Atomen, Aryl mit 5 bis 20 C-Atomen oder Heteroaryl, das kein Stickstoffatom enthält, mit 4 bis 20 C-Atomen, bedeuten.

Weiter bevorzugt sind bei der Verbindung mit der Formel (3) die Reste Q¹ und Q^{1a} oder bei der Verbindung mit der Formel (101) die Reste Q³ und Q^{3a} jeweils unabhängig voneinander ein aromatischer Rest der allgemeinen Formel (11a), (12a) oder (13a): 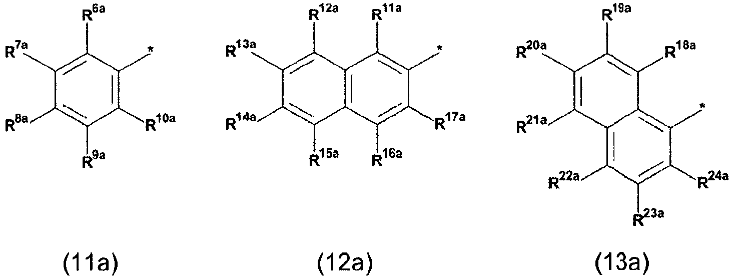 und wobei bei der Verbindung mit der Formel (3) die Reste Q² und Q^{2a} oder bei der Verbindung mit der Formel (101) die Reste Q⁴ und Q^{4a} jeweils unabhängig voneinander ein aromatischer Rest der allgemeinen Formel (11b), (12b) oder (13b): 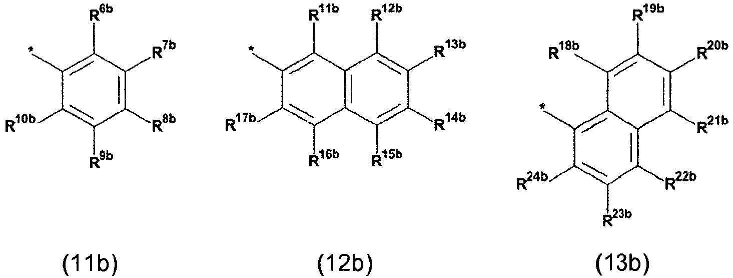 wobei jeweils mindestens 1, vorzugsweise 1 bis 9, weiter bevorzugt 1 bis 7, weiter bevorzugt 1 bis 5, weiter bevorzugt 1 bis 4, weiter bevorzugt 2 bis 3, Rest(e) R^{6a} bis R^{10a}, R^{11a} bis R^{17a}, R^{18a} bis R^{24a}, R^{6b} bis R^{10b}, R^{11b} bis R^{17b} oder R^{18b} bis R^{24b} jeweils unabhängig voneinander ein organischer Rest W1 oder ein organischer Rest W1a oder ein organischer Rest W1b oder ein organischer Rest W1c ist, wobei
die Reste R^{10a}, R^{11a} bis R^{17a}, R^{18a} bis R^{24a}, R^{6b} bis R^{10b}, R^{11b} bis R^{17b} und R^{18b} bis R^{24b}, die nicht ein organischer Rest W1 oder ein organischer Rest W1a oder ein organischer Rest W1b oder ein organischer Rest W1c sind, jeweils unabhängig voneinander gleich oder verschieden sind und Wasserstoff, Halogen, Nitro, Carboxylat, Aldehyd mit 1 bis 8 C-Atomen, Keton mit 2 bis 8 C-Atomen, O-Alkyl mit 1 bis 12 C-Atomen, O-Alkenyl mit 2 bis 12 C-Atomen, O-Aryl mit 5 bis 20 C-Atomen, Ether mit 2 bis 12 C-Atomen, Carbonsäureester mit 1 bis 12 C-Atomen, Carbonsäureamid mit 1 bis 12 C-Atomen, wobei vorzugsweise jeweils das C-Atom der Carboxamidgruppe an einen der Reste Q¹ bis Q^{4a} gebunden ist, Alkyl mit 1 bis 12 C-Atomen, Alkenyl mit 2 bis 12 C-Atomen, Cycloalkyl mit 3 bis 12 C-Atomen, Cycloalkenyl mit 3 bis 12 C-Atomen, Alkylaryl mit 1 bis 12 C-Atomen, Aryl mit 5 bis 20 C-Atomen oder Heteroaryl, das kein Stickstoffatom enthält, mit 4 bis 20 C-Atomen, vorzugsweise Wasserstoff, Halogen, Nitro, Carboxylat, Aldehyd mit 1 bis 8 C-Atomen, Keton mit 2 bis 8 C-Atomen, O-Alkyl mit 1 bis 12 C-Atomen, O-Alkenyl mit 2 bis 12 C-Atomen, O-Aryl mit 5 bis 20 C-Atomen, Ether mit 2 bis 12 C-Atomen, Carbonsäureester mit 1 bis 12 C-Atomen, Alkyl mit 1 bis 12 C-Atomen, Alkenyl mit 2 bis 12 C-Atomen, Cycloalkyl mit 3 bis 12 C-Atomen, Cycloalkenyl mit 3 bis 12 C-Atomen, Alkylaryl mit 1 bis 12 C-Atomen, Aryl mit 5 bis 20 C-Atomen oder Heteroaryl, das kein Stickstoffatom enthält, mit 4 bis 20 C-Atomen, bedeuten.

Bei einer bevorzugten Ausführungsform der Verbindung mit der Formel (100), der Verbindung mit der Formel (101) und/oder der Verbindung mit der Formel (103) ist der organische Rest X jeweils unabhängig voneinander ein Rest der Formel (20a), (20b), (21), (22a), (22b), (23a), (23b), oder (24): 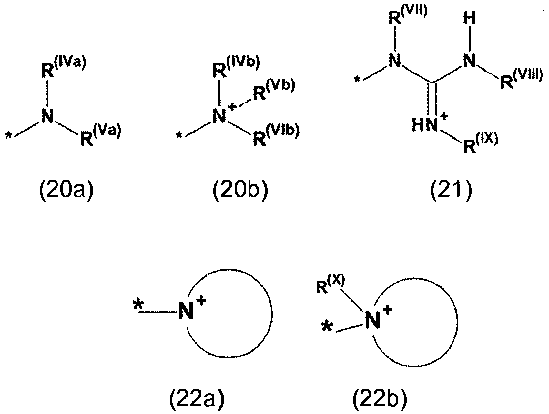 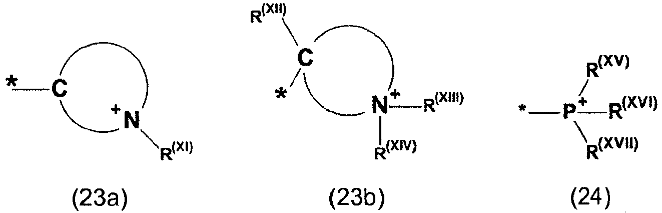 bedeutet,
wobei jeder der Reste R^{(IVa)}, R^{(Va)}, R^{(IVb)}, R^{(Vb)}, R^{(VIb)}, R^{(VII)} , R^{(VIII)}, R^{(IX)}, R^{(X)}, R^{(XI)}, R^{(XII)}, R^{(XIII)}, und R^{(XIV)}, jeweils unabhängig voneinander Wasserstoff, ein Arylrest mit 5 bis 12 C-Atomen, ein Alkylrest, der geradkettig oder verzweigt sein kann, mit 1 bis 8 C-Atomen, oder ein Etherrest, der geradkettig oder verzweigt sein kann, mit 1 bis 8 C-Atomen bedeutet, und
wobei jeder der Reste R^{(XV)}, R^{(XVI)}, und R^{(XVII)} jeweils unabhängig voneinander ein Arylrest mit 5 bis 12 C-Atomen, ein Alkylrest, der geradkettig oder verzweigt sein kann, mit 1 bis 8 C-Atomen, oder ein Etherrest, der geradkettig oder verzweigt sein kann, mit 1 bis 8 C-Atomen bedeutet, und
wobei der Rest mit der Formel (22a) und der Rest mit der Formel (23a): 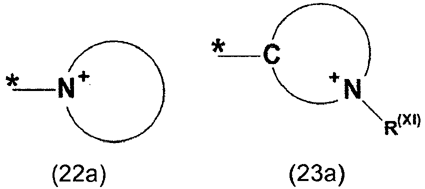 einen substituierten oder unsubstituierten heterocyclischen Rest mit 5 bis 7 Ringatomen, die mindestens 1 Kohlenstoffatom und mindestens 1 Stickstoffatom sowie optional 1 oder 2 Sauerstoffatome umfassen, wobei 1 Stickstoffatom eine Doppelbindung ausbildet, darstellt, und
wobei der Rest mit der Formel (22b) und der Rest mit der Formel (23b): 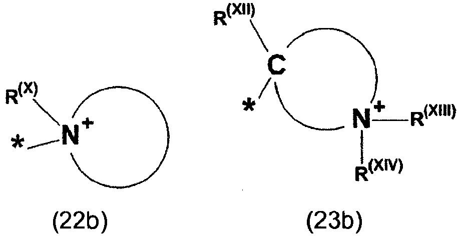 einen substituierten oder unsubstituierten heterocyclischen Rest mit 5 bis 7 Ringatomen, die mindestens 1 Kohlenstoffatom und mindestens 1 Stickstoffatom sowie optional 1 oder 2 Sauerstoffatome umfassen, wobei 1 Stickstoffatom eine Einfachbindung ausbildet, darstellt.

Bei einer bevorzugten Ausführungsform der Verbindung mit der Formel (3) ist der organische Rest X^{c} jeweils unabhängig voneinander ein Rest der Formel (20a), (20b), (21), (22a), (22b), (23a), (23b), oder (24): 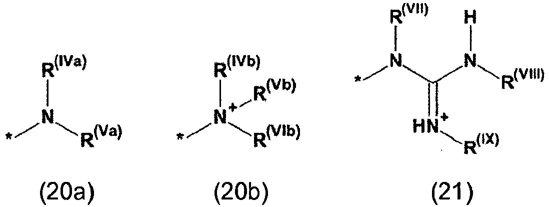 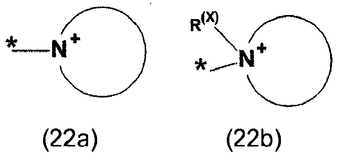 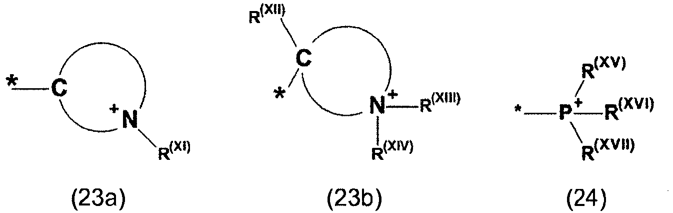 bedeutet,
wobei jeder der Reste R^{(IVa)}, R^{(Va)}, R^{(IVb)}, R^{(Vb)}, R^{(VIb)}, R^{(VII)}, R^{(VIII)}, R^{(IX)}, R^{(X)}, R^{(XI)}, R^{(XII)}, R^{(XIII)}, und R^{(XIV)}, jeweils unabhängig voneinander Wasserstoff, ein Arylrest mit 5 bis 12 C-Atomen, ein Alkylrest, der geradkettig oder verzweigt sein kann, mit 1 bis 8 C-Atomen, oder ein Etherrest, der geradkettig oder verzweigt sein kann, mit 1 bis 8 C-Atomen bedeutet, und
wobei jeder der Reste R^{(XV)}, R^{(XVI)}, und R^{(XVII)} jeweils unabhängig voneinander ein Arylrest mit 5 bis 12 C-Atomen, ein Alkylrest, der geradkettig oder verzweigt sein kann, mit 1 bis 8 C-Atomen, oder ein Etherrest, der geradkettig oder verzweigt sein kann, mit 1 bis 8 C-Atomen bedeutet, und
wobei der Rest mit der Formel (22a) und der Rest mit der Formel (23a): 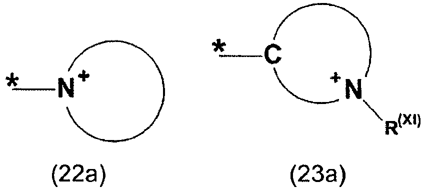 einen substituierten oder unsubstituierten heterocyclischen Rest mit 5 bis 7 Ringatomen, die mindestens 1 Kohlenstoffatom und mindestens 1 Stickstoffatom sowie optional 1 oder 2 Sauerstoffatome umfassen, wobei 1 Stickstoffatom eine Doppelbindung ausbildet, darstellt, und
wobei der Rest mit der Formel (22b) und der Rest mit der Formel (23b): 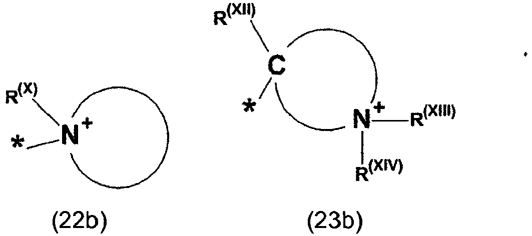 einen substituierten oder unsubstituierten heterocyclischen Rest mit 5 bis 7 Ringatomen, die mindestens 1 Kohlenstoffatom und mindestens 1 Stickstoffatom sowie optional 1 oder 2 Sauerstoffatome umfassen, wobei 1 Stickstoffatom eine Einfachbindung ausbildet, darstellt.

Weiter bevorzugt wird jeweils der Rest der Formel (23a) aus der Gruppe, die aus Resten der Formeln (25a), (25b) und (25c): 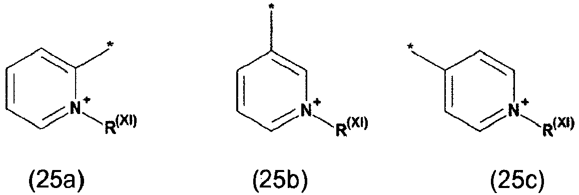 besteht, ausgewählt, wobei R^{(XI)} jeweils ein Arylrest mit 5 bis 20 C-Atomen, beispielsweise Phenyl oder Benzyl, ein Alkylrest, der geradkettig oder verzweigt sein kann, mit 1 bis 20 C-Atomen, beispielsweise Methyl, Ethyl, n-Propyl i-Propyl n-Butyl oder t-Butyl, ein Alkenylrest, der geradkettig oder verzweigt sein kann, mit 2 bis 20 C-Atomen, ein Hydroxyalkylrest, der geradkettig oder verzweigt sein kann, mit 1 bis 20 C-Atomen, beispielsweise Hydroxymethyl, 2-Hydroxyethyl, 3-Hydroxypropyl oder 1-Hydroxy-1-methyl-ethyl, oder ein Etherrest, der geradkettig oder verzweigt sein kann, mit 2 bis 20 C-Atomen, beispielsweise Methoxymethyl, Methoxyethyl, Methoxypropyl, Ethoxymethyl, Ethoxyethyl, Ethoxypropyl, Propoxymethyl, Propoxyethyl oder Propoxypropyl, sein kann.

Bei einer weiteren bevorzugten Ausführungsform wird jeweils der Rest der Formel (22a) aus der Gruppe, die aus Resten der Formeln (26): 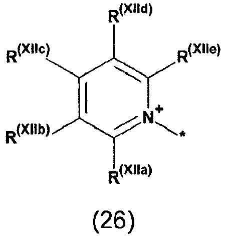 besteht, ausgewählt, wobei die Reste R^{(XIIa)} bis R^{(XIIe)}jeweils unabhängig von einander Wasserstoff, ein Arylrest mit 5 bis 20 C-Atomen, beispielsweise Phenyl oder Benzyl, ein Alkylrest, der geradkettig oder verzweigt sein kann, mit 1 bis 20 C-Atomen, beispielsweise Methyl, Ethyl, n-Propyl, i-Propyl, n-Butyl oder t-Butyl, ein Alkenylrest, der geradkettig oder verzweigt sein kann, mit 2 bis 20 C-Atomen, ein Hydroxyalkylrest, der geradkettig oder verzweigt sein kann, mit 1 bis 20 C-Atomen, beispielsweise Hydroxymethyl, 2-Hydroxyethyl, 3-Hydroxypropyl oder 1-Hydroxy-1-methyl-ethyl, oder ein Etherrest, der geradkettig oder verzweigt sein kann, mit 2 bis 20 C-Atomen, beispielsweise Methoxymethyl, Methoxyethyl, Methoxypropyl, Ethoxymethyl, Ethoxyethyl, Ethoxypropyl, Propoxymethyl, Propoxyethyl oder Propoxypropyl, sein kann. Vorzugsweise sind die Reste R^{(XIIa)} bis R^{(XIIe)}jeweils unabhängig von einander Wasserstoff, Methyl, Ethyl, Prop-1-yl, But-1-yl, Pent-1-yl, Hex-1-yl, Hept-1-oder Oct-1-yl.

Bei einer weiteren bevorzugten Ausführungsform ist der Rest der Formel (21 b) 1-Methylpyrrolidin-1-ium-1-yl, 1-Methylpiperazin-1-ium-1-yl oder 4-Methylmorpholin-4-ium-4-yl.

Bei einer weiteren bevorzugten Ausführungsform der Verbindung mit der Formel (100), der Verbindung mit der Formel (101) und/oder der Verbindung mit der Formel (103) ist der organische Rest X jeweils unabhängig voneinander ein Rest der Formel (20c), (20d), (21), oder (24), vorzugsweise ein Rest der Formel (20c), (20d), oder (21), weiter bevorzugt ein Rest der Formel (24), bedeutet: 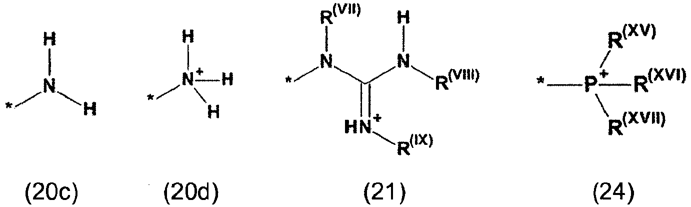 wobei jeder der Reste R^{(VII)}, R^{(VIII)}, und R^{(IX)} jeweils unabhängig voneinander Wasserstoff, ein Arylrest mit 5 bis 12 C-Atomen, ein Alkylarylrest mit 5 bis 12 C-Atomen, ein Alkylrest, der geradkettig oder verzweigt sein kann, mit 1 bis 8 C-Atomen, oder ein Etherrest, der geradkettig oder verzweigt sein kann, mit 1 bis 8 C-Atomen, vorzugsweise Wasserstoff, bedeutet und wobei jeder der Reste R^{(XV)}, R^{(XVI)}, und R^{(XVII)} jeweils unabhängig voneinander ein Arylrest mit 5 bis 12 C-Atomen, ein Alkylarylrest mit 5 bis 12 C-Atomen, ein Alkylrest, der geradkettig oder verzweigt sein kann, mit 1 bis 8 C-Atomen, oder ein Etherrest, der geradkettig oder verzweigt sein kann, mit 1 bis 8 C-Atomen, vorzugsweise ein Arylrest mit 5 bis 12 C-Atomen, ein Alkylarylrest mit 5 bis 12 C-Atomen, oder ein Alkylrest, der geradkettig oder verzweigt sein kann, mit 1 bis 8 C-Atomen, bedeutet.

Bei einer weiteren bevorzugten Ausführungsform werden in der Verbindung mit der Formel (100), der Verbindung mit der Formel (101) und/oder der Verbindung mit der Formel (103) die Reste R^{(IVa)}, R^{(Va)}, R^{(IVb)}, R^{(Vb)}, R^{(VIb)}, R^{(VII)}, R^{(VIII)}, R^{(IX)}, R^{(X)}, R^{(XI)}, R^{(XII)}, R^{(XIII)}, R^{(XIV)}, R^{(XV)}, R^{(XVI)}, und R^{(XVII)} unabhängig voneinander aus der Gruppe, die aus Wasserstoff und Alkylgruppen der allgemeinen Formel -(CH₂)ₙ-CH₃, wobei n eine ganze Zahl von 0 bis 19, vorzugsweise von 1 bis 17, ist, besteht, ausgewählt.

Bei einer weiteren bevorzugten Ausführungsform werden in der Verbindung mit der Formel (1), und/oder der Verbindung mit der Formel (2), und/oder der Verbindung mit der Formel (3), und/oder der Verbindung mit der Formel (3a), und/oder der Verbindung mit der Formel (100), und/oder der Verbindung mit der Formel (101), und/oder der Verbindung mit der Formel (102) die Reste R^{(IVa)}, R^{(Va)}, R^{(IVb)}, R^{(Vb)}, R^{(VIb)}, R^{(VII)}, R^{(VIII)}, R^{(IX)}, R^{(X)}, R^{(XI)}, R^{(XII)}, R^{(XIII)}, und R^{(XIV)} unabhängig voneinander ausgewählt aus der Gruppe, die aus Wasserstoff, Methyl, Ethyl, Prop-1-yl, Prop-2-yl, But-1-yl, But-2-yl, 2-Methylprop-1-yl, 2-Methyl-prop-2-yl, Pent-1-yl, Pent-2-yl, Pent-3-yl, 2-Methylbut-1-yl, 2-Methylbut-2-yl, 2-Methylbut-3-yl, 2-Methylbut-4-yl, 2,2-Dimethylprop-1-yl, Hex-1-yl, Hex-2-yl, Hex-3-yl, Hept-1-yl, Oct-1-yl, 2-Methylpent-1-yl, 2-Methylpent-2-yl, 2-Methylpent-3-yl, 2-Methylpent-4-yl, 2-Methylpent-5-yl, 3-Methylpent-1-yl, 3-Methylpent-2-yl, 3-Methylpent-3-yl, 2,2-Dimethylbut-1-yl, 2,2-dimethylbut-3-yl, 2,2-Dimethylbut-4-yl, 2,3-Dimethylbut-1-yl, 2,3-Dimethylbut-2-yl, Phenyl und Benzyl besteht, ausgewählt.

Bei einer besonders bevorzugten Ausführungsform des erfindungsgemäßen 1,7-Diaryl-1,6-heptadien-3,5-dion-Derivats der Formel (3) sowie dem erfindungsgemäß zu verwendenden 1,7-Diaryl-1,6-heptadien-3,5-dion-Derivats der Formel (100), der Formel (101), und/oder der Formel (102) werden werden die Reste R^{(XV)}, R^{(XVI)}, und R^{(XVII)} des Restes der Formel (24), 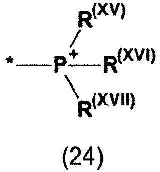 unabhängig voneinander aus der Gruppe, die aus Methyl, Ethyl, Prop-1-yl, Prop-2-yl, But-1-yl, But-2-yl, 2-Methylprop-1-yl, 2-Methyl-prop-2-yl, Pent-1-yl, Pent-2-yl, Pent-3-yl, 2-Methylbut-1-yl, 2-Methylbut-2-yl, 2-Methylbut-3-yl, 2-Methylbut-4-yl, 2,2-Dimethylprop-1-yl, Hex-1-yl, Hex-2-yl, Hex-3-yl, Hept-1-yl, Oct-1-yl, 2-Methylpent-1-yl, 2-Methylpent-2-yl, 2-Methylpent-3-yl, 2-Methylpent-4-yl, 2-Methylpent-5-yl, 3-Methylpent-1-yl, 3-Methylpent-2-yl, 3-Methylpent-3-yl, 2,2-Dimethylbut-1-yl, 2,2-dimethylbut-3-yl, 2,2-Dimethylbut-4-yl, 2,3-Dimethylbut-1-yl und 2,3-Dimethylbut-2-yl, Phenyl und Benzyl besteht, ausgewählt.

Bei einer weiteren bevorzugten Ausführungsform sind in der Verbindung mit der Formel (100), der Verbindung mit der Formel (101) und/oder der Verbindung mit der Formel (103) die Reste R^{(IVa)}, R^{(Va)}, R^{(IVb)}, R^{(Vb)}, R^{(VIb)}, R^{(VII)}, R^{(VIII)}, R^{(IX)}, R^{(X)}, R^{(XI)}, R^{(XII)}, R^{(XIII)}, R^{(XIV)}, R^{(XV)}, R^{(XVI)}, und R^{(XVII)} unabhängig voneinander Wasserstoff oder der Rest mit der Formel (10): 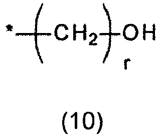 wobei r jeweils eine ganze Zahl von 1 bis 20, vorzugsweise von 1 bis 8, weiter bevorzugt von 1 bis 4, bedeutet, ausgewählt.

Bei einer weiteren bevorzugten Ausführungsform des erfindungsgemäß zu verwendenden 1,7-Diaryl-1,6-hepta-dien-3,5-dion-Derivates der Formel (100) und/oder der Formel (101) und/oder der Formel (102) bedeutet der organische Rest W2 und/oder W1 jeweils unabhängig voneinander ein organischer Rest der allgemeinen Formel (30a), (30b), (30c), (30d), (30e), (30f), (30g), (30h), (30i), (30k), (30m), (30n), (30p), (31a), (31b), (32), (33), (34), (35), (36), (37a) oder (37b): 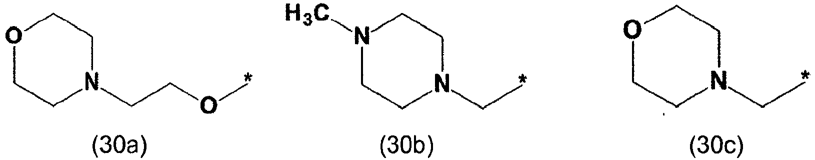 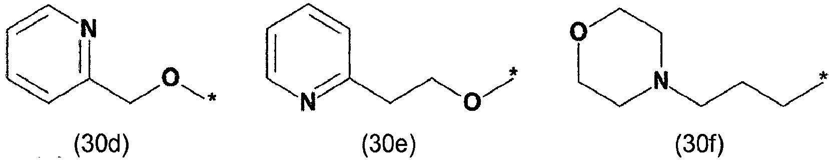 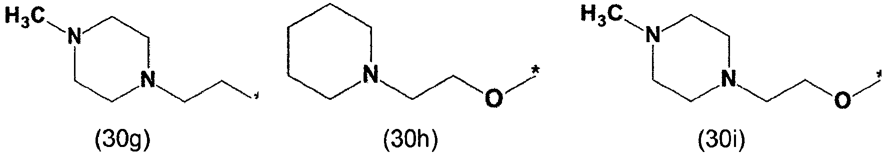 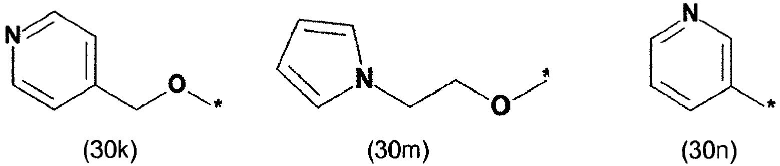 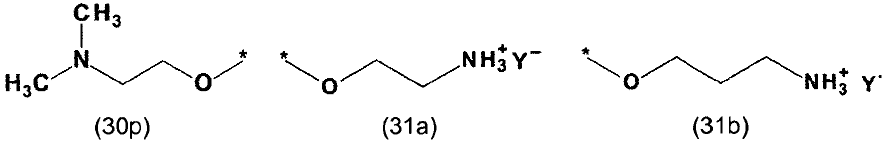 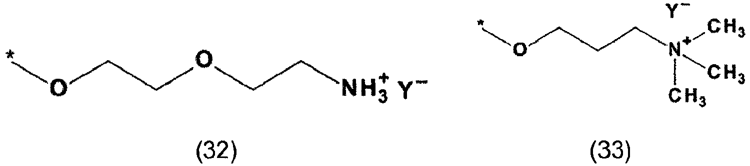 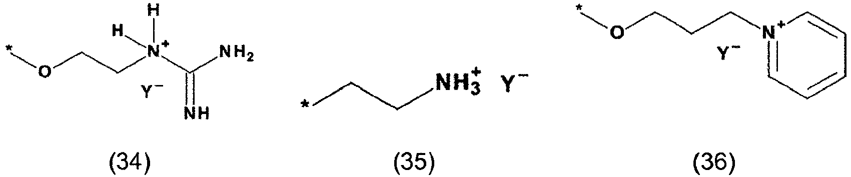 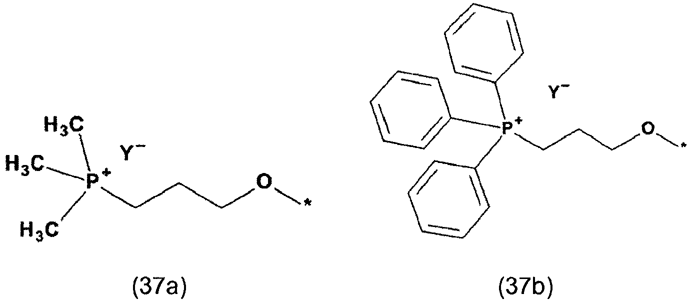 wobei Y⁻ ein Anion ist, das jeweils unabhängig voneinander Fluorid, Chlorid, Bromid, lodid, Sulfat, Hydrogensulfat, Phosphat, Hydrogenphosphat, Dihydrogenphosphat, Tosylat, Mesylat, oder wenigstens ein Carboxylation einer Carbonsäure mit 1 bis 15 Kohlenstoffatomen bedeutet. Vorzugsweise ist ein Carboxylation einer Carbonsäure mit 1 bis 15 Kohlenstoffatomen unabhängig voneinander Formiat, Acetat, n-Propionat, Lactat, Oxalat, Fumarat, Maleinat, Tartrat, Succinylat, Benzoat, Salicylat, oder Citrat.

Bei einer weiteren bevorzugten Ausführungsform des erfindungsgemäßen 1,7-Diaryl-1,6-hepta-dien-3,5-dion-Derivates der Formel (1) und/oder der Formel (2) und/oder der Formel (3) und/oder der Formel (3a) bedeutet der organische Rest W1a jeweils unabhängig voneinander ein organischer Rest der allgemeinen Formel (31a), (31b), (32) oder (34): 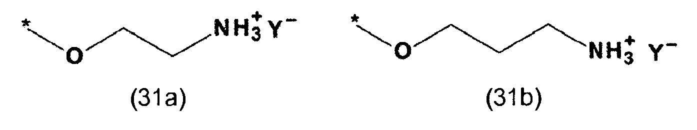 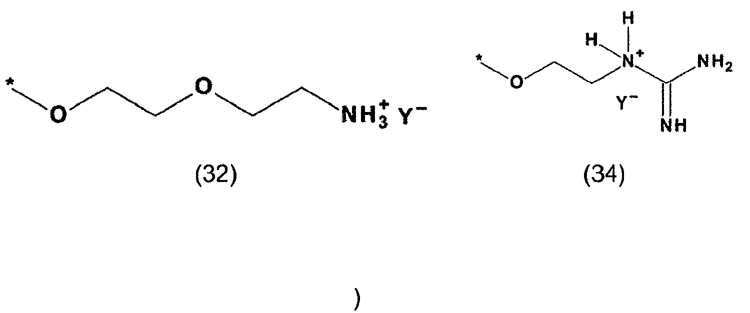 wobei Y⁻ ein Anion ist, das jeweils unabhängig voneinander Fluorid, Chlorid, Bromid, lodid, Sulfat, Hydrogensulfat, Phosphat, Hydrogenphosphat, Dihydrogenphosphat, Tosylat, Mesylat, oder wenigstens ein Carboxylation einer Carbonsäure mit 1 bis 15 Kohlenstoffatomen bedeutet. Vorzugsweise ist ein Carboxylation einer Carbonsäure mit 1 bis 15 Kohlenstoffatomen unabhängig voneinander Formiat, Acetat, n-Propionat, Lactat, Oxalat, Fumarat, Maleinat, Tartrat, Succinylat, Benzoat, Salicylat, oder Citrat.

Bei einer weiteren bevorzugten Ausführungsform des erfindungsgemäßen 1,7-Diaryl-1,6-hepta-dien-3,5-dion-Derivates der Formel (3) bedeutet der organische Rest W1c jeweils unabhängig voneinander ein organischer Rest der allgemeinen Formel (31a), (31b), (32), (34), (35), (37a) oder (37b): 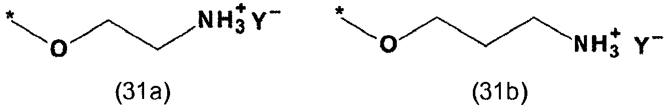 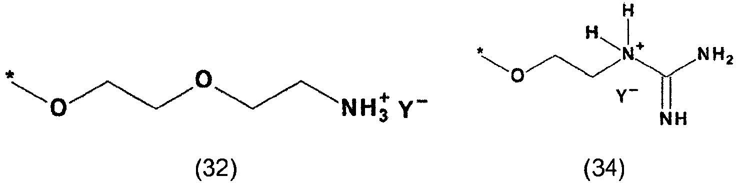 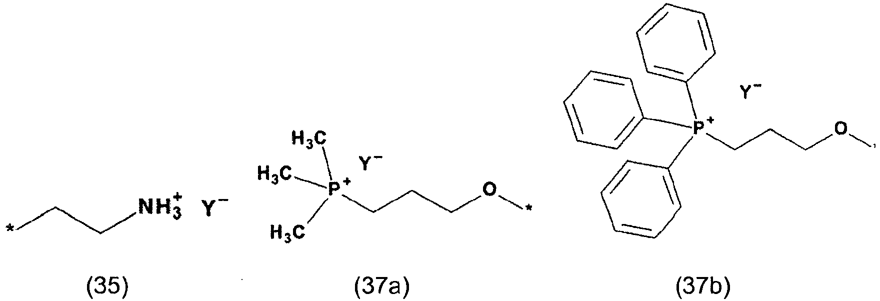 wobei Y⁻ ein Anion ist, das jeweils unabhängig voneinander Fluorid, Chlorid, Bromid, lodid, Sulfat, Hydrogensulfat, Phosphat, Hydrogenphosphat, Dihydrogenphosphat, Tosylat, Mesylat, oder wenigstens ein Carboxylation einer Carbonsäure mit 1 bis 15 Kohlenstoffatomen bedeutet. Vorzugsweise ist ein Carboxylation einer Carbonsäure mit 1 bis 15 Kohlenstoffatomen unabhängig voneinander Formiat, Acetat, n-Propionat, Lactat, Oxalat, Fumarat, Maleinat, Tartrat, Succinylat, Benzoat, Salicylat, oder Citrat.

Bei einer weiter bevorzugten Ausführungsform des erfindungsgemäß zu verwendenden 1,7-Diaryl-1,6-heptadien-3,5-dion-Derivates der Formel (100) ist die Verbindung wenigstens eine Verbindung der Formel (40) bis (67):

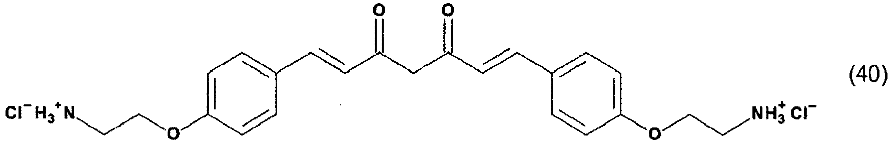

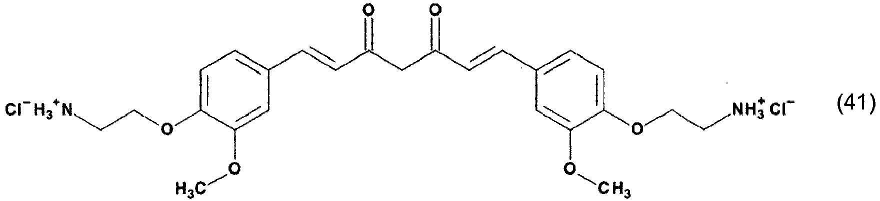

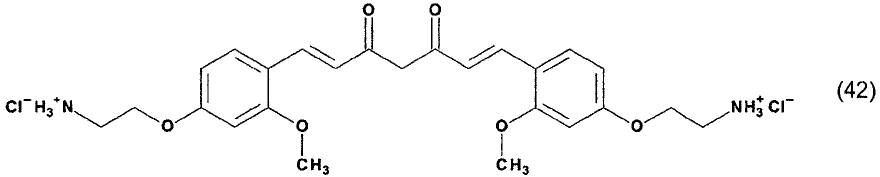

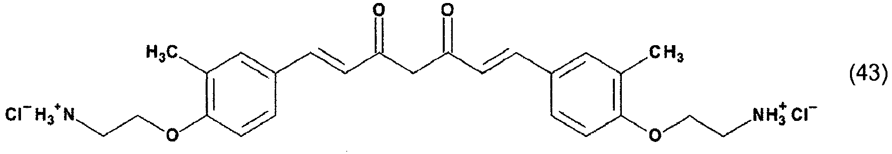

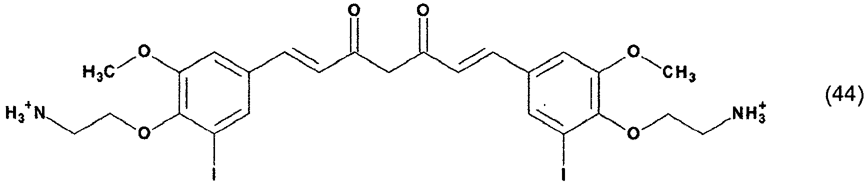

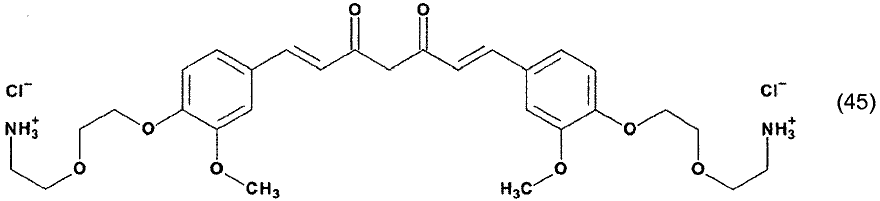

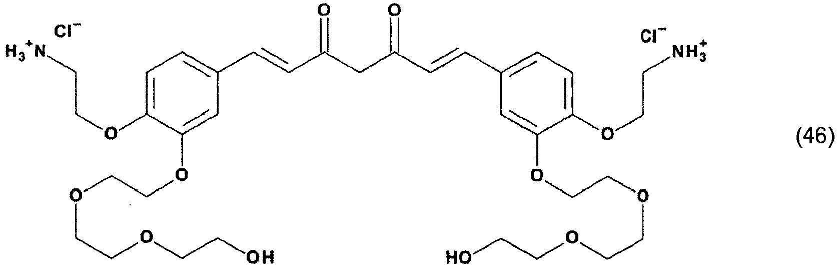

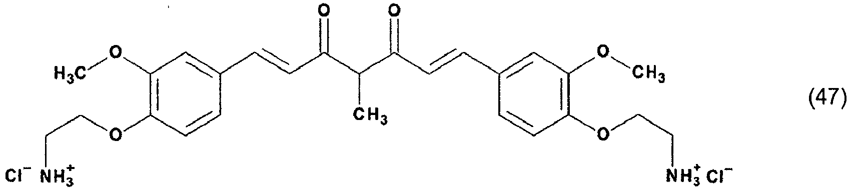

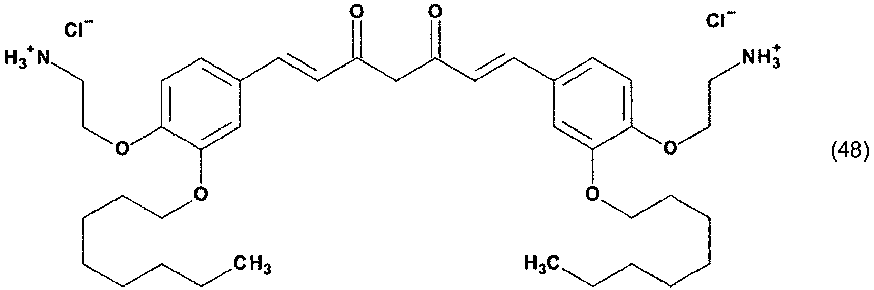

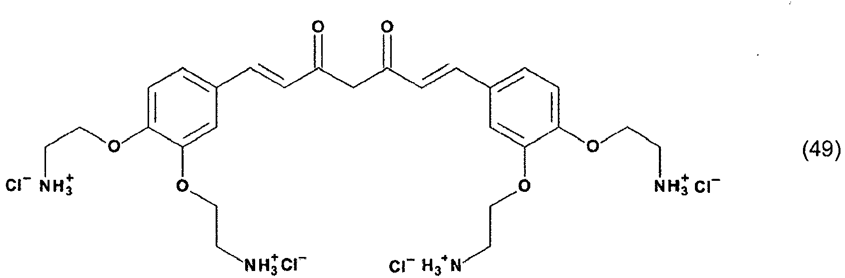

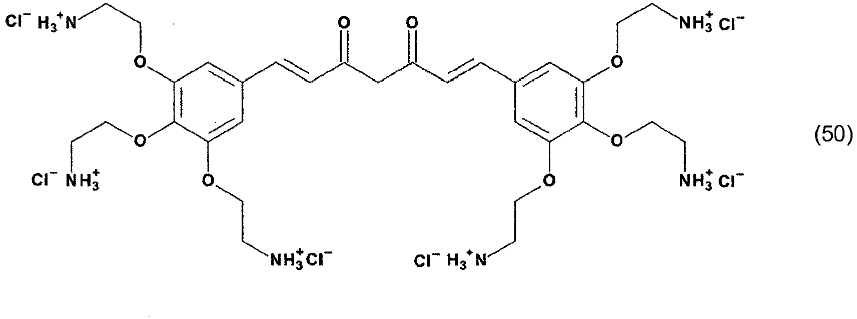

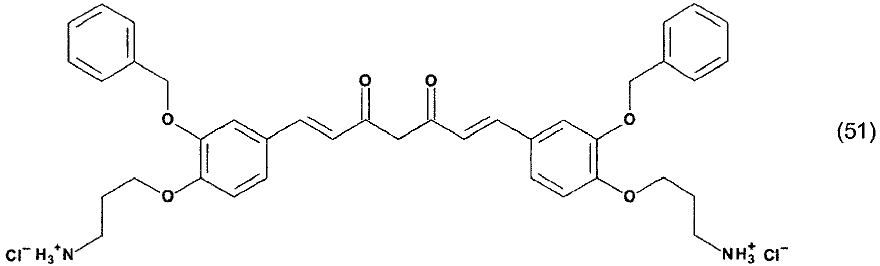

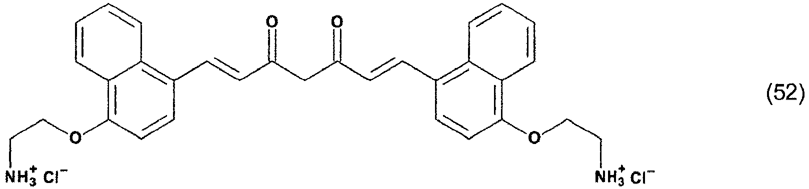

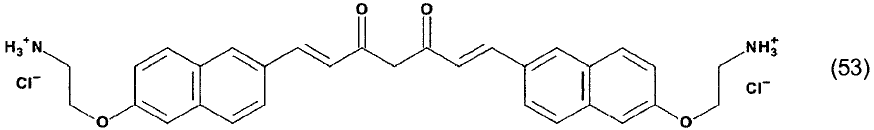

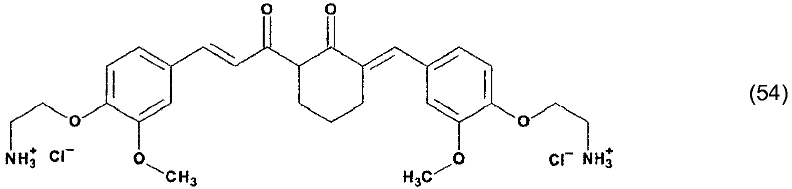

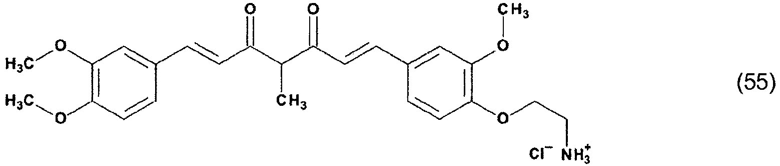

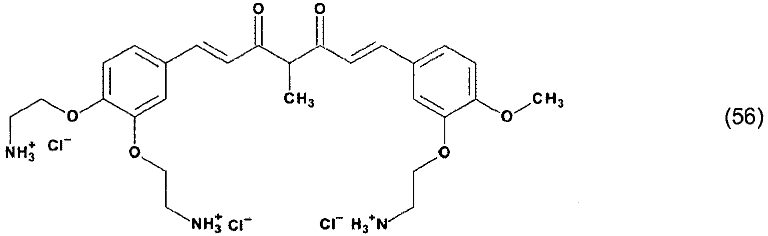

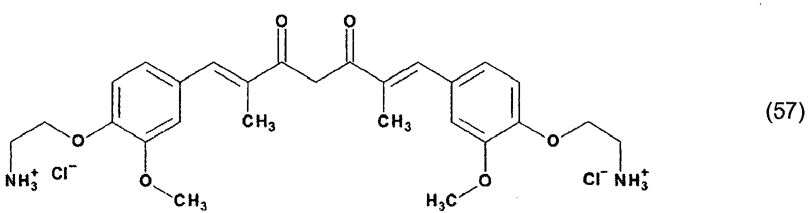

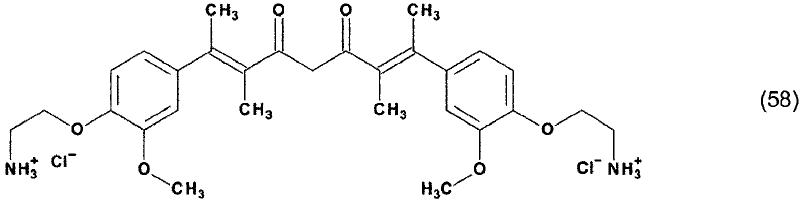

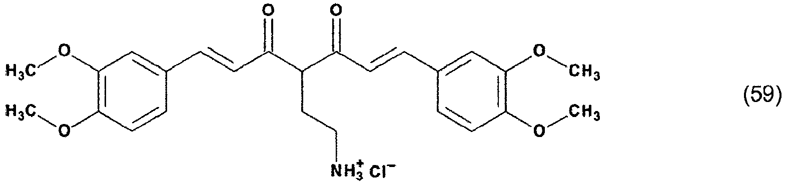

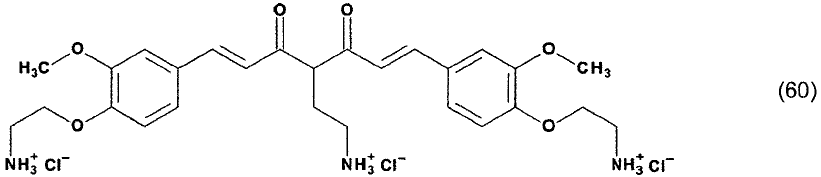

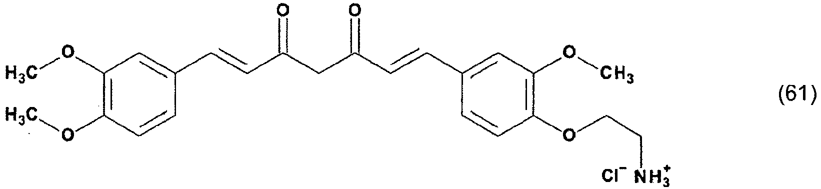

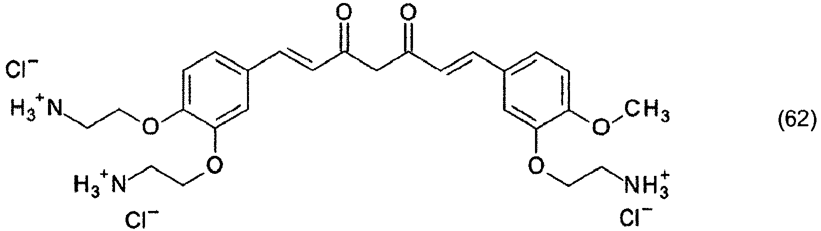

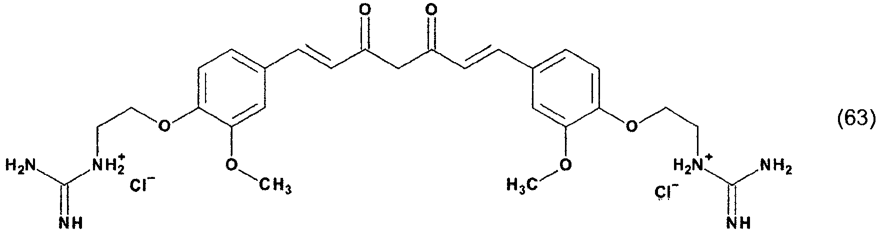

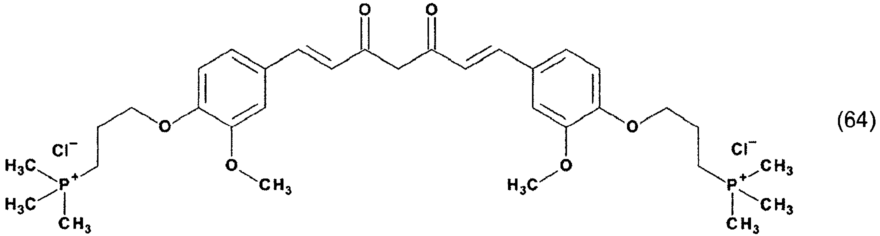

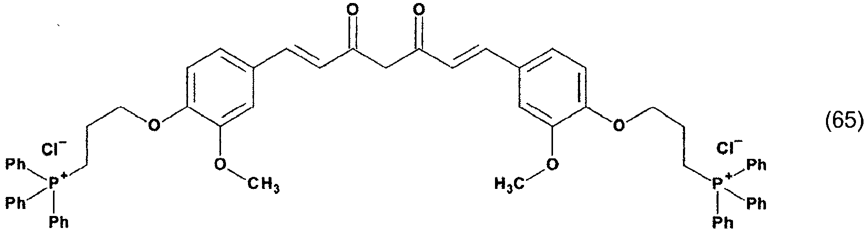

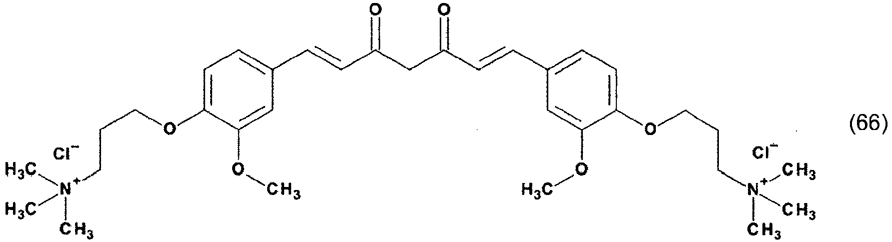

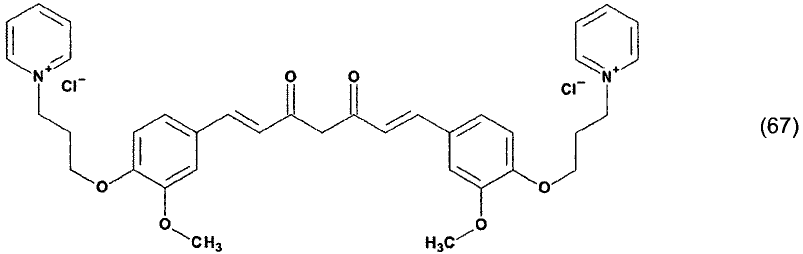

Bei einer weiter bevorzugten Ausführungsform des erfindungsgemäß zu verwendenden 1,7-Diaryl-1,6-heptadien-3,5-dion-Derivates der Formel (100) ist die Verbindung wenigstens eine Verbindung der Formel (40) bis (67) und (110) bis (229).

Bei einer weiter bevorzugten Ausführungsform des erfindungsgemäßen 1,7-Diaryl-1,6-heptadien-3,5-dion-Derivates der Formel (1) ist die Verbindung wenigstens eine Verbindung der Formel (40) bis (63), vorzugsweise wenigstens eine Verbindung der Formel (40) bis (62):

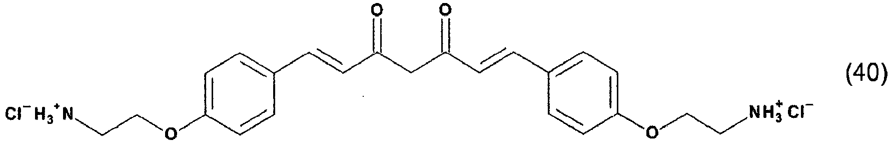

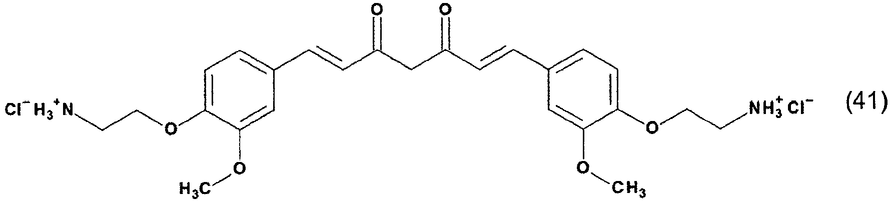

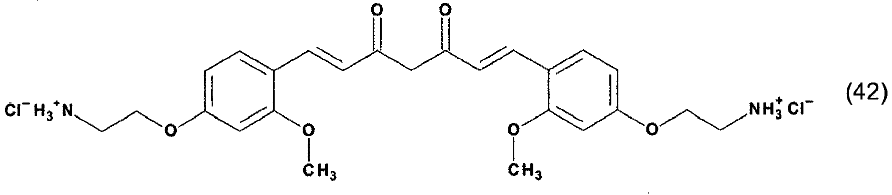

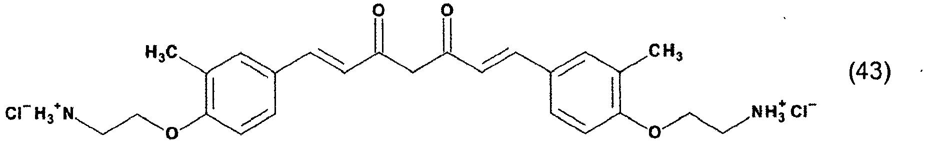

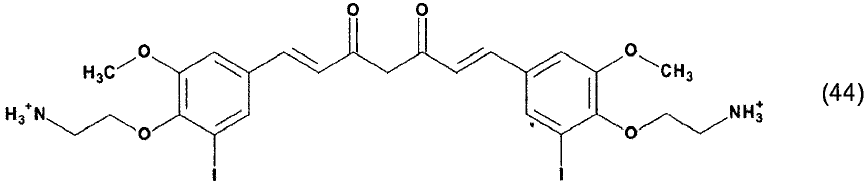

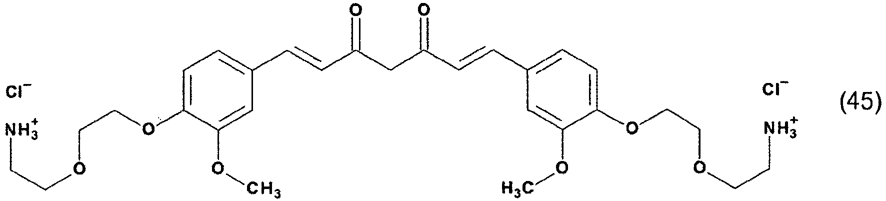

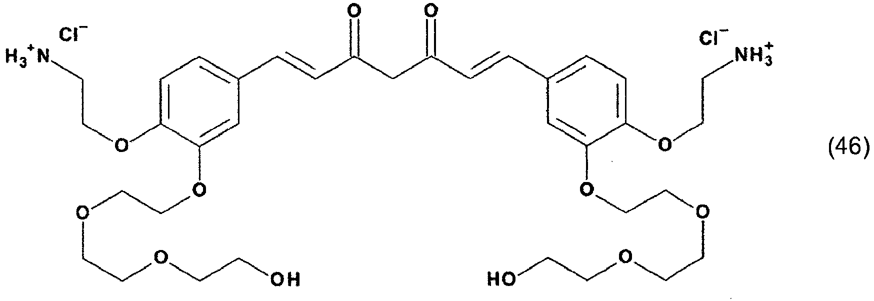

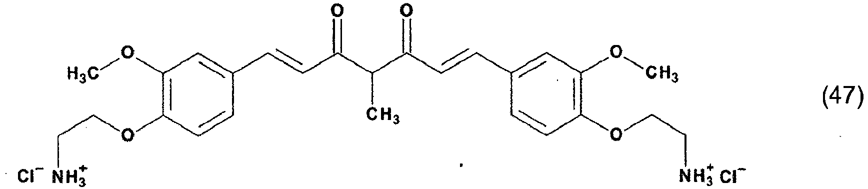

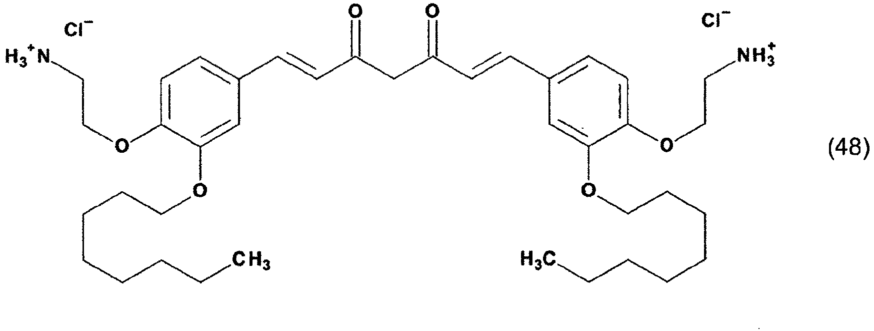

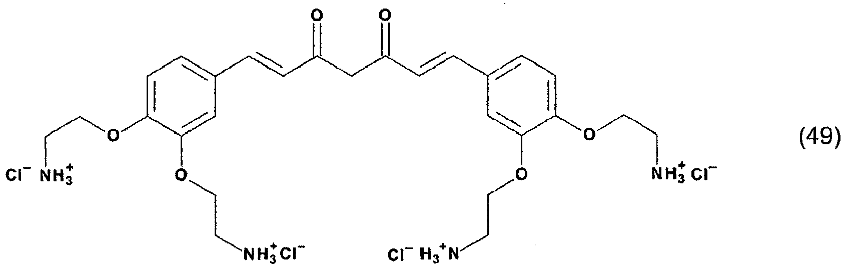

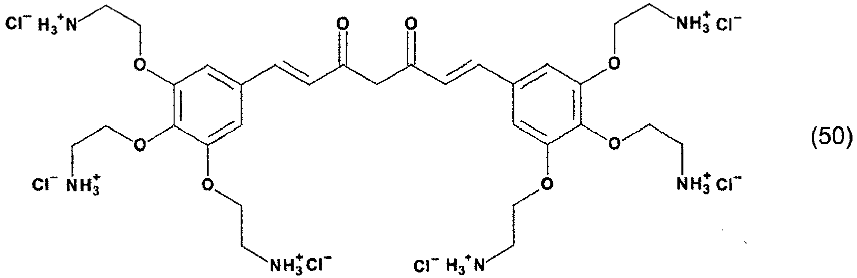

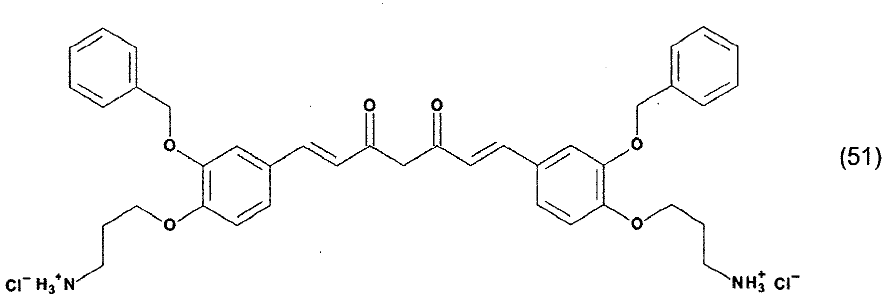

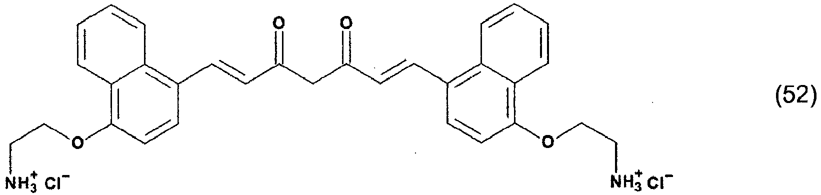

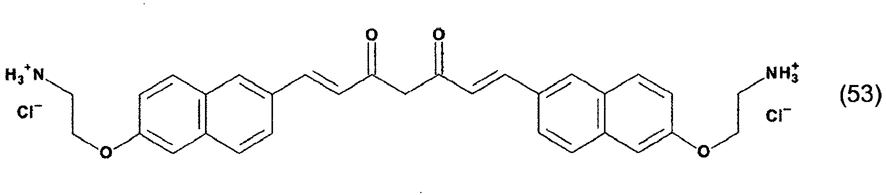

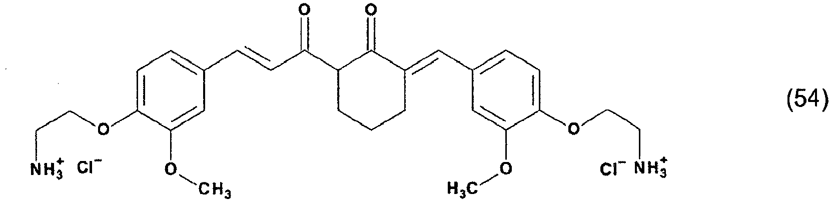

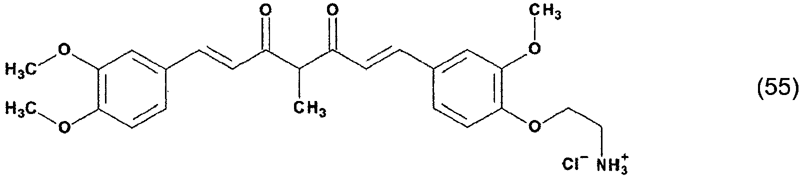

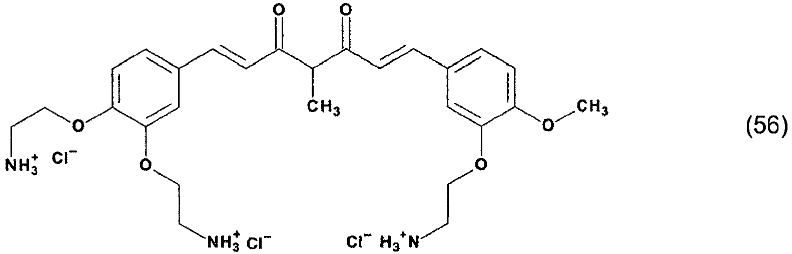

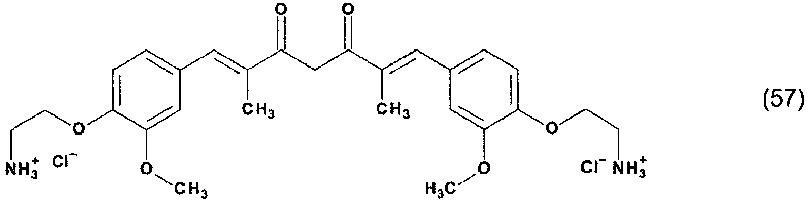

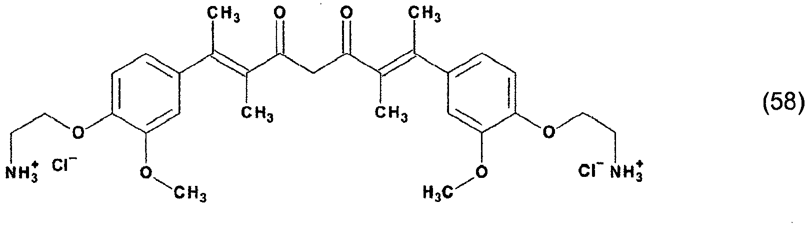

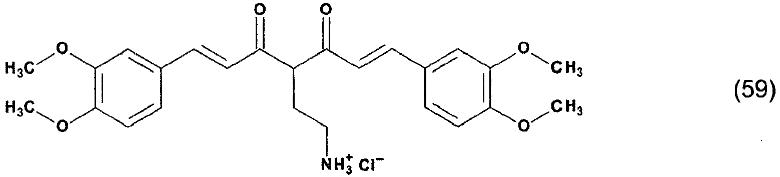

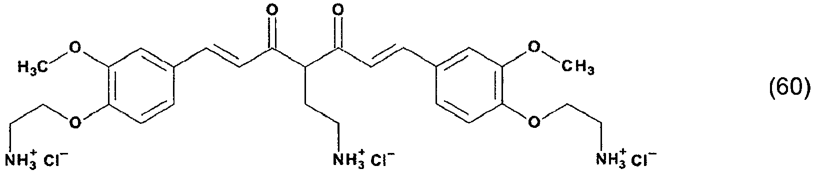

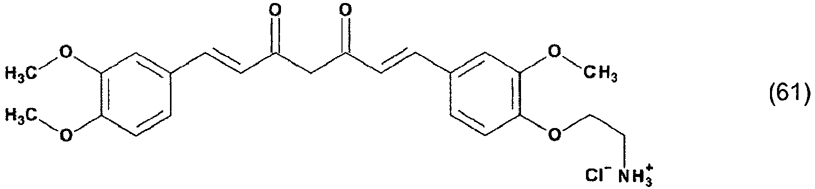

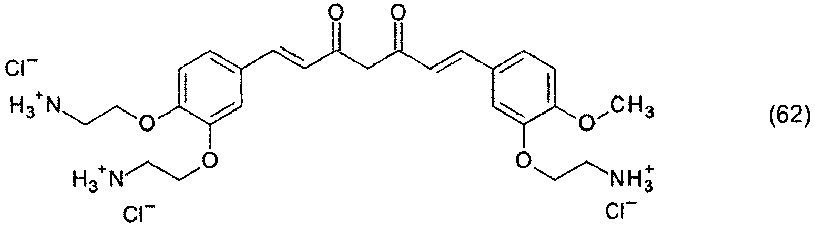

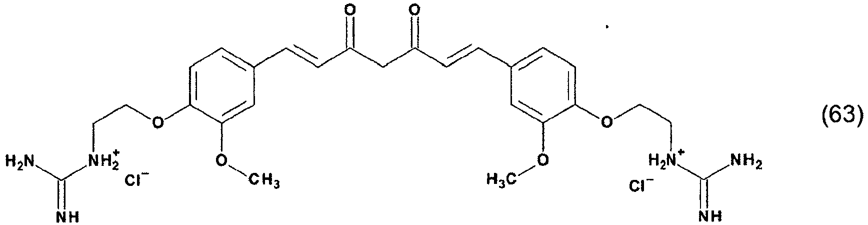

Bei einer weiter bevorzugten Ausführungsform des erfindungsgemäßen 1,7-Diaryl-1,6-heptadien-3,5-dion-Derivates der Formel (2) oder des erfindungsgemäß zu verwendenden 1,7-Diaryl-1,6-heptadien-3,5-dion-Derivates der Formel (102) ist die Verbindung wenigstens eine Verbindung der Formel (68), (69a) oder (69b):

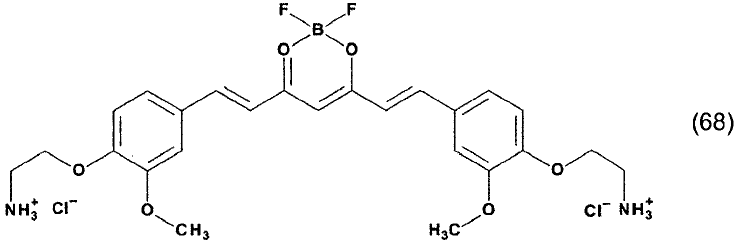

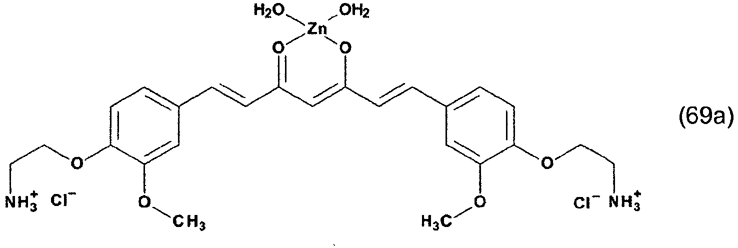

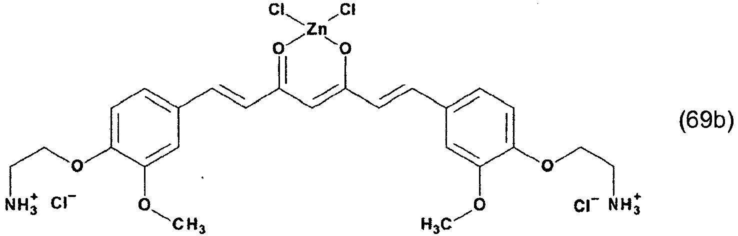

Bei einer weiter bevorzugten Ausführungsform des erfindungsgemäßen 1,7-Diaryl-1,6-heptadien-3,5-dion-Derivates der Formel (3) oder des erfindungsgemäß zu verwendenden 1,7-Diaryl-1,6-heptadien-3,5-dion-Derivates der Formel (101) ist die Verbindung wenigstens eine Verbindung der Formel (70):

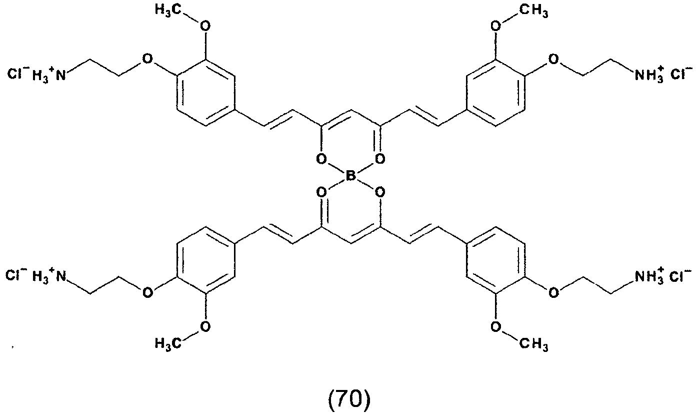

Beim erfindungsgemäßen 1,7-Diaryl-1,6-heptadien-3,5-dion-Derivates der Formel (1), und/oder der Formel (2) und/oder der Formel (3) und/oder der Formel (3a) sowie beim erfindungsgemäß zu verwendenden 1,7-Diaryl-1,6-heptadien-3,5-dion-Derivates der Formel (100), und/oder der Formel (101) und/oder der Formel (102) und/oder der Formel (3a) können vorgenannte Aldehydreste, Ketonreste, Carbonsäurereste, Carbonsäureamidreste, Cycloalkylreste, Cycloalkenylreste, Alkylreste und Akenylreste geradkettig oder verzweigt, vorzugsweise geradkettig, sein und sowohl unsubstituiert oder mit wenigstens einem Rest, der Halogen, vorzugsweise Chlor, Brom, lod oder Fluor, Nitro, Hydroxy, Alkyloxy, vorzugsweise Methoxy, Ethoxy, n-Propyloxy, i-Propyloxy, n-Butyloxy oder n-Pentyloxy, oder Alkanoyloxy, vorzugsweise Formyloxy, Acetoxy oder n-Propanoyloxy ist, substituiert sein.

Bei einer weiter bevorzugten Ausführungsform werden die vorgenannten Alkylreste jeweils unabhängig voneinander aus der Gruppe, die aus Methyl, Ethyl, n-Propyl, iso-Propyl, n-Butyl, iso-Butyl, tert-Butyl, n-Pentyl, 3-Methylbutyl, 2,2-Dimethylpropyl, n-Hexyl, n-Heptyl und n-Octyl besteht, ausgewählt.

Bei einer weiter bevorzugten Ausführungsform können vorgenannten Alkylreste nichtcyclische Polyolreste der allgemeinen Formel -CH₂(CH(OH))_{g}CH₂OH sein, wobei g eine ganze Zahl von 1 bis 10, vorzugsweise 1 bis 4, ist. Weiter bevorzugt werden die nichtcyclischen Polyolreste aus der Gruppe, die aus Arabityl, Ribityl, Xylityl, Erythrityl, Threityl, Lactityl, Mannityl und Sorbityl, weiter bevorzugt D-Ribityl und D-Arabityl, besteht, ausgewählt.

Bei einer weiter bevorzugten Ausführungsform können vorgenannte Cycloalkylreste und Cycloalkenylreste Sauerstoffatome als Ringatome aufweisen und sowohl unsubstituiert oder mit wenigstens einem Rest, der aus Hydroxyl, Alkyloxy, vorzugsweise Methoxy, Ethoxy, n-Propyloxy, i-Propyloxy, n-Butyloxy oder n-Pentyloxy, oder Alkanoyloxy, vorzugsweise Formyloxy, Acetoxy oder n-Propanoyloxy ist, substituiert sein.

Bei einer weiter bevorzugten Ausführungsform werden die vorgenannten Cycloalkylreste und Cycloalkenylreste, die Sauerstoffatome als Ringatome aufweisen, jeweils unabhängig voneinander aus der Gruppe, die aus Tetrahydrofuranyl, Tetrahydropyranyl, Dioxolanyl und Dioxanyl besteht, ausgewählt.

Bei einer weiter bevorzugten Ausführungsform weisen die vorgenannten Arylreste jeweils höchstens 4, weiter bevorzugt höchstens 3, weiter bevorzugt höchstens 2, anellierte Ringe auf. Noch weiter bevorzugt weisen die Arylreste jeweils 1 Ring auf.

Vorzugsweise werden die vorgenannten Arylreste jeweils unabhängig voneinander aus der Gruppe, die aus Phenyl, Benzyl, Naphthyl, Anthracenyl, Phenanthrenyl und Pyrenyl besteht, ausgewählt.

Bei einer bevorzugten Ausführungsform weisen die vorgenannten Alkenylreste jeweils unabhängig voneinander 2 bis 17 C-Atome, weiter bevorzugt 2 bis 13 C-Atome, weiter bevorzugt 2 bis 9 C-Atome, weiter bevorzugt 2 bis 5 C-Atome, auf. Bei einer weiter bevorzugten Ausführungsform werden die vorgenannten Alkenylreste jeweils unabhängig voneinander aus der Gruppe, die aus Ethenyl, n-Propenyl und n-Butenyl besteht, ausgewählt.

Bei einer weiteren bevorzugten Ausführungsform weisen die vorgenannten Aldehyde jeweils unabhängig voneinander 1 bis 17 C-Atome, weiter bevorzugt 1 bis 13 C-Atome, weiter bevorzugt 1 bis 9 C-Atome, weiter bevorzugt 1 bis 5 C-Atome, auf. Bei einer weiter bevorzugten Ausführungsform werden die vorgenannten Aldehyde jeweils unabhängig voneinander aus der Gruppe, die aus Methanal-1-yl (Formyl), Ethanal-1-yl (2-Oxoethyl), n-Propanal-1-yl (3-Oxopropyl) und n-Butanal-1-yl (4-Oxobutyl) besteht, ausgewählt.

Bei einer weiteren bevorzugten Ausführungsform weisen die vorgenannten Ketone jeweils unabhängig voneinander 2 bis 17 C-Atome, weiter bevorzugt 3 bis 14 C-Atome, weiter bevorzugt 3 bis 9 C-Atome, auf. Bei einer weiter bevorzugten Ausführungsform werden die vorgenannte Ketone jeweils unabhängig voneinander aus der Gruppe, die aus Dimethylketyl, Methyl-Ethyl-ketyl, Ethyl-Methyl-ketyl, Diethylketyl, Methyl-Propyl-ketyl, Ethyl-Propyl-ketyl, Propyl-Methyl-ketyl, Propyl-Ethyl-ketyl und Dipropyl-ketyl, das geradkettig oder verzweigt sein kann, besteht, ausgewählt.

Bei einer weiter bevorzugten Ausführungsform können vorgenannte Aldehydreste und/oder Ketonreste Monosaccharidreste, vorzugsweise Pentose- oder Ketosereste, sein.

Vorzugsweise haben geeignete Monosaccharidreste jeweils unabhängig voneinander 3 bis 7 Kohlenstoffatome, vorzugsweise 5 bis 6 Kohlenstoffatome, und weisen eine Carbonylgruppe, vorzugsweise Aldehydgruppe oder Ketogruppe, sowie mindestens eine Hydroxylgruppe auf und können offenkettig oder cyclisch, vorzugsweise als Furanose oder Pyranose, vorliegen.

Bevorzugt leiten sich geeignete Monosaccharidreste von Monosacchariden ab, die jeweils unabhängig voneinander aus der Gruppe, die aus D-Glycerinaldehyd, L-Glycerinaldehyd, D-Erythrose, L-Erythrose, D-Threose, L-Threose, D-Ribose, L-Ribose, D-Arabinose, L-Arabinose, D-Xylose, L-Xylose, D-Lyxose, L-Lyxose, D-Allose, L-Allose, D-Altrose, L-Altrose, D-Glucose, L-Glucose, D-Mannose, L-Mannose, D-Gulose, L-Gulose, D-Idose, L-Idose, D-Galactose, L-Galactose, D-Talose, L-Talose, Dihydroxyaceton, D-Erythrulose, L-Erythrulose, D-Ribulose, L-Ribulose, D-Xylulose, L-Xylulose, D-Psicose, L-Psicose, D-Fructose, L-Fructose, D-Sorbose, L-Sorbose, D-Tagatose und L-Tagatose besteht, ausgewählt. Weiter bevorzugt werden geeignete Monosaccharide aus der Gruppe, die aus D-Ribose, L-Ribose, D-Arabinose, L-Arabinose, D-Xylose, L-Xylose, D-Lyxose, L-Lyxose, D-Allose, L-Allose, D-Altrose, L-Altrose, D-Glucose, L-Glucose, D-Mannose, L-Mannose, D-Gulose, L-Gulose, D-Idose, L-Idose, D-Galactose, L-Galactose, D-Talose, L-Talose, D-Ribulose, L-Ribulose, D-Xylulose, L-Xylulose, D-Psicose, L-Psicose, D-Fructose, L-Fructose, D-Sorbose, L-Sorbose, D-Tagatose und L-Tagatose besteht, ausgewählt werden.
Bei einer weiteren bevorzugten Ausführungsform weisen die vorgenannten Carbonsäureester jeweils unabhängig voneinander 1 bis 17 C-Atome, weiter bevorzugt 1 bis 15 C-Atome, weiter bevorzugt 1 bis 12 C-Atome, auf, wobei vorzugsweise das C-Atom der Carboxylatgruppe an einem der Reste Q¹ bis Q^{4a} gebunden ist. Bei einer weiter bevorzugten Ausführungsform werden die vorgenannten Carbonsäureester jeweils unabhängig voneinander aus der Gruppe, die aus Ethylester, n-Propylester, i-Propylester, n-Butylester, sec.-Butylester, tert.-Butylester und Benzylester besteht, ausgewählt. Weiter bevorzugt werden vorgenannte Carbonsäureester aus der Gruppe, die aus Methoxycarboyl, 2-Methoxy-2-oxo-eth-1 -yl, 3-Methoxy-3-oxo-prop-1-yl, 4-Methoxy-4-oxo-but-1-yl, Ethoxycarboyl, 2-Ethoxy-2-oxo-eth-1-yl, 3-Ethoxy-3-oxo-prop-1-yl, 4-Ethoxy-4-oxo-but-1-yl, Propoxycarboyl, 2-Oxy-2-propoxy-eth-1-yl, 3-Oxo-3-propoxy-prop-1-yl, 4-Oxo-4-propoxy-but-1-yl, Isopropoxycarbonyl, 2-Isopropoxy-2-oxo-eth-1-yl, 3-Isopropoxy-3-oxo-prop-1-yl, 4-Isopropoxy-4-oxo-but-1-yl, Butoxycarbonyl, 2-Butoxy-2-oxo-eth-1-yl, 3-Butoxy-3-oxo-prop-1-yl, 4-Butoxy-4-oxo-but-1-yl, Isobutoxycarbonyl, 2-Isobutoxy-2-oxo-eth-1-yl, 3-Isobutoxy-3-oxo-prop-1-yl, 4-Isobutoxy-4-oxo-but-1-yl, tert-Butoxycarbonyl, 2-tert-Butoxy-2-oxo-eth-1-yl, 3-tert-Butoxy-3-oxo-prop-1-yl, 4-tert-Butoxy-4-oxo-but-1-yl, Phenoxycarbonyl, 2-Oxy-2-phenoxy-eth-1 -yl, 3-Oxo-3-phenoxy-prop-1-yl, 4-Oxo-4-phenoxy-but-1-yl, Benzyloxycarbonyl, 2-Benzyloxy-2-oxo-eth-1-yl, 3-Benzyloxy-3-oxo-prop-1-yl, und 4-Benzyloxy-4-oxo-but-1-yl besteht, ausgewählt.

Bei einer weiteren bevorzugten Ausführungsform weisen die vorgenannten Carbonsäureamide jeweils unabhängig voneinander 1 bis 17 C-Atome, weiter bevorzugt 1 bis 15 C-Atome, weiter bevorzugt 1 bis 12 C-Atome, auf, wobei vorzugsweise das C-Atom der Carboxamidgruppe jeweils an einen der Reste Q¹ bis Q^{4a} gebunden ist.

Die vorgenannten Carbonsäureamide sind vorzugsweise keine Carbamide.

Bei einer weiteren bevorzugten Ausführungsform werden die vorgenannten Carbonsäureamide jeweils unabhängig voneinander aus der Gruppe, die aus Carbamoyl, 2-Amino-2-oxo-eth-1-yl, 3-Amino-3-oxo-prop-1-yl, 4-Amino-4-oxo-but-1-yl, Methylcarbamoyl, 2-(Methylamino)-2-oxo-eth-1-yl, 3-(Methylamino)-3-oxo-prop-1-yl, 4-(Methylamino)-4-oxo-but-1-yl, Dimethylcarbamoyl, 2-(Dimethylamino)-2-oxo-eth-1-yl, 3-(Dimethylamino)-3-oxo-prop-1-yl, 4-(Dimethylamino)-4-oxo-but-1-yl, Ethylcarbamoyl, 2-(Ethylamino)-2-oxo-eth-1-yl, 3-(Ethylamino)-3-oxo-prop-1-yl, 4-(Ethylamino)-4-oxo-but-1-yl, Ethyl(methyl)carbamoyl, 2-[Ethyl(methyl)amino]-2-oxo-eth-1-yl, 3-[Ethyl(methyl)amino]-3-oxo-prop-1-yl, 4-[Ethyl(methyl)amino]-4-oxo-but-1-yl, Dimethylcarbamoyl, 2-(Dimethylamino)-2-oxo-eth-1-yl, 3-(Dimethylamino)-3-oxo-prop-1-yl, 4-(Dimethylamino)-4-oxo-but-1-yl, Dipropylcarbamoyl, 2-(Dipropylamino)-2-oxo-eth-1-yl, 3-(Dipropylamino)-3-oxo-prop-1-yl, 4-(Dipropylamino)-4-oxo-but-1-yl, Diisopropylcarbamoyl, 2-(Diisopropylamino)-2-oxo-eth-1-yl, 3-(Diisopropylamino)-3-oxo-prop-1-yl, 4-(Diisopropylamino)-4-oxo-but-1-yl, Dibutylcarbamoyl, 2-(Dibutylamino)-2-oxo-eth-1-yl, 3-(Dibutylamino)-3-oxo-propyl, 4-(Dibutylamino)-4-oxo-but-1-yl, Di-tert-butylcarbamoyl, 2-(Di-tert-butylamino)-2-oxo-eth-1-yl, 3-(Di-tert-butylamino)-3-oxo-propyl, und 4-(Di-tert-butylamino)-4-oxo-but-1-yl besteht, ausgewählt.

Bei einer weiteren bevorzugten Ausführungsform wird der vorgenannte Heteroarylrest, der kein Stickstoffatom enthält, mit 4 bis 20 C-Atomen aus der Gruppe, die aus, Furanyl, und Benzofuranyl besteht, ausgewählt.

Bei einer weiteren bevorzugten Ausführungsform weisen die vorgenannten Etherreste jeweils unabhängig von einander 2 bis 17 C-Atome, weiter bevorzugt 2 bis 13 C-Atome, weiter bevorzugt 2 bis 9 C-Atome, auf. Bei einer weiter bevorzugten Ausführungsform werden die vorgenannten Etherreste beispielsweise aus der Gruppe, die aus Methoxymethyl, Methoxyethyl, Methoxy-n-propyl, Ethoxymethyl, n-Propoxymethyl, 2-Ethoxyethoxymethyl, 2-(2-Ethoxyethoxy)ethyl, i-Propoxymethyl, tert.-Butyloxymethyl, Dioxa-3,6-heptyl und Benzyloxymethyl besteht, ausgewählt. Bei einer weiter bevorzugten Ausführungsform können die vorgenannten Etherreste einfache Etherreste, Oligoetherreste, Polyetherreste oder Mischungen davon sein.

Erfindungsgemäß wird unter einem Halogen jeweils unabhängig voneinander Fluor, Chlor, Brom oder lod verstanden. Erfindungsgemäß wird unter einem Halogenid jeweils unabhängig von einander Fluorid, Chlorid, Bromid oder lodid verstanden.

Chiralitätszentren können, wenn nicht anders angegeben, in der R- oder in der S-Konfiguration vorliegen. Die Erfindung betrifft sowohl die optisch reinen Verbindungen als auch Stereoisomerengemische, wie Enantiomerengemische und Diasteromerengemische, in jedem Verhältnis.

Die Erfindung betrifft vorzugsweise auch Mesomere und/oder Tautomere der Verbindung mit der Formel (1) und/oder der Verbindung mit der Formel (2) und/oder der Verbindung mit der Formel (3) und/oder der Verbindung mit der Formel (100) und/oder der Verbindung mit der Formel (101) und/oder der Verbindung mit der Formel (102), sowohl die reinen Verbindungen als auch die Isomerengemische in jedem Verhältnis.

Beispielsweise kann die Verbindung der Formel (1) in der Keto-Form (1a) oder der Enol-Form (1b) vorliegen:

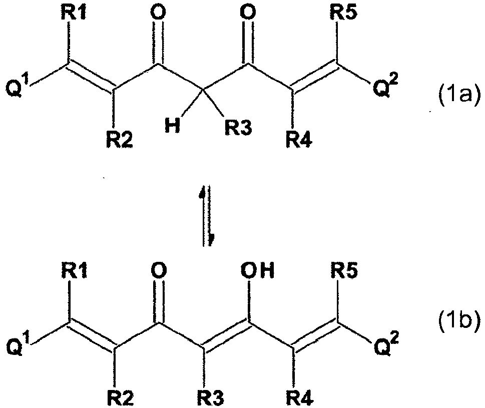

Eine Variante des Verfahrens zur Herstellung eines 1,7-Diaryl-1,6-heptadion-3,6-dion-Derivats der Formel (1), umfasst folgende Schritte:
(A) Umsetzen eines substituierten Arylketons der Formel (80) mit einem substituierten Acetylacetons der Formel (81) in Gegenwart eines Lösungsmittels, vorzugsweise Ethylacetat, Dimethylsulfoxid (DMSO), Dimethylformiat (DMF) oder Mischungen davon, in Gegenwart eines Dehydratisierungsmittels, vorzugsweise Tributylborat, B₂O₃ oder Mischungen davon, und eine primäre und/oder sekundären Amins, vorzugsweise n-Butylamin, Morpholin, Piperidin oder Mischungen davon, unter Erhalt einer Verbindung mit der Formel (82), 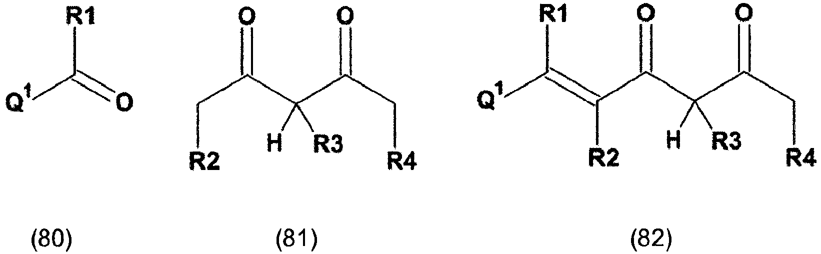 wobei Q¹ ein substituierter oder unsubstituierter monozyklischer oder polyzyklischer, aromatischer Rest bedeutet, und
   wobei der Reste Q¹ mit mindestens einem organischen Rest W1a, der die allgemeine Formel (5a), (6a), (7a), (8a), oder (9a) aufweist:

   -A-(C(D)(E))ₕ-X^{a}, (5a)

   -(C(D)(E))ₖ-Aryl-(C(D)(E))ₗ-X^{a}, (6a)

   -A-(C(D)(E))ₖ-Aryl-(C(D)(E))ₗ-X^{a}, (7a)

   -((C(D)(E))ₘ-A)ₚ-(C(D)(E))ₙ-X^{a}, (8a)

   -A-((C(D)(E))ₘ-A)ₚ-(C(D)(E))ₙ-X^{a}, (9a)

   substituiert ist,
   wobei h eine ganze Zahl von 1 bis 20, vorzugsweise von 2 bis 8, bedeutet, wobei k eine ganze Zahl von 0 bis 10, vorzugsweise von 1 bis 8, vorzugsweise von 2 bis 6, bedeutet, wobei l eine ganze Zahl von 0 bis 10, vorzugsweise von 1 bis 8, vorzugsweise von 2 bis 6, bedeutet, und wobei m, n und p jeweils unabhängig voneinander eine ganze Zahl von 1 bis 6, vorzugsweise von 2 bis 4, bedeuten, und
   wobei A jeweils unabhängig voneinander Sauerstoff oder Schwefel bedeutet,
   wobei D und E jeweils unabhängig voneinander Wasserstoff, Halogen, G-R^{(I)}, oder G-C(=G)-R^{(II)}, bedeuten, wobei G jeweils unabhängig voneinander Sauerstoff oder Schwefel bedeutet, und wobei die Reste R^{(I)} und R^{(II)} jeweils unabhängig voneinander Wasserstoff, Methyl, Ethyl, n-Propyl, n-Butyl, n-Pentyl, Phenyl oder Benzyl bedeutet, wobei Phenyl und Benzyl unsubstituiert oder substituiert sein können,
   wobei Aryl einen substituierten oder unsubstituierten Aromaten oder einen substituierten oder unsubstituierten Heteroaromaten, der kein Stickstoffatom enthält, bedeutet,
   wobei X^{a} jeweils unabhängig voneinander ein Rest der Formel (20c), (20d), oder (21) bedeutet: 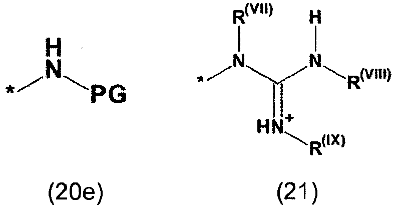 wobei jeder der Reste R^{(VII)}, R^{(VIII)}, und R^{(IX)} jeweils unabhängig voneinander eine Schutzgruppe PG, ein Arylrest mit 5 bis 12 C-Atomen, ein Alkylarylrest mit 5 bis 12 C-Atomen, ein Alkylrest, der geradkettig oder verzweigt sein kann, mit 1 bis 8 C-Atomen, oder ein Etherrest, der geradkettig oder verzweigt sein kann, mit 1 bis 8 C-Atomen bedeutet, und
   wobei die Reste R1, R2, R3, und R4 jeweils unabhängig voneinander Wasserstoff, Halogen, Alkyl mit 1 bis 12 C-Atomen, Cycloalkyl mit 1 bis 12 C-Atomen, Alkylaryl mit 1 bis 12 C-Atomen, Aryl mit 5 bis 20 C-Atomen, Ether mit 2 bis 12 C-Atomen oder Glykol mit 2 bis 12 C-Atomen bedeuten,
(B) Umsetzen der Verbindung mit der Formel (82) mit einem substituierten Arylketons der Formel (83) in Gegenwart eines Lösungsmittels, vorzugsweise Ethylacetat, Dimethylsulfoxid (DMSO), Dimethylformiat (DMF) oder Mischungen davon, in Gegenwart eines Dehydratisierungsmittels, vorzugsweise Tributylborat, B₂O₃ oder Mischungen davon, und eine primäre und/oder sekundären Amins, vorzugsweise n-Butylamin, Morpholin, Piperidin oder Mischungen davon, unter Erhalt eines 1,7-Diaryl-1,6-heptadion-3,6-dion-Derivats der Formel (1a): 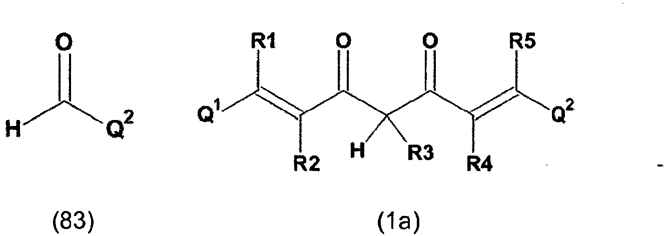 wobei Q² ein substituierter oder unsubstituierter monozyklischer oder polyzyklischer, aromatischer Rest bedeutet, und
   wobei der Rest Q² optional mit mindestens einem organischen Rest W1a, der die allgemeine Formel (5a), (6a), (7a), (8a), oder (8a) aufweist, und
   wobei der Rest R5 Wasserstoff, Halogen, Alkyl mit 1 bis 12 C-Atomen, Cycloalkyl mit 1 bis 12 C-Atomen, Alkylaryl mit 1 bis 12 C-Atomen, Aryl mit 5 bis 20 C-Atomen, Ether mit 2 bis 12 C-Atomen oder Glykol mit 2 bis 12 C-Atomen bedeutet,
(C) Optional Entfernen gegebenenfalls vorhandener Schutzgruppen PG.

Alternativ, kann ein erfindungsgemäßes 1,7-Diaryl-1,6-heptadion-3,6-dion-Derivat der Formel (1) hergestellt werden durch:
(A1) Umsetzen von Curcumin mit einer Verbindung der allgemeinen Formel X^{a}-(C(D)(E))ₕ-OH in Gegenwart von Diethylazodicarboxylat (DEAD) und Triphenylphosphan (PPh₃) sowie eines Lösungsmittels, vorzugsweise Ethylacetat, Dimethylsulfoxid (DMSO), Dimethylformiat (DMF) oder Mischungen davon, oder
(A2) Umsetzen von Curcumin mit einer Verbindung der allgemeinen Formel X^{a}-(C(D)(E))ₕ-Z in Gegenwart von K₂CO₃ und KI sowie eines Lösungsmittels, vorzugsweise Ethylacetat, Dimethylsulfoxid (DMSO), Dimethylformiat (DMF) oder Mischungen davon,
   wobei h eine ganze Zahl von 1 bis 20, vorzugsweise von 2 bis 8, bedeutet, wobei der Rest Z unabhängig von einander Cl, Br, I, Tosylat (OTs) oder Mesylat (OMs) bedeutet, und wobei X^{a} jeweils unabhängig voneinander ein Rest der Formel (20c), (20d), oder (21), bedeutet: 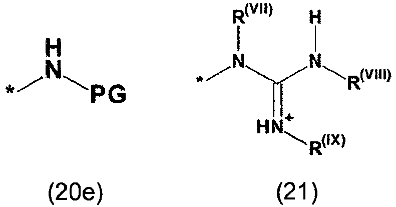 wobei jeder der Reste R^{(VII)}, R^{(VIII)}, und R^{(IX)} jeweils unabhängig voneinander eine Schutzgruppe PG, ein Arylrest mit 5 bis 12 C-Atomen, ein Alkylarylrest mit 5 bis 12 C-Atomen, ein Alkylrest, der geradkettig oder verzweigt sein kann, mit 1 bis 8 C-Atomen, oder ein Etherrest, der geradkettig oder verzweigt sein kann, mit 1 bis 8 C-Atomen bedeutet, und
(B) Entfernen vorhandener Schutzgruppen PG.

Eine weitere Variante des Verfahrens zur Herstellung eines 1,7-Diaryl-1,6-heptadion-3,6-dion-Derivats der Formel (1), umfasst folgende Schritte:
(A) Umsetzen eines unsubstituierten oder unsubstituierten Arylketons der Formel (80a) mit einem substituierten Acetylacetons der Formel (81a) in Gegenwart eines Lösungsmittels, vorzugsweise Ethylacetat, Dimethylsulfoxid (DMSO), Dimethylformiat (DMF) oder Mischungen davon, in Gegenwart eines Dehydratisierungsmittels, vorzugsweise Tributylborat, B₂O₃ oder Mischungen davon, und eine primäre und/oder sekundären Amins, vorzugsweise n-Butylamin, Morpholin, Piperidin oder Mischungen davon, unter Erhalt einer Verbindung mit der Formel (82a), 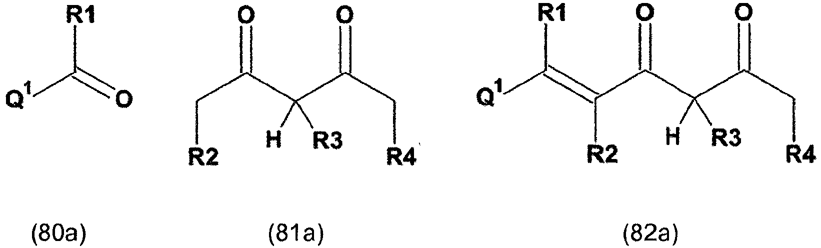 wobei der Rest R3 ein organischer Rest W2a ist, der die allgemeine Formel (4b), (5b), (6b), (7b), (8b), oder (9b):

   -(C(D)(E))ₕ-X^{b}, (4b)

   -A-(C(D)(E))ₕ-X^{b}, (5b)

   -(C(D)(E))ₖ-Aryl-(C(D)(E))ₗ-X^{b}, (6b)

   -A-(C(D)(E))ₖ-Aryl-(C(D)(E))ₗ-X^{b}, (7b)

   -[(C(D)(E))ₘ-A]ₚ-(C(D)(E))ₙ-X^{b}, (8b)

   -A-[(C(D)(E))ₘ-A]ₚ-(C(D)(E))ₙ-X^{b}, (9b)

   aufweist,
   wobei Q¹ ein substituierter oder unsubstituierter monozyklischer oder polyzyklischer, aromatischer Rest bedeutet, und
   wobei der Rest Q¹ optional mit mindestens einem organischen Rest W1b substituiert ist, der die allgemeine Formel (4b), (5b), (6b), (7b), (8b), oder (9b) aufweist, und
   wobei h eine ganze Zahl von 1 bis 20, vorzugsweise von 2 bis 8, bedeutet, wobei k eine ganze Zahl von 0 bis 10, vorzugsweise von 1 bis 8, vorzugsweise von 2 bis 6, bedeutet, wobei I eine ganze Zahl von 0 bis 10, vorzugsweise von 1 bis 8, vorzugsweise von 2 bis 6, bedeutet, und wobei m, n und p jeweils unabhängig voneinander eine ganze Zahl von 1 bis 6, vorzugsweise von 2 bis 4, bedeuten, und
   wobei A jeweils unabhängig voneinander Sauerstoff oder Schwefel bedeutet,
   wobei D und E jeweils unabhängig voneinander Wasserstoff, Halogen, G-R^{(I)}, oder G-C(=G)-R^{(II)}, bedeuten, wobei G jeweils unabhängig voneinander Sauerstoff oder Schwefel bedeutet, und wobei die Reste R^{(I)} und R^{(II)} jeweils unabhängig voneinander Wasserstoff, Methyl, Ethyl, n-Propyl, n-Butyl, n-Pentyl, Phenyl oder Benzyl bedeutet, wobei Phenyl und Benzyl unsubstituiert oder substituiert sein können,
   wobei Aryl einen substituierten oder unsubstituierten Aromaten oder einen substituierten oder unsubstituierten Heteroaromaten, der kein Stickstoffatom enthält, bedeutet,
   wobei X^{b} jeweils unabhängig von einander ein organischer Rest ist, der (i) wenigstens ein neutrales, protonierbares Stickstoffatom, oder (ii) wenigstens ein positiv geladenes, vorzugsweise quartäres, Stickstoffatom, oder (iii) wenigstens ein positiv geladenes, vorzugsweise quartäres, Phosphoratom enthält, wobei vorzugsweise X^{b} jeweils unabhängig voneinander ein Rest der Formel (20e), (21), oder (24), vorzugsweise ein Rest der Formel (20c), (20d), oder (21), weiter bevorzugt ein Rest der Formel (24), bedeutet: 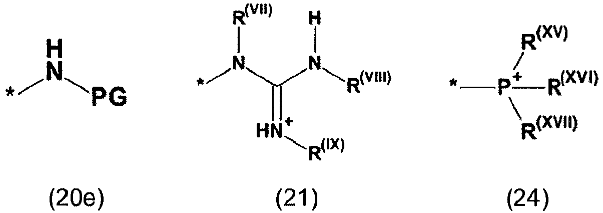 wobei jeder der Reste R^{(VII)}, R^{(VIII)}, und R^{(IX)} jeweils unabhängig voneinander eine Schutzgruppe PG, ein Arylrest mit 5 bis 12 C-Atomen, ein Alkylarylrest mit 5 bis 12 C-Atomen, ein Alkylrest, der geradkettig oder verzweigt sein kann, mit 1 bis 8 C-Atomen, oder ein Etherrest, der geradkettig oder verzweigt sein kann, mit 1 bis 8 C-Atomen bedeutet und wobei jeder der Reste R^{(XV)}, R^{(XVI)}, und R^{(VII)} jeweils unabhängig voneinander ein Arylrest mit 5 bis 12 C-Atomen, ein Alkylarylrest mit 5 bis 12 C-Atomen, ein Alkylrest, der geradkettig oder verzweigt sein kann, mit 1 bis 8 C-Atomen, oder ein Etherrest, der geradkettig oder verzweigt sein kann, mit 1 bis 8 C-Atomen bedeutet, und
   wobei die Reste R1, R2, und R4 jeweils unabhängig voneinander Wasserstoff, Halogen, Alkyl mit 1 bis 12 C-Atomen, Cycloalkyl mit 1 bis 12 C-Atomen, Alkylaryl mit 1 bis 12 C-Atomen, Aryl mit 5 bis 20 C-Atomen, Ether mit 2 bis 12 C-Atomen oder Glykol mit 2 bis 12 C-Atomen bedeuten,
(B) Umsetzen der Verbindung mit der Formel (82a) mit einem substituierten Arylketons der Formel (83a) in Gegenwart eines Lösungsmittels, vorzugsweise Ethylacetat, Dimethylsulfoxid (DMSO), Dimethylformiat (DMF) oder Mischungen davon, in Gegenwart eines Dehydratisierungsmittels, vorzugsweise Tributylborat, B₂O₃ oder Mischungen davon, und eine primäre und/oder sekundären Amins, vorzugsweise n-Butylamin, Morpholin, Piperidin oder Mischungen davon, unter Erhalt eines 1,7-Diaryl-1,6-heptadion-3,6-dion-Derivats der Formel (1a): 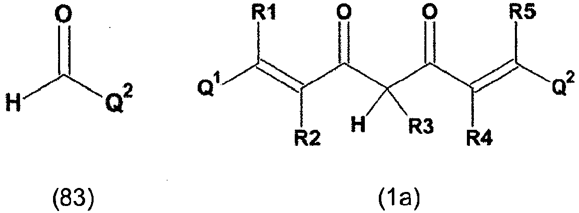 wobei Q² ein substituierter oder unsubstituierter monozyklischer oder polyzyklischer, aromatischer Rest bedeutet, und
   wobei der Rest Q² optional mit mindestens einem organischen Rest W1a substituiert ist, der die allgemeine Formel (4b), (5b), (6b), (7b), (8b), oder (9b) aufweist, und
   wobei der Rest R5 Wasserstoff, Halogen, Alkyl mit 1 bis 12 C-Atomen, Cycloalkyl mit 1 bis 12 C-Atomen, Alkylaryl mit 1 bis 12 C-Atomen, Aryl mit 5 bis 20 C-Atomen, Ether mit 2 bis 12 C-Atomen oder Glykol mit 2 bis 12 C-Atomen bedeutet,
(C) Optional Entfernen gegebenenfalls vorhandener Schutzgruppen PG.

Ein Verfahren zur Herstellung eines 1,7-Diaryl-1,6-heptadion-3,6-dion-Derivats der Formel (2), umfasst folgende Schritte:
(A) Umsetzen einer Verbindung mit der Formel (1) mit einem Metallsalz M(L¹)(L²) 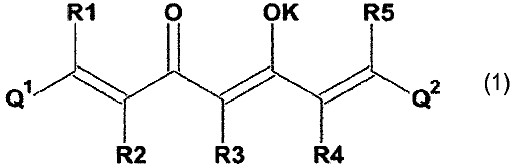 unter Erhalt eines 1,7-Diaryl-1,6-heptadion-3,6-dion-Derivats der Formel (2): 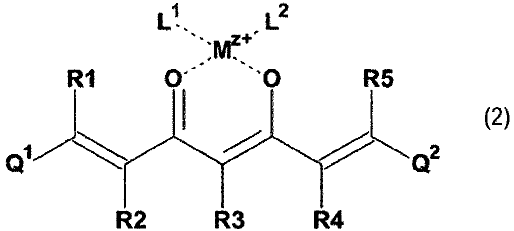 wobei L¹ und L² jeweils unabhängig voneinander Wasser, Halogenid, Cyanid, Thiocyanat, Phosphat, Hydrogenphosphat, oder ein Carboxylation einer Carbonsäure mit 1 bis 10 Kohlenstoffatomen, vorzugsweise Formiat, Acetat, n-Propionat, Lactat, Oxalat, Fumarat, Maleinat, Tartrat, Succinylat, Benzoat, Salicylat, oder Citrat, bedeuten, und wobei die Reste Q¹ und Q² jeweils unabhängig voneinander 1 substituierter oder unsubstituierter monozyklischer oder polyzyklischer, aromatischer Rest bedeuten, und wobei
   (a) wenigstens einer der Reste Q¹ und Q² jeweils unabhängig voneinander mit mindestens einem organischen Rest W1a substituiert ist, der die allgemeine Formel (5a), (6a), (7a), (8a), oder (9a) aufweist:

      -A-(C(D)(E))ₕ-X^{a}, (5a)

      -(C(D)(E))ₖ-Aryl-(C(D)(E))ₗ-X^{a}, (6a)

      -A-(C(D)(E))ₖ-Aryl-(C(D)(E))ₗ-X^{a}, (7a)

      -((C(D)(E))ₘ-A)ₚ-(C(D)(E))ₙ-X^{a}, (8a)

      -A-((C(D)(E))ₘ-A)ₚ-(C(D)(E))ₙ-X^{a}, (9a)

      wobei h eine ganze Zahl von 1 bis 20 bedeutet, wobei k eine ganze Zahl von 0 bis 10 bedeutet, wobei I eine ganze Zahl von 0 bis 10 bedeutet, und wobei m, n und p jeweils unabhängig voneinander eine ganze Zahl von 1 bis 6 bedeuten, und
      wobei A jeweils unabhängig voneinander Sauerstoff oder Schwefel beudeutet
      wobei D und E jeweils unabhängig voneinander Wasserstoff, Halogen, G-R^{(I)}, oder G-C(=G)-R^{(II)}, bedeuten, wobei G jeweils unabhängig voneinander Sauerstoff oder Schwefel bedeutet, und wobei die Reste R^{(I)} und R^{(II)} jeweils unabhängig voneinander Wasserstoff, Methyl, Ethyl, n-Propyl, n-Butyl, n-Pentyl, Phenyl oder Benzyl bedeutet, wobei Phenyl und Benzyl unsubstituiert oder substituiert sein können,
      wobei Aryl einen substituierten oder unsubstituierten Aromaten oder einen substituierten oder unsubstituierten Heteroaromaten, der kein Stickstoffatom enthält, bedeutet,
      wobei X^{a} jeweils unabhängig voneinander ein Rest der Formel (20e), (21), oder (24), vorzugsweise ein Rest der Formel (20c), (20d), oder (21), weiter bevorzugt ein Rest der Formel (24), bedeutet: 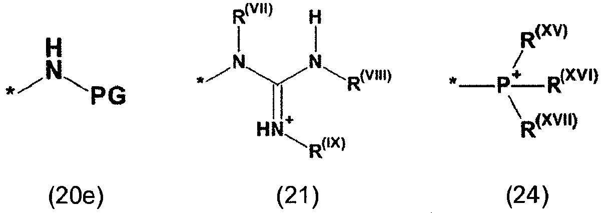 wobei jeder der Reste R^{(VII)}, R^{(VIII)}, und R^{(IX)} jeweils unabhängig voneinander eine Schutzgruppe PG, ein Arylrest mit 5 bis 12 C-Atomen, ein Alkylarylrest mit 5 bis 12 C-Atomen, ein Alkylrest, der geradkettig oder verzweigt sein kann, mit 1 bis 8 C-Atomen, oder ein Etherrest, der geradkettig oder verzweigt sein kann, mit 1 bis 8 C-Atomen bedeutet und wobei jeder der Reste R^{(XV)}, R^{(XVI)}, und R^{(XVII)} jeweils unabhängig voneinander ein Arylrest mit 5 bis 12 C-Atomen, ein Alkylarylrest mit 5 bis 12 C-Atomen, ein Alkylrest, der geradkettig oder verzweigt sein kann, mit 1 bis 8 C-Atomen, oder ein Etherrest, der geradkettig oder verzweigt sein kann, mit 1 bis 8 C-Atomen bedeutet, und
      wobei die Reste R1, R2, R3, R4 und R5 jeweils unabhängig voneinander Wasserstoff, Halogen, Alkyl mit 1 bis 12 C-Atomen, Cycloalkyl mit 1 bis 12 C-Atomen, Alkylaryl mit 1 bis 12 C-Atomen, Aryl mit 5 bis 20 C-Atomen, Ether mit 2 bis 12 C-Atomen oder Glykol mit 2 bis 12 C-Atomen bedeuten,
      oder wobei
   (b) der Rest R3 ein organischer Rest W2a ist, der die allgemeine Formel (4b), (5b), (6b), (7b), (8b), oder (9b):

      -(C(D)(E))ₕ-X^{b}, (4b)

      -A-(C(D)(E))ₕ-X^{b}, (5b)

      -(C(D)(E))ₖ-Aryl-(C(D)(E))ₗ-X^{b}, (6b)

      -A-(C(D)(E))ₖ-Aryl-(C(D)(E))ₗ-X^{b}, (7b)

      -[(C(D)(E))ₘ-A]ₚ-(C(O)(E))ₙ-X^{b}, (8b)

      -A-[(C(D)(E))ₘ-A]ₚ-(C(D)(E))ₙ-X^{b}, (9b)

      aufweist, und
      wobei, optional, wenigstens einer der Reste Q¹ und Q² jeweils unabhängig voneinander mit mindestens einem organischen Rest W1b, der die allgemeine Formel (4b), (5b), (6b), (7b), (8b), oder (9b) aufweist, substituiert ist,
      wobei h eine ganze Zahl von 1 bis 20 bedeutet, wobei k eine ganze Zahl von 0 bis 10, bedeutet, wobei I eine ganze Zahl von 0 bis 10 bedeutet, und wobei m, n und p jeweils unabhängig voneinander eine ganze Zahl von 1 bis 6 bedeuten, und
      wobei A jeweils unabhängig voneinander Sauerstoff oder Schwefel bedeutet, wobei D und E jeweils unabhängig voneinander Wasserstoff, Halogen, G-R^{(I)}, oder G-C(=G)-R^{(II)}, bedeuten, wobei G jeweils unabhängig voneinander Sauerstoff oder Schwefel bedeutet, und wobei die Reste R^{(I)} und R^{(II)} jeweils unabhängig voneinander Wasserstoff, Methyl, Ethyl, n-Propyl, n-Butyl, n-Pentyl, Phenyl oder Benzyl bedeutet, wobei Phenyl und Benzyl unsubstituiert oder substituiert sein können,
      wobei Aryl einen substituierten oder unsubstituierten Aromaten oder einen substituierten oder unsubstituierten Heteroaromaten, der kein Stickstoffatom enthält, bedeutet,
      wobei X^{b} jeweils unabhängig von einander ein organischer Rest ist, der (i) wenigstens ein neutrales, protonierbares Stickstoffatom, oder (ii) wenigstens ein positiv geladenes, vorzugsweise quartäres, Stickstoffatom, oder (iii) wenigstens ein positiv geladenes, vorzugsweise quartäres, Phosphoratom enthält, wobei vorzugsweise X^{b} jeweils unabhängig voneinander ein Rest der Formel (20e), (21), oder (24), vorzugsweise ein Rest der Formel (20c), (20d), oder (21), weiter bevorzugt ein Rest der Formel (24), bedeutet: 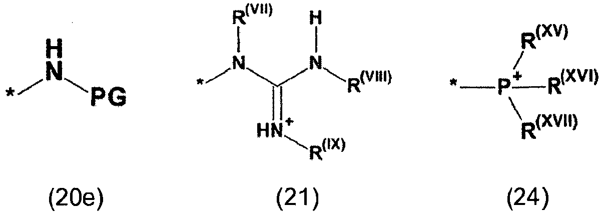 wobei jeder der Reste R^{(VII)}, R^{(VIII)}, und R^{(IX)} jeweils unabhängig voneinander eine Schutzgruppe PG, ein Arylrest mit 5 bis 12 C-Atomen, ein Alkylarylrest mit 5 bis 12 C-Atomen, ein Alkylrest, der geradkettig oder verzweigt sein kann, mit 1 bis 8 C-Atomen, oder ein Etherrest, der geradkettig oder verzweigt sein kann, mit 1 bis 8 C-Atomen bedeutet und wobei jeder der Reste R^{(XV)}, R^{(XVI)}, und R^{(XVII)} jeweils unabhängig voneinander ein Arylrest mit 5 bis 12 C-Atomen, ein Alkylarylrest mit 5 bis 12 C-Atomen, ein Alkylrest, der geradkettig oder verzweigt sein kann, mit 1 bis 8 C-Atomen, oder ein Etherrest, der geradkettig oder verzweigt sein kann, mit 1 bis 8 C-Atomen bedeutet, und
      wobei die Reste R1, R2, R4 und R5 jeweils unabhängig voneinander Wasserstoff, Halogen, Alkyl mit 1 bis 12 C-Atomen, Alkylaryl mit 1 bis 12 C-Atomen, Aryl mit 5 bis 20 C-Atomen, Ether mit 2 bis 12 C-Atomen oder Glykol mit 2 bis 12 C-Atomen bedeuten.

Ein Verfahren zur Herstellung eines 1,7-Diaryl-1,6-heptadion-3,6-dion-Derivats der Formel (3), umfasst folgende Schritte:
(A) Umsetzen einer Verbindung mit der der Formel (2): 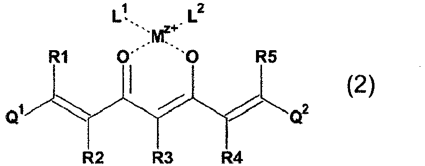 mit einer Verbindung der Formel (1e): 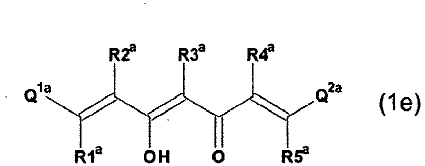 unter Erhalt eines 1,7-Diaryl-1,6-heptadion-3,6-dion-Derivats der Formel (3) 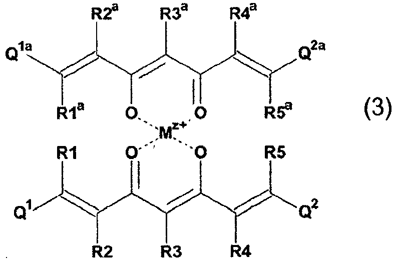 wobei L¹ und L² jeweils unabhängig voneinander Wasser, Fluorid, Chlorid, Bromid, lodid, Phosphat, Hydrogenphosphat, Dihydrogenphosphat, Sulfat, Hydrogensulfat, Tosylat, Mesylat oder wenigstens ein Carboxylation einer Carbonsäure mit 1 bis 15 Kohlenstoffatomen und/oder Mischungen davon bedeuten,
   wobei M^{z+} ein Kation eines Metalls bedeutet, wobei z die formale Oxidationszahl des Metalls M ist und wobei z eine ganze Zahl von 1 bis 7, vorzugsweise von 2 bis 5, bedeutet, und
   wobei die Reste Q¹ und Q² jeweils unabhängig voneinander 1 substituierter oder unsubstituierter monozyklischer oder polyzyklischer, aromatischer Rest bedeuten, und
   wobei die Reste Q^{1a} und Q^{2a} jeweils unabhängig voneinander 1 substituierter oder unsubstituierter monozyklischer oder polyzyklischer, aromatischer Rest oder 1 substituierter oder unsubstituierter monozyklischer oder polyzyklischer, heteroaromatischer Rest bedeuten,
   und wobei
   (a) wenigstens einer der Reste Q¹ und Q² jeweils unabhängig voneinander mit mindestens einem organischen Rest W1a substituiert ist, wobei der mindestens eine organische Rest W1a die allgemeine Formel (5a), (6a), (7a), (8a), oder (9a) aufweist:

      -A-(C(D)(E))ₕ-X^{a}, (5a)

      -(C(D)(E))ₖ-Aryl-(C(D)(E))ₗ-X^{a}, (6a)

      -A-(C(D)(E))ₖ-Aryl-(C(D)(E))ₗ-X^{a}, (7a)

      -((C(D)(E))ₘ-A)ₚ-(C(D)(E))ₙ-X^{a}, (8a)

      -A-((C(D)(E))ₘ-A)ₚ-(C(D)(E))ₙ-X^{a}, (9a)

      wobei h eine ganze Zahl von 1 bis 20 bedeutet, wobei k eine ganze Zahl von 0 bis 10 bedeutet, wobei I eine ganze Zahl von 0 bis 10 bedeutet, und wobei m, n und p jeweils unabhängig voneinander eine ganze Zahl von 1 bis 6 bedeuten, und
      wobei A jeweils unabhängig voneinander Sauerstoff oder Schwefel bedeutet,
      wobei D und E jeweils unabhängig voneinander Wasserstoff, Halogen, G-R^{(I)}, oder G-C(=G)-R^{(II)}, bedeuten, wobei G jeweils unabhängig voneinander Sauerstoff oder Schwefel bedeutet, und wobei die Reste R^{(I)} und R^{(II)} jeweils unabhängig voneinander Wasserstoff, Methyl, Ethyl, n-Propyl, n-Butyl, n-Pentyl, Phenyl oder Benzyl bedeutet, wobei Phenyl und Benzyl unsubstituiert oder substituiert sein können,
      wobei Aryl einen substituierten oder unsubstituierten Aromaten oder einen substituierten oder unsubstituierten Heteroaromaten, der kein Stickstoffatom enthält, bedeutet,
      wobei X^{a} jeweils unabhängig voneinander ein Rest der Formel (20c), (20d), (21), oder (24), weiter bevorzugt ein Rest der Formel (20c), (20d), oder (21), weiter bevorzugt ein Rest der Formel (24), bedeutet: 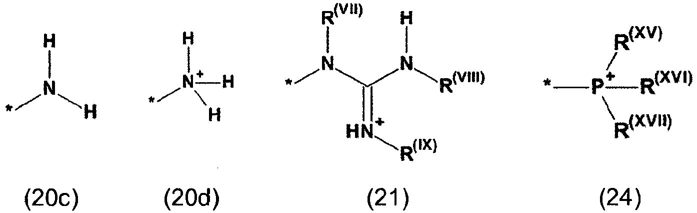 wobei jeder der Reste R^{(VII)}, R^{(VIII)}, und R^{(IX)} jeweils unabhängig voneinander Wasserstoff, ein Arylrest mit 5 bis 12 C-Atomen, ein Alkylarylrest mit 5 bis 12 C-Atomen, ein Alkylrest, der geradkettig oder verzweigt sein kann, mit 1 bis 8 C-Atomen, oder ein Etherrest, der geradkettig oder verzweigt sein kann, mit 1 bis 8 C-Atomen bedeutet und wobei jeder der Reste R^{(XV)}, R^{(XVI)}, und R^{(XVII)} jeweils unabhängig voneinander ein Arylrest mit 5 bis 12 C-Atomen, ein Alkylarylrest mit 5 bis 12 C-Atomen, ein Alkylrest, der geradkettig oder verzweigt sein kann, mit 1 bis 8 C-Atomen, oder ein Etherrest, der geradkettig oder verzweigt sein kann, mit 1 bis 8 C-Atomen bedeutet, und
      wobei wenigstens einer der Reste Q^{1a} und Q^{2a} jeweils unabhängig voneinander mit mindestens einem organischen Rest W1c substituiert ist, wobei der mindestens eine organische Rest W1c die allgemeine Formel (5c), (6c), (7c), (8c), oder (9c) aufweist:

      -A-(C(D)(E))ₕ-X^{c}, (5c)

      -(C(D)(E))ₖ-Aryl-(C(D)(E))ₗ-X^{c}, (6c)

      -A-(C(D)(E))ₖ-Aryl-(C(D)(E))ₗ-X^{c}, (7c)

      -((C(D)(E))ₘ-A)ₚ-(C(D)(E))ₙ-X^{c}, (8c)

      -A-((C(D)(E))ₘ-A)ₚ-(C(D)(E))ₙ-X^{c}, (9c)

      wobei h eine ganze Zahl von 1 bis 20 bedeutet, wobei k eine ganze Zahl von 0 bis 10 bedeutet, wobei I eine ganze Zahl von 0 bis 10 bedeutet, und wobei m, n und p jeweils unabhängig voneinander eine ganze Zahl von 1 bis 6 bedeuten, und
      wobei A jeweils unabhängig voneinander Sauerstoff oder Schwefel bedeutet,
      wobei D und E jeweils unabhängig voneinander Wasserstoff, Halogen, G-R^{(I)}, oder G-C(=G)-R^{(II)}, bedeuten, wobei G jeweils unabhängig voneinander Sauerstoff oder Schwefel bedeutet, und wobei die Reste R^{(I)} und R^{(II)} jeweils unabhängig voneinander Wasserstoff, Methyl, Ethyl, n-Propyl, n-Butyl, n-Pentyl, Phenyl oder Benzyl bedeutet, wobei Phenyl und Benzyl unsubstituiert oder substituiert sein können,
      wobei Aryl einen substituierten oder unsubstituierten Aromaten oder einen substituierten oder unsubstituierten Heteroaromaten, der kein Stickstoffatom enthält, bedeutet,
      wobei X^{c} jeweils unabhängig voneinander ein organischer Rest ist, der (i) wenigstens ein neutrales, protonierbares Stickstoffatom, oder (ii) wenigstens ein positiv geladenes, vorzugsweise quartäres, Stickstoffatom, oder (iii) wenigstens ein positiv geladenes, vorzugsweise quartäres, Phosphoratom enthält, wobei vorzugsweise X^{c} jeweils unabhängig voneinander ein Rest der Formel (20c), (20d), (21), oder (24), weiter bevorzugt ein Rest der Formel (20c), (20d), oder (21), weiter bevorzugt ein Rest der Formel (24), bedeutet: 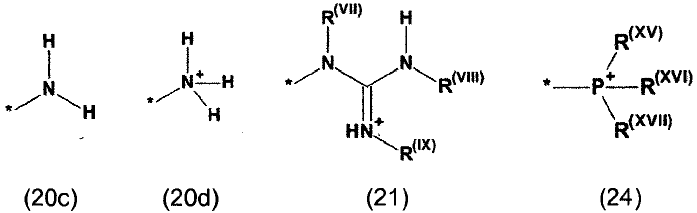 wobei jeder der Reste R^{(VII)}, R^{(VIII)}, und R^{(IX)} jeweils unabhängig voneinander Wasserstoff, ein Arylrest mit 5 bis 12 C-Atomen, ein Alkylarylrest mit 5 bis 12 C-Atomen, ein Alkylrest, der geradkettig oder verzweigt sein kann, mit 1 bis 8 C-Atomen, oder ein Etherrest, der geradkettig oder verzweigt sein kann, mit 1 bis 8 C-Atomen bedeutet und wobei jeder der Reste R^{(XV)}, R^{(XVI)}, und R^{(XVII)} jeweils unabhängig voneinander ein Arylrest mit 5 bis 12 C-Atomen, ein Alkylarylrest mit 5 bis 12 C-Atomen, ein Alkylrest, der geradkettig oder verzweigt sein kann, mit 1 bis 8 C-Atomen, oder ein Etherrest, der geradkettig oder verzweigt sein kann, mit 1 bis 8 C-Atomen bedeutet,
      wobei die Reste R1, R1^{a}, R2, R2^{a}, R3, R3^{a}, R4, R4^{a}, R5 und R5^{a} jeweils unabhängig voneinander Wasserstoff, Halogen, Alkyl mit 1 bis 12 C-Atomen, Cycloalkyl mit 1 bis 12 C-Atomen, Alkylaryl mit 1 bis 12 C-Atomen, Aryl mit 5 bis 20 C-Atomen, Ether mit 2 bis 12 C-Atomen oder Glykol mit 2 bis 12 C-Atomen bedeuten,oder wobei
   (b) der Rest R3 oder R3^{a} jeweils unabhängig von einander ein organischer Rest W2a ist, wobei der eine organische Rest W2a die allgemeine Formel (4b), (5b), (6b), (7b), (8b), oder (9b):

      -(C(D)(E))ₕ-X^{b}, (4b)

      -A-(C(D)(E))ₕ-X^{b}, (5b)

      -(C(D)(E))ₖ-Aryl-(C(D)(E))ₗ-X^{b}, (6b)

      -A-(C(D)(E))ₖ-Aryl-(C(D)(E))ₗ-X^{b}, (7b)

      -[(C(D)(E))ₘ-A]ₚ-(C(D)(E))ₙ-X^{b}, (8b)

      -A-[(C(D)(E))ₘ-A]ₚ-(C(D)(E))ₙ-X^{b}, (9b)

      aufweist, und
      wobei, optional, wenigstens einer der Reste Q¹, Q^{1a}, Q² und Q^{2a} jeweils unabhängig voneinander mit mindestens einem organischen Rest W1b, der die allgemeine Formel (4b), (5b), (6b), (7b), (8b), oder (9b) aufweist, substituiert sind,
      wobei h eine ganze Zahl von 1 bis 20 bedeutet, wobei k eine ganze Zahl von 0 bis 10 bedeutet, wobei I eine ganze Zahl von 0 bis 10 bedeutet, und wobei m, n, und p jeweils unabhängig voneinander eine ganze Zahl von 1 bis 6 bedeuten, und
      wobei A jeweils unabhängig voneinander Sauerstoff oder Schwefel, vorzugsweise Sauerstoff, bedeutet,
      wobei D und E jeweils unabhängig voneinander Wasserstoff, Halogen, G-R^{(I)}, oder G-C(=G)-R^{(II)}, bedeuten, wobei G jeweils unabhängig voneinander Sauerstoff oder Schwefel bedeutet, und wobei die Reste R^{(I)} und R^{(II)} jeweils unabhängig voneinander Wasserstoff, Methyl, Ethyl, n-Propyl, n-Butyl, n-Pentyl, Phenyl oder Benzyl bedeutet, wobei Phenyl und Benzyl unsubstituiert oder substituiert sein können,
      wobei Aryl einen substituierten oder unsubstituierten Aromaten oder einen substituierten oder unsubstituierten Heteroaromaten, der kein Stickstoffatom enthält, bedeutet,
      wobei X^{b} jeweils unabhängig voneinander ein organischer Rest ist, der (i) wenigstens ein neutrales, protonierbares Stickstoffatom, oder (ii) wenigstens ein positiv geladenes, vorzugsweise quartäres, Stickstoffatom, oder (iii) wenigstens ein positiv geladenes, vorzugsweise quartäres, Phosphoratom enthält, wobei vorzugsweise X^{b} jeweils unabhängig voneinander ein Rest der Formel (20c), (20d), (21), oder (24), weiter bevorzugt ein Rest der Formel (20c), (20d), oder (21), weiter bevorzugt ein Rest der Formel (24), bedeutet: 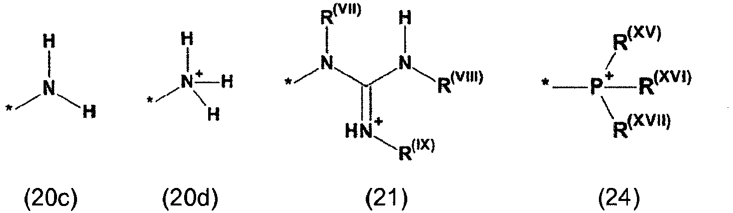 wobei jeder der Reste R^{(VII)}, R^{(VIII)}, und R^{(IX)} jeweils unabhängig voneinander Wasserstoff, ein Arylrest mit 5 bis 12 C-Atomen, ein Alkylarylrest mit 5 bis 12 C-Atomen, ein Alkylrest, der geradkettig oder verzweigt sein kann, mit 1 bis 8 C-Atomen, oder ein Etherrest, der geradkettig oder verzweigt sein kann, mit 1 bis 8 C-Atomen bedeutet und wobei jeder der Reste R^{(XV)}, R^{(XVI)}, und R^{(XVII)} jeweils unabhängig voneinander ein Arylrest mit 5 bis 12 C-Atomen, ein Alkylarylrest mit 5 bis 12 C-Atomen, ein Alkylrest, der geradkettig oder verzweigt sein kann, mit 1 bis 8 C-Atomen, oder ein Etherrest, der geradkettig oder verzweigt sein kann, mit 1 bis 8 C-Atomen bedeutet, und
      wobei die Reste R1, R1^{a}, R2, R2^{a}, R4, R4^{a}, R5 und R5^{a} jeweils unabhängig voneinander Wasserstoff, Halogen, Alkyl mit 1 bis 12 C-Atomen, Alkylaryl mit 1 bis 12 C-Atomen, Aryl mit 5 bis 20 C-Atomen, Ether mit 2 bis 12 C-Atomen oder Glykol mit 2 bis 12 C-Atomen bedeuten.

Bei der Verwendung unterschiedlicher Amino-Schutzgruppe PG in einer Synthese, ergibt sich die Möglichkeit der orthogonalen Schutzgruppenstrategie, wobei unterschiedliche Amino-Funktionen eines Moleküls gezielt nacheinander freigesetzt können und zur Reaktion gebracht werden.

Geeignete Methoden zur Entfernung der Amino-Schutzgruppe PG sind aus dem Stand der Technik bekannt. Beispielsweise kann Benzyloxycarbonyl (Cbz) durch Behandlung mit Lewis-Säuren, beispielsweise ZnBr₂ in Essigsäure, wieder entfernt werden. Di-tert-butyloxycarbonyl (Boc) kann beispielsweise durch saure Hydrolyse entfernt werden. Allyloxycarbonyl (Alloc) kann beispielsweise durch Einwirkung von Tetrakis(triphenylphosphan)palladium(0) und einem Nukleophil abgespalten werden.

Das aktive oder passive Eindringen, Anhaften und Vermehren von Krankheitserregern in einen Wirt wird als Infektion bezeichnet. Quellen für infektiöse Partikel treten überall auf. So wird beispielsweise der menschliche Körper von einer großen Anzahl von Mikroorganismen besiedelt, die im Regelfall durch den normalen Metabolismus und ein intaktes Immunsystem unter Kontrolle gehalten werden. Allerdings kann es, beispielsweise bei einer Schwächung des Immunsystems, zu einer starken Vermehrung der Krankheiterreger kommen und je nach Art des Erregers zu unterschiedlichen Krankheitssymptomen. Die Medizin hält für viele Erreger-bedingte Krankheiten spezifische Gegenmittel bereit, beispielsweise Antibiotika gegen Bakterien oder Antimikotika gegen Pilze oder Virustatika gegen Viren. Allerdings ist bei der Verwendung dieser Gegenmittel vermehrt das Auftreten von resistenten Krankheitserregern zu beobachten, die teilweise gleichzeitig gegen mehrere Gegenmittel Resistenzen aufweisen. Durch das Auftreten dieser resistenten oder multiresistenten Krankheitserreger hat sich die Therapie von Infektionserkrankungen zunehmend erschwert. Die klinische Konsequenz der Resistenz zeigt sich durch ein Versagen der Behandlung, vor allem bei immunsupprimierten Patienten.

Einzellige oder mehrzellige Mikroorganismen können Auslöser von infektiösen Erkrankungen sein. Durch Applikation wenigstens eines Erreger-spezifischen Gegenmittels, beispielsweise Antibiotikum, Antimikotikum oder Virustatikum, kann die Anzahl der Erreger reduziert und/oder der Erreger inaktiviert werden. Die Applikation eines Erreger-spezifischen Gegenmittels kann systemisch und/oder topisch erfolgen.

Bei der systemischen Applikation wird das Erreger-spezifische Gegenmittel in das Blut- und/oder Lymphsystem des zu behandelnden Körpers übertragen und hierüber im gesamten Körper verteilt. Bei einer systemischen Aufnahme des Erreger-spezifischen Gegenmittels kann es zu einem Abbau des Gegenmittels und/oder zu Nebenwirkungen, beispielsweise durch eine biochemische Umwandlung (Metabolisierung) des Gegenmittels, kommen.

Bei der topischen Applikation des Erreger-spezifischen Gegenmittels erfolgt die Anwendung des Gegenmittels dort, wo es therapeutisch wirken soll, beispielsweise auf einer infizierten Hautpartie, während die gesunde Haut nicht belastet wird. Somit können systemische Nebenwirkungen weitgehend vermieden werden.

Oberflächliche Haut- oder Weichteilinfektionen müssen nicht notwendigerweise mit, einer systemischen Applikation eines Erreger-spezifischen Gegenmittels behandelt werden, da das Gegenmittel direkt auf die infizierte Hautpartien aufgetragen werden kann.

Die bisher bekannten Erreger-spezifischen Gegenmittel weisen sowohl bei systemischer als auch topischer Applikation teilweise starke Neben- und Wechselwirkungen auf. Darüber hinaus kann es auch bei topischer Applikation durch eine unzuverlässige Medikamenteneinnahme (Compliance) des Patienten, insbesondere bei Verwendung von Antibiotika, zur Resistenzbildung kommen.

Eine Alternative stellt hier die photodynamische Inaktivierung von Mikroorganismen dar, bei der Resistenzen gegenüber der photodynamischen Inaktivierung unbekannt sind. Unabhängig von der Art der zu bekämpfenden Mikroorganismen und der damit verbundenen infektiösen Erkrankungen wird die Anzahl der Erreger reduziert und/oder die Erreger werden abtötet. Beispielsweise können Mischungen aus verschieden Mikroorganismen, beispielsweise Pilze und Bakterien oder unterschiedliche Bakterienstämme, bekämpft werden.

Die Aufgabe der vorliegenden Erfindung wird durch die Bereitstellung eines Verfahrens zur Inaktivierung von Mikroorganismen, die vorzugsweise Viren, Archäeen, Bakterien, Bakteriensporen, Pilzen, Pilzsporen, Protozoen, Algen, blutübertragbaren Parasiten oder Kombinationen davon umfassen, gelöst, wobei das Verfahren folgende Schritte umfasst:
(A) in Kontakt bringen der Mikroorganismen mit wenigstens einem Photosensibilisator, wobei der Photosensibilisator wenigstens ein 1,7-Diaryl-1,6-heptadien-3,5-dion-Derivat der Formel (100) und/oder wenigstens ein 1,7-Diaryl-1,6-heptadien-3,5-dion-Derivat der Formel (101) und/oder wenigstens ein 1,7-Diaryl-1,6-heptadien-3,5-dion-Derivat der Formel (1) und/oder wenigstens ein 1,7-Diaryl-1,6-heptadien-3,5-dion-Derivat der Formel (2) und/oder wenigstens ein 1,7-Diaryl-1,6-heptadien-3,5-dion-Derivat der Formel (3) oder jeweils ein pharmakologisch verträgliches Salz und/oder Ester und/oder Komplex davon ist, und
(B) Bestrahlen der Mikroorganismen und des wenigstens einen Photosensibilisators mit elektromagnetischer Strahlung geeigneter Wellenlänge und Energiedichte.

Verzugsweise wird das erfindungsgemäße Verfahren zur Inaktivierung von Mikroorganismen bei der photodynamischen Therapie eines Patienten und/oder photodynamischen Entkeimung wenigstens einer Oberfläche eines Gegenstandes und/oder wenigstens einer Oberfläche eines Raumes durchgeführt.

Bei einer bevorzugten Ausführungsform des erfindungsgemäßen Verfahrens erfolgt die Bestrahlung der Mikroorganismen und des wenigstens einen Photosensibilisators mit elektromagnetischer Strahlung geeigneter Wellenlänge und Energiedichte in Gegenwart wenigstens einer Sauerstoff-abgebenden Verbindung, vorzugsweise Peroxid, und/oder wenigstens eines Sauerstoff-haltigen Gases, vorzugsweise Sauerstoff.

Die wenigstens eine Sauerstoff-abgebende Verbindung und/oder das wenigstens eine Sauerstoff-haltige Gas können vorzugsweise vor, oder während Schritt (B) des erfindungsgemäßen Verfahrens appliziert werden.

Durch eine zusätzliche Gabe von Sauerstoff in Form wenigstens einer Sauerstoff-haltigen Verbindung und/oder wenigstens eines Sauerstoff-haltigen Gases vor oder während der Bestrahlung der Mikroorganismen und des wenigstens einen Photosensibilisators mit elektromagnetischer Strahlung geeigneter Wellenlänge und Energiedichte wird die Ausbeute an entstandenen reaktiven Sauerstoffspezies (ROS), vorzugsweise Sauerstoffradikale und/oder Singulett-Sauerstoff erhöht.

Vorzugweise ist der Photosensibilisator wenigstens eine Verbindung mit der Formel (100), wenigstens eine Verbindung die Formel (101), wenigstens eine Verbindung mit der Formel (102), wenigstens eine Verbindung mit der Formel (1), wenigstens eine Verbindung die Formel (2), wenigstens eine Verbindung mit der Formel (3) oder eine Kombination davon oder jeweils ein pharmakologisch verträgliches Salz und/oder Ester und/oder Komplex davon.

Weiter bevorzugt ist der Photosensibilisator ein erfindungsgemäßes 1,7-Diaryl-1,6-heptadien-3,5-dion-Derivat der Formel (1), und/oder ein erfindungsgemäßes 1,7-Diaryl-1,6-heptadien-3,5-dion-Derivat der Formel (2), und/oder ein erfindungsgemäßes 1,7-Diaryl-1,6-heptadien-3,5-dion-Derivat der Formel (3), und/oder ein erfindungsgemäßes 1,7-Diaryl-1,6-heptadien-3,5-dion-Derivat der Formel (3a) oder jeweils ein pharmakologisch verträgliches Salz und/oder Ester und/oder Komplex davon.

Die Aufgabe der vorliegenden Erfindung wir ebenfalls gelöst durch die Verwendung wenigstens eines 1,7-Diaryl-1,6-heptadien-3,5-dion-Derivats der Formel (100), und/oder wenigstens eines 1,7-Diaryl-1,6-heptadien-3,5-dion-Derivats der Formel (101), und/oder wenigstens eines 1,7-Diaryl-1,6-heptadien-3,5-dion-Derivat der Formel (102) oder jeweils ein pharmakologisch verträgliches Salz und/oder Ester und/oder Komplex davon als Photosensibilisator zur Inaktivierung von Mikroorganismen, die vorzugsweise Viren, Archäeen, Bakterien, Bakteriensporen, Pilzen, Pilzsporen, Protozoen, Algen, blutübertragbaren Parasiten oder Kombinationen davon umfassen.

Weiter bevorzugt wird ein erfindungsgemäßes 1,7-Diaryl-1,6-heptadien-3,5-dion-Derivat der Formel (1), und/oder ein erfindungsgemäßes 1,7-Diaryl-1,6-hepladien-3,5-dion-Derivat der Formel (2), und/oder ein erfindungsgemäßes 1,7-Diaryl-1,6-heptadien-3,5-dion-Derivat der Formel (3), und/oder ein erfindungsgemäßes 1,7-Diaryl-1,6-heptadien-3,5-dion-Derivat der Formel (3a) oder jeweils ein pharmakologisch verträgliches Salz und/oder Ester und/oder Komplex davon als Photosensibilisator zur Inaktivierung von Mikroorganismen, die vorzugsweise Viren, Archäeen, Bakterien, Bakteriensporen, Pilzen, Pilzsporen, Protozoen, Algen, blutübertragbaren Parasiten oder Kombinationen davon umfassen, verwendet.

Ein erfindungsgemäß zu verwendendes1,7-Diaryl-1,6-heptadien-3,5-dion-Derivat der Formel (100), und/oder der Formel (101), und/oder der Formel (102), und/oder ein erfindungsgemäßes 1,7-Diaryl-1,6-heptadien-3,5-dion-Derivat der Formel (1), und/oder ein erfindungsgemäßes 1,7-Diaryl-1,6-heptadien-3,5-dion-Derivat der Formel (2), und/oder ein erfindungsgemäßes 1,7-Diaryl-1,6-heptadien-3,5-dion-Derivat der Formel (3), und/oder ein erfindungsgemäßes 1,7-Diaryl-1,6-heptadien-3,5-dion-Derivat der Formel (3a) weist nach Bestrahlung mit elektromagnetischer Strahlung geeigneter Wellenlänge und Energiedichte eine hohe Ausbeute an Singulett-Sauerstoff auf.

Bei dem erfindungsgemäßen Verfahren und/oder der erfindungsgemäßen Verwendung liegt vorzugsweise die elektromagnetische Strahlung im sichtbaren Spektralbereich, ultravioletten und/oder infraroten Bereich. Weiter bevorzugt weist die elektromagnetische Strahlung eine Wellenlänge aus einem Bereich von 280 bis 1000 nm, weiter bevorzugt von 380 bis 1000 nm, auf.

Weiter bevorzugt weist die elektromagnetische Strahlung eine Energiedichte aus einem Bereich von 1 µW/cm² bis 1 kW/cm², weiter bevorzugt von 1 mW/cm² bis 100 W/cm², weiter bevorzugt von 2 mW/cm² bis 50 W/cm², weiter bevorzugt von 6 mW/cm² bis 30 W/cm², weiter bevorzugt von 7 mW/cm² bis 25 W/cm², auf.

Die Bestrahlungszeit kann in Abhängigkeit von der Art der Mikroorganismen und/oder der Schwere der Infektion variiert werden. Vorzugsweise liegt die Bestrahlungszeit in einem Bereich von 1 µs bis 1 h, weiter bevorzugt von 1 ms bis 1000 s.

Beispielsweise kann zur Bestrahlung eine in der WO 96/29943 A1, der EP 0 437 183 B1 oder der WO 2013/172977 A1 beschriebene Bestrahlungsvorrichtung verwendet werden.

Vorzugsweise umfasst die Bestrahlungsvorrichtung weiterhin eine Vorrichtung zur Abgabe der wenigstens einen Sauerstoff-haltigen Verbindung, vorzugsweise Peroxid, und/oder des wenigstens einen Sauerstoff-haltigen Gases, vorzugsweise von Sauerstoff

Vorzugsweise wird die elektromagnetische Strahlung durch eine Strahlungsquelle erzeugt, die aus der Gruppe, die aus künstlichen Strahlungsquellen, beispielsweise UV-Lampe, IR-Lampe, Leuchtstofflampen, Leuchtdioden, Laser oder chemisches Licht, besteht, ausgewählt wird.

Darüber hinaus haben die Erfinder überraschenderweise festgestellt, dass ein erfindungsgemäß zu verwendendes 1,7-Diaryl-1,6-heptadien-3,5-dion-Derivat der Formel (100), und/oder der Formel (101), und/oder der Formel (102), und/oder ein erfindungsgemäßes 1,7-Diaryl-1,6-heptadien-3,5-dion-Derivat der Formel (1), und/oder ein erfindungsgemäßes 1,7-Diaryl-1,6-heptadien-3,5-dion-Derivat der Formel (2), und/oder ein erfindungsgemäßes 1,7-Diaryl-1,6-heptadien-3,5-dion-Derivat der Formel (3), und/oder ein erfindungsgemäßes 1,7-Diaryl-1,6-heptadien-3,5-dion-Derivat der Formel (3a) und/oder jeweils pharmakologisch verträgliche Salze und/oder Ester und/oder Komplexe davon vorzugsweise eine hohe Affinität zu Mikroorganismen aufweist.

Auf Grund der Affinität kann das erfindungsgemäß zu verwendende wenigstens eine 1,7-Diaryl-1,6-heptadien-3,5-dion-Derivat der Formel (100), und/oder der Formel (101), und/oder der Formel (102), und/oder das wenigstens eine erfindungsgemäße 1,7-Diaryl-1,6-heptadien-3,5-dion-Derivat der Formel (1), und/oder der Formel (2), und/oder der Formel (3), und/oder der Formel (3a) und/oder jeweils pharmakologisch verträgliche Salze und/oder Ester und/oder Komplexe davon an Mikroorganismen effektiv binden und lokal ausreichend Singulett-Sauerstoff erzeugen, um die Mikroorganismen zu inaktivieren, vorzugsweise abzutöten.

Bei einer bevorzugten Verwendung als Photosensibilisator wird wenigstens ein erfindungsgemäß zu verwendendes 1,7-Diaryl-1,6-heptadien-3,5-dion-Derivat der Formel (100), und/oder der Formel (101), und/oder der Formel (102), und/oder wenigstens ein erfindungsgemäßes 1,7-Diaryl-1,6-heptadien-3,5-dion-Derivat der Formel (1), und/oder der Formel (2), und/oder der Formel (3), und/oder der Formel (3a) und/oder jeweils pharmakologisch verträgliche Salze und/oder Ester und/oder Komplexe davon von Mikroorganismen gebunden. Nach Bestrahlung mit elektromagnetischer Strahlung geeigneter Wellenlänge und Energiedichte werden die Mikroorganismen durch die entstandenen reaktiven Sauerstoffspezies (ROS), vorzugsweise Sauerstoffradikale und/oder Singulett-Sauerstoff, inaktiviert, vorzugsweise abgetötet.

Vorzugsweise erlaubt die Bindung wenigstens eines erfindungsgemäß zu verwendendes 1 ,7-Diaryl-1,6-heptadien-3,5-dion-Derivat der Formel (100), und/oder der Formel (101), und/oder der Formel (102), und/oder wenigstens eines erfindungsgemäßen 1,7-Diaryl-1,6-heptadien-3,5-dion-Derivat der Formel (1), und/oder der Formel (2), und/oder der Formel (3), und/oder der Formel (3a) und/oder jeweils pharmakologisch verträgliche Salze und/oder Ester und/oder Komplexe davon an Mikroorganismen ebenfalls eine Anfärbung oder Lokalisierung von Mikroorganismen. Dadurch kann vorzugsweise auch der Verlauf der Anaktivierung von Mikroorganismen oder der Dekolonisation verfolgt werden. Erfindungsgemäß wird unter dem Begriff "Dekolonisation" das Entfernen, vorzugsweise vollständige Entfernen, von Mikroorganismen verstanden.

Bei einer weiteren bevorzugten Ausführungsform wird wenigstens ein erfindungsgemäß zu verwendendes 1,7-Diaryl-1,6-heptadien-3,5-dion-Derivat der Formel (100), und/oder der Formel (101), und/oder der Formel (102), und/oder wenigstens ein erfindungsgemäßes 1,7-Diaryl-1,6-heptadien-3,5-dion-Derivat der Formel (1), und/oder der Formel (2), und/oder der Formel (3), und/oder der Formel (3a) und/oder jeweils pharmakologisch verträgliche Salze und/oder Ester und/oder Komplexe davon bei der Inaktivierung von einzelligen oder mehrzelligen Mikroorganismen, die vorzugsweise aus der Gruppe, die aus Viren, Archäeen, Bakterien, Bakteriensporen, Pilzen, beispielsweise Myzelpilze und Hefen, Pilzsporen, Protozoen, Algen und blutübertragbaren Parasiten besteht, ausgewählt werden, verwendet.

Vorzugsweise können Körperoberflächen, beispielsweise Haut oder Schleimhaut, von Menschen und Tieren, vorzugsweise Säugetieren, behandelt werden. Bei dieser bevorzugten Ausführungsform wird wenigstens ein erfindungsgemäß zu verwendendes 1 ,7-Diaryl-1,6-heptadien-3,5-dion-Derivat der Formel (100), und/oder der Formel (101), und/oder der Formel (102), und/oder wenigstens ein erfindungsgemäßes 1,7-Diaryl-1,6-heptadien-3,5-dion-Derivat der Formel (1), und/oder der Formel (2), und/oder der Formel (3), und/oder der Formel (3a) und/oder jeweils pharmakologisch verträgliche Salze und/oder Ester und/oder Komplexe davon, vorzugsweise in einer pharmazeutischen Zubereitung, bei der Entkeimung und/oder Dekolonisierung von Haut- oder Weichteiloberflächen verwendet, wobei vorzugsweise die Hautintegrität erhalten bleibt.

Bei einer weiter bevorzugten Ausführungsform ist wenigstens ein erfindungsgemäß zu verwendendes 1,7-Diaryl-1,6-heptadien-3,5-dion-Derivat der Formel (100), und/oder der Formel (101), und/oder der Formel (102), und/oder wenigstens ein erfindungsgemäßes 1,7-Diaryl-1,6-heptadien-3,5-dion-Derivat der Formel (1), und/oder der Formel (2), und/oder der Formel (3), und/oder der Formel (3a) und/oder jeweils pharmakologisch verträgliche Salze und/oder Ester und/oder Komplexe davon in einer pharmazeutischen Zubereitung zur lokalen und/oder topischen, vorzugsweise nasalen, oralen, analen, vaginalen oder dermalen, Applikation vorhanden.

Unter topischer Applikation wird auch die Anwendung am oder im Ohr, vorzugsweise Außenohr, verstanden. Das Außenohr umfasst den Ohrknorpel, die Ohrmuschel, das Ohrläppchen, den äußeren Gehörgang oder auch Ohrkanal und die Außenseite des Trommelfells.

Unter topischer Applikation wird auch die Anwendung an oder in der Nase und/oder den Nasennebenhöhlen, wie beispielsweise der Kieferhöhle, der Stirnhöhle und/oder Keilbeinhöhle, verstanden.

Unter topischer Applikation wird auch die Anwendung auf der Oberfläche des Auges, vorzugsweise der äußeren, apikalen Seite der Epithelschicht der Hornhaut, und/oder der aüßeren Oberfläche der Anhangsorgane des Auges, vorzugsweise des Tränenapparates, der Bindehaut und/oder der Augenlider, verstanden.

Unter topischer Applikation wird auch die Anwendung auf die äußere, apikale Seite des Epithels von Hohlorganen, beispielsweise der Speiseröhre, den Magen-DarmTrakt, der Gallenblase, der Gallengänge, des Kehlkopfs, der Luftröhre, der Bronchien, der Eileiter, der Gebärmutter, der Vagina, der Harnleiter, der Harnblase, oder der Harnröhre, verstanden.

Unter topischer Applikation wird auch die Anwendung an oder in Zähnen, beispielsweise in einem Wurzelkanal und/oder einer Wurzelkavität und/oder Zahnfissur, oder Zahnfleischtaschen und/oder Knochentaschen verstanden.

Bei einer weiteren bevorzugten Ausführungsform wird wenigstens ein erfindungsgemäß zu verwendendes 1,7-Diaryl-1,6-heptadien-3,5-dion-Derivat der Formel (100), und/oder der Formel (101), und/oder der Formel (102), und/oder wenigstens ein erfindungsgemäßes 1,7-Diaryl-1,6-heptadien-3,5-dion-Derivat der Formel (1), und/oder der Formel (2), und/oder der Formel (3), und/oder der Formel (3a) und/oder jeweils pharmakologisch verträgliche Salze und/oder Ester und/oder Komplexe davon zur Herstellung einer pharmazeutischen Zubereitung bei der Prophylaxe und/oder Behandlung einer infektiösen, vorzugsweise viralen, bakteriellen und/oder mykotischen, Hautkrankheit, die vorzugsweise ausgewählt wird aus der Gruppe, die aus Staphylococcal scalded skin syndrome, Impetigo, Hautabszess, Furunkel, Karbunkel, Phlegmone, Cellulitis, Akute Lymphadenitis, Pilonidalzyste, Pyodermie, Dermatitis purulenta, Dermatitis septica, Dermatitis suppurativa, Erythrasma, Erysipelas, Akne vulgaris oder Pilzinfektion besteht, verwendet.

Bei einer weiteren bevorzugten Ausführungsform wird wenigstens ein erfindungsgemäß zu verwendendes 1,7-Diaryl-1,6-heptadien-3,5-dion-Derivat der Formel (100), und/oder der Formel (101), und/oder der Formel (102), und/oder wenigstens ein erfindungsgemäßes 1,7-Diaryl-1,6-heptadien-3,5-dion-Derivat der Formel (1), und/oder der Formel (2), und/oder der Formel (3), und/oder der Formel (3a) und/oder jeweils pharmakologisch verträgliche Salze und/oder Ester und/oder Komplexe davon zur Herstellung einer pharmazeutischen Zubereitung bei der Wundheilung, beispielsweise bei Heilungsstörungen nach chirurgischen Interventionen, verwendet.

Vorzugsweise wird wenigstens ein erfindungsgemäß zu verwendendes 1,7-Diaryl-1,6-heptadien-3,5-dion-Derivat der Formel (100), und/oder der Formel (101), und/oder der Formel (102), und/oder wenigstens ein erfindungsgemäßes 1,7-Diaryl-1,6-heptadien-3,5-dion-Derivat der Formel (1), und/oder der Formel (2), und/oder der Formel (3), und/oder der Formel (3a) und/oder jeweils pharmakologisch verträgliche Salze und/oder Ester und/oder Komplexe davon bzw. eine pharmazeutische Zubereitung enthaltend wenigstens ein erfindungsgemäß zu verwendendes 1 ,7-Diaryl-1,6-heptadien-3,5-dion-Derivat der Formel (100), und/oder der Formel (101), und/oder der Formel (102), und/oder wenigstens ein erfindungsgemäßes 1,7-Diaryl-1,6-heptadien-3,5-dion-Derivat der Formel (1), und/oder der Formel (2), und/oder der Formel (3), und/oder der Formel (3a) und/oder jeweils pharmakologisch verträgliche Salze und/oder Ester und/oder Komplexe davon bei der Entkeimung und/oder Reduktion der Keimzahl in infizierten Wunden verwendet.

Bei einer weiteren bevorzugten Ausführungsform wird wenigstens ein erfindungsgemäß zu verwendendes 1,7-Diaryl-1,6-heptadien-3,5-dion-Derivat der Formel (100), und/oder der Formel (101), und/oder der Formel (102), und/oder wenigstens ein erfindungsgemäßes 1,7-Diaryl-1,6-heptadien-3,5-dion-Derivat der Formel (1), und/oder der Formel (2), und/oder der Formel (3), und/oder der Formel (3a) und/oder jeweils pharmakologisch verträgliche Salze und/oder Ester und/oder Komplexe davon zur Herstellung einer pharmazeutischen Zubereitung bei der Prophylaxe und/oder Behandlung von infektiösen, vorzugsweise viralen, bakteriellen und/oder mykotischen, Erkrankungen des Ohrs, der oberen Luftwege, der Mundhöhle, des Rachens, des Kehlkopfes, der unteren Luftwege und/oder der Speiseröhre verwendet.

Das Vorherrschen pathogener Mikroorganismen ist beispielsweise die Hauptursache für Infektionen in der Mundhöhle. Dabei tritt das Problem auf, dass die Mikroorganismen in äußerst komplex aufgebauten Biofilmen synergetisch organisiert sind. Diese Biofilme, beispielsweise Plaque oder Zahnbelag, bestehen aus mehreren, komplex aufgebauten Schichten und enthalten Eiweiße, Kohlenhydrate, Phosphate und Mikroorganismen. Zahnbelag entsteht besonders dort, wo Zahnflächen nicht durch natürliche oder künstliche Reinigung belagfrei gehalten werden können. Dieser Umstand macht es schwierig, einen Zugang zu den im Biofilm eingebundenen Mikroorganismen zu finden.

Konventionelle Therapien, wie beispielsweise Antibiotika und Spüllösungen oder mechanische Zahnreinigung, können nur begrenzt eingesetzt werden, da sie entweder die Bakterien nicht direkt beeinflussen, beispielsweise bei der Zahnreinigung, nur schwer dosiert und appliziert werden können, beispielsweise bei Antibiotika und Spüllösungen, oder eine generelle Anwendung aufgrund von negativen Begleiterscheinungen nicht zu rechtfertigen ist.

Beispielsweise werden in den Vereinigten Staaten jährlich 20 Millionen Wurzelkanäle behandelt, wobei etwa mehr als 2 Millionen endodontische Wiederbehandlungen durchgeführt werden, die sich durch eine verbesserte Entkeimung der Wurzelkanäle vermeiden ließen.

Vorzugsweise eignet sich das erfindungsgemäße Verfahren und die erfindungsgemäße Verwendung zu einer wirkungsvollen Eliminierung von Mikroorganismen im Wurzelkanalsystem eines humanen Zahnes, wobei die Wurzelkanäle und Dentinkanälchen verstanden werden.

Bei einer bevorzugten Ausführungsform wird wenigstens ein erfindungsgemäß zu verwendendes 1 ,7-Diaryl-1,6-heptadien-3,5-dion-Derivat der Formel (100), und/oder der Formel (101), und/oder der Formel (102), und/oder wenigstens ein erfindungsgemäßes 1,7-Diaryl-1,6-heptadien-3,5-dion-Derivat der Formel (1), und/oder der Formel (2), und/oder der Formel (3), und/oder der Formel (3a) und/oder jeweils pharmakologisch verträgliche Salze und/oder Ester und/oder Komplexe davon als Photosensibilisator bei der photodynamischen Inaktivierung von Mikroorganismen in der Mundhöhle verwendet.

Bei einer weiteren bevorzugten Ausführungsform wird wenigstens ein erfindungsgemäß zu verwendendes 1,7-Diaryl-1,6-heptadien-3,5-dion-Derivat der Formel (100), und/oder der Formel (101), und/oder der Formel (102), und/oder wenigstens ein erfindungsgemäßes 1,7-Diaryl-1,6-heptadien-3,5-dion-Derivat der Formel (1), und/oder der Formel (2), und/oder der Formel (3), und/oder der Formel (3a) und/oder jeweils pharmakologisch verträgliche Salze und/oder Ester und/oder Komplexe davon zur Herstellung einer pharmazeutischen Zubereitung bei der Behandlung und/oder Prophylaxe einer infektiösen, vorzugsweise viralen, bakteriellen und/oder mykotischen, Erkrankung des Zahngewebes, vorzugsweise Plaque, Karies oder Pulpitis, und/oder infektiösen, vorzugsweise viralen, bakteriellen und/oder mykotischen, Erkrankung des Zahnhalteapparates, vorzugsweise Gingivitis, Paradontitis, Endodontitis oder Periimplantitis, verwendet.

Bei einer weiteren bevorzugten Ausführungsform wird wenigstens ein erfindungsgemäß zu verwendendes 1,7-Diaryl-1,6-heptadien-3,5-dion-Derivat der Formel (100), und/oder der Formel (101), und/oder der Formel (102), und/oder wenigstens ein erfindungsgemäßes 1,7-Diaryl-1,6-heptadien-3,5-dion-Derivat der Formel (1), und/oder der Formel (2), und/oder der Formel (3), und/oder der Formel (3a) und/oder jeweils pharmakologisch verträgliche Salze und/oder Ester und/oder Komplexe davon bzw. eine pharmazeutische Zubereitung enthaltend wenigstens ein erfindungsgemäß zu verwendendes 1,7-Diaryl-1,6-heptadien-3,5-dion-Derivat der Formel (100), und/oder der Formel (101), und/oder der Formel (102), und/oder wenigstens ein erfindungsgemäßes 1,7-Diaryl-1,6-heptadien-3,5-dion-Derivat der Formel (1), und/oder der Formel (2), und/oder der Formel (3), und/oder der Formel (3a) und/oder jeweils pharmakologisch verträgliche Salze und/oder Ester und/oder Komplexe davon bei der Reinigung von Zähnen, Zahnersatz und/oder Zahnspangen verwendet.

Bei einer weiteren bevorzugten Ausführungsform wird wenigstens ein erfindungsgemäß zu verwendendes 1,7-Diaryl-1,6-heptadien-3,5-dion-Derivat der Formel (100), und/oder der Formel (101), und/oder der Formel (102), und/oder wenigstens ein erfindungsgemäßes 1,7-Diaryl-1,6-heptadien-3,5-dion-Derivat der Formel (1), und/oder der Formel (2), und/oder der Formel (3), und/oder der Formel (3a) und/oder jeweils pharmakologisch verträgliche Salze und/oder Ester und/oder Komplexe davon bzw. eine pharmazeutische Zubereitung enthaltend wenigstens ein erfindungsgemäß zu verwendendes 1,7-Diaryl-1,6-heptadien-3,5-dion-Derivat der Formel (100), und/oder der Formel (101), und/oder der Formel (102), und/oder wenigstens ein erfindungsgemäßes 1,7-Diaryl-1,6-heptadien-3,5-dion-Derivat der Formel (1), und/oder der Formel (2), und/oder der Formel (3), und/oder der Formel (3a) und/oder jeweils pharmakologisch verträgliche Salze und/oder Ester und/oder Komplexe davon bei der nasalen Dekolonisierung von Mikroorganismen verwendet.

Beispielsweise besitzen Methicillin-resistente Staphylococcus aureus (MRSA)-Stämme eine monatelange Persistenz bei nasaler Kolonisierung sowie eine hohe Umweltresistenz. Daher reduziert eine nasale Dekolonisierung, d.h. Entfernung der Mikroorganismen, in der Regel auch die Kolonisation an anderen Körperstellen.

Des Weiteren betrifft die vorliegende Erfindung eine pharmazeutische Zusammensetzung enthaltend wenigstens ein erfindungsgemäß zu verwendendes 1 ,7-Diaryl-1,6-heptadien-3,5-dion-Derivat der Formel (100), und/oder der Formel (101), und/oder der Formel (102), und/oder wenigstens ein erfindungsgemäßes 1,7-Diaryl-1,6-heptadien-3,5-dion-Derivat der Formel (1), und/oder der Formel (2), und/oder der Formel (3), und/oder der Formel (3a) und/oder jeweils pharmakologisch verträgliche Salze und/oder Ester und/oder Komplexe davon und einen oder mehrere physiologisch annehmbare Hilfsstoff(e).

Vorzugsweise umfasst die pharmazeutische Zusammensetzung wenigstens ein erfindungsgemäßes 1,7-Diaryl-1,6-heptadien-3,5-dion-Derivat der Formel (1), und/oder der Formel (2), und/oder der Formel (3), und/oder der Formel (3a) und/oder jeweils pharmakologisch verträgliche Salze und/oder Ester und/oder Komplexe davon und einen oder mehrere physiologisch annehmbare Hilfsstoff(e).

Vorzugsweise wird die pharmazeutische Zusammensetzung durch Mischen von mindestens einer Verbindung der Formel (100) und/oder der Formel (101) und/oder der Formel (102) und/oder der Formel (1) und/oder der Formel (2) und/oder der Formel (3), und/oder der Formel (3a) oder jeweils pharmakologisch verträglicher Salze und/oder Ester und/oder Komplexe davon mit einem oder mehreren physiologisch annehmbaren Hilfsstoff(en) hergestellt und in eine geeignete Darreichungsform gebracht.

Eine geeignete Darreichungsformen der erfindungsgemäßen pharmazeutischen Zusammensetzung wird vorzugsweise aus der Gruppe, die aus Salbe, Creme, Gel, Lotion, Schüttelmixtur, Lösung, beispielsweise in Tropfen- oder Sprayform, Puder, Mikrokapsel und Paste besteht, ausgewählt.

Die erfindungsgemäße pharmazeutische Zusammensetzung kann lokal oder topisch, vorzugsweise nasal, oral, anal, vaginal oder dermal, angewendet werden.

Als physiologisch annehmbare Hilfsstoffe kommen die pharmazeutisch üblichen flüssigen oder festen Füllstoffe und Streckmittel, Lösemittel, Emulgatoren, Gleitstoffe, Geschmackskorrigentien, Farbstoffe und/oder Puffersubstanzen in Frage.

Vorzugsweise ist der physiologisch annehmbaren Hilfsstoff Polyvinylpyrrolidon (PVP), Cyclodextrin, PolyethylenGlykol (PEG) oder eine Mischungen davon.

Weiter bevorzugt umfasst die pharmazeutischen Zusammensetzung Liposomen.

Bei einer weiteren bevorzugten Ausführungsform enthält die pharmazeutische Zusammensetzung eine effektive Menge wenigstens eines erfindungsgemäß zu verwendenden 1 ,7-Diaryl-1,6-heptadien-3,5-dion-Derivat der Formel (100), und/oder der Formel (101), und/oder der Formel (102), und/oder wenigstens eines erfindungsgemäßen 1,7-Diaryl-1,6-heptadien-3,5-dion-Derivat der Formel (1), und/oder der Formel (2), und/oder der Formel (3), und/oder der Formel (3a) und/oder jeweils pharmakologisch verträglicher Salze und/oder Ester und/oder Komplexe davon, wobei die effektive Menge jeweils von 0,01 µg bis 1000 µg pro Gramm der Zusammensetzung, vorzugsweise jeweils von 0,1 µg bis 500 µg pro Gramm der Zusammensetzung, umfasst.

Bei einer bevorzugten Ausführungsform der Erfindung umfasst die pharmazeutische Zusammensetzung wenigstens ein erfindungsgemäß zu verwendendes 1,7-Diaryl-1,6-heptadien-3,5-dion-Derivat der Formel (100), und/oder der Formel (101), und/oder der Formel (102), und/oder wenigstens ein erfindungsgemäßes 1,7-Diaryl-1,6-heptadien-3,5-dion-Derivat der Formel (1), und/oder der Formel (2), und/oder der Formel (3), und/oder der Formel (3a) und/oder jeweils pharmakologisch verträgliche Salze und/oder Ester und/oder Komplexe davon und wenigstens einen weiteren pharmazeutisch aktiven Bestandteil.

Vorzugsweise wird der wenigstens eine weitere pharmazeutisch aktive Bestandteil aus der Gruppe, die aus Antibiotika, Antimikotika, Virustatika, Antihistaminika, Sympathomimetika, Antihämorrhagika, Emmolientia und Hautschutzmittel, Analgetika, Desinfektionsmittel, Immunsera und Immunglobuline, Antiparasitäre Substanzen, Insektizide, Repellenzien und Corticosteroide besteht, ausgewählt.

Bei einer weiteren bevorzugten Ausführungsform wird wenigstens ein erfindungsgemäß zu verwendendes 1,7-Diaryl-1,6-heptadien-3,5-dion-Derivat der Formel (100), und/oder der Formel (101), und/oder der Formel (102), und/oder wenigstens ein erfindungsgemäßes 1,7-Diaryl-1,6-heptadien-3,5-dion-Derivat der Formel (1), und/oder der Formel (2), und/oder der Formel (3), und/oder der Formel (3a) und/oder jeweils pharmakologisch verträgliche Salze und/oder Ester und/oder Komplexe davon bzw. eine pharmazeutische Zubereitung enthaltend wenigstens ein erfindungsgemäß zu verwendendes 1,7-Diaryl-1,6-heptadien-3,5-dion-Derivat der Formel (100), und/oder der Formel (101), und/oder der Formel (102), und/oder wenigstens ein erfindungsgemäßes 1,7-Diaryl-1,6-heptadien-3,5-dion-Derivat der Formel (1), und/oder der Formel (2), und/oder der Formel (3), und/oder der Formel (3a) und/oder jeweils pharmakologisch verträgliche Salze und/oder Ester und/oder Komplexe davon durch den Anwender selbst aufgebracht und, optional, nachfolgend mit einer geeigneten Strahlenquelle, die elektromagnetische Strahlung geeigneter Wellenlänge und Energiedichte erzeugt, bestrahlt.

Bei einer weiteren bevorzugten Ausführungsform wird wenigstens ein erfindungsgemäß zu verwendendes 1,7-Diaryl-1,6-heptadien-3,5-dion-Derivat der Formel (100), und/oder der Formel (101), und/oder der Formel (102), und/oder wenigstens ein erfindungsgemäßes 1,7-Diaryl-1,6-heptadien-3,5-dion-Derivat der Formel (1), und/oder der Formel (2), und/oder der Formel (3), und/oder der Formel (3a) und/oder jeweils pharmakologisch verträgliche Salze und/oder Ester und/oder Komplexe davon bzw. eine Zubereitung enthaltend wenigstens ein erfindungsgemäß zu verwendendes 1,7-Diaryl-1,6-heptadien-3,5-dion-Derivat der Formel (100), und/oder der Formel (101), und/oder der Formel (102), und/oder wenigstens ein erfindungsgemäßes 1,7-Diaryl-1,6-heptadien-3,5-dion-Derivat der Formel (1), und/oder der Formel (2), und/oder der Formel (3), und/oder der Formel (3a) und/oder jeweils pharmakologisch verträgliche Salze und/oder Ester und/oder Komplexe davon bei der Inaktivierung von Mikroorganismen in einem biologischen Flüssigkeiten, vorzugsweise medizinischen Blutprodukten, verwendet.

Geeignete Vorrichtungen zur Bestrahlung einer biologischen Flüssigkeit sind dem Fachmann bekannt und werden beispielsweise in der WO 99/43790 A1, US 2009/0010806 A1 oder der WO 2010/141564 A2 beschrieben.

Geeignete biologische Flüssigkeiten umfassen beispielsweise Blut und Blutprodukte, einschließlich gefrorenem Frischplasma, Erythrozyten-Konzentrat, Thrombozyten-Konzentrat, Granulozyten-Konzentrat, thrombozytenreiches Plasma, Stammzellpräparate, Konzentrate von einzelnen Gerinnungsfaktoren, Humanalbumin, Immunglobuline, Fibrinkleber, Antithrombin, Protein C, Protein S, Fibrinolytika oder Kombinationen davon.

Bei einer bevorzugten Ausführungsform wird wenigstens ein erfindungsgemäß zu verwendendes 1 ,7-Diaryl-1,6-heptadien-3,5-dion-Derivat der Formel (100), und/oder der Formel (101), und/oder der Formel (102), und/oder wenigstens ein erfindungsgemäßes 1,7-Diaryl-1,6-heptadien-3,5-dion-Derivat der Formel (1), und/oder der Formel (2), und/oder der Formel (3), und/oder der Formel (3a) und/oder jeweils pharmakologisch verträgliche Salze und/oder Ester und/oder Komplexe davon zur photodynamischen Entkeimung von Oberflächen aller Art verwendet. Eine photodynamische Entkeimung von Oberflächen bewirkt eine photodynamische Inaktivierung von Mikroorganismen auf der behandelten Oberfläche.

Geeignete Oberflächen umfassen beispielsweise Oberflächen aus Kunststoff, Metall, Glas, Textilien, Holz, Stein oder Kombinationen davon.

Weiter bevorzugt wird das erfindungsgemäße 1,7-Diaryl-1,6-heptadien-3,5-dion-Derivat der Formel (1) und/oder der Formel (3), und/oder der Formel (3a) oder jeweils ein pharmakologisch verträgliches Salz und/oder Ester und/oder Komplex davon bei der photodynamische Entkeimung, Oberflächenreinigung und/oderbeschichtung, vorzugsweise von Medizinprodukten, elektronische Geräte, Hygieneartikeln, Lebensmittelverpackungen, Lebensmitteln, Möbeln, Baustoffen, oder Räumen verwendet.

Weiter bevorzugt wird wenigstens ein erfindungsgemäß zu verwendendes 1,7-Diaryl-1,6-heptadien-3,5-dion-Derivat der Formel (100), und/oder der Formel (101), und/oder der Formel (102), und/oder wenigstens ein erfindungsgemäßes 1,7-Diaryl-1,6-heptadien-3,5-dion-Derivat der Formel (1), und/oder der Formel (2), und/oder der Formel (3), und/oder der Formel (3a) und/oder jeweils pharmakologisch verträgliche Salze und/oder Ester und/oder Komplexe davon auf Oberflächen aufgebracht und/oder eingebracht und, optional, nachfolgend mit einer geeigneten Strahlenquelle, die elektromagnetische Strahlung geeigneter Wellenlänge und Energiedichte erzeugt, bestrahlt. Vorzugsweise bewirkt das wenigstens ein erfindungsgemäß zu verwendendes 1,7-Diaryl-1,6-heptadien-3,5-dion-Derivat der Formel (100), und/oder der Formel (101), und/oder der Formel (102), und/oder wenigstens ein erfindungsgemäßes 1,7-Diaryl-1,6-heptadien-3,5-dion-Derivat der Formel (1), und/oder der Formel (2), und/oder der Formel (3), und/oder der Formel (3a) und/oder jeweils pharmakologisch verträgliche Salze und/oder Ester und/oder Komplexe davon während der Bestrahlung eine "Selbstdesinfektion" der Oberfläche.

Die Bestrahlung kann dabei direkt nach der Behandlung der Oberfläche mit wenigstens einem erfindungsgemäß zu verwendendes 1 ,7-Diaryl-1,6-heptadien-3,5-dion-Derivat der Formel (100), und/oder der Formel (101), und/oder der Formel (102), und/oder wenigstens einem erfindungsgemäßen 1,7-Diaryl-1,6-heptadien-3,5-dion-Derivat der Formel (1), und/oder der Formel (2), und/oder der Formel (3), und/oder der Formel (3a) und/oder jeweils pharmakologisch verträglicher Salze und/oder Ester und/oder Komplexe davon, vorzugsweise nach dem Aufbringen des wenigstens einen erfindungsgemäß zu verwendendes 1 ,7-Diaryl-1,6-heptadien-3,5-dion-Derivat der Formel (100), und/oder der Formel (101), und/oder der Formel (102), und/oder wenigstens ein erfindungsgemäßen 1,7-Diaryl-1,6-heptadien-3,5-dion-Derivat der Formel (1), und/oder der Formel (2), und/oder der Formel (3), und/oder der Formel (3a) und/oder jeweils pharmakologisch verträglicher Salze und/oder Ester und/oder Komplexe davon auf die Oberfläche und/oder Einbringen des wenigstens einen erfindungsgemäß zu verwendendes 1 ,7-Diaryl-1,6-heptadien-3,5-dion-Derivat der Formel (100), und/oder der Formel (101), und/oder der Formel (102), und/oder wenigstens einen erfindungsgemäßen 1,7-Diaryl-1,6-heptadien-3,5-dion-Derivat der Formel (1), und/oder der Formel (2), und/oder der Formel (3), und/oder der Formel (3a) und/oder jeweils pharmakologisch verträglicher Salze und/oder Ester und/oder Komplexe davon in die Oberfläche, erfolgen und/oder zu einem späteren Zeitpunkt.

Weiter bevorzugt werden Gegenstände behandelt, die eine thermisch begrenzte Haltbarkeit aufweisen, beispielsweise Gegenstände aus thermoplastischen Kunststoffen, oder von Desinfektionsmitteln angegriffen werden.

Gegenstände, die eine thermisch begrenzte Haltbarkeit aufweisen, können beispielsweise nur unzureichend sterilisiert werden, da sie bei höheren Temperaturen ihre Form verlieren oder spröde werden.

Darüber hinaus kann es bei einer unsachgemäßen und/oder übermäßigen Anwendung von Desinfektionsmitteln zu Resistenzbildung durch Selektion robuster Mikroorganismen kommen, wenn beispielsweise die Wirkstoffkonzentration und Einwirkzeit und damit die keimreduzierende Wirkung zu gering ist.

Bei einer weiter bevorzugten Ausführungsform wird das erfindungsgemäße Verfahren zur Vermeidung einer bakteriellen Infektion verwendet, beispielsweise vor einer Implantation oder nach erfolgter Dekolonisierung beispielsweise zur Vorbeugung vor der Neubesiedlung mit krankheitserregenden Mikroorganismen, wie beispielsweise paradontalpathogene Mikroorganismen.

Zur Vermeidung von Infektionen durch Mikroorganismen kann das erfindungsgemäße Verfahren ebenfalls zur Dekolonisierung von Oberflächen verwendet werden.

Beispielsweise führt der Kontakt von immunsupprimierten Patienten mit kontaminierten Gegenständen häufig zur Ausbildung einer Infektion, da immunsupprimierte Patienten in der Regel anfällig sind gegenüber Infektionen, beispielsweise auch von geringen Keimzahlen. Insbesondere Oberflächen von Medizinprodukten, vorzugweise medizinischen Hilfsmitteln oder zahnmedizinischen Hilfsmitteln, weiter bevorzugt invasiven medizinischen Hilfsmitteln wie etwa Kathetern, Hohlsonden, Schläuchen oder Nabeln, müssen vor dem Eindringen in den menschlichen Körper desinfiziert werde.

Daher wird in einer weiteren bevorzugten Ausführungsform wenigstens ein erfindungsgemäß zu verwendendes 1,7-Diaryl-1,6-heptadien-3,5-dion-Derivat der Formel (100), und/oder der Formel (101), und/oder der Formel (102), und/oder wenigstens ein erfindungsgemäßes 1,7-Diaryl-1,6-heptadien-3,5-dion-Derivat der Formel (1), und/oder der Formel (2), und/oder der Formel (3), und/oder der Formel (3a) und/oder jeweils pharmakologisch verträgliche Salze und/oder Ester und/oder Komplexe davon zur Inaktivierung von Mikroorganismen auf Oberflächen von Medizinprodukten, vorzugsweise invasiven medizinischen Hilfsmitteln wie etwa Kontaktlinsen, chirurgische Instrumente, zahnmedizinische Bohrer, Mundspiegel, Küretten, zahnmedizinische Feilen, Kathetern, Hohlsonden, Schläuchen oder Nadeln, verwendet.

Vorzugsweise werden die Medizinprodukte aus Wundauflagen, Verbände, chirurgische Instrumenten, Kathetern, Hohlsonden, Schläuchen oder Nadeln ausgewählt.

Weiter bevorzugt werden unter Medizinprodukten auch zahnärztliche Abdrücke, Abdrucklöffel, Aufbissschienen oder Zahnersatz, beispielsweise Prothesen, Kronen oder Implantate, verstanden.

Vorzugsweise wird durch eine Behandlung der Oberfläche von Gegenständen aller Art mit wenigstens einem erfindungsgemäß zu verwendenden 1,7-Diaryl-1,6-heptadien-3,5-dion-Derivat der Formel (100), und/oder der Formel (101), und/oder der Formel (102), und/oder wenigstens einem erfindungsgemäßen 1,7-Diaryl-1,6-heptadien-3,5-dion-Derivat der Formel (1), und/oder der Formel (2), und/oder der Formel (3), und/oder der Formel (3a) und/oder jeweils pharmakologisch verträglichen Salzen und/oder Estern und/oder Komplexen davon und/oder Beschichtung und/oder Immobilisierung wenigstens eines erfindungsgemäß verwendeten 1,7-Diaryl-1,6-heptadien-3,5-dion-Derivat der Formel (100), und/oder der Formel (101), und/oder der Formel (102), und/oder wenigstens eines erfindungsgemäßen 1,7-Diaryl-1,6-heptadien-3,5-dion-Derivat der Formel (1), und/oder der Formel (2), und/oder der Formel (3), und/oder der Formel (3a) und/oder jeweils pharmakologisch verträglicher Salze und/oder Ester und/oder Komplexe davon auf der Oberfläche von Medizinprodukten und nachfolgender Bestrahlung mit elektromagnetische Strahlung geeigneter Wellenlänge und Energiedichte die Besiedelung von Mikroorganismen auf der behandelten Oberflächen reduziert, vorzugsweise verhindert.

Vorzugsweise erfolgt die Behandlung der Oberfläche durch Sprühen, Streichen, Spritzen, Sprayen, Tauchen oder Kombinationen davon.

Die Bestrahlung kann dabei direkt nach der Behandlung der Oberfläche mit wenigstens einem erfindungsgemäß zu verwendenden 1 ,7-Diaryl-1,6-heptadien-3,5-dion-Derivat der Formel (100), und/oder der Formel (101), und/oder der Formel (102), und/oder wenigstens einem erfindungsgemäßen 1,7-Diaryl-1,6-heptadien-3,5-dion-Derivat der Formel (1), und/oder der Formel (2), und/oder der Formel (3), und/oder der Formel (3a) und/oder jeweils pharmakologisch verträglicher Salze und/oder Ester und/oder Komplexe davon, vorzugsweise nach dem Aufbringen des wenigstens einen erfindungsgemäß zu verwendenden 1 ,7-Diaryl-1,6-heptadien-3,5-dion-Derivat der Formel (100), und/oder der Formel (101), und/oder der Formel (102), und/oder wenigstens ein erfindungsgemäßen 1,7-Diaryl-1,6-heptadien-3,5-dion-Derivat der Formel (1), und/oder der Formel (2), und/oder der Formel (3), und/oder der Formel (3a) und/oder jeweils pharmakologisch verträglichen Salzes und/oder Esters und/oder Komplexes davon auf die Oberfläche und/oder Einbringen des wenigstens einen erfindungsgemäß zu verwendenden 1 ,7-Diaryl-1,6-heptadien-3,5-dion-Derivat der Formel (100), und/oder der Formel (101), und/oder der Formel (102), und/oder wenigstens eines erfindungsgemäßen 1,7-Diaryl-1,6-heptadien-3,5-dion-Derivat der Formel (1), und/oder der Formel (2), und/oder der Formel (3), und/oder der Formel (3a) und/oder jeweils pharmakologisch verträglichen Salzes und/oder Esters und/oder Komplexes davon in die Oberfläche, erfolgen und/oder zu einem späteren Zeitpunkt, vor oder während der Verwendung des behandelten Gegenstandes, beispielsweise Medizinproduktes.

Bei einer weiter bevorzugten Verwendung des wenigstens einen erfindungsgemäß zu verwendenden 1,7-Diaryl-1,6-heptadien-3,5-dion-Derivat der Formel (100), und/oder der Formel (101), und/oder der Formel (102), und/oder des wenigstens einen erfindungsgemäßen 1,7-Diaryl-1,6-heptadien-3,5-dion-Derivat der Formel (1), und/oder der Formel (2), und/oder der Formel (3), und/oder der Formel (3a) und/oder jeweils pharmakologisch verträglichen Salzes und/oder Esters und/oder Komplexes davon in Wundauflagen und/oder Verbänden, beispielsweise Baumwollgaze, kann während oder nach dem Aufbringen einer Wundauflage und/oder Verbandes, die bzw. der wenigstens ein erfindungsgemäß zu verwendendes 1 ,7-Diaryl-1,6-heptadien-3,5-dion-Derivat der Formel (100), und/oder der Formel (101), und/oder der Formel (102), und/oder wenigstens ein erfindungsgemäßes 1,7-Diaryl-1,6-heptadien-3,5-dion-Derivat der Formel (1), und/oder der Formel (2), und/oder der Formel (3), und/oder der Formel (3a) und/oder jeweils pharmakologisch verträgliche Salze und/oder Ester und/oder Komplexe davon enthält, eine Bestrahlung mit elektromagnetische Strahlung geeigneter Wellenlänge und Energiedichte erfolgen wodurch es nachfolgend zu einer Reduzierung, vorzugsweise Inaktivierung, von Mikroorganismen im Wundbereich oder behandelten Hautpartien kommen.

Bei einer weiteren bevorzugten Ausführungsform umfasst die Wundauflage und/oder Verband neben wenigstens einem erfindungsgemäß zu verwendenden 1 ,7-Diaryl-1,6-heptadien-3,5-dion-Derivat der Formel (100), und/oder der Formel (101), und/oder der Formel (102), und/oder wenigstens einem erfindungsgemäßen 1,7-Diaryl-1,6-heptadien-3,5-dion-Derivat der Formel (1), und/oder der Formel (2), und/oder der Formel (3), und/oder der Formel (3a) und/oder jeweils pharmakologisch verträgliche Salze und/oder Ester und/oder Komplexe davon weitere Bestandteile, vorzugsweise Absorbtionsmittel, beispielsweise Calciumalginat oder Polyurethanschaum, oder weitere pharmazeutisch wirksame Substanzen.

Bei einer weiteren bevorzugten Ausführungsform wird wenigstens ein erfindungsgemäß zu verwendendes 1,7-Diaryl-1,6-heptadien-3,5-dion-Derivat der Formel (100), und/oder der Formel (101), und/oder der Formel (102), und/oder wenigstens ein erfindungsgemäßes 1,7-Diaryl-1,6-heptadien-3,5-dion-Derivat der Formel (1), und/oder der Formel (2), und/oder der Formel (3), und/oder der Formel (3a) und/oder jeweils pharmakologisch verträgliche Salze und/oder Ester und/oder Komplexe davon zur Inaktivierung von Mikroorganismen auf Oberflächen von Lebensmittelverpackungen verwendet.

Geeignete Lebensmittelverpackungen schließen beispielsweise Behälter aus Glas, Metall, Kunststoff, Papier, Karton oder Kombinationen davon ein.

Geeignete Behälter können beispielsweise vor dem Befüllen mit einem Lebensmittel oder Getränk mit wenigstens einem erfindungsgemäß zu verwendenden 1,7-Diaryl-1,6-heptadien-3,5-dion-Derivat der Formel (100), und/oder der Formel (101), und/oder der Formel (102), und/oder wenigstens einem erfindungsgemäßen 1,7-Diaryl-1,6-heptadien-3,5-dion-Derivat der Formel (1), und/oder der Formel (2), und/oder der Formel (3), und/oder der Formel (3a) und/oder jeweils pharmakologisch verträgliche Salze und/oder Ester und/oder Komplexe davon behandelt und nachfolgend mit einer geeigneten Strahlenquelle, die elektromagnetische Strahlung geeigneter Wellenlänge und Energiedichte erzeugt, bestrahlt werden. Anschließend kann das entsprechende Lebensmittel oder Getränk in den entkeimten Behälter gefüllt und der Behälter verschlossen werden.

Bei einer weiteren bevorzugten Ausführungsform wird wenigstens ein erfindungsgemäß zu verwendendes 1,7-Diaryl-1,6-heptadien-3,5-dion-Derivat der Formel (100), und/oder der Formel (101), und/oder der Formel (102), und/oder wenigstens ein erfindungsgemäßes 1,7-Diaryl-1,6-heptadien-3,5-dion-Derivat der Formel (1), und/oder der Formel (2), und/oder der Formel (3), und/oder der Formel (3a) und/oder jeweils pharmakologisch verträgliche Salze und/oder Ester und/oder Komplexe davon zur Inaktivierung von Mikroorganismen auf Oberflächen von Lebensmitteln verwendet.

Curcumin ist ein zugelassener Lebensmittelzusatzstoff mit der E-Nummer E100. Daher sind modifizierte Curcumine, beispielsweise ein erfindungsgemäß zu verwendendes 1 ,7-Diary)-1,6-heptadien-3,5-dion-Derivat der Formel (100), und/oder der Formel (101), und/oder der Formel (102), und/oder ein erfindungsgemäßes 1,7-Diaryl-1,6-heptadien-3,5-dion-Derivat der Formel (1), und/oder der Formel (2), und/oder der Formel (3), und/oder der Formel (3a) und/oder jeweils pharmakologisch verträgliche Salze und/oder Ester und/oder Komplexe davon, vorzugsweise ebenfalls geeignete Lebensmittelzusätze.

Geeignete Lebensmittel umfassen beispielsweise Lebensmittel wie Fleisch, Fisch, Eier, Samen, Saaten, Nüssen, Beeren, Gewürzen, Obst oder Gemüse, die mit pathogenen Bakterien wie etwa Salmonellen, Clostridien, Escherichia coli - oder Camphylobacter - Spezies in Kontakt kommen können. Vorzugsweise können ebenfalls Bruteier photodynamisch entkeimt werden.

Als Magen-Darm-Infektionen wird eine Gruppe von Erkrankungen bezeichnet, die sich hauptsächlich durch Symptome im oberen Magen-Darm-Trakt wie Erbrechen, Durchfälle und Bauchschmerzen äußern. Magen-Darm-Infektionen werden durch Viren, Bakterien oder Parasiten verursacht. Die Erreger werden meist über kontaminiertes Wasser und/oder kontaminierte Lebensmittel aufgenommen.

Zu den bekanntesten Verursachern von Magen-Darm-Infektionen gehören beispielsweise Salmonellen, Campylobacter - Spezies oder Escherichia coli - Spezies wie z. B. enterohämorrhagische Escherichia coli (EHEC). Brechdurchfälle durch Lebensmittelvergiftungen werden vor allem durch Staphylokokken verursacht.

Am häufigsten gelangen Erreger von Magen-Darm-Infektionen, wie beispielsweise Salmonellen, über Lebensmittel in den Verdauungstrakt des Menschen. Die Erfinder haben festgestellt, dass durch das erfindungsgemäße Verfahren Mikroorganismen auf der Oberfläche von Lebensmitteln effizient beseitigen lassen.

Salmonellen sind beispielsweise Bakterien, die weltweit vorkommen. Eine Salmonellen-Erkrankung ist eine typische Lebensmittelinfektion, die Durchfall verursacht. Die Erreger vermehren sich im Magen-Darm-Trakt von Menschen und Tieren. Salmonellen können sich schnell auf ungekühlten Lebensmitteln vermehren. Die Bakterien gelangen unter Umständen auch durch schlechte Küchenhygiene ins Essen, zum Beispiel über verunreinigte Schneidebretter und/oder Messer.

Häufig mit Salmonellen belastete Lebensmittel sind beispielsweise rohe, bzw. nicht vollständig durchgegarte Eier und Eiprodukte, wie beispielsweise Mayonnaise, Cremes oder Salate auf Eierbasis oder roher Kuchenteig. Häufig mit Salmonellen belastete Lebensmittel sind ebenfalls Speiseeis, rohes Fleisch, zum Beispiel rohes Hack oder Tatar, Rohwurstsorten, zum Beispiel Mett oder Salami. Auch pflanzliche Lebensmittel können mit Salmonellen besiedelt sein.

Campylobacter sind beispielsweise weltweit auftretende Bakterien, die ansteckende Durchfall-Erkrankungen auslösen. Campylobacter-Spezies leben vor allem im Verdauungstrakt von Tieren, die meist nicht selbst erkranken. Campylobacter sind in Deutschland die häufigsten bakteriellen Erreger von Durchfall-Erkrankungen.

Die Hauptansteckungsquelle für Campylobacter ist der Verzehr von Lebensmitteln, die mit den Bakterien belastet sind. Häufig erfolgt die Übertragung über Geflügelfleisch. Zwar können sich Campylobacter in Lebensmitteln nicht vermehren, allerdings können Campylobacter einige Zeit in der Umwelt überleben. Auch mangelnde Küchenhygiene kann zu einer Ansteckung führen, beispielsweise über Schneidebretter und/oder Messer, die nach der Zubereitung von rohem Fleisch nicht ausreichend gereinigt werden.

Häufige mit Campylobacter belastet Lebensmittel sind beispielsweise nicht ausreichend erhitztes Geflügelfleisch und Geflügelprodukte, Rohmilch oder Rohmilchprodukte, nicht durchgegartes Hackfleisch oder frische Rohwurstsorten, wie beispielsweise Mettwurst, und verunreinigtes Trinkwasser, zum Beispiel aus einer Brunnenanlage.

Enterohämorrhagische Escherichia coli (EHEC) finden sich im Darm von Wiederkäuern wie Rindern, Schafen, Ziegen, Rehen oder Hirschen. Die Bakterien werden mit dem Kot infizierter Tiere ausgeschieden. Da EHEC relativ unempfindlich sind, können sie in der Umwelt wochenlang überleben. Sie sind hoch ansteckend und schon eine geringe Menge an Keimen reicht für eine Übertragung aus. Das Fell von Rindern und anderen Wiederkäuern kann mit Kotspuren verunreinigt sein. Durch Berühren und Streicheln der Tiere können die Bakterien an die Hände und von dort in den Mund gelangen. Auch das Spielen auf Wiesen, auf denen Wiederkäuer gehalten werden, stellt beispielsweise ein Ansteckungsrisiko für Kinder dar.

Durch Anwendung des erfindungsgemäßen Verfahrens können ebenfalls Oberflächen von Schuhen, beispielsweise Sohlen, auf einfache Weise photodynamisch entkeimt werden.

Weiterhin haben die Erfinder festgestellt, dass das erfindungsgemäße Verfahren auch zur photodynamischen Entkeimung der Oberfläche von Tierprodukten, beispielsweise Fellen, Leder, Haaren, Fasern, oder Wolle, geeignet ist.

Die EHEC-Bakterien können beispielsweise bei mangelnder Händehygiene auch auf berührten Gegenständen haften bleiben und von dort indirekt weiterverbreitet werden.

Eine Übertragung auf den Menschen kann auch durch roh verzehrte oder unzureichend erhitzte Lebensmittel erfolgen. Häufig mit EHEC belastet Lebensmittel sind beispielsweise Rohmilch und Rohmilchprodukte, rohe oder nicht ausreichend durchgegarte Fleischerzeugnisse, wie beispielsweise Rinderhackfleisch (z.B. Hamburger) und streichfähige Rohwurstsorten, beispielsweise Teewurst. Häufig mit EHEC belastet Lebensmittel sindebenfalls pflanzliche Lebensmittel wie Gemüse, das durch Düngen oder über verunreinigtes Wasser mit den Erregern belastet wurden, unpasteurisierte Fruchtsäfte, die aus verunreinigtem Obst hergestellt wurden, Samen, die für das Ziehen von Sprossenkeimlingen verwendet werden, und alle Speisen, auf die der Erreger von verunreinigten Lebensmitteln direkt oder indirekt durch verunreinigte Hände oder Küchenutensilien übertragen wurde.

Clostridium difficile ist beispielsweise ein Bakterium, das weltweit vorkommt. Bei gesunden Menschen ist Clostridium difficile ein harmloses Darmbakterium. Werden konkurrierende Arten der normalen Darmflora durch Antibiotika zurückgedrängt, kann sich Clostridium. difficile vermehren und Toxine produzieren, die zu einer unter Umständen lebensbedrohenden Durchfallserkrankung, beispielsweise Antibiotikaassoziierte Kolitis, führen können, insbesondere wenn bereits vorher eine Antibiotika-assoziierte Diarrhoe eingetreten ist.

Clostridium difficile ist einer der häufigsten Krankenhauskeime (nosokomialen Erreger). Darüber hinaus kann Clostridium difficile widerstandsfähige Dauerformen, sogenannte Sporen, bilden, durch die die Bakterien auch außerhalb des Magen-Darm-Traktes ggf. jahrelang überleben können. Eine Übertragung kann deshalb auch über Gegenstände und Flächen erfolgen, an denen die Erreger haften, wie zum Beispiel Toiletten, Türklinken, Griffe und/oder Handläufe.

Die oben beschriebenen Probleme können bei Anwendung des erfindungsgemäßen Verfahrens vermieden werden, da krankheitsauslösende Erreger auf kontaminierten Flächen nach Anwendung des erfindungsgemäßen Verfahrens wirksam beseitigt werden.

Bei weiteren bevorzugten Ausführungsform wird wenigstens ein erfindungsgemäß zu verwendendes 1,7-Diaryl-1,6-heptadien-3,5-dion-Derivat der Formel (100), und/oder der Formel (101), und/oder der Formel (102), und/oder wenigstens ein erfindungsgemäßes 1,7-Diaryl-1,6-heptadien-3,5-dion-Derivat der Formel (1), und/oder der Formel (2), und/oder der Formel (3), und/oder der Formel (3a) und/oder jeweils pharmakologisch verträgliche Salze und/oder Ester und/oder Komplexe davon zur Inaktivierung von Mikroorganismen in einem Raum, beispielsweise einem Reinraum oder einem Operationssaal verwendet. Nach Einbringen in den Raum, beispielsweise durch Vernebeln, Versprühen, Verspritzen oder Verdampfen, kann der Raum mit einer geeigneten Strahlenquelle, die elektromagnetische Strahlung geeigneter Wellenlänge und Energiedichte erzeugt, bestrahlt werden, wodurch vorhandene Mikroorganismen inaktiviert werden.

Bei weiteren bevorzugten Ausführungsform wird wenigstens ein erfindungsgemäß zu verwendendes 1,7-Diaryl-1,6-heptadien-3,5-dion-Derivat der Formel (100), und/oder der Formel (101), und/oder der Formel (102), und/oder wenigstens ein erfindungsgemäßes 1,7-Diaryl-1,6-heptadien-3,5-dion-Derivat der Formel (1), und/oder der Formel (2), und/oder der Formel (3), und/oder der Formel (3a) und/oder jeweils pharmakologisch verträgliche Salze und/oder Ester und/oder Komplexe davon zur Inaktivierung von Mikroorganismen in einer Flüssigkeit oder flüssige Zubereitungen verwendet. Geeignete Flüssigkeit oder flüssige Zubereitungen sind beispielsweise Dispersionsfarben, Kühlstoffe, Kühlschmierstoffe, Schmierstoffe, Bremsflüssigkeiten, Lacke, Klebstoffe, oder Ölen. Vorzugsweise ist die flüssige Zubereitung eine wässrige Zubereitung.

Vorzugsweise handelt es sich bei der Flüssigkeit um Wasser.

Dabei kann wenigstens ein erfindungsgemäß zu verwendendes 1,7-Diaryl-1,6-heptadien-3,5-dion-Derivat der Formel (100), und/oder der Formel (101), und/oder der Formel (102), und/oder wenigstens ein erfindungsgemäßes 1,7-Diaryl-1,6-heptadien-3,5-dion-Derivat der Formel (1), und/oder der Formel (2), und/oder der Formel (3), und/oder der Formel (3a) und/oder jeweils pharmakologisch verträgliche Salze und/oder Ester und/oder Komplexe davon zur Aufbereitung von Wasser für die Getränke- und Lebensmittelindustrie, die Pharma-, Chemie- und Kosmetikindustrie, die Elektroindustrie verwendet werden. Ferner kann wenigstens ein erfindungsgemäß zu verwendendes 1,7-Diaryl-1,6-heptadien-3,5-dion-Derivat der Formel (100), und/oder der Formel (101), und/oder der Formel (102), und/oder wenigstens ein erfindungsgemäßes 1,7-Diaryl-1,6-heptadien-3,5-dion-Derivat der Formel (1), und/oder der Formel (2), und/oder der Formel (3), und/oder der Formel (3a) und/oder jeweils pharmakologisch verträgliche Salze und/oder Ester und/oder Komplexe davon bei der Trinkwasser- und Regenwasseraufbereitung, der Behandlung von Abwasser oder bei der Aufbereitung von Wasser für den Einsatz in der Klimatechnik eingesetzt werden.

Bei dieser bevorzugten Verwendung des wenigstens einen erfindungsgemäß zu verwendenden 1 ,7-Diaryl-1,6-heptadien-3,5-dion-Derivat der Formel (100), und/oder der Formel (101), und/oder der Formel (102), und/oder des wenigstens einen erfindungsgemäßen 1,7-Diary)-1,6-heptadien-3,5-dion-Derivat der Formel (1), und/oder der Formel (2), und/oder der Formel (3), und/oder der Formel (3a) und/oder jeweils pharmakologisch verträglicher Salze und/oder Ester und/oder Komplexe davon kann die Flüssigkeit oder flüssige Zubereitung nachfolgend mit einer geeigneten Strahlenquelle, die elektromagnetische Strahlung geeigneter Wellenlänge und Energiedichte erzeugt, bestrahlt werden. Vorzugsweise bewirkt das wenigstens eine erfindungsgemäß zu verwendende 1,7-Diaryl-1,6-heptadien-3,5-dion-Derivat der Formel (100), und/oder der Formel (101), und/oder der Formel (102), und/oder das wenigstens eine erfindungsgemäße 1,7-Diaryl-1,6-heptadien-3,5-dion-Derivat der Formel (1), und/oder der Formel (2), und/oder der Formel (3), und/oder der Formel (3a) und/oder jeweils pharmakologisch verträgliche Salze und/oder Ester und/oder Komplexe davon während der Bestrahlung eine "Selbstdesinfektion" der Flüssigkeit oder der flüssigen Zubereitung.

Bei einer weiteren bevorzugten Verwendung des wenigstens einen erfindungsgemäß zu verwendenden 1,7-Diary)-1,6-heptadien-3,5-dion-Derivat der Formel (100), und/oder der Formel (101), und/oder der Formel (102), und/oder des wenigstens einen erfindungsgemäßen 1,7-Diaryl-1,6-heptadien-3,5-dion-Derivat der Formel (1), und/oder der Formel (2), und/oder der Formel (3), und/oder der Formel (3a) und/oder jeweils pharmakologisch verträgliche Salze und/oder Ester und/oder Komplexe davon kann das erfindungsgemäß zu verwendende 1,7-Diaryl-1,6-heptadien-3,5-dion-Derivat der Formel (100), und/oder der Formel (101), und/oder der Formel (102), und/oder das erfindungsgemäße 1,7-Diaryl-1,6-heptadien-3,5-dion-Derivat der Formel (1), und/oder der Formel (2), und/oder der Formel (3), und/oder der Formel (3a) und/oder jeweils pharmakologisch verträgliche Salze und/oder Ester und/oder Komplexe davon an einen festen Träger gebunden vorliegen und so als Teil einer festen Matrix verwendet werden.

Besonders bevorzugt wird wenigstens ein an einen festen Träger gebundenes erfindungsgemäß zu verwendendes 1,7-Diaryl-1,6-heptadien-3,5-dion-Derivat der Formel (100), und/oder der Formel (101), und/oder der Formel (102), und/oder wenigstens ein an einen festen Träger gebundenes erfindungsgemäßes 1,7-Diaryl-1,6-heptadien-3,5-dion-Derivat der Formel (1), und/oder der Formel (2), und/oder der Formel (3), und/oder der Formel (3a) und/oder jeweils pharmakologisch verträgliche Salze und/oder Ester und/oder Komplexe davon in die zu behandelnde Flüssigkeit, vorzugsweise Wasser oder Blut, eingebracht.

Besonders bevorzugt ist als Träger ein Polymer, welches wenigstens ein erfindungsgemäß zu verwendendes 1,7-Diaryl-1,6-heptadien-3,5-dion-Derivat der Formel (100), und/oder der Formel (101), und/oder der Formel (102), und/oder wenigstens ein erfindungsgemäßes 1,7-Diaryl-1,6-heptadien-3,5-dion-Derivat der Formel (1), und/oder der Formel (2), und/oder der Formel (3), und/oder der Formel (3a) und/oder jeweils pharmakologisch verträgliche Salze und/oder Ester und/oder Komplexe davon daran in kovalent gebundener Weise trägt. Diese Zusammensetzung, umfassend den Träger und wenigstens ein erfindungsgemäß zu verwendendes 1,7-Diaryl-1,6-heptadien-3,5-dion-Derivat der Formel (100), und/oder der Formel (101), und/oder der Formel (102), und/oder wenigstens ein erfindungsgemäßes 1,7-Diaryl-1,6-heptadien-3,5-dion-Derivat der Formel (1), und/oder der Formel (2), und/oder der Formel (3), und/oder der Formel (3a) und/oder jeweils pharmakologisch verträgliche Salze und/oder Ester und/oder Komplexe davon, entwickelt eine antimikrobielle Aktivität sobald sie elektromagnetischer Strahlung geeigneter Wellenlänge und Energiedichte ausgesetzt wird.

Des Weiteren betrifft die vorliegende Erfindung einen beschichteten Gegenstand, der wenigstens ein erfindungsgemäß zu verwendendes 1 ,7-Diaryl-1,6-heptadien-3,5-dion-Derivat der Formel (100), und/oder der Formel (101), und/oder der Formel (102), und/oder wenigstens ein erfindungsgemäßes 1,7-Diaryl-1,6-heptadien-3,5-dion-Derivat der Formel (1), und/oder der Formel (2), und/oder der Formel (3), und/oder der Formel (3a) und/oder jeweils pharmakologisch verträgliche Salze und/oder Ester und/oder Komplexe davon enthält und/oder damit beschichtet ist. Vorzugsweise enthält der Gegenstand wenigstens ein erfindungsgemäßes 1,7-Diaryl-1,6-heptadien-3,5-dion-Derivat der Formel (1), und/oder der Formel (2), und/oder der Formel (3), und/oder der Formel (3a) und/oder jeweils pharmakologisch verträgliche Salze und/oder Ester und/oder Komplexe davon und/oder ist damit beschichtet.

Vorzugsweise weist die Oberfläche des beschichteten Gegenstands wenigstens ein erfindungsgemäß zu verwendendes 1,7-Diaryl-1,6-heptadien-3,5-dion-Derivat der Formel (100), und/oder der Formel (101), und/oder der Formel (102), und/oder wenigstens ein erfindungsgemäßes 1,7-Diaryl-1,6-heptadien-3,5-dion-Derivat der Formel (1), und/oder der Formel (2), und/oder der Formel (3), und/oder der Formel (3a) und/oder jeweils pharmakologisch verträgliche Salze und/oder Ester und/oder Komplexe davon auf.

Weiter bevorzugt weist die Oberfläche des beschichteten Gegenstands wenigstens ein erfindungsgemäßes 1,7-Diaryl-1,6-heptadien-3,5-dion-Derivat der Formel (1), und/oder der Formel (2), und/oder der Formel (3), und/oder der Formel (3a) und/oder jeweils pharmakologisch verträgliche Salze und/oder Ester und/oder Komplexe davon auf.

Der beschichtete Gegenstand kann nachfolgend mit einer geeigneten Strahlenquelle, die elektromagnetische Strahlung geeigneter Wellenlänge und Energiedichte erzeugt, bestrahlt werden. Vorzugsweise bewirkt das erfindungsgemäß zu verwendende 1,7-Diaryl-1,6-heptadien-3,5-dion-Derivat der Formel (100), und/oder der Formel (101), und/oder der Formel (102), und/oder das erfindungsgemäße 1,7-Diaryl-1,6-heptadien-3,5-dion-Derivat der Formel (1), und/oder der Formel (2), und/oder der Formel (3), und/oder der Formel (3a) und/oder jeweils pharmakologisch verträgliche Salze und/oder Ester und/oder Komplexe davon während der Bestrahlung eine "Selbst-desinfektion" der Oberfläche des beschichteten Gegenstandes.

Die Bestrahlung kann dabei direkt nach der Behandlung des beschichteten Gegenstandes mit wenigstens einem erfindungsgemäß zu verwendenden 1 ,7-Diaryl-1,6-heptadien-3,5-dion-Derivat der Formel (100), und/oder der Formel (101), und/oder der Formel (102), und/oder wenigstens einem erfindungsgemäßen 1,7-Diaryl-1,6-heptadien-3,5-dion-Derivat der Formel (1), und/oder der Formel (2), und/oder der Formel (3), und/oder der Formel (3a) und/oder jeweils pharmakologisch verträgliche Salze und/oder Ester und/oder Komplexe davon, vorzugsweise nach dem Aufbringen des wenigstens einen erfindungsgemäß zu verwendenden 1 ,7-Diary)-1,6-heptadien-3,5-dion-Derivat der Formel (100), und/oder der Formel (101), und/oder der Formel (102), und/oder des wenigstens einen erfindungsgemäßen 1,7-Diaryl-1,6-heptadien-3,5-dion-Derivat der Formel (1), und/oder der Formel (2), und/oder der Formel (3), und/oder der Formel (3a) und/oder jeweils pharmakologisch verträglicher Salze und/oder Ester und/oder Komplexe davon auf die Oberfläche des beschichteten Gegenstandes und/oder Einbringen des wenigstens einen erfindungsgemäß zu verwendenden 1 ,7-Diaryl-1,6-heptadien-3,5-dion-Derivat der Formel (100), und/oder der Formel (101), und/oder der Formel (102), und/oder des wenigstens einen erfindungsgemäßen 1,7-Diaryl-1,6-heptadien-3,5-dion-Derivat der Formel (1), und/oder der Formel (2), und/oder der Formel (3), und/oder der Formel (3a) und/oder jeweils pharmakologisch verträglicher Salze und/oder Ester und/oder Komplexe davon in die Oberfläche des beschichteten Gegenstandes, erfolgen und/oder zu einem späteren Zeitpunkt, vorzugsweise vor oder während der Verwendung des beschichteten Gegenstandes.

Geeignete Gegenstände sind beispielsweise Medizinprodukte, Lebensmittelverpackungen, Hygieneartikel, Textilien, Handgriffe, Handläufe, Kontaktlinsen, Baustoffe, Geldscheine, Münzen, Spielchips, Karten, Sportgeräte, Textilien, Geschirr, Besteck, oder elektronische Geräte.

Geeignete Gegenstände sind ebenfalls Geräte oder Anlagen mit wasserführenden Leitungen und/oder wasserführenden Behältern, bei denen beispielsweise während des Betriebs des Gerätes oder der Anlage Kondenswasserbildung auftritt.

Geeignete Gegenstände sind beispielsweise Dichtungen, Membranen, Siebe, Filter, Behälter und/oder Rohre von Warmwassererzeugungsanlagen, Warmwasserverteilungsanlagen, Wärmeaustauschern, Klimaanlagen, Luftbefeuchtern, Kühlanlagen, Kühlschränken, Getränkeautomaten, Waschmaschinen oder Trocknern.

Beispielsweise können geringe Mengen von Mikroorganismen trotz Filterung der von Außen zugeführten Luft in eine Klimaanlage gelangen und dort zumindest kurzzeitig existieren. Die Stoffwechselprodukte dieser Mikroorganismen können zu modrigen und muffigen Gerüchen führen.

Weiterhin wird beispielsweise beim Betrieb einer Klimaanlage der Luft Feuchtigkeit entzogen und aufgefangen. Ein Großteil des Kondenswassers wird entfernt und läuft beispielsweise durch einen Kondenswasserablauf ab. Dennoch bleibt eine Restfeuchte auf der Oberfläche des Verdampfers der Klimaanlage zurück, insbesondere dann, wenn die Klimaanlage beispielsweise in einem PKW erst mit dem Abschalten des Motors ausgeschaltet wird und sich die Temperatur nicht mehr ausgleichen kann.

Durch die Luft auf den Verdampfer gelangte Mikroorganismen, beispielsweise Pilzsporen und/oder Bakterien, finden nun ein ideales, feucht-warmes Klima vor und können sich ungehindert ausbreiten.

Da beispielsweise Schimmelpilze ein Gesundheitsrisiko darstellen, sollte die Klimaanlage regelmäßig entkeimt und vorhandene Mikroorganismen durch Anwendung des erfindungsgemäßen Verfahrens abgetötet werden.

Bei einem Wechsel der Filter der Klimaanlage, beispielsweise Staub- und/oder Pollenfilter, kann weiterhin das Filtergehäuse und die angrenzenden Luftkanäle der Klimaanlage durch Anwendung des erfindungsgemäßen Verfahrens gereinigt werden. Mittels der Reinigung des Verdampfers der Klimaanlage durch Anwendung des erfindungsgemäßen Verfahrens können weiterhin Geruchsbelästigungen, die durch die Klimaanlage entstehen, beseitigt werden.

Legionellen sind beispielsweise Bakterien, die beim Menschen unterschiedliche Krankheitsbilder verursachen, beispielsweise grippeartige Beschwerden oder schwere Lungenentzündungen. Legionellen vermehren sich vorzugsweise bei Temperaturen zwischen 25 °C und 45 °C. Besonders in künstlichen Wassersystemen wie Wasserleitungen in Gebäuden finden die Erreger aufgrund der vorherrschenden Temperaturen gute Wachstumsbedingungen. In Ablagerungen und/oder Belägen eines Rohrsystems können sich Legionellen ebenfalls gut vermehren. Daher kann das erfindungsgemäße verfahren beispielsweise in Kombination mit einem Verfahren zur Beseitigung der Ablagerungen und/oder Belägen verwendet werden.

Legionellen werden durch zerstäubtes, vernebeltes Wasser übertragen. Die erregerhaltigen Tröpfchen können sich in der Luft verbreiten und eingeatmet werden. Mögliche Ansteckungsquellen sind beispielsweise Warmwasserversorgungen, insbesondere Duschen, Luftbefeuchter oder Wasserhähne, ebenso Kühltürme oder Klimaanlagen oder sonstige Anlagen, die Wasser zu Wassertröpfchen zerstäuben, beispielsweise Nebelerzeuger, Nebelbrunnen, Zierspringbrunnen oder Ähnliches. Auch in Schwimmbädern ist über Wasserfälle, Rutschen, Warmsprudelbecken (Whirlpools) und/oder Fontänen eine Übertragung möglich. Eine Ansteckung mit Legionellen wird durch Anwendung des erfindungsgemäßen Verfahrens auf Oberflächen kontaminierter Gegenstände verhindert.

Das erfindungsgemäße Verfahren kann beispielsweise in Geräten oder Anlagen mit wasserführenden Leitungen und/oder wasserführenden Behältern, beispielsweise Geräte oder Anlagen, die in der Fischzucht verwendet werden, angewendet werden.

Epidemieartige Fischkrankheiten sind beispielsweise eine große wirtschaftliche Bedrohung für alle intensiv bewirtschafteten Fischfarmen, wo Nutzfische auf engem Raum gehalten werden. Zur Bekämpfung von Fischkrankheiten werden beispielsweise Antibiotika und/oder chemische Additive eingesetzt. Als chemische Additive werden beispielsweise Löschkalk (Calciumhydroxid), Wasserstoffperoxid, Peressigsäurepräparate, Kupfersulfat, Chloramine, Natriumcarbonat, Natriumchlorid oder Formaldehyd verwendet.

Um den Einsatz von Antibiotika und/oder oben genannte chemische Additive zu verringern, kann wenigstens einem erfindungsgemäß zu verwendendes 1,7-Diaryl-1,6-heptadien-3,5-dion-Derivat der Formel (100), und/oder der Formel (101), und/oder der Formel (102), und/oder wenigstens ein erfindungsgemäßes 1,7-Diaryl-1,6-heptadien-3,5-dion-Derivat der Formel (1), und/oder der Formel (2), und/oder der Formel (3), und/oder der Formel (3a) und/oder jeweils pharmakologisch verträgliche Salze und/oder Ester und/oder Komplexe davon zur photodynamischen Entkeimung von Geräten oder Anlagen der Fischzucht, beispielsweise Fischbecken, Teichen, Pumpen, Filtern, Rohren, Netzen, Haken oder Fußmatten, verwendet werden. Ebenso können beispielsweise Fische und/oder Fischeier photodynamisch entkeimt werden.

Ebenso können Terrarien, Aquarium-Behälter, Sand, Kies, und/oder Grünpflanzen vor und/oder während ihrer Verwendung photodynamisch entkeimt werden.

Geeignete elektronische Geräte umfassen beispielsweise Herdplatten, Fernbedienungen, Kopfhöhrer, Freisprecheinrichtungen, Headsets, Mobiltelefone, Bedienelemente, wie Knöpfe, Schalter, Touch-Screens, oder Tastaturen. Geeignete Baustoffe umfassen beispielsweise Beton, Glas, Sand, Kies, Wandverkleidungen, Putz, Estrich oder Ähnliches.

Geeignete Wandverkleidung umfassen beispielsweise Holzvertäfelungen, Fliesen, Massivholzplatten, mitteldichte Holzfaserplatten, Sperrholzplatten, Multiplex-Platten, Faserbetonplatten, Gipskartonplatten, Gipsfaserplatten, Tapeten aus Kunststoff, Schaumstoff und/oder Zellulose

Beispielsweise kann wenigstens ein erfindungsgemäß zu verwendendes 1 ,7-Diaryl-1,6-heptadien-3,5-dion-Derivat der Formel (100), und/oder der Formel (101), und/oder der Formel (102), und/oder wenigstens ein erfindungsgemäßes 1,7-Diaryl-1,6-heptadien-3,5-dion-Derivat der Formel (1), und/oder der Formel (2), und/oder der Formel (3), und/oder der Formel (3a) und/oder jeweils ein pharmakologisches verträgliches Salz und/oder Ester und/oder Komplex davon zur Beseitigung von Schimmelpilzen verwendet werden.

Vorzugsweise wird eine mit Schimmelpilz behaftete Oberfläche mit wenigstens einem erfindungsgemäß zu verwendenden 1 ,7-Diaryl-1,6-heptadien-3,5-dion-Derivat der Formel (100), und/oder der Formel (101), und/oder der Formel (102), und/oder wenigstens einem erfindungsgemäßen 1,7-Diaryl-1,6-heptadien-3,5-dion-Derivat der Formel (1), und/oder der Formel (2), und/oder der Formel (3), und/oder der Formel (3a) und/oder jeweils pharmakologisch verträgliche Salze und/oder Ester und/oder Komplexe davon behandelt und nachfolgend mit einer geeigneten Strahlenquelle, die elektromagnetische Strahlung geeigneter Wellenlänge und Energiedichte erzeugt, bestrahlt werden, wodurch es nachfolgend zu einer Reduzierung, vorzugsweise Inaktivierung, von Schimmelpilz auf der behandelten Oberfläche kommt.

Bei einer weiteren bevorzugten Ausführungsform des beschichteten Gegenstandes handelt es sich um mit wenigstens einem erfindungsgemäß zu verwendenden 1,7-Diaryl-1,6-heptadien-3,5-dion-Derivat der Formel (100), und/oder der Formel (101), und/oder der Formel (102), und/oder wenigstens einem erfindungsgemäßen 1,7-Diaryl-1,6-heptadien-3,5-dion-Derivat der Formel (1), und/oder der Formel (2), und/oder der Formel (3), und/oder der Formel (3a) und/oder jeweils pharmakologisch verträgliche Salze und/oder Ester und/oder Komplexe davon beschichtete Partikel, beispielsweise anorganische oder organische Partikel.

Weiter bevorzugt umfassen die Partikel wenigstens ein erfindungsgemäß zu verwendendes 1 ,7-Diaryl-1,6-heptadien-3,5-dion-Derivat der Formel (100), und/oder der Formel (101), und/oder der Formel (102), und/oder wenigstens ein erfindungsgemäßes 1,7-Diaryl-1,6-heptadien-3,5-dion-Derivat der Formel (1), und/oder der Formel (2), und/oder der Formel (3), und/oder der Formel (3a) und/oder jeweils ein pharmakologisch verträgliches Salz und/oder Ester und/oder Komplex davon, das an die Partikel kovalent gebunden vorliegt.

Bei den vorgenannte bevorzugten Ausführungsform der erfindungsgemäßen Verwendung oder des erfindungsgemäßen Verfahrens erfolgt die Bestrahlung der Mikroorganismen und des wenigstens ein erfindungsgemäß zu verwendendes 1,7-Diaryl-1,6-heptadien-3,5-dion-Derivat der Formel (100), und/oder der Formel (101), und/oder der Formel (102), und/oder wenigstens ein erfindungsgemäßes 1,7-Diaryl-1,6-heptadien-3,5-dion-Derivat der Formel (1), und/oder der Formel (2), und/oder der Formel (3), und/oder der Formel (3a) und/oder jeweils ein pharmakologisch verträgliches Salz und/oder Ester und/oder Komplex davon mit elektromagnetischer Strahlung geeigneter Wellenlänge und Energiedichte in Gegenwart wenigstens einer Sauerstoff-abgebenden Verbindung, vorzugsweise Peroxid, und/oder wenigstens eines Sauerstoff-haltigen Gases, vorzugsweise Sauerstoff.

Die wenigstens eine Sauerstoff-abgebende Verbindung und/oder das wenigstens eine Sauerstoff-haltige Gas können vorzugsweise vor, oder während der Bestrahlung mit elektromagnetischer Strahlung geeigneter Wellenlänge und Energiedichte appliziert werden.

Durch eine zusätzliche Gabe von Sauerstoff in Form wenigstens einer Sauerstoff-haltigen Verbindung und/oder wenigstens eines Sauerstoff-haltigen Gases vor oder während der Bestrahlung der Mikroorganismen und des wenigstens einen Photosensibilisators mit elektromagnetischer Strahlung geeigneter Wellenlänge und Energiedichte wird die Ausbeute an entstandenen reaktiven Sauerstoffspezies (ROS), vorzugsweise Sauerstoffradikale und/oder Singulett-Sauerstoff erhöht.

Erfindungsgemäß kann wenigstens ein erfindungsgemäßes 1,7-Diaryl-1,6-heptadien-3,5-dion-Derivat der Formel (1), und/oder der Formel (2), und/oder der Formel (3), und/oder der Formel (3a) und/oder jeweils pharmakologisch verträgliche Salze und/oder Ester und/oder Komplexe davon als Medikament verwendet werden.

Bei vorgenannten pharmakologisch verträglichen Komplexen handelt es sich vorzugsweise um Einschlusskomplexe des wenigstens einen erfindungsgemäß zu verwendenden 1 ,7-Diaryl-1,6-heptadien-3,5-dion-Derivat der Formel (100), und/oder der Formel (101), und/oder der Formel (102), und/oder des wenigstens einen erfindungsgemäßen 1,7-Diaryl-1,6-heptadien-3,5-dion-Derivat der Formel (1), und/oder der Formel (2), und/oder der Formel (3), und/oder der Formel (3a), vorzugsweise des wenigstens einen erfindungsgemäßen 1,7-Diaryl-1,6-heptadien-3,5-dion-Derivat der Formel (1), und/oder der Formel (2), und/oder der Formel (3) und/oder der Formel (3a), mit Polyvinylpyrrolidon (PVP), Cyclodextrinen oder Mischungen davon.

Ein geeignetes Verfahren zur Herstellung von Einschlusskomplexen der entsprechenden 1,7-Diaryl-1,6-heptadien-3,5-dion-Derivate wird beispielsweise beschrieben in:
S. Winter, N. Tortik, A. Kubin, B. Krammer, K. Plaetzer, Back to the roots: photodynamic inactivation of bacteria based on water-soluble curcumin bound to polyvinylpyrrolidone as a photosensitizer, Photochem. Photobiol. Sci., 2013,12, 1795-1802. DOI: 10.1039/C3PP50095K

Die Erfindung wird nachfolgend durch Figuren und Beispiele erläutert, ohne hierauf beschränkt zu sein.

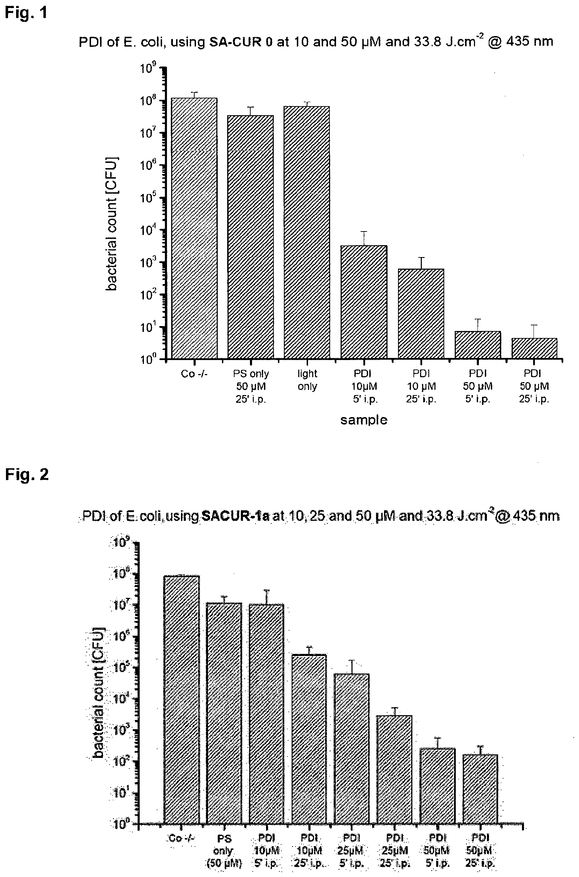

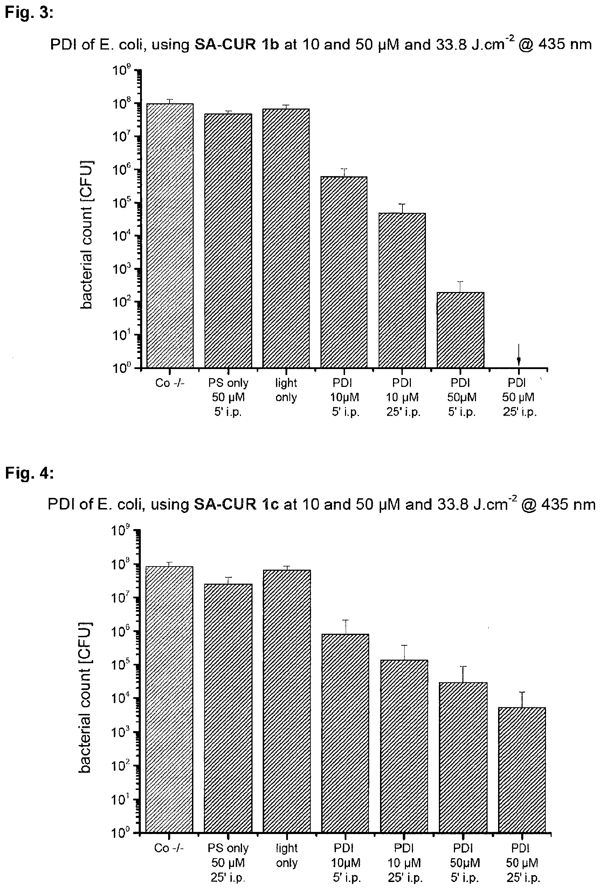

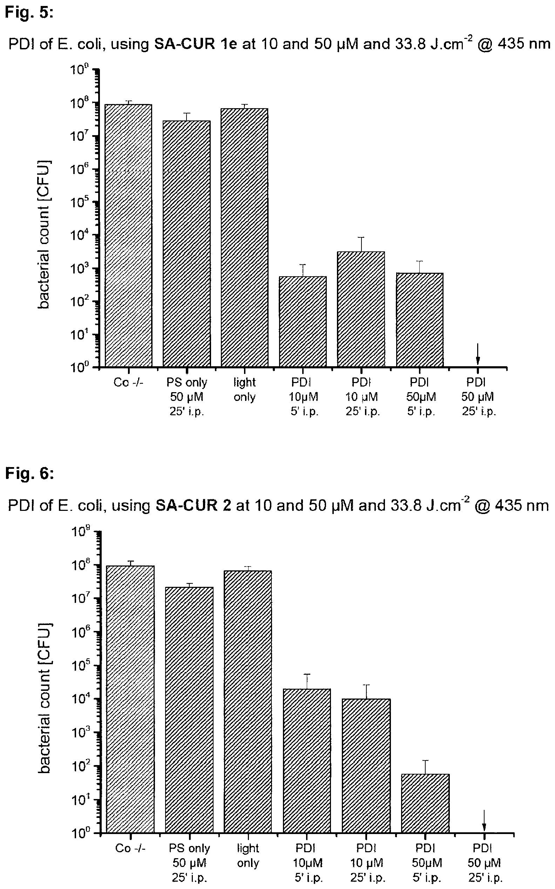

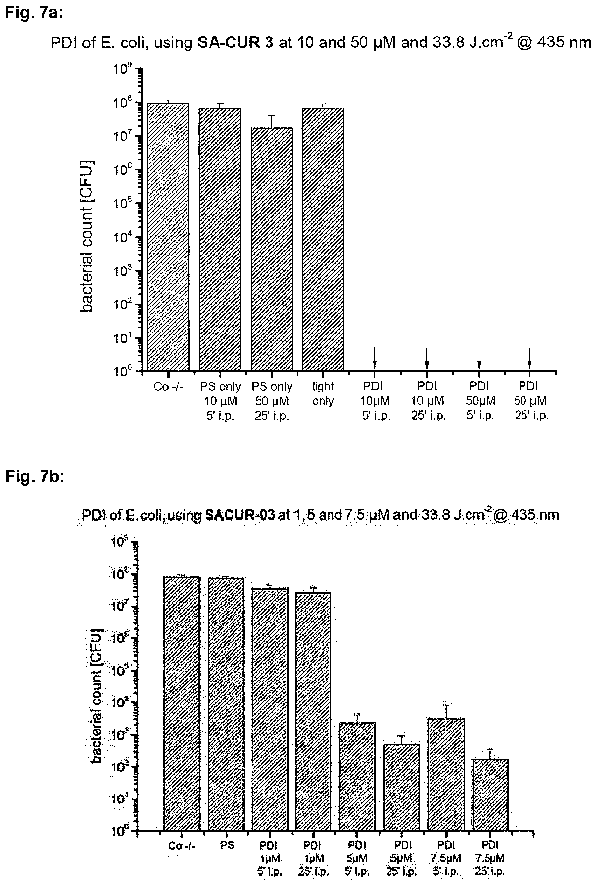

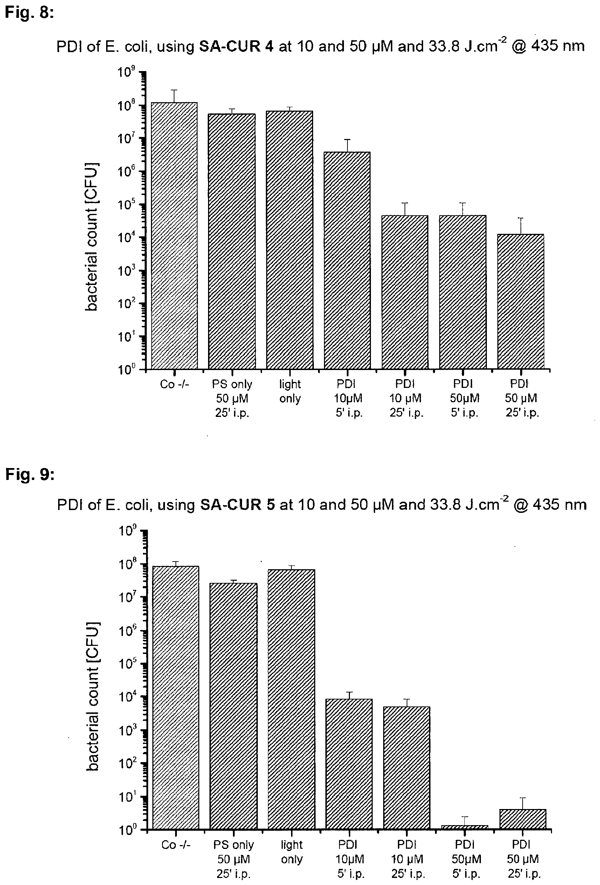

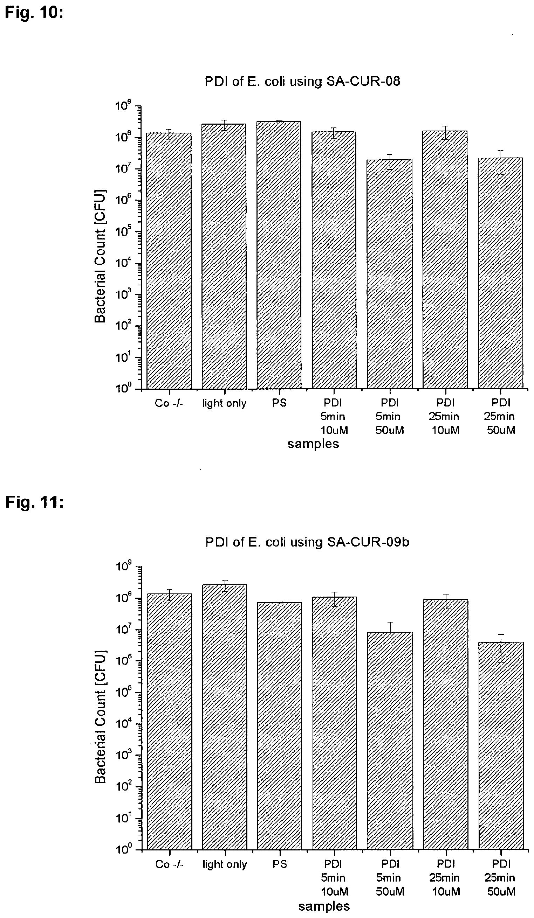

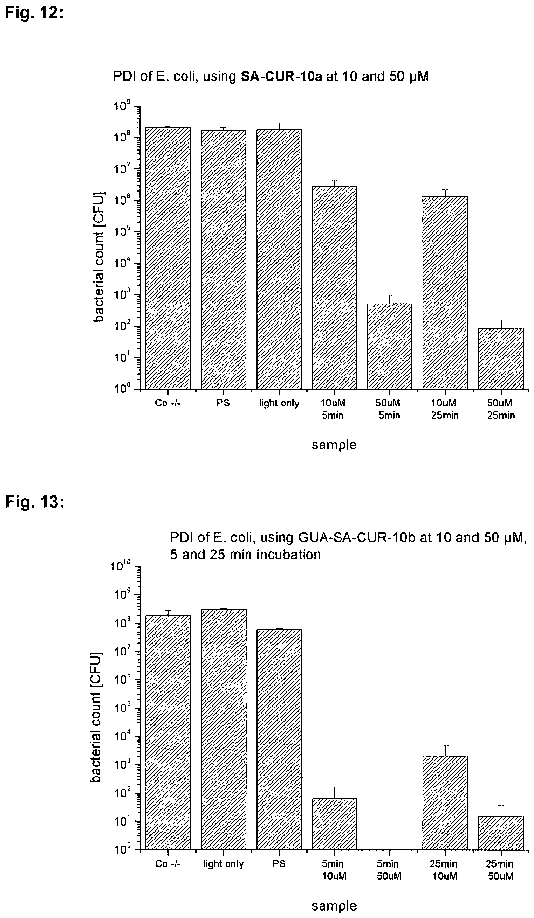

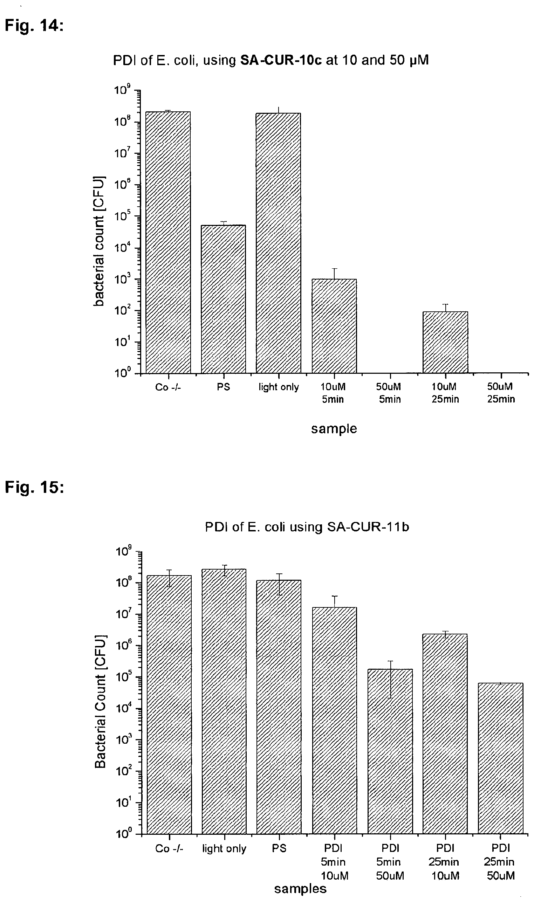

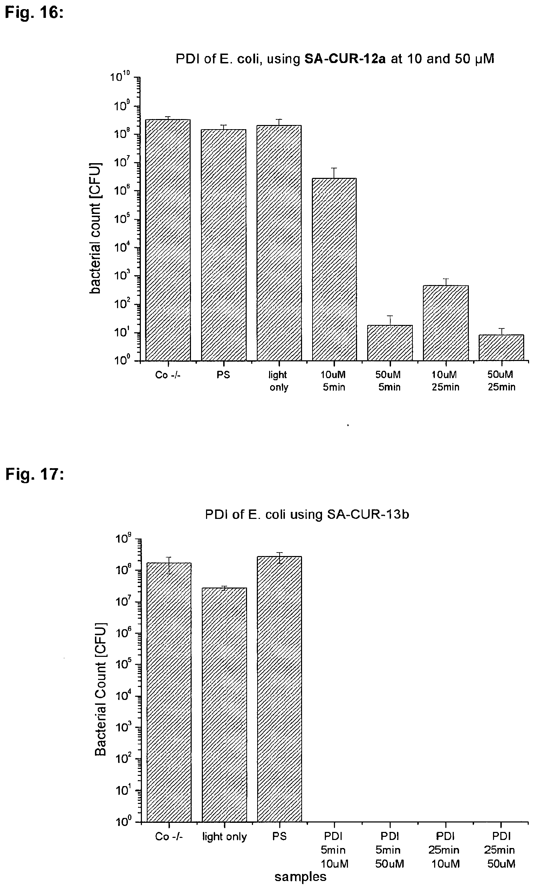

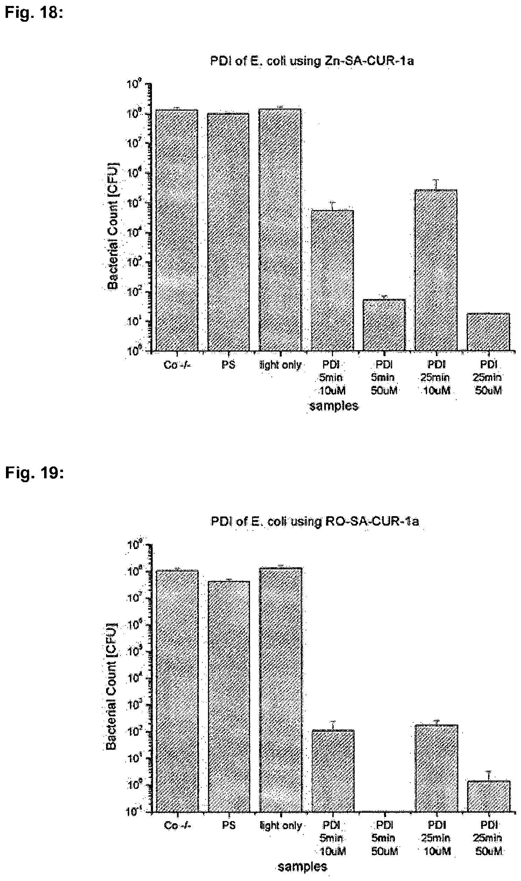

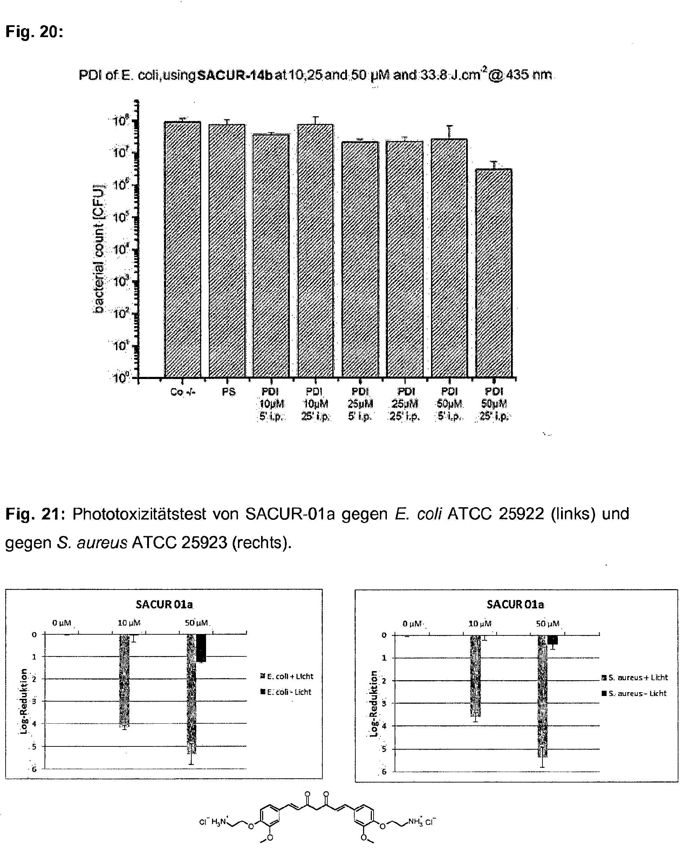

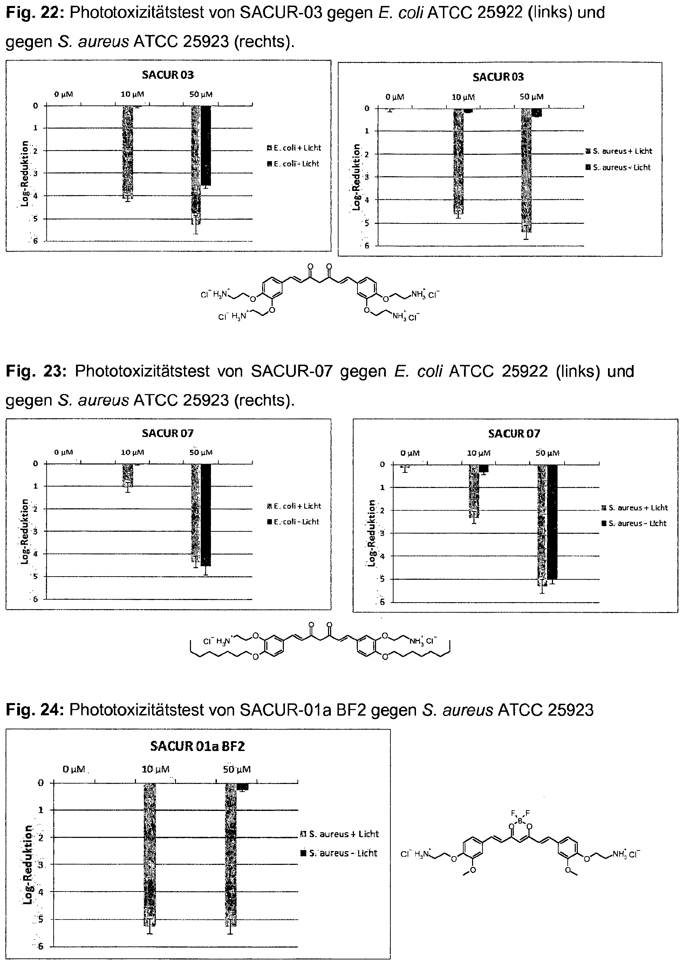

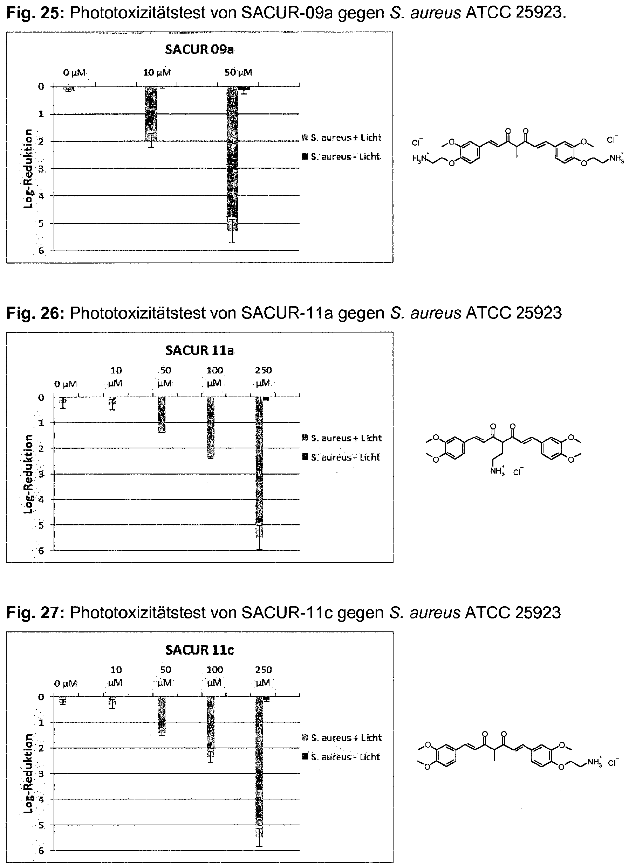

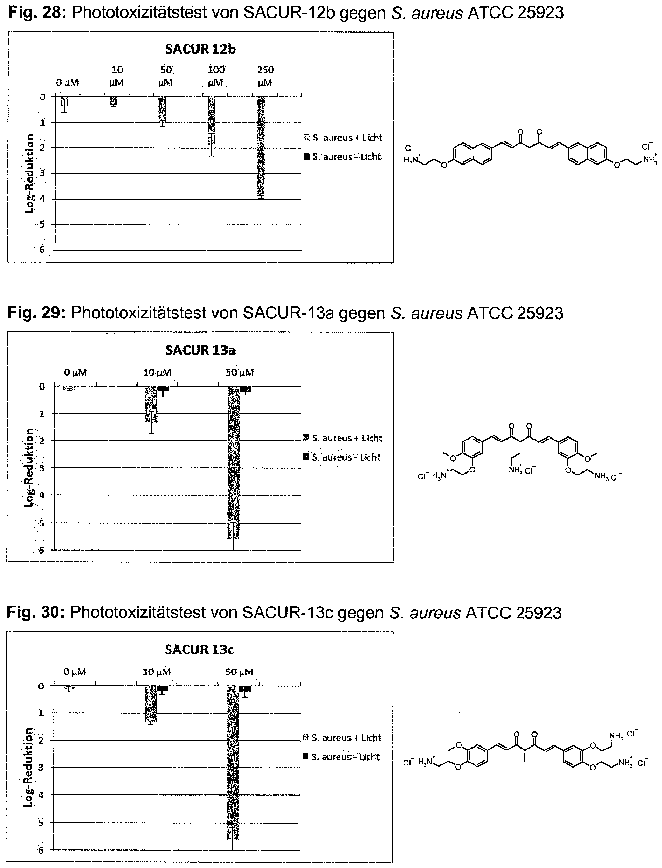

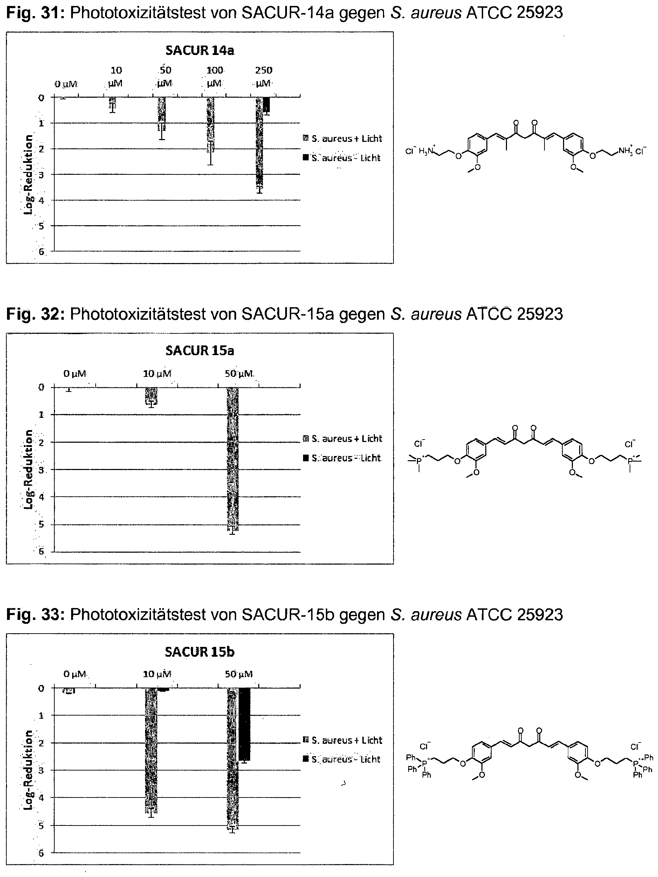

**Figur 1** zeigt die photodynamische Inaktivierung (PDI) von E.coli durch SACUR-0 Hydrochlorid im Vergleich zu den Kontrollen (PS only / light only). **Figur 2** zeigt die photodynamische Inaktivierung (PDI) von E.coli durch SACUR-01a Hydrochlorid im Vergleich zu den Kontrollen (PS only / light only). **Figur 3** zeigt die photodynamische Inaktivierung ((PDI) von E.coli durch SACUR-01b Hydrochlorid im Vergleich zu den Kontrollen (PS only / light only). **Figur 4** zeigt die photodynamische Inaktivierung (PDI) von E.coli durch SACUR-01c Hydrochlorid im Vergleich zu den Kontrollen (PS only / light only). **Figur 5** zeigt die photodynamische Inaktivierung (PDI) von E.coli durch SACUR-01e Hydrochlorid im Vergleich zu den Kontrollen (PS only / light only). **Figur 6** zeigt die photodynamische Inaktivierung (PDI) von E.coli durch SACUR-02 Hydrochlorid im Vergleich zu den Kontrollen (PS only / light only). **Figur 7a** und **7b** zeigen die photodynamische Inaktivierung (PDI) von E.coli durch SACUR-03 Hydrochlorid im Vergleich zu den Kontrollen (PS only / light only). **Figur 8** zeigt die photodynamische Inaktivierung (PDI) von E.coli durch SACUR-04 Hydrochlorid im Vergleich zu den Kontrollen (PS only / light only). **Figur 9** zeigt die photodynamische Inaktivierung (PDI) von E.coli durch SACUR-05 Hydrochlorid im Vergleich zu den Kontrollen (PS only / light only). **Figur 10** zeigt die photodynamische Inaktivierung (PDI) von E.coli durch SACUR-08 Hydrochlorid im Vergleich zu den Kontrollen (PS only / light only). **Figur 11** zeigt die photodynamische Inaktivierung (PDI) von E.coli durch SACUR-09b Hydrochlorid im Vergleich zu den Kontrollen (PS only / light only). **Figur 12** zeigt die photodynamische Inaktivierung (PDI) von E.coli durch SACUR-10a chlorid im Vergleich zu den Kontrollen (PS only / light only). **Figur 13** zeigt die photodynamische Inaktivierung (PDI) von E.coli durch SACUR-10b Hydrochlorid im Vergleich zu den Kontrollen (PS only / light only). **Figur 14** zeigt die photodynamische Inaktivierung (PDI) von E.coli durch SACUR-10c chlorid im Vergleich zu den Kontrollen (PS only / light only). **Figur 15** zeigt die photodynamische Inaktivierung (PDI) von E.coli durch SACUR-11b Hydrochlorid im Vergleich zu den Kontrollen (PS only / light only). **Figur 16** zeigt die photodynamische Inaktivierung (PDI) von E.coli durch SACUR-12a Hydrochlorid im Vergleich zu den Kontrollen (PS only / light only). **Figur 17** zeigt die photodynamische Inaktlvlerung (PDI) von E.coli durch SACUR-13b Hydrochlorid im Vergleich zu den Kontrollen (PS only / light only). **Figur 18** zeigt die photodynamische Inaktivierung (PDI) von E.coli durch Zn-SACUR-1a Hydrochlorid gegen E.coli im Vergleich zu den Kontrollen (PS only / light only). **Figur 19** zeigt die photodynamische Inaktivierung (PDI) von E.coli durch RO-SACUR-1a Hydrochlorid im Vergleich zu den Kontrollen (PS only / light only). **Figur 20** zeigt die photodynamische Inaktivierung (PDI) von E.coli durch SACUR-14b Hydrochlorid im Vergleich zu den Kontrollen (PS only / light only). **Figur 21** zeigt das Ergebnis des Phototoxizitätstests von SACUR-01a gegen *E. coli* ATCC 25922 (links) und gegen *S. aureus* ATCC 25923 (rechts). **Figur 22** zeigt das Ergebnis des Phototoxizitätstests von SACUR-03 gegen *E. coli* ATCC 25922 (links) und gegen *S. aureus* ATCC 25923 (rechts). **Figur 23** zeigt das Ergebnis des Phototoxizitätstests von SACUR-07 gegen *E. coli* ATCC 25922 (links) und gegen *S. aureus* ATCC 25923 (rechts). **Figur 24** zeigt das Ergebnis des Phototoxizitätstests von SACUR-01a BF2 gegen *S. aureus* ATCC 25923. **Figur 25** zeigt das Ergebnis des Phototoxizitätstests von SACUR-09a gegen *S. aureus* ATCC 25923. **Figur 26** zeigt das Ergebnis des Phototoxizitätstests von SACUR-11a gegen *S*. *aureus* ATCC 25923. **Figur 27** zeigt das Ergebnis des Phototoxizitätstests von SACUR-11c gegen *S. aureus* ATCC 25923. **Figur 28** zeigt das Ergebnis des Phototoxizitätstests von SACUR-12b gegen *S. aureus* ATCC 25923. **Figur 29** zeigt das Ergebnis des Phototoxizitätstests von SACUR-13a gegen *S. aureus* ATCC 25923. **Figur 30** zeigt das Ergebnis des Phototoxizitätstests von SACUR-13c gegen *S. aureus* ATCC 25923. **Figur 31** zeigt das Ergebnis des Phototoxizitätstests von SACUR-14a gegen *S. aureus* ATCC 25923. **Figur 32** zeigt das Ergebnis des Phototoxizitätstests von SACUR-15a gegen *S. aureus* ATCC 25923. **Figur 33** zeigt das Ergebnis des Phototoxizitätstests von SACUR-15b gegen *S. aureus* ATCC 25923.

### Beispiel 1) Herstellung verschiedener 1,7-Diaryl-1,6-heptadien-3,5-dion-Derivate.

### Überblick über die Synthesen

Alle verwendeten Chemikalien wurden von gängigen Anbietern käuflich erworben (TCI, ABCR, Acros, Merck und Fluka) und ohne weitere Reinigung eingesetzt. Lösemittel wurden vor Gebrauch destilliert und bei Bedarf auf übliche Art und Weise getrocknet. Trockenes DMF wurde bei Fluka (Taufkirchen, DE) käuflich erworben.

Dünnschichtchromatographien wurden auf Dünnschicht-Aluminiumfolien, beschichtet mit Kieselgel 60 F254, der Firma Merck (Darmstadt, DE) durchgeführt. Präparative Dünnschichtchromatographien wurden auf kommerziell erhältlichen, mit Kieselgel 60 beschichteten Glasplatten (20cm x 20 cm, Carl Roth GmbH & Co. KG, Karlsruhe, DE)) durchgeführt. Die Verbindungen wurden durch UV-Licht (λ = 254 nm, 333 nm) und teilweise mit bloßem Auge detektiert oder mit Ninhydrin angefärbt. Chromatographien wurden mit Kieselgel (0.060 - 0.200) der Firma Acros (Waltham, US) durchgeführt.

NMR-Spektren wurden auf einem Bruker-Spektrometer Avance 300 (300 MHz [¹H-NMR], 75 MHz [¹³C-NMR]) (Bruker Corporation, Billerica, US) gemessen.

Alle chemischen Verschiebungen sind in δ [ppm] relativ zum externen Standard (Tetramethylsilan, TMS) angegeben. Die Kopplungskonstanten sind jeweils in Hz angegeben; Charakterisierung der Signale: s = Singulett, d = Dublett, t = Triplett, m = Multiplett, dd = Dublett vom Dublett, br = breit. Die Integration bestimmt die relative Anzahl an Atomen. Die eindeutige Bestimmung der Signale in den Kohlenstoffspektren erfolgte mittels der DEPT-Methode (Pulswinkel: 135°). Fehlergrenzen: 0.01 ppm für ¹H-NMR, 0.1 ppm für ¹³C-NMR und 0.1 Hz für Kopplungskonstanten. Das verwendete Lösemittel ist jeweils für jedes Spektrum aufgeführt.

Die IR-Spektren wurden an einem Biorad Spektrometer Excalibur FTS 3000 (BioRad Laboratories GmbH, München, DE) aufgenommen.

ES-MS wurden mit einem ThermoQuest Finnigan TSQ 7000 Spektrometer gemessen, sämtliche HR-MS auf einem ThermoQuest Finnigan MAT 95 (jeweils Thermo Fisher Scientific Inc, Waltham, US) Spektrometer ermittelt, Argon diente als lonisierungsgas für FAB.

Schmelzpunkte wurden mit Hilfe des Schmelzpunktmessgerätes Büchi SMP-20 (Büchi Labortechnik GmbH, Essen, DE) unter Verwendung einer Glaskapillare bestimmt.

Sämtliche UV/Vis-Spektren wurden mit einem Varian Cary 50 Bio UV/VIS Spektrometer, Fluoreszenzspektren mit einem Varian Cary Eclipse Spektrometer aufgenommen.

Die Lösungsmittel für Absorbtions- und Emissionsmessungen wurden in spezieller spektroskopischer Reinheit von Acros oder Baker bzw. Uvasol von Merck gekauft. Milliporewasser (18 MΩ, Milli Q_{Plus}) wurde für alle Messungen verwendet.

### 1. Synthese der Aldehydbausteine

Die entsprechenden in Tabelle 1 dargestellten Edukte und Alkylierungsmittel wurden kommerziell bezogen.

Als Alkylierungsmittel wurden die ensprechenden Alkylbromide oder Alkyltosylate verwendet.

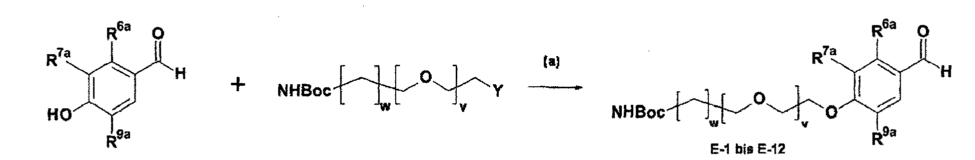

**Übersicht 1:** Synthese der Synthesebausteine; Bedingungen: (a) K₂CO₃, KI, DMF oder DMSO, T = 60 - 80°C

**Tabelle 1: Synthese der Aldehydbausteine; Bedingungen: (a) K₂CO₃, KI, DMF oder DMSO, T = 60 - 80°C**

| Beispiel | | Edukt | | | | Alkylierungsmittel | | | | Produkt | | | | | Ausbeute |
|---|---|---|---|---|---|---|---|---|---|---|---|---|---|---|---|
| | | R^{6a} = | R^{7a} = | R^{9a} = | | W | *V* | Y | | R^{6a} = | R^{7a} = | R^{9a} = | w | v | |
| E-1 | | H | H | H | | 1 | 0 | Br | | H | H | H | 1 | 0 | 76 % |
| E-2 | | H | Me | H | | 1 | 0 | Br | | H | Me | H | 1 | 0 | 73 % |
| E-3 | | H | OMe | H | | 1 | 0 | Br | | H | OMe | H | 1 | 0 | 79 % |
| E-4 | | OMe | H | H | | 1 | 0 | Br | | OMe | H | H | 1 | 0 | 71 % |
| E-5 | | H | OMe | | | 1 | 0 | Br | | H | OMe | I | 1 | 0 | 66 % |
| E-6 | | H | OMe | H | | 1 | 1 | TsO | | H | OMe | H | 1 | 1 | 72 % |
| E-7 | | H | OBz | H | | 2 | 0 | Br | | H | OBz | H | 2 | 0 | 82 % |
| E-8 | | H | OH | H | | 1 | 0 | Br | | H | OH | H | 1 | 0 | 17 % |
| E-9 | | H | OH | H | | 1 | 0 | Br | | H | OC₈H₁₇ | H | 1 | 0 | 39 % (2 Schritte) |
| E-10 | | H | OH | H | | 1 | 0 | Br | | H | O(CH₂CH₂)₃OH | H | 1 | 0 | 33% (2 Schritte) |
| E-11 | | H | OH | H | | 1 | 0 | Br | | H | OCH₂CH₂NHBoc | H | 1 | 0 | 69 % |
| E-12 | | H | OH | OH | | 1 | 0 | Br | | H | OCH₂CH₂NHBoc | OCH₂CH₂NHBoc | 1 | 0 | 61 % |

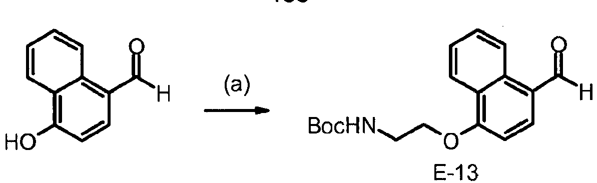

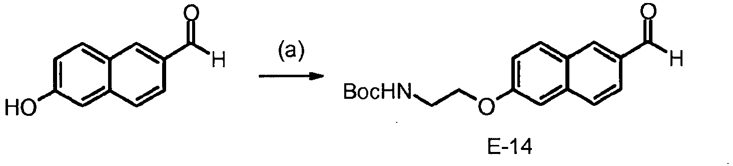

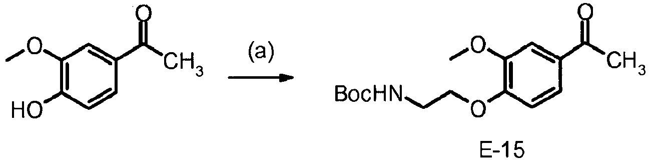

**Übersicht** 2: Synthese weiterer Aldehydbausteine; Bedingungen: (a) K₂CO₃, KI, DMF oder DMSO, T = 60 - 80°C

### 1.1 Substituierte Vanilline

### 1.1.1 Allgemeine Vorschrift:

10 mmol des entsprechenden Eduktes wurde unter Stickstoff in 20 mL DMF vorgelegt:
(I) 4-Hydroxybenzaldehyd (1.22 g, 10mmol)
(II) Vanillin (1.52 g, 10 mmol)
(III) Benzyloxyvanilin (2.28 g, 10 mmol)
(IV) 6-Hydroxy-2-naphthaldehyd (1.72 g, 10 mmol)
(V) 4-Hydroxy-3-methoxy-acetophenon (1.66 g, 10 mmol)

Das entsprechende Alkylierungsreagenz (12 - 15 mmol, 1.2 - 1.5 eq) in DMF (10 mL) und K₂CO₃ (2.76 g, 20 mmol) wurden nacheinander zugegeben. Der Ansatz wurde unter Stickstoffatmosphäre 24h bei 80°C gerührt. Nach Abkühlen auf Raumtemperatur wurde die Reaktionsmischung mit 60 mL Ethylacetat verdünnt und die organische Phase dreimal mit je 40 mL gesättigter wässriger Kochsalzlösung und einmal mit 50 mL Wasser ausgeschüttelt. Die organische Phase wurde abgetrennt, über MgSO₄ getrocknet und einrotiert.

Zur Reinigung des Produkts wurde eine Säulenchromatographie an Kieselgel durchgeführt.

### E-1: 4-(2-N-tert-butv/oxvcarbonvl-aminoethoxy)benzaldehyd

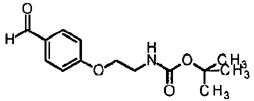

Edukt: 4-Hydroxybenzaldehyd.

Als Alkylierungsreagenz wurde 2-(tert-Butoxycarbonylamino)ethylbromid (3.34 g, 15 mmol) eingesetzt.
**Ausbeute:** 76% der Theorie, 2.5 g blassgelber Feststoff
**Molekülmasse** = 265.30 g/mol; **Summenformel** = C₁₄H₁₉NO₄
***¹H NMR** (300 MHz, CDCl₃), δ* = *9.87 (s, 1H), 7.82 (d, J* = *8.8 Hz, 2H), 6.98 (d, J* = *8.7 Hz, 2H), 5.05 (s, 1H), 4.09 (t, J = 5.2 Hz, 2H), 3.55 (dd, J = 10.4, 5.1 Hz, 2H), 1.44 (s, 9H). **MS** (ESI, CH₂Cl₂*/*MeOH* + *10 mmol NH₄OAc): 288.1 (17%, MNa*⁺*), 265.1 (1%, MH*⁺*), 210.1 (100%, MH*⁺-*C₄H₉), 166.1 (19%, MH*⁺-*boc)*

### E-3: 3-Mothoxy-4-(2-N-tert-butyloxycarbonyl-aminoethoxy)benzaldehyd

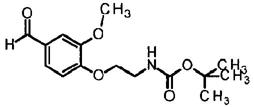

Edukt: Vanilin (4-Hydroxy-3-methoxybenzaldehyd).

Als Alkylierungsreagenz wurde 2-(tert-Butoxycarbonylamino)ethylbromid (3.34 g, 15 mmol) eingesetzt.
**Ausbeute:** 79% der Theorie, 2.54 g blassgelber Feststoff
**Molekülmasse** = 295.34 g/mol; **Summenformel** = C₁₅H₂₁NO₅
**¹H NMR:** (300 MHz, CDCl₃), δ = 9.85 (s, 1H), 7.43 (d, J = 1.8 Hz, 1H), 7.42 (m, 1H), 6.98 (d, J = 8.0 Hz, 1H), 5.10 (s, 1H), 4.15 (t, J = 5.1 Hz, 2H), 3.92 (s, 3H), 3.60 (dd, J = 10.6, 5.3 Hz, 2H), 1.44 (s, 9H). **MS:** (ESI, CH₂Cl₂/MeOH + 10 mmol NH₄OAc): 318.1 (13%, MNa⁺), 296.1 (100%, MH⁺), 240.1 (45%, MH⁺-C₄H₉), 196.1 (9%, MH⁺-boc).

### E-15: 3-Methoxy-4-(2-N-tert-butyloxycarbony/-aminoethoxy)acetophenon

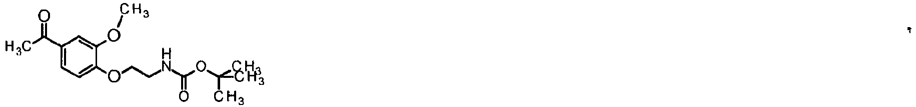

Herstellung wie unter A.1) beschrieben.
Edukt: 4-Hydroxy-3-methoxyacetophenon (Acetovanillon).

Als Alkylierungsreagenz wurde 2-(tert-Butoxycarbonylamino)ethylbromid (3.34 g, 15 mmol) eingesetzt.
**Ausbeute:** 77% der Theorie, 2.75 g blassgelber Feststoff
**Molekülmasse** = 309.36 g/mol; **Summenformel** = C₁₆H₂₃NO₅
**¹H NMR:** (300 MHz, CDCl₃), δ = 7.51 (d, J = 1.9 Hz, 1H), 7.49 (s, 1H), 6.94 (d, J = 7.9 Hz, 1H), 5.19 (s, 1H), 4.12 (t, J = 5.1 Hz, 2H), 3.89 (s, 3H), 3.58 (q, 5.3 Hz, 2H), 2.52 (s, 3H), 1.43 (s, 9H). **MS:** (ESI, CH₂Cl₂/MeOH + 10 mmol NH₄OAc): 332.1 (29%, MNa⁺), 309.1 (2%, MH⁺), 254.1 (33%, MH⁺-C₄H₉), 210.1 (100%, MH⁺-boc).

### E-14: 6-(2-N-tert-butyloxycarbonyl-aminoethoxy)naphthalen-2-carbaldehyd

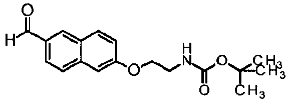

Edukt: 6-Hydroxy-2-naphthaldehyd.

Als Alkylierungsreagenz wurde 2-(tert-Butoxycarbonylamino)ethylbromid (3.34 g, 15 mmol) eingesetzt.
**Ausbeute:** 71% der Theorie, 2.30 g blassgelber Feststoff
**Molekülmasse** = 315.37 g/mol; **Summenformel** = C₁₈H₂₁NO₄
**¹H NMR:** (300 MHz, CDCl₃), δ = 10.09 (s, 1H), 8.25 (s, 1H), 7.94 - 7.85 (m, 2H), 7.79 (d, *J* = 8.5 Hz, 1H), 7.23 (dd, *J* = 8.9, 2.5 Hz, 1H), 7.17 (d, *J* = 2.3 Hz, 1H), 5.05 (s, 1H), 4.18 (t, *J* = 5.1 Hz, 2H), 3.62 (dd, *J* = 10.5, 5.3 Hz, 2H), 1.46 (s, 9H). **MS:** (ESI, CH₂Cl₂/MeOH + 10 mmol NH₄OAc): 338.2 (11%, MNa⁺), 316.2 (12%, MH⁺), 260.1 (100%, MH⁺-C₄H₉), 216.1 (14%, MH⁺-boc)

### E-7: 3-Benzyloxy-4-(2-N-tert-butyloxycarbonyl-aminopropyloxy)benzaldehyd

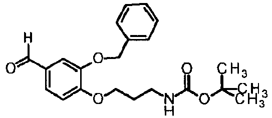

Edukt: Benzyloxyvanilin (4-Benzyloxy-3-methoxybenzaldehyd).

Als Alkylierungsreagenz wurde 2-(tert-Butoxycarbonylamino)propylbromid (3.34 g, 15 mmol) eingesetzt.
**Ausbeute:** 82% der Theorie, 2.64 g blassgelber Feststoff
**Molekülmasse** = 385.45 g/mol; **Summenformel** = C₂₂H₂₇NO₅
**¹H NMR:** (300 MHz, CDCl₃), δ = 9.74 (s, 1H), 7.44 - 7.16 (m, 7H), 6.91 (dd, J = 8.1, 1.4 Hz, 1H), 5.47 (s, 1H), 5.21 (m, 2H), 4.08 (td, J = 5.6, 2.2 Hz, 2H), 3.33 (d, J = 5.2 Hz, 2H), 2.03 - 1.92 (m, 2H), 1.36 (s, 9H). **MS:** (ESI, CH₂Cl₂/MeOH + 10 mmol NH₄OAc): 408.2 (37%, MNa⁺), 386.1 (2%, MH⁺), 330.1 (63%, MH⁺-C₄H₉), 286.1 (100%, MH⁺-boc).

### E-6: 3-Methoxy-4-(2-N-tert-butyloxycarbonyl-aminoethoxy-ethoxy)benzaldehyd

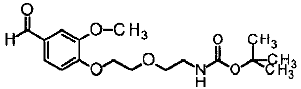

Edukt: Vanilin (4-Hydroxy-3-methoxybenzaldehyd).

Als Alkylierungsreagenz wurde 2-[2-(tert-Butoxycarbonylamino)ethoxy]ethyl-4-methylbenzensulfonat (4.26 g, 12 mmol) eingesetzt.
**Ausbeute:** 72% der Theorie; 2.78 g blassgelber Feststoff
**Molekülmasse** = 339.39 g/mol; **Summenformel** = C₁₇H₂₅NO₆
**¹H NMR:** (300 MHz, CDCl₃), δ = 9.76 (s, 1H), 7.42 - 7.26 (m, 2H), 6.92 (d, J = 8.1 Hz, 1H), 5.08 (s, 1H), 4.21 - 4.12 (m, 2H), 3.86 - 3.78 (m, 2H), 3.84 (s, 3H), 3.54 (dd, J = 7.4, 2.6 Hz, 2H), 3.26 (m, 2H), 1.35 (s, 9H). **MS:** (ESI, CH₂Cl₂/MeOH + 10 mmol NH₄OAc): 356.9 (100%, MNH₄⁺), 339.9 (41%, MH⁺), 239.9 (6%, MH⁺-boc).

### 1.1.2 Allgemeine Vorschrift:

15 mmol des entsprechenden Eduktes wurde unter Stickstoff in 10 mL DMF vorgelegt:
(VI) 4-Hydroxy-3-methyl-benzaldehyd (2.04 g, 15 mmol)
(VII) 4-Hydroxy-2-methoxy-benzaldehyd (2.28 g, 15 mmol)

Das entsprechende Alkylierungsreagenz (12 - 15 mmol, 1.2 - 1.5 eq) in DMF (20 mL) und K₂CO₃ (4.14 g, 30 mmol) wurden nacheinander zugegeben. Der Ansatz wurde unter Stickstoffatmosphäre 24h bei 80°C gerührt. Nach Abkühlen auf Raumtemperatur wurde die Reaktionsmischung mit 120 mL Ethylacetat verdünnt und die organische Phase dreimal mit je 40 mL gesättigter wässriger Kochsalzlösung und einmal mit 40 mL Wasser ausgeschüttelt. Die organische Phase wurde abgetrennt, über MgSO₄ getrocknet und einrotiert. Zur Reinigung des Produkts wurde eine Säulenchromatographie an Kieselgel durchgeführt.

### E-2: 3-Methyl-4-(2-N-tert-butyloxycarbonyl-aminoethoxy)benzaldehyd

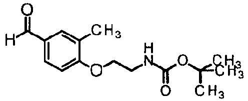

Edukt: 4-Hydroxy-3-methyl-benzaldehyd.

Als Alkylierungsreagenz wurde 2-(tert-Butoxycarbonylamino)ethylbromid (5.02 g, 22.5 mmol) eingesetzt.
**Ausbeute:** 73% der Theorie, 3,44 g gelblicher Feststoff
**Molekülmasse** = 279,34 g/mol; **Summenformel** = C₁₅H₂₁NO₄
**¹H NMR:** (300 MHz, CDCl₃), δ = 9.85 (s, 1H), 7.75 - 7.66 (m, 2H), 6.90 (d, *J* = 8.9 Hz, 1H), 4.95 (s, 1H), 4.11 (t, *J* = 5.2 Hz, 2H), 3.59 (m, 2H), 2.27 (s, 3H), 1.45 (s, 9H). **MS:** (ESI, CH₂Cl₂/MeOH + 10 mmol NH₄OAc): 302.1 (11%, MNa⁺), 280.1 (9%, MH⁺), 224.1 (100%, MH⁺-C₄H₉), 180.1 (4%, MH⁺-boc).

### E-4: 2-Methoxy-4-(2-N-tert-butyloxycarbonyl-aminoethoxy)benzaldehyd

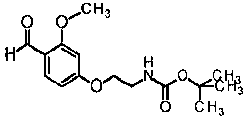

Edukt: 4-Hydroxy-2-methoxy-benzaldehyd.

Als Alkylierungsreagenz wurde 2-(tert-Butoxycarbonylamino)ethylbromid (5.02 g, 22.5 mmol) eingesetzt.
**Ausbeute:** 71% der Theorie, 3,94 g blassgelber Feststoff
**Molekülmasse** = 295.34 g/mol; **Summenformel** = C₁₅H₂₁NO₅
**¹H NMR:** (300 MHz, CDCl₃), δ = 10.25 (d, *J* = 1.2 Hz, 1H), 7.76 (dd, *J* = 8.6, 1.5 Hz, 1H), 6.58 - 6.46 (m, 1H), 6.44 (s, 1H), 5.06 (s, 1H), 4.07 (t, *J* = 5.0 Hz, 2H), 3.87 (s, 3H), 3.53 (m, 2H), 1.43 (s, 9H). **MS:** (ESI, CH₂Cl₂/MeOH + 10 mmol NH₄OAc): 318.1 (8%, MNa⁺), 296.1 (100%, MH⁺), 240.1 (87%, MH⁺-C₄H₉), 196.1 (3%, MH⁺-boc).

### E-5: 3-Methoxy-4-(2-N-tert-butyloxycarbonyl-aminoethoxy)-5-iodo-benzaldehyd

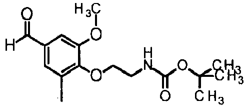

5-lodovanilin (4.17 g, 15 mmol) wurde unter Stickstoff in 10 mL DMF vorgelegt. Als Alkylierungsreagenz wurde 2-(tert-Butoxycarbonylamino)ethylbromid (5.02 g, 22.5 mmol) eingesetzt, das in 20 mL DMF gelöst wurde. Das Alkylierungsreagenz und K₂CO₃ (4.14 g, 30 mmol) wurden nacheinander zugegeben. Der Ansatz wurde unter Stickstoffatmosphäre 24h bei 80°C gerührt. Nach Abkühlen auf Raumtemperatur wurde die Reaktionsmischung mit 120 mL Ethylacetat verdünnt und die organische Phase dreimal mit je 40 mL gesättigter wässriger Kochsalzlösung und einmal mit 40 mL Wasser ausgeschüttelt. Die organische Phase wurde abgetrennt, über MgSO₄ getrocknet und einrotiert. Zur Reinigung des Produkts wurde eine Säulenchromatographie an Kieselgel durchgeführt.
**Ausbeute:** 66% der Theorie, 3,07 g gelber Feststoff
**Molekülmasse** = 421,23 g/mol; **Summenformel** = C₁₅H₂₀INO₅
**¹H NMR:** (300 MHz, CDCl₃), δ = 9.82 (s, 1H), 7.84 (d, *J* = 1.7 Hz, 1H), 7.39 (d, *J* = 1.8 Hz, 1H), 5.40 (s, 1H), 4.17 (t, *J* = 4.9 Hz, 2H), 3.91 (s, 3H), 3.52 (dd, *J* = 10.3, 5.3 Hz, 2H), 1.45 (s, 9H). **MS:** (ESI, CH₂Cl₂/MeOH + 10 mmol NH₄OAc): 444.1 (6%, MNa⁺), 422.1 (3%, MH⁺), 366.0 (63%, MH⁺-C₄H₉), 322.0 (100%, MH⁺-boc).

### 1.2 Mehrfach substituierte Aldehyde

Das entsprechende Edukt wurde unter Stickstoff in 10 mL DMF vorgelegt:
(VIII) 3,4-Dihydroxybenzaldehyd (3.45 g, 25 mmol)
(IX) 3,4,5-Trihydroxybenzaldehyd (2.31 g, 15 mmol)

Als Alkylierungsreagenz wurde 2-(tert-Butoxycarbonylamino)ethylbromid (11.2 g, 50 mmol, 2eq) eingesetzt, das in 30 mL DMF gelöst wurde. Das Alkylierungsreagenz und K₂CO₃ (13.8 g, 100 mmol) wurden nacheinander zugegeben. Der Ansatz wurde unter Stickstoffatmosphäre 16 h bei 80°C gerührt. Eine zweite Portion des Alkylierungsreagenz (50 mmol, 2 eq) in 20 mL DMF wurde zugegeben und der Ansatz weitere 24 h bei 80°C gerührt. Nach Abkühlen auf Raumtemperatur wurde die Reaktionsmischung mit 250 mL Ethylacetat verdünnt und die organische Phase dreimal mit je 150 mL gesättigter wässriger Kochsalzlösung und einmal mit 100 mL Wasser ausgeschüttelt. Die organische Phase wurde abgetrennt, über MgSO₄ getrocknet und einrotiert. Zur Reinigung des Produkts wurde eine Säulenchromatographie an Kieselgel durchgeführt (Laufmittel: anfangs PE:EE = 3:1, dann PE:EE = 3:2).

### E-11: 3,4-bis-(2-N-tert-butyloxycarbonyl-aminoethoxy)benzaldehyd

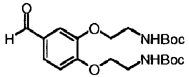

Edukt: 3,4-Dihydroxybenzaldehyd
**Ausbeute:** 8.1 g blassgelbes Glas, 69% der Theorie
**Molekülmasse** = 424.50 g/mol; **Summenformel** = C₂₁H₃₂N₂O₇
**¹H NMR:** (300 MHz, CDCl₃), δ = 9.83 (s, 1H), 7.52 - 7.37 (m, 2H), 6.99 (d, *J* = 8.2 Hz, 1H), 5.20 (s, 2H), 4.17-4.06 (m, 4H), 3.56 (m, 4H), 1.44 (s, 9H), 1.43 (s, 9H). **MS:** (ESI, CH₂Cl₂/MeOH + 10 mmol NH₄OAc): 447.2 (100%, MNa⁺), 425.2 (70%, MH⁺), 369.2 (49%, MH⁺-C₄H₉), 269.1 (78%, MH⁺-boc).

### E-12: 3,4,5-tris-(2-N-tert-butyloxycarbonyl-aminoethoxy)benzaldehyd

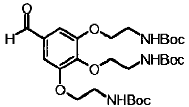

Edukt: 3,4,5-Trihydroxybenzaldehyd
**Ausbeute:** 6.22 g blassgelbes Glas, 61% der Theorie
**Molekülmasse** = 583.68 g/mol; **Summenformel** = C₂₈H₄₅N₃O₁₀
**¹H NMR:** (300 MHz, CDCl₃), δ = 9.82 (s, 1H), 7.11 (s, 2H), 5.71 (s, 1H), 5.21 (s, 2H), 4.12 (t, *J* = 4.6 Hz, 6H), 3.62 - 3.51 (m, 4H), 3.42 (d, *J* = 4.9 Hz, 2H), 1.44 (d, *J* = 8.7 Hz, 27H). **MS:** (ESI, CH₂Cl₂/MeOH + 10 mmol NH₄OAc): 606.3 (47%, MNa⁺), 584.3 (100%, MH⁺), 528.3 (4%, MH⁺-C₄H₉), 484.3 (23%, MH⁺-boc).

### 1.3 Gemischtsubstituierte Benzaldehyde

### Sufe 1:

3,4-Dihydroxybenzaldehyd (3.45 g, 25 mmol) wurde unter Stickstoff in 20 mL DMF vorgelegt. 2-(tert-Butoxycarbonylamino)ethylbromid (6.72 g, 30 mmol) in 30 mL DMF und K₂CO₃ (4.6 g, 33 mmol) wurden nacheinander zugegeben. Der Ansatz wurde unter Stickstoffatmosphäre 24 h bei 80°C gerührt. Nach Abkühlen auf Raumtemperatur wurde die Reaktionsmischung mit 150 mL Ethylacetat verdünnt und die organische Phase dreimal mit je 100 mL gesättigter wässriger Kochsalzlösung und einmal mit 100 mL Wasser ausgeschüttelt. Die organische Phase wurde abgetrennt, über MgSO₄ getrocknet und einrotiert. Zur Reinigung der Produkte wurde eine Säulenchromatographie an Kieselgel durchgeführt.

### E-8: 3-Hydroxy-4-(2-N-tert-butyloxycarbonyl-aminoethoxy)benzaldehyd

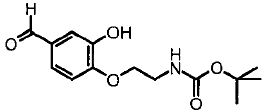

**Ausbeute:** 1.48 g blassgelbes Glas, 17% der Theorie
**Molekülmasse** = 281.31 g/mol; **Summenformel** = C₁₄H₁₉NO₅
**¹H NMR:** (300 MHz, CDCl₃), δ = 9.78 (s, 1H), 7.42 (d, *J* = 1.9 Hz, 1H), 7.36 (dd, *J* = 8.2, 1.9 Hz, 1H), 7.14 (s, 1H), 6.89 (d, *J* = 8.2 Hz, 1H), 5.53 (s, 1H), 4.13 (t, *J* = 4.9 Hz, 2H), 3.58 (dd, *J* = 10.6, 5.2 Hz, 2H), 1.42 (s, 9H). **MS:** (ESI, CH₂Cl₂/MeOH + 10 mmol NH₄OAc): 304.1 (19%, MNa⁺), 282.1 (2%, MH⁺), 226.1 (100%, MH⁺-C₄H₉), 182.1 (42%, MH⁺-boc).

### E-8a: 4-Hydroxy-3-(2-N-tert-butyloxycarbonyl-aminoethoxy)benzaldehyd

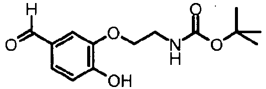

**Ausbeute:** 2.60 g blassgelbes Glas, 37% der Theorie
**Molekülmasse** = 281.31 g/mol; - **Summenformel** = C₁₄H₁₉NO₅

### E-11: 3,4-bis-(2-N-tert-butyloxcarbonyl-aminoethoxy)benzaldehyd

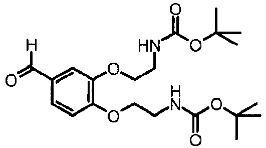

**Ausbeute:** 1.38 g blassgelbes Glas, 13% der Theorie
**Molekülmasse** = 424.50 g/mol; **Summenformel** = C₂₁H₃₂N₂O₇
¹H-NMR und MS wie oben.

### Sufe 2:

1.41 g (5 mmol) 3-Hydroxy-4-(2-N-tert-butyloxycarbonyl-aminoethoxy)benzaldehyd (E-8) wurde unter Stickstoff in 10 mL DMF vorgelegt. Das jeweilige in 10 mL DMF gelöste Alkylierungsreagenz Triethylenglykol-monotosylat oder 1-Bromooctanol sowie K₂CO₃ (2.07 g, 15 mmol) wurden nacheinander zugegeben. Der Ansatz wurde unter Stickstoffatmosphäre 24 h bei 80°C gerührt. Nach Abkühlen auf Raumtemperatur wurde die Reaktionsmischung mit 80 mL Ethylacetat verdünnt und die organische Phase dreimal mit je 50 mL gesättigter wässriger Kochsalzlösung und einmal mit 50 mL Wasser ausgeschüttelt. Die organische Phase wurde abgetrennt, über MgSO₄ getrocknet und einrotiert. Zur Reinigung des Produkts wurde eine Säulenchromatographie an Kieselgel durchgeführt.

### E-10: 3-(2-(2-(2-Hydroxy-ethoxy)ethoxy)ethoxy)-4-(2-N-tert-butyloxycarbonyl-aminoethoxy)benzaldehyd

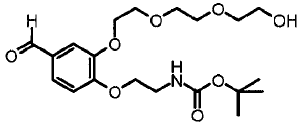

Als Alkylierungsreagenz wurde Triethylenglykol-monotosylat (3.04 g, 10 mmol) eingesetzt. Säulenchromatographie mit EE → EE/EtOH = 3:1
**Ausbeute:** 1.53 g blassgelbes Glas, 74% der Theorie
**Molekülmasse** = 413.47 g/mol; **Summenformel** = C20H31N08
**¹H NMR:** (300 MHz, CDCl₃), δ = 9.83 (s, 1H), 7.48 - 7.38 (m, 2H), 6.96 (d, *J* = 8.1 Hz, 1H), 4.24 (dd, *J* = 5.3, 3.4 Hz, 2H), 4.12 (t, *J* = 4.8 Hz, 2H), 3.92 (dd, *J* = 5.3, 3.4 Hz, 2H), 3.74 (t, *J* = 4.6 Hz, 6H), 3.66 - 3.61 (m, 2H), 3.58 (t, *J* = 4.7 Hz, 2H), 1.43 (s, 9H). **MS:** (ESI, CH₂Cl₂/MeOH + 10 mmol NH₄OAc): 436.2 (35%, MNa⁺), 414.2 (33%, MH⁺), 370.2 (5%, MH⁺-C₄H₉), 314.2 (100%, MH⁺-boc).

### E-9: 3-Octyloxy-4-(2-N-tert-butyloxycarbonyl-aminoethoxy)benzaldehyd

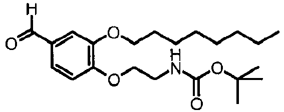

Als Alkylierungsreagenz wurde 1-Bromooctanol (1.93 g, 10 mmol) eingesetzt. Säulenchromatographie mit PE:EE = 3:1
**Ausbeute:** 1.53 g blassgelbes Glas, 78% der Theorie
**Molekülmasse** = 393.53 g/mol; **Summenformel** = C₂₂H₃₅NO₅
**¹H NMR:** (300 MHz, CDCl₃), δ = 9.81 (s, 1H), 7.43 - 7.32 (m, 2H), 6.96 (d, *J* = 8.4 Hz, 1H), 4.11 (t, *J* = 5.3 Hz, 2H), 4.02 (t, *J* = 6.7 Hz, 2H), 3.55 (dd, *J* = 10.2, 5.0 Hz, 2H), 1.91 -1.76 (m, 2H), 1.47 - 1.39 (m, 2H), 1.42 (s, 9H), 1.36 - 1.16 (m, 8H), 0.90-0.80 (m, 3H). **MS:** (ESI, CH₂Cl₂/MeOH + 10 mmol NH₄OAc): 416.2 (51%, MNa⁺), 396.2 (4%, MH⁺), 338.2 (7%, MH⁺-C₄H₉), 294.2 (100%, MH⁺-boc).

### 1.4 Substituiertes Naphthaldehyd

### E-13: 4-(2-N-tert-butyloxycarbonyl-aminoethoxy)naphthalen-1-carbaldehyd

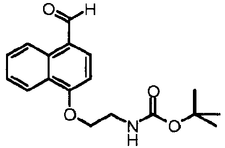

4-Hydroxynaphth-1-aldehyd (1.74 g, 10 mmol) wurde unter Stickstoff in 10 mL DMF vorgelegt. 2-(tert-Butoxycarbonylamino)ethylbromid (4.4 g, 20 mmol) in 10ml DME Kaliumiodid (10 mmol, 1.66 g) und Cs₂CO₃ (6.5 g,20 mmol) wurden nacheinander zugegeben. Der Ansatz wurde unter Stickstoffatmosphäre 24h bei 80°C gerührt. Nach Abkühlen auf Raumtemperatur wurde die Reaktionsmischung mit 80 mL Ethylacetat verdünnt und die organische Phase dreimal mit je 50 mL gesättigte wässriger Kochsalzlösung und einmal mit 100 mL Wasser ausgeschüttelt. Die organische Phase wurde abgetrennt, über MgSO₄ getrocknet und einrotiert. Das Rohprodukt wurde mit EE/PE 1:19 gewaschen, um den Überschuss an Reaktand zu entfernen. Anschließend wurde der orange Feststoff mit EE/PE 1:2 extrahiert (3x je 100 mL) und vom Niederschlag abfiltriert. Die Lösung wurde einrotiert und der Rückstand in 250 mL Diethylether gelöst. Die organische Phase wurde zweimal mit je 50 mL 3 M NaOH und einmal mit 100 mL Wasser ausgeschüttelt. Die organische Phase wurde abgetrennt, über MgSO₄ getrocknet und einrotiert. Zur weiteren Reinigung des Produkts wurde eine Säulenchromatographie an Kieselgel durchgeführt. **Ausbeute:** 2.08 g blassgelber Feststoff, 47% der Theorie **Molekülmasse** = 315.37 g/mol; **Summenformel** = C₁₈H₂₁NO₄
**¹H NMR:** (300 MHz, CDCl₃), δ = 10.19 (s, 1H), 9.30 (d, *J* = 8.5 Hz, 1H), 8.33 (d, *J* = 8.4 Hz, 1H), 7.94 - 7.86 (m, 1H), 7.76 - 7.64 (m, 1H), 7.62 - 7.51 (m, 1H), 6.94 - 6.82 (m, 1H), 5.07 (s, 1H), 4.29 (dd, *J* = 6.9, 3.2 Hz, 2H), 3.73 (m, 2H), 1.46 (s, 9H).
**MS:** (ESI, CH₂Cl₂/MeOH + 10 mmol NH₄OAc): 338.2 (16%, MNa⁺), 316.2 (19%, MH⁺), 260.1 (100%, MH⁺-C₄H₉), 216.1 (4%, MH⁺-boc).

### 2. Synthese substituierter Curcumine

Aus den oben beschriebenen Vorstufen wurden die entsprechenden Curcumine als tert-Butyloxycarbonyl (Boc) geschützte Curcumine synthetisiert. Die Boc-Schutzgruppe wurde anschließend entfernt.

### 2.1 Synthese symmetrisch substituierter Curcumine

**Übersicht 3:** Synthese verschiedener symmetrisch substituierter Curcumine; Bedingungen: (a) Acetylaceton, B₂O₃, B(OBu)₃, n-Butylamin, Ethylacetat, 80°C, 6h, dann Hydrolyse mit HOAc 40% über Nacht, RT; (b) Acetylaceton, B₂O₃, B(OBu)₃, n-Butylamin, DMF, 80°C, 6h, dann Hydrolyse mit HOAc 40% über Nacht, RT; (c) 3,5-Heptandion, B₂O₃, B(OBu)₃, n-Butylamin, DMF, 80°C, 6h, dann Hydrolyse mit HOAc 40% über Nacht, RT; (d) DCM, TFA, RT, 5h; dann lonentauscher Amberlite IRA-958, Wasser.

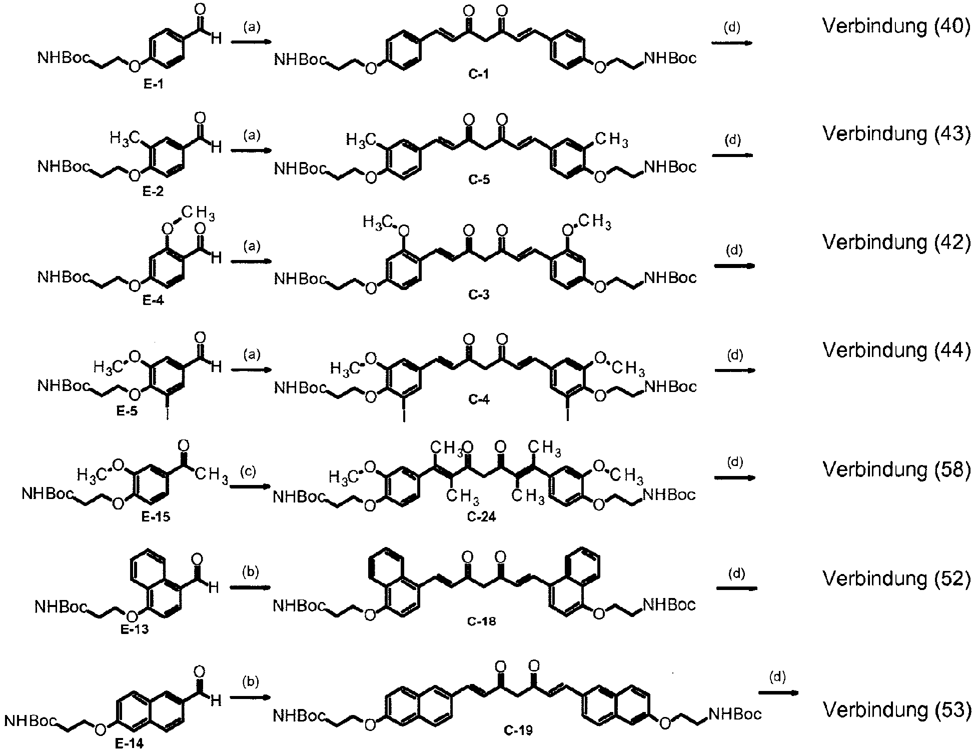

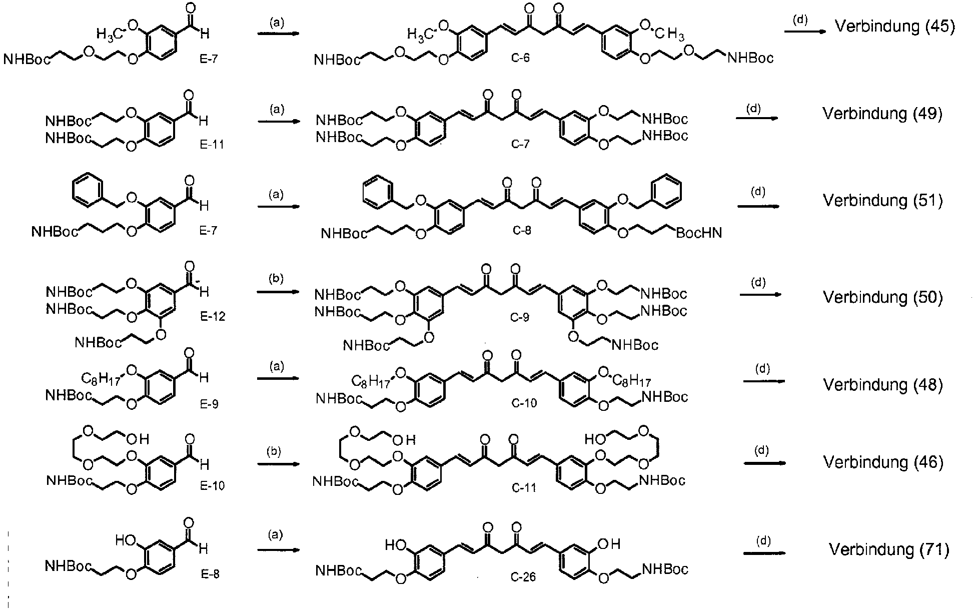

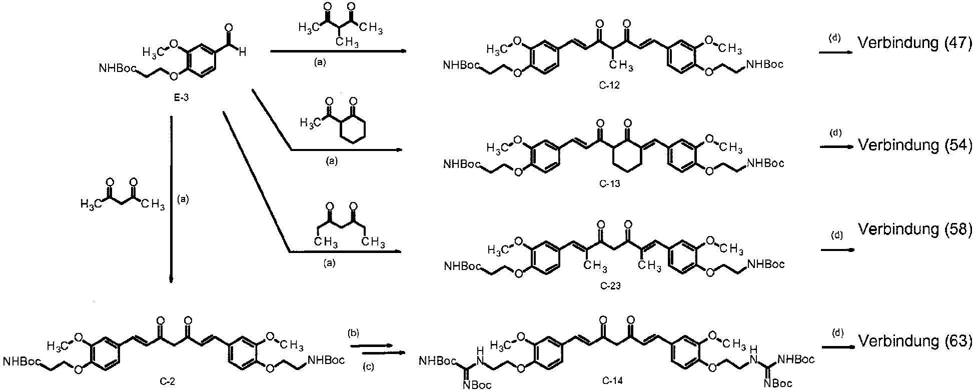

**Übersicht 4:** Synthese symmetrisch substituierter Curcumine ausgehend von E-3; Bedingungen: (a) B₂O₃, B(OBu)₃, n-Butylamin, DMF oder Ethylacetat, 80°C, 6h, dann Hydrolyse mit HOAc 40% über Nacht, RT; (b) DCM, TFA, RT, 5h; (c) 1,3-Di-boc-2-(trifluoromethylsulfonyl)guanidine, DCM, Triethylamin, 0°C → RT, 4h; (d) DCM, TFA, RT, 5h; dann lonentauscher Amberlite IRA-958, Wasser

### 2.1.1 Allgemeine Vorschrift:

Acetylaceton (0.1 g, 1 mmol) und Boroxid B₂O₃ (0.07 g, 1 mmol) wurden in Ethylacetat (2 mL) gelöst und 30 Minuten bei 50°C gerührt. Das substituierte Benzaldehyd (20 mmol) in Ethylacetat (3 mL) sowie Tributylborat (0.7 g, 3 mmol) wurden nacheinander zugegeben und der Ansatz für eine weitere halbe Stunde gerührt. Nun wurde über 5 Minuten n-Butylamin (0.1 mL in 1 mL EE) zugetropft. Nach weiteren fünf Stunden Rühren bei 50°C wurde die Reaktion über Nacht stehen gelassen. Die Lösung wurde einrotiert und der Rückstand kurz im Hochvakuum getrocknet. Pro 1 g Rohprodukt wurden 10 mL Ethylacetat zugegeben und das Rohprodukt gelöst. Für die anschließende Hydrolyse des Borkomplexes wurde das doppelte Volumen 50%ige Essigsäure zugegeben (20 mL pro 1 g Rohprodukt). Nach Rühren für 24h bei Raumtemperatur unter Lichtschutz wurde das Lösungsmittelgemisch bei vermindertem Druck abgezogen. Der Rückstand wurde dreimal mit EE extrahiert (3x 20 mL) und das unlösliche Salz abfiltriert. Die vereinigten organischen Phasen wurden mit Wasser (30 mL) gewaschen, über MgSO₄ getrocknet und schließlich das Lösungsmittel bei vermindertem Druck abgezogen. Die Aufreinigung erfolgt durch Säulenchromatographie an Kieselgel mit Aceton / PE. Die Reinfraktion des erhaltenen Curcumins wurde anschließend in möglichst wenig Ethylacetat gelöst. Durch Eintropfen dieser Lösung in einen Überschuss an Petrolether wurde das Produkt als feines gelboranges Pulver gefällt.

### 1.7-bis(3,4-dimethoxyphenyl)hepta-1,6-diene-3,5-dione (Tetramethoxvcurcumin)

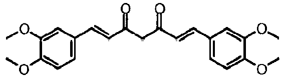

Vom entsprechenden Aldehyd wurden eingesetzt: 334 mg = 2 mmol Säulenchromatographie an Kieselgel mit Aceton / PE = 2:5 → 1:2
**Ausbeute:** 289 mg, 73% der Theorie, oranger, zäher Feststoff bzw. orangegelbes Pulver. Molekülmasse = 396,44 g/mol; Summenformel = C₂₃H₂₄O₆

### C-1: [2-(4-(7-[4-(2-tert-Butoxycarbonylamino-ethoxy)-phenyl]-3,5-dioxo-hepta-1,6-dienyl}-phenoxy)-ethyl]-carbaminsäure-tert-butylester

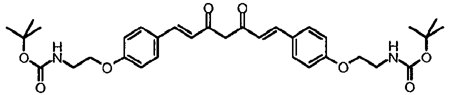

Vom entsprechenden Aldehyd **E-1** wurden eingesetzt: 530 mg = 2 mmol Säulenchromatographie an Kieselgel mit Aceton / PE = 1:3 → 2:3
**Ausbeute:** 363 mg, 67% der Theorie, gelber Feststoff bzw. gelbes Pulver
**Molekülmasse** = 594,71 g/mol; **Summenformel** = C₃₃H₄₂N₂O₈
**¹H NMR:** (300 MHz, CDCl₃), δ = 7.61 (d, *J* = 15.8 Hz, 2H), 7.50 (d, *J* = 8.8 Hz, 4H), 6.89 (d, *J* = 8.7 Hz, 4H), 6.50 (d, *J* = 15.8 Hz, 2H), 5.02 (s, 2H), 4.05 (t, *J* = 5.1 Hz, 4H), 3.60 - 3.48 (m, 4H), 1.45 (s, 18H). **MS:** (ESI, CH₂Cl₂/MeOH + 10 mmol NH₄OAc): 595.3 (100%, MH⁺), 539.2 (18%, MH⁺-C₄H₉).

### C-5: [2-(4-{7-[4-(2-tert-Butoxycarbonylamino-ethoxy)-3-methyl-phenyl]-3,5-dioxo-hepta-1,6-dienyl}-2-methyl-phenoxy)-ethyl]-carbaminsäure-tert-butylester

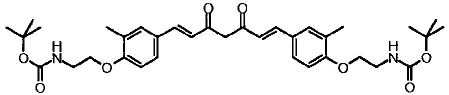

Vom entsprechenden Aldehyd **E-2** wurden eingesetzt: 559 mg = 2 mmol Säulenchromatographie an Kieselgel mit Aceton / PE = 1:3 → 2:3
**Ausbeute:** 442 mg, 59% der Theorie, orangegelber Feststoff bzw. orangegelbes Pulver.
**Molekülmasse** = 622,77 g/mol; **Summenformel** = C₃₅H₄₆N₂O₈
**¹H NMR:** (300 MHz, CDCl₃), δ = 7.59 (d, *J* = 15.8 Hz, 2H), 7.36 (d, *J* = 10.8 Hz, 4H), 6.80 (d, *J* = 8.3 Hz, 2H), 6.49 (d, *J* = 15.8 Hz, 2H), 4.94 (s, 2H), 4.06 (t, *J* = 5.0 Hz, 4H), 3.58 (m, 4H), 2.25 (s, 6H), 1.46 (s, 18H). **MS:** (ESI, CH₂Cl₂/MeOH + 10 mmol NH₄OAc): 623.3 (100%, MH⁺), 567.3 (21%, MH⁺-C₄H₉).

### C-3: [2-(4-{7-[4-(2-tert-Butoxycarbonylamino-ethoxy)-2-octyloxy-phenyl]-3,5-dioxo-hepta-1,6-dienyl}-3-octyloxy-phenoxy)-ethyl-carbaminsäure-tert-butylester

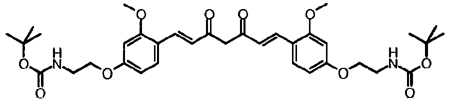

Vom entsprechenden Aldehyd **E-4** wurden eingesetzt: 591 mg = 2 mmol Säulenchromatographie an Kieselgel mit Aceton / PE = 1:3 → 2:3
**Ausbeute:** 393 mg, 58% der Theorie, oranger, zäher Feststoff bzw. orangegelbes Pulver.
**Molekülmasse** = 654,76 g/mol; **Summenformel** = C₃₅H₄₆N₂O₁₀
**¹H NMR:** (300 MHz, CDCl₃), δ = 7.89 (d, *J* = 16.0 Hz, 2H), 7.47 (d, *J* = 8.5 Hz, 2H), 6.62 (d, *J* = 16.0 Hz, 2H), 6.52 - 6.39 (m, 4H), 4.99 (s, 2H), 4.06 (t, *J* = 5.1 Hz, 4H), 3.88 (s, 6H), 3.55 (m, 4H), 1.45 (s, 18H). **MS:** (ESI, CH₂Cl₂/MeOH + 10 mmol NH₄OAc): 655.3 (100%, MH⁺).

### C-4: [2-(4-{7-[4-(2-tert-Butoxycarbonylamino-ethoxy)-3-methoxy-5-iodo-phenyl]-3,5 dioxo-hepta-1,6-dienyl}-2-octyloxy-6-iodo-phenoxy)-ethyl]-carbaminsäure-tert-butylester

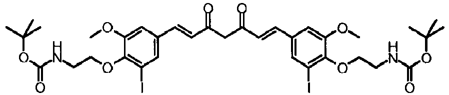

Vom entsprechenden Aldehyd **E-5** wurden eingesetzt: 842 mg = 2 mmol Säulenchromatographie an Kieselgel mit Aceton / PE = 1:4 → 1:2
**Ausbeute:** 580 mg, 64% der Theorie, oranger, zäher Feststoff bzw. orangegelbes Pulver. **Molekülmasse** = 906,56 g/mol; **Summenformel** = C₃₅H₄₄I₂N₂O₁₀
**¹H NMR:** (300 MHz, CDCl₃), δ = 7.57 (d, *J* = 1.5 Hz, 2H), 7.50 (d, *J* = 15.8 Hz, 2H), 7.02 (d, *J* = 1.5 Hz, 2H), 6.51 (d, *J* = 15.8 Hz, 2H), 5.47 (s, 2H), 4.11 (t, *J* = 4.7 Hz, 4H), 3.90 (s, 6H), 3.52 (m, 4H), 1.46 (s, 18H). **MS:** (ESI, CH₂Cl₂/MeOH + 10 mmol NH₄OAc): 929.1 (100%, MNa⁺), 907.1 (100%, MH⁺), 807.1 (42%, MH⁺-boc).

### C-2: [2-(4-{7-[4-(2-tert-Butoxycarbonylamino-ethoxy)-3-methoxy-phenyl]-3,5-dioxo-hepota-1,6-dienyl}-2-methoxy-phenoxy)-ethyl]-carbaminsäure-tert-butylester

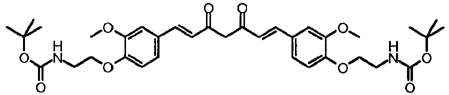

Vom entsprechenden Aldehyd **E-15** wurden eingesetzt: 590 mg = 2 mmol Säulenchromatographie an Kieselgel mit Aceton / PE = 1:3 → 2:3
**Ausbeute:** 354 mg, 54% der Theorie, oranger, zäher Feststoff bzw. orangegelbes Pulver.
**Molekülmasse** = 654,76 g/mol, **Summenformel** = C₃₅H₄₆N₂O₁₀
**¹H NMR:** (300 MHz, CDCl₃), δ = 7.58 (d, *J* = 15.7 Hz, 2H), 7.14 - 6.99 (m, 4H), 6.87 (d, *J* = 8.3 Hz, 2H), 6.48 (d, *J* = 15.8 Hz, 2H), 5.16 (s, 2H), 4.09 (t, *J* = 4.9 Hz, 4H), 3.90 (s, 6H), 3.56 (m, 4H), 1.44 (s, 18H). **MS:** (ESI, CH₂Cl₂/MeOH + 10 mmol NH₄OAc): 655.3 (100%, MH⁺), 555.3 (26%, MH⁺-boc).

### C-6: (2-{2-[4-(7-{4-[2-(2-tert-Butoxycarbonylamino-ethoxy)-ethoxy]-3-methoxy-phenyl}-3,5-dioxo-hepta-1,6-dienyl)-2-methoxy-phenoxyl-ethoxy}-ethyl)-carbaminsäure-tert-butylester

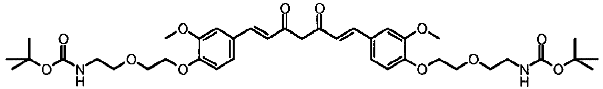

Vom entsprechenden Aldehyd **E-7** wurden eingesetzt: 679 mg = 2 mmol
**Ausbeute:** 349 mg, 47% der Theorie, oranger, zäher Feststoff bzw. oranges Pulver Säulenchromatographie an Kieselgel mit Aceton / PE = 1:2 → 1:1
**Molekülmasse** = 742,87 g/mol; **Summenformel** = C₃₉H₅₄N₂O₁₂
**¹H NMR:** (300 MHz, CDCl₃), δ = 7.59 (d, *J* = 15.8 Hz, 2H), 7.18 - 7.03 (m, 4H), 6.90 (d, *J* = 8.3 Hz, 2H), 6.49 (d, *J* = 15.8 Hz, 2H), 5.07 (s, 2H), 4.24 - 4.15 (m, 4H), 3.91 (s, 6H), 3.88 - 3.83 (m, 4H), 3.61 (t, *J* = 5.1 Hz, 4H), 3.33 (m, 4H), 1.43 (s, 18H). **MS:** (ESI, CH₂Cl₂/MeOH + 10 mmol NH₄OAc): 671.3 (51%, MNa⁺), 655.3 (86%, MH⁺), 555.3 (100%, MH⁺-boc).

### C-7: (2-[4-{7-[3,4-Bis-(2-tert-butoxycarbonylamino-ethoxy)-phenyl]-3,5-dioxo-hepta-1,6-dienyl}-2-(2-tert-butoxycarbonylamino-ethoxy)-phenoxy]-ethyl}-carbaminsäure-tert-butylester

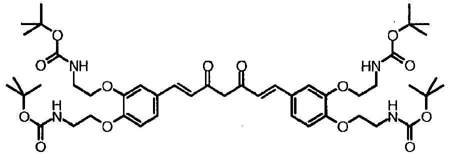

Vom entsprechenden Aldehyd **E-11** wurden eingesetzt: 850 mg = 2 mmol
**Ausbeute:** 374 mg, 41% der Theorie, oranger, sehr zäher Feststoff bzw. orangerötliches Pulver
Säulenchromatographie an Kieselgel mit Aceton / PE = 1:3 → 1:2
**Molekülmasse** = 913,08 g/mol; **Summenformel** = C₄₇H₆₈N₄O₁₄
**¹H NMR** (300 MHz, CDCl₃), δ = 7.57 (d, *J* = 15.8 Hz, 2H), 7.19 - 7.12 (m, 4H), 6.98 - 6.86 (m, 2H), 6.50 (d, *J* = 15.8 Hz, 2H), 5.28 (s, 4H), 4.10 (t, *J* = 4.4 Hz, 8H), 3.55 (m, 8H), 1.46 (s, 18H), 1.45 (s, 18H). **MS** (ESI, CH₂Cl₂/MeOH + 10 mmol NH₄OAc): 913.5 (100%, MH⁺), 457.3 (7%, (M+2H⁺)²⁺):

### C-8: [2-(4-{7-[4-(2-tert-Butoxycarbonylamino-ethoxy)-3-benzyloxy-phenyl]-3,5-dioxo-hepta-1,6-dienyl}-2-benzyloxy-phenoxy)-ethyl]-carbaminsäure-tert-butylester

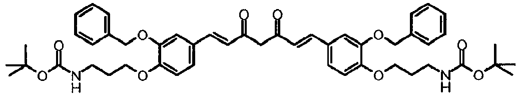

Vom entsprechenden Aldehyd **E-7** wurden eingesetzt: 738 mg = 2 mmol
**Ausbeute:** 518 mg, 62% der Theorie, oranger Feststoff bzw. oranges Pulver Säulenchromatographie an Kieselgel mit Aceton / PE = 1:3 → 2:3
**Molekülmasse** = 835.02 g/mol; **Summenformel** = C₄₉H₅₈N₂O₁₀
**¹H NMR** (300 MHz, CDCl₃), δ = 7.55 (d, *J* = 15.8 Hz, 2H), 7.45-7.31 (m, 10H), 7.14-7.04 (m, 4H), 6.86 (d, *J* = 8.3 Hz, 2H), 6.46 (d, *J* = 15.8 Hz, 2H), 5.39 (s, 2H), 5.22 (s, 4H), 4.14 (t, *J* = 5.9 Hz, 4H), 3.39 (m, 4H), 2.09 - 2.00 (m, 4H), 1.41 (s, 18H). **MS** (ESI, CH₂Cl₂/MeOH + 10 mmol NH₄OAc): 857.4 (100%, MNa⁺), 835.4 (76%, MH⁺), 735.4 (53%, MH⁺-boc).

### C-9: {2-[4-{7-[3,4,5-Tris-(2-tert-butoxycarbonylamino-ethoxy)-phenyl]-3,5-dioxo-hepta-1,6-dienyl}-2,6-bis-(2-tert-butoxycarbonylamino-ethoxy)-phenoxy]-ethyl}-carbaminsäure-tert-butylester

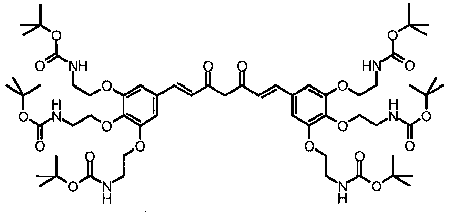

Vom entsprechenden Aldehyd **E-11** wurden eingesetzt: 1166 mg = 2 mmol
**Ausbeute:** 554 mg, 45% der Theorie, oranger, sehr zäher Feststoff bzw. orangerötliches Pulver. Säulenchromatographie an Kieselgel mit Aceton / PE = 1:4 → 1:2. **Molekülmasse** = 1231,46 g/mol; **Summenformel** = C₆₁H₉₄N₆O₂₀
**¹H NMR** (300 MHz, CDCl₃), δ = 7.52 (d, *J* = 15.1 Hz, 2H), 6.79 (s, 4H), 6.51 (d, *J* = 15.1 Hz, 2H), 5.75 (s, 2H), 5.25 (s, 3H), 4.10 (m, 12H), 3.57 (m, 8H), 3.41 (m, 4H), 1.47 (s, 18H), 1.47 (s, 36H).**MS** (ESI, CH₂Cl₂/MeOH + 10 mmol NH₄OAc): 1253.6 (100%, MNa⁺), 1231.6 (49%, MH⁺), 1131.3 (51%, MH⁺-boc).

### C-10: [2-54-{7-[4-(2-tert-Butoxycarbonylamino-ethoxy)-3-octyloxy-phenyl-3,5-dioxo-hepta-1,6-dienyl}-2-octyloxy-phenoxy)-ethyl]-carbaminsäure-tert-butylester

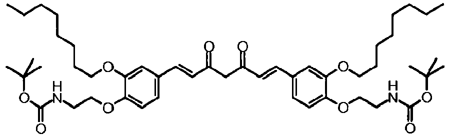

Vom entsprechenden Aldehyd **E-10** wurden eingesetzt: 787 mg = 2 mmol Säulenchromatographie an Kieselgel mit Aceton / PE = 1:4 → 1:2
Ausbeute: 400 mg, 47% der Theorie, oranger, zäher Feststoff bzw. orangegelbes Pulver.
**Molekülmasse** = 851,14 g/mol; **Summenformel** = C₄₉H₇₄N₂O₁₀
**¹H NMR** (300 MHz, CDCl₃), δ = 7.58 (d, *J* = 15.7 Hz, 2H), 7.15 - 7.05 (m, 4H), 6.89 (d, *J* = 8.2 Hz, 2H), 6.48 (d, *J* = 15.8 Hz, 2H), 5.17 (s, 2H), 4.09 (t, *J* = 5.0 Hz, 4H), 4.03 (t, *J* = 6.7 Hz, 4H), 3.54 (m, 4H), 1.91 - 1.80 (m, 4H), 1.41 (s, 18H), 1.51 - 1.26 (m, 20H), 0.89 (t, 4.5 Hz, 6H). **MS** (ESI, CH₂Cl₂/MeOH + 10 mmol NH₄OAc): 873.5 (100%, MNa⁺), 851.4 (13%, MH⁺), 751.5 (38%, MH⁺-boc).

### 2.1.2 Allgemeine Vorschrift:

Die Reaktion wurde unter Stickstoffatmosphäre und geschützt von Licht ausgeführt. Als beta-Diketon wurden verwendet:
(X) Acetylaceton (0.1 g, 1 mmol)
(XI) 3-Methyl-2,4-pentanedion (0.12 g, 1 mmol)
(XII) 3,5-Heptandion (0.13 g, 0.137 mL, 1 mmol), oder
(XIII) 2-Acetylcyclohexanon (0.14 g, 1 mmol).

Das jeweilige beta-Diketon und Boroxid B₂O₃ (0.05 g, 0.7 mmol) wurden in trockenem DMF (2 mL) gelöst und 30 Minuten bei 70°C gerührt. Das entsprechende substituierte Aldehyd (2 mmol) sowie Tributylborat (0.46 g, 2 mmol) wurden in trockenem DMF (5 mL) gelöst. Diese Lösung wurde zum Ansatz gegeben und es wurde für eine weitere halbe Stunde bei 85°C gerührt. Nun wurde über 5 Minuten n-Butylamin (0.1 mL in 1 mL DMF) zugetropft und der Ansatz bei 70°C für 4 Stunden gerührt. Nach Abkühlen auf Raumtemperatur, wurden alle flüchtigen Bestandteile im Stickstoffstrom über Nacht abgetrieben (Abzug, Schlauch hinter der Prallwand).

Je 1 g Rohprodukt wurden 10 mL Ethylacetat zugegeben und das Rohprodukt gelöst. Für die anschließende Hydrolyse des Borkomplexes wurde das doppelte Volumen 50%ige Essigsäure zugegeben (20 mL pro 1 g Rohprodukt). Nach Rühren für 24h bei Raumtemperatur unter Lichtschutz wurde das Lösungsmittelgemisch bei vermindertem Druck abgezogen (max. 50°C Wasserbadtemperatur). Der Rückstand wurde dreimal mit EE extrahiert (3x 50 mL) und das unlösliche Salz abfiltriert. Die vereinigten organischen Phasen wurden mit Wasser (50 mL) gewaschen, über MgSO₄ getrocknet und schließlich das Lösungsmittel bei vermindertem Druck abgezogen. Die Aufreinigung erfolgt durch Säulenchromatographie an Kieselgel mit Aceton / PE. Die Reinfraktion des Curcumins wurde anschließend in möglichst wenig Ethylacetat gelöst. Durch Eintropfen dieser Lösung in einen Überschuss an Petrolether wurde das Produkt als feines gelboranges Pulver gefällt.

### C-26: [2-(4-{7-[4-(2-tert-Butoxycarbonylamino-ethoxy)-3-hydroxy-phenyl]-3,5-dioxo-hepta-1,6-dienyl}-2-hydroxy-phenoxy)-ethyl]-carbaminsäure-tert-butylester

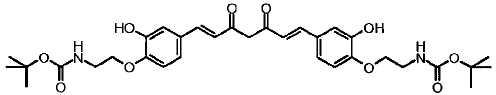

beta-Diketon: Acetylaceton
Vom entsprechenden Aldehyd **E-8** wurden eingesetzt: 560 mg = 2 mmol Säulenchromatographie an Kieselgel mit Aceton / PE = 1:2 → 2:3
**Ausbeute:** 28% der Theorie, oranger, zäher Feststoff (180 mg).
**Molekülmasse** = 626,71 g/mol; **Summenformel** = C₃₃H₄₂N₂O₁₀
**¹H NMR** (300 MHz, CDCl₃), δ = 7.57 (d, J = 15.8 Hz, 2H), 7.34 (m, H), 7.19 - 7.08 (m, 2H), 6.85 (d, J = 8.4 Hz, 2H), 6.64 (d, J = 15.8 Hz, 2H), 5.17 (s, 2H), 4.07 (t, J = 5.0 Hz, 4H), 3.58 - 3.44 (m, 4H), 1.43 (s, 18H). **MS** (ESI, CH₂Cl₂/MeOH + 10 mmol NH₄OAc): 627.2 (100%, MH⁺), 527.2 (37%, MH⁺-boc).

### C-18: [2-(4-{7-[4-(2-tert-Butoxycarbonylamino-ethoxy)-napthyl]-3,5-dioxo-hepta-1,6 dienyl}-naphthoxy)-ethyl]-carbaminsäure-tert-butylester

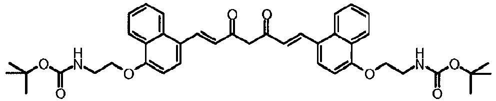

beta-Diketon: Acetylaceton
Vom entsprechenden Aldehyd **E-13** wurden eingesetzt: 630 mg = 2 mmol Säulenchromatographie an Kieselgel mit Aceton / PE = 2:7 → 1:2
**Ausbeute:** 62% der Theorie, oranger, zäher Feststoff (430 mg).
**Molekülmasse** = 694,83 g/mol; **Summenformel** = C₄₁H₄₆N₂O₈
**¹H NMR** (300 MHz, CDCl₃), δ = 8.48 (d, *J* = 15.5 Hz, 2H), 8.33 (d, *J* = 8.1 Hz, 2H), 8.25 (d, *J* = 8.0 Hz, 2H), 7.80 (d, *J* = 8.1 Hz, 2H), 7.63 (t, *J* = 6.9 Hz, 2H), 7.55 (t, *J* = 6.8 Hz, 2H), 6.86 (d, *J* = 8.2 Hz, 2H), 6.68 (d, *J* = 15.5 Hz, 2H), 5.04 (s, 2H), 4.26 (t, *J* = 5.0 Hz, 4H), 3.72 (q, *J* = 5.0 Hz, 5H), 1.47 (s, 18H). **MS** (ESI, CH₂Cl₂/MeOH + 10 mmol NH₄OAc): 717.3 (100%, MNa⁺), 695.3 (56%, MH⁺), 639.3 (73%, MH⁺-C₄H₉).

### C-19: [2-(6-{7-[6-(2-tert-Butoxycarbonylamino-ethoxy)-napthyl]-3,5-dioxo-hepta-1,6-dienyl}-naphthoxy)-ethyl]-carbaminsäure-tert-butylester

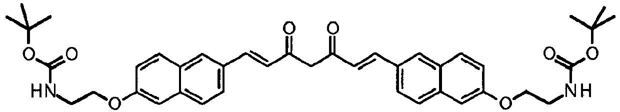

beta-Diketon: Acetylaceton
Vom entsprechenden Aldehyd **E-14** wurden eingesetzt: 1.26 g = 4 mmol
**Ausbeute:** 820 mg, 59 % der Theorie, rötlicher, zäher Feststoff bzw. oranges Pulver.
**Molekülmasse** = 694,83 g/mol; **Summenformel** = C₄₁H₄₆N₂O₈
**¹H NMR** (300 MHz, CDCl₃), δ = 7.85 (d, *J* = 15.8 Hz, 2H), 7.79 - 7.63 (m, 8H), 7.20 - 7.08 (m, 4H), 6.71 (d, *J* = 15.8 Hz, 2H), 5.06 (s, 2H), 4.15 (t, *J* = 5.0 Hz, 4H), 3.61 (m, 4H), 1.46 (s, 18H). **MS** (ESI, CH₂Cl₂/MeOH + 10 mmol NH₄OAc): 717.3 (58%, MNa⁺), 695.3 (100%, MH⁺), 639.3 (97%, MH⁺-C₄H₉).

### C-13: tert-Butyl-N-[2-[4-[(E)-3-[(3E)-3-[[4-[2-(tert-butoxycarbonylamino)ethoxy]-3-methoxy-phenyl]methylene]1-2-oxo-cyclohexyl]-3-oxo-prop-1-enyl]-2-methoxy-phenoxy]ethyl]carbamat

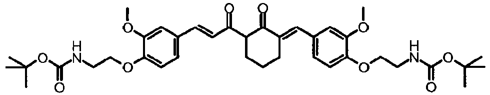

beta-Diketon: 2-Acetylcyclohexanon
Vom entsprechenden Aldehyd wurden eingesetzt: 580 mg = 2 mmol Flashchromatographie an Kieselgel mit Aceton / PE = 2:5 → 1:2
Produktenthaltende Fraktionen wurden einrotiert und das Rohprodukt aus Aceton/PE 1:3 umkristallisiert. Nach langsamen Abkühlen auf RT wurde zunächst im Kühlschrank über Nacht, danach 3 h im Gefrierfach gekühlt. Der Feststoff wurde abgesaugt, mehrmals mit wenig eiskaltem Aceton/PE 1:3 und zuletzt mit PE gewaschen. Das Produkt wurde an der Luft getrocknet. Durch Einengen der Mutterlauge kann eine weitere, kleine Kristallfraktion erhalten wurden.
**Ausbeute:** 68% der Theorie, orange, verfilzende Kristallnadeln (472 mg).
**Molekülmasse** = 694,83 g/mol; **Summenformel** = C₃₈H₅₀N₂O₁₀
**¹H NMR** (300 MHz, CDCl₃), 5 = 7.76 - 7.62 (m, 2H), 7.17 (dd, *J* = 8.3, 1.5 Hz, 1H), 7.09 (d, *J* = 1.7 Hz, 1H), 7.06 - 6.85 (m, 5H), 5.22 - 5.08 (m, 2H), 4.11 (t, *J* = 4.5 Hz, 4H), 3.92 (s, 3H), 3.89 (s, 3H), 3.57 (m, 4H), 2.83 - 2.73 (m, 2H), 2.71 - 2.64 (m, 2H), 1.86 - 1.77 (m, 2H), 1.45 (s, 18H). **MS** (ESI, CH₂Cl₂/MeOH + 10 mmol NH₄OAc): 717.4 (24%, MNa⁺), 695.4 (100%, MH⁺), 595.3 (30%, MH⁺-boc).

### C-9: {2-[4-{7-[3,4,5-Tris-(2-tert-butoxycarbonylamino-ethoxy)-phenyl]-3,5-dioxo-hepta-1,6-dienyl}-2,6-bis-(2-tert-butoxycarbonylamino-ethoxy]-phenoxy]-ethy/}-carbaminsäure-tert-butylester

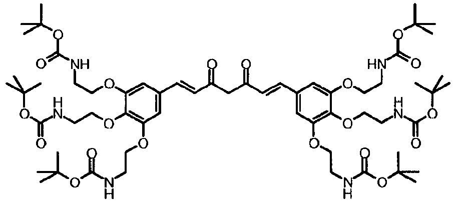

beta-Diketon: Acetylaceton
Vom entsprechenden Aldehyd **E-12** wurden eingesetzt: 1160 mg = 2 mmol
**Ausbeute:** 66% der Theorie, oranger, zäher Feststoff bzw. orangerötliches Pulver (810 mg). Säulenchromatographie an Kieselgel mit Aceton / PE = 1:4 → 2:3. **Molekülmasse** = 1231,46 g/mol; **Summenformel** = C₆₁H₉₄N₆O₂₀
¹H-NMR und MS wie oben.

### C-11: tert-Butyl-N-[2-[4-[(1E,6E)-7-[4-[2-(tert-butoxycarbonylamino)ethoxy]-3-[2-[2-(2-hydroxyethoxy)ethoxy]ethoxy]phenyl-3,5-dioxo-hepta-1,6-dienyl)-2-[2-[2-(2-hydroxvethoxy)ethoxy]ethoxy]phenoxy]ethyl]carbamate

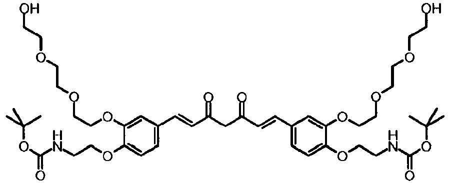

beta-Diketon: Acetylaceton
Vom entsprechenden Aldehyd **E-10** wurden eingesetzt: 830 mg = 2 mmol
**Ausbeute:** 37% der Theorie, oranger, sehr zäher Feststoff (330 mg) Säulenchromatographie an Kieselgel, Aceton / PE = 1:2 → 1:1; präp. DC, Aceton / Petrolether (PE) = 1:1. **Molekülmasse** = 891,03 g/mol; **Summenformel** = C₄₅H₆₆N₂O₁₆
**¹H NMR** (600 MHz, CDCl₃), δ = 7.58 (d, *J* = 15.7 Hz, 2H), 7.20 - 7.06 (m, 4H), 6.88 (d, *J* = 8.1 Hz, 2H), 6.48 (d, *J* = 15.8 Hz, 2H), 5.91 (s, 2H), 4.25 - 4.17 (m, 4H), 4.12 - 4.04 (m, 4H), 3.93 - 3.86 (m, 4H), 3.83 - 3.46 (m, 20H), 1.44 (s, 18H). **MS** (ESI, CH₂Cl₂/MeOH + 10 mmol NH₄OAc): 891.7 (92%, MH⁺), 446.3 (100%, (M+2H⁺)²⁺).

### C-23: tert-Butyl-N-[2-[4-[(1E,6E)-7-[4-[2-(tert-butoxycarbonylamino)ethoxyl-3-methoxyphenyl]-2,6-dimethyl-3,5-dioxo-hepta-1,6-dienyl]-2-methoxyphenoxy]ethyl]carbamat

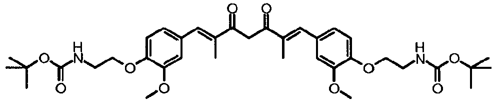

beta-Diketon: 3,5-Heptandion.
Vom entsprechenden Acetophenon **E-3** wurden eingesetzt: 608 mg = 2 mmol
**Ausbeute:** 72% der Theorie, oranger Feststoff (490 mg). Säulenchromatographie an Kieselgel, Aceton / PE = 2:5 → 1:2; präp. DC, Aceton / PE = 1:2. **Molekülmasse** = 682,82 g/mol; **Summenformel** = C₃₇H₅₀N₂O₁₀
**¹H NMR** (300 MHz, CDCl₃), δ = 7.55 (s, 2H), 7.07 - 6.88 (m, 6H), 6.31 (s, 1H), 5.16 (s, 2H), 4.12 (t, *J* = 5.0 Hz, 4H), 3.89 (s, 6H), 3.57 (q, *J* = 5.1 Hz, 4H), 2.18 (s, 6H), 1.45 (s, 18H). **MS** (ESI, CH₂Cl₂/MeOH + 10 mmol NH₄OAc): 705.2 (MNa⁺, 67%), 683.4 (MH⁺, 100%), 627.3 (MH⁺ - C₄H₉, 4%), 583.3 (MH⁺-boc, 63%), 527.2 (MH⁺ - boc -C₄H₉, 27%).

### C-24: tert-Butyl-N-[2-[4-[(1E,6E)-7-[4-[2-(tert-butoxycarbonylamino)ethoxy]-3-methoxyphenyl]-1,2,6-trimethyl-3,5-dioxo-octa-1,6-dienyl]-2-methoxyphenoxy]ethyl]carbamat

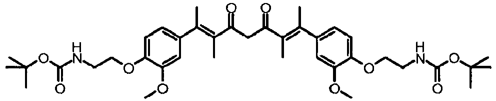

beta-Diketon: 3,5-Heptandion
Vom entsprechenden Acetophenon **E-15** wurden eingesetzt: 608 mg = 2 mmol
**Ausbeute:** 19% der Theorie, oranger, sehr zäher Feststoff (137 mg) Säulenchromatographie an Kieselgel, Aceton / PE = 2:5 → 1:2; präp. DC, Aceton / PE = 1:2. **Molekülmasse** = 710,87 g/mol; **Summenformel** = C₃₉H₅₄N₂O₁₀
**¹H NMR** (300 MHz, CDCl₃), δ = 7.12 - 6.91 (m, 6H), 6.26 (s, 1H), 5.14 (s, 2H), 4.13 (t, *J* = 5.3 Hz, 4H), 3.91 (s, 6H), 3.56 (q, *J* = 5.2 Hz, 4H), 2.17 (s, 6H), 2.12 (s, 6H), 1.44 (s, 18H). **MS** (ESI, CH₂Cl₂/MeOH + 10 mmol NH₄OAc): 732.4 (MNa⁺, 88%), 710.4 (MH+, 100%), 654.4 (8%, MH⁺-C₄H₉), 610.4 (53%, MH⁺-boc).

### C-12: [2-(4-{7-[4-(2-tert-Butoxycarbonylamino-ethoxy)-3-methoxy-phenyl]-3,5-dioxohepta-4-methyl-1,6-dieny/}-2-methoxy-phenoxy)-ethyl]-carbaminsäure-tert-butylester

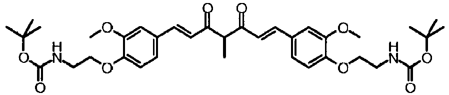

beta-Diketon: 3-Methyl-2,4-pentanedion
Vom entsprechenden Aldehyd **E-3** wurden eingesetzt: 2.94 g = 0.01 mol Säulenchromatographie an Kieselgel mit Aceton / PE = 1:3 → 2:3
**Ausbeute:** 59% der Theorie, oranger, zäher Feststoff bzw. orangegelbes Pulver (403 mg). **Molekülmasse** = 682,82 g/mol; **Summenformel** = C₃₆H₄₈N₂O₁₀
**¹H NMR** (300 MHz, CDCl₃), δ = 7.74 - 7.56 (m, 2H), 7.20 - 6.82 (m, 7H), 6.70 (d, *J* = 15.9 Hz, 1H), 5.13 (s, 2H), 4.10 (dd, *J* = 10.7, 5.3 Hz, 4H), 3.92 (s, 3H), 3.88 (s, 3H), 3.56 (m, 4H), 2.17 (m, 3H), 1.44 (s, 9H), 1.43 (s, 9H). **MS** (ESI, CH₂Cl₂/MeOH + 10 mmol NH₄OAc): 691.3 (43%, MNa⁺), 669.3 (100%, MH⁺), 569.3 (17%, MH⁺-boc).

### 2.2 Modifizierung mit Guanidin

### C-14: tert-Butyl-(2,2'-(4,4'-((1E,6E)-3,5-dioxohepta-1,6-diene-1,7-diyl)bis(2-methoxy-4,1-phenylene))bis(oxy)bis(ethane-2,1-diyl))bis(azanediyl)bis((tert-butoxycarbonylamino)methan-1-yl-1-ylidene)dicarbamat

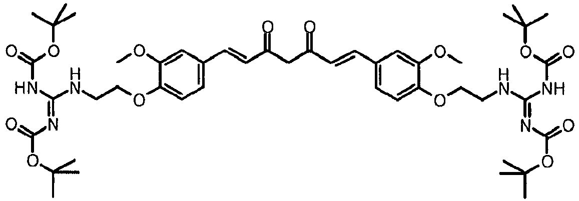

N,N'-di-Boc-N"-triflylguanidin wurde analog der in Organic Syntheses, Coll. Vol. 10, p.266 (2004); Vol. 78, p.91 (2002) beschriebenen Methode hergestellt.

Curcumin **C-2** wurde in Dichlormethan (DCM) bei Raumtempemperatur (RT) für 5 h mit Triflouressigsäure (TFA) gefällt. Das erhaltene Trifluoroacetate-Salz wurde abzentrifugiert. Zu N,N'-di-Boc-N"-triflylguanidin (0.82 g, 2 mmol) in Dichloromethan (10 mL) wurde langsam Triethylamin (0.51 g, 0.66 mL, 5 mmol) mittels Spritze bei 2-5°C zugetropft. Curcumin C-2 trifluoroacetate (550 mg, 0.8 mmol) wurde zugegeben. Nach 5 h Rühren bei Raumtemperatur, wurde mit Dichlormethan (30 mL) verdünnt und die organische Phase mit wässrigem Kaliumhydrogensulfat (3 %, 20 mL) und Wasser (20 mL) gewaschen. Nach Trocknen über MgSO₄, wurde die Lösung gefiltert und einrotiert. Das Rohmaterial wurde durch Säulenchromatographie an Kieselgel mit Aceton/Petroleumether (PE) gereinigt (Aceton / PE = 1:3 → 1:2). Ausbeute: 47% der Theorie, oranger, zäher Feststoff bzw. orangegelbes Pulver (437 mg).
**Molekülmasse** = 930,08 g/mol; **Summenformel** = C₄₇H₆₆N₆O₁₄
**¹H NMR** (300 MHz, CDCl₃), δ = 11.47 (s, 2H), 8.79 (s, 2H), 7.59 (d, *J* = 15.8 Hz, 2H), 7.16 - 7.05 (m, 4H), 7.01 (d, *J* = 8.3 Hz, 2H), 6.50 (d, *J* = 15.8 Hz, 2H), 4.20 (t, *J* = 5.3 Hz, 4H), 3.92 (s, 6H), 3.89 - 3.81 (m, 4H), 1.51 (s, 18H), 1.49 (s, 18H). **MS** (ESI, CH₂Cl₂/MeOH + 10 mmol NH₄OAc): 939.6 (9%, MH⁺), 470.4 (100%, (M+2H⁺)²⁺).

### 2.3 Synthese substituierter Curcumine durch Mitsonubo-Reaktion

Die Synthese wurde analog der in Lepore, S. D. und He, Y.: ("Use of Sonication for the Coupling of Sterically Hindered Substrates in the Phenolic Mitsunobu Reaction"; J.Org.Chem. 68, 2003, Seiten 8261 bis 8263) beschriebenen Methode durchgeführt.

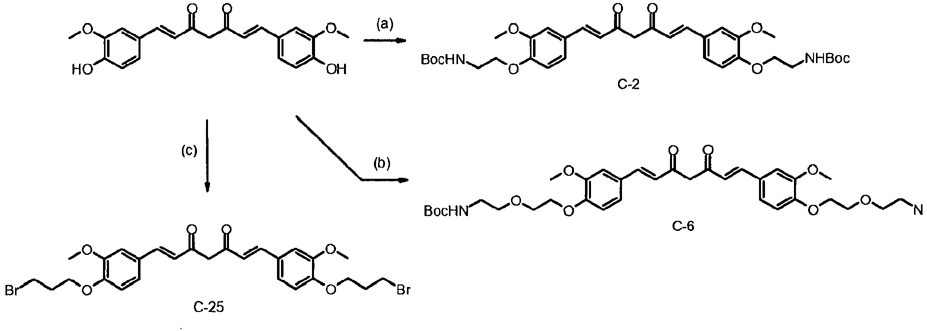

**Übersicht 5**: Synthese substituierter Curcumine durch Mitsonubo-Reaktion ausgehend von Curcumin; Bedingungen: (a) 2-N-tert-Butyloxycarbonyl-aminoethanol, DEAD, PPh₃, DMF oder THF oder DCM, 0°C → RT; (b) 2-(2-N-tert-Butoxycarbonyl-aminoethoxy)ethanol, DEAD, PPh₃, DMF oder THF oder DCM, 0°C → RT; (c) 3-Bromo-propan-1-ol, DEAD, PPh₃, THF oder DCM, 0°C → RT;

### 2.3.1 Allgemeine Vorschrift:

Curcumin (0.36 g, 1 mmol) wurde zusammen mit Triphenylphosphin (1.04 g, 4 mmol) und dem entsprechenden Boc-geschützten Aminoalkohol (1 mmol bzw. 3 mmol) in trockenem THF vorgelegt (4 mL). Diethylazodicarboxylat (DEAD) (0.7 g, 4 mmol, 40% in Toluen) in trockenem THF (6 mL) wurde über 20 Minuten bei ca 2°C bis 5°C zugetropft und der Ansatz dann 4 h bei RT im Dunklen gerührt. Der Ansatz wurde mit 40 mL Essigsäureethylester (EE) verdünnt und die organische Lösung dreimal mit je 20 mL Wasser ausgeschüttelt. Die organische Phase wurde abgetrennt, über MgSO₄ getrocknet und einrotiert. Der Rückstand wurde durch SC an Kieselgel mit Aceton/PE gereinigt (dryload). Das Produkt wurde zur weiteren Reinigung in möglichst wenig EE gelöst und durch Zugabe der 10fachen Menge Petrolether gefällt.

### [2-(4-{7-[4-hydroxy-3-methoxy-phenyl]-3,5-dioxo-hepta-1,6-dienyl}-2-methoxy-phenoxy)-ethyl]-carbaminsäure-tert-butylester

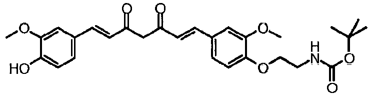

Vom entsprechenden Boc-geschützten Aminoalkohol tert-Butyl-N-(2-hydroxyethyl)carbamat wurden eingesetzt: 161 mg = 1 mmol. Säulenchromatographie an Kieselgel mit Aceton / Petrolether (PE) = 1:2; Präparative Dünnschicht-Chromatografie mit Aceton / Petrolether (PE) = 2:3.
**Ausbeute:** 41% der Theorie, oranger, zäher Feststoff bzw. orangegelbes Pulver (210 mg). **Molekülmasse** = 511,58 g/mol; **Summenformel** = C₂₈H₃₃NO₈

### C-2: [2-(4-{7-[4-(2-tert-Butoxycarbonylamino-ethoxy)-3-methoxy-phenyl]-3,5-dioxo-hepta-1,6-dienyl}-2-methoxy-phenoxy)-ethyl]-carbaminsäure-tert-butyl ester

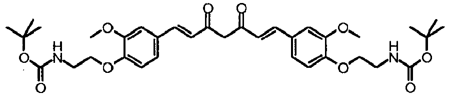

Vom entsprechenden Boc-geschützten Aminoalkohol tert-Butyl-N-(2-hydroxyethyl)carbamat wurden eingesetzt: 483 mg = 3 mmol. Säulenchromatographie an Kieselgel mit Aceton / Petrolether (PE) = 1:3 → 1:2.
**Ausbeute:** 71% der Theorie, oranger, zäher Feststoff (465 mg).
**Molekülmasse** = 654,76 g/mol; **Summenformel** = C₃₅H₄₆N₂O₁₀
¹H-NMR und MS wie oben.

### C-6: (2-{2-[4-(7-{4-[2-(2-tert-Butoxycarbonylamino-ethoxy)-ethoxy]-3-methoxy-phenyl}-3,5-dioxo-hepta-1,6-dienyl)-2-methoxy-phenoxy]-ethoxy}-ethyl)-carbaminsäure-tert-butylester

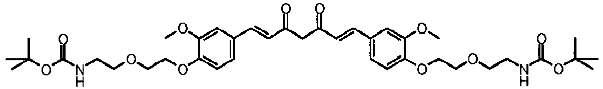

Vom entsprechenden Boc-geschützten Aminoalkohol 2-(2-N-tert-Butoxycarbonyl-aminoethoxy)ethanol wurden eingesetzt: 715 mg = 3 mmol.
**Ausbeute:** 52% der Theorie, oranger, zäher Feststoff bzw. oranges Pulver (386 mg) Säulenchromatographie an Kieselgel mit Aceton / PE = 1:2 → 2:3
**Molekülmasse** = 742,87 g/mol; **Summenformel** = C₃₉H₅₄N₂O₁₂
¹H-NMR und MS wie oben.

### C-25: (1E,6E)-1,7-bis[4-(3-bromopropoxy)-3-methoxy-phenyl]hepta-1,6-diene-3,5-dion

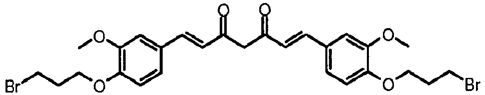

Curcumin (2.00 g, 5.4 mmol) wurde zusammen mit Triphenylphosphin (5.26 g, 20 mmol) und 3-Bromo-propan-1-ol (2.25 g, 1.51 mL, 16.2 mmol) in trockenem THF (40 mL) vorgelegt und bei 0°C entgast. Diethylazodicarboxylat (DEAD) (7 mL, 40% in Toluen, 20 mmol) wurde über 20 Minuten zugetropft und der Ansatz über Nacht im auftauenden Eisbad unter Feuchtigkeitsausschluss im Dunklen gerührt. Der Ansatz wurde in die dreifache Menge Diethylether eingegossen. Nach dem Absetzen des Niederschlags absetzen wurde die überstehende Lösung vorsichtig abdekantiert.

Der Rückstand wurde zweimal mit je 50 mL Diethylether ausgelaugt. Die vereinigten organischen Lösungen wurden mit 100 mL Wasser ausgeschüttelt. Die organische Phase wurde abgetrennt, über MgSO₄ getrocknet und einrotiert. Der Rückstand wurde in Aceton/PE 1:2 suspendiert und die gelborange Lösung von den farblosen Kristallen abfiltriert. Der Filterkuchen wurde mehrmals mit kleinen Portionen des kalten Lösungsmittelgemisches nachgewaschen und das Filtrat einrotiert. Der Rückstand wurde durch plug-filtration an Kieselgel mit Aceton/PE 1:2 vorgereinigt. Nach Abziehen des Lösungsmittelgemisches wurde der zurückbleibende Feststoff in Ethanol im Ultraschallbad suspendiert (30 mL), zentrifugiert und die überstehende Lösung abgegossen. Dieser Waschschritt wurde insgesamt dreimal wiederholt, danach wurde das Produkt an der Luft getrocknet. **Ausbeute:** 2,08 g oranges Pulver, 63 % der Theorie. **Molekülmasse** = 610,34 g/mol; **Summenformel** = C₂₇H₃₀Br₂O₆
**¹H NMR** (300 MHz, CDCl₃), δ = 7.61 (d, *J* = 15.8 Hz, 2H), 7.16 - 7.04 (m, 4H), 6.92 (d, *J* = 8.3 Hz, 2H), 6.50 (d, *J* = 15.8 Hz, 2H), 4.20 (t, *J* = 6.0 Hz, 4H), 3.91 (s, 6H), 3.64 (t, *J* = 6.4 Hz, 4H), 2.39 (p, *J* = 6.1 Hz, 4H). **MS** (ESI, CH₂Cl₂/MeOH + 10 mmol NH₄OAc): 609.1 (57%, MH⁺), 611.0 (100%, MH⁺).

Ausgehend von Bis-(3-bromo-propoxy)curcumin **C-25** wurden die Curcumine **C-27** bis **C-30** hergestellt:

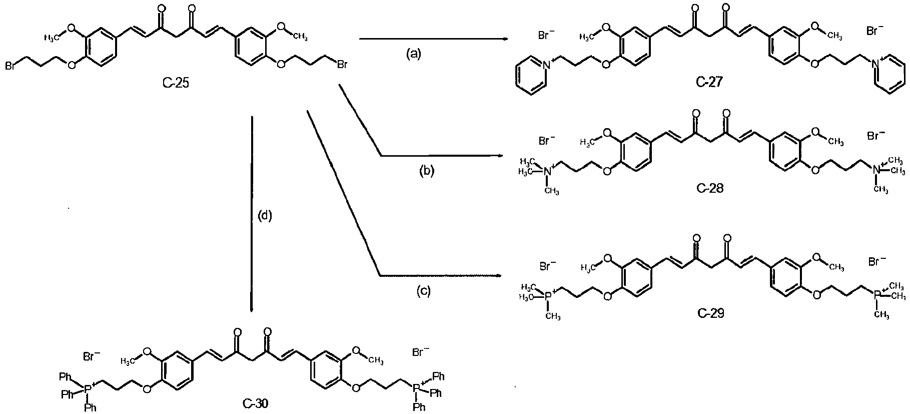

**Übersicht 6**: Synthese symmetrisch substituierter Curcumine ausgehend von C-25; Bedingungen: (a) Pyridin, DMF, 50°C, über Nacht; (b) Trimethylamin in Ethanol, DMF, 50°C, über Nacht; (c) Trimethylphosphin in Toluol, DMF, Argon, 50°C, über Nacht; (d) Triphenylphosphin, DMF, 50°C, über Nacht;

### 2.4 Curcumine mit quaternären Ladungen

Das Bis-(3-bromo-propoxy)curcumin (61 mg, 0.1 mmol) C-25 wurde in trockenem DMF (3 mL) vorgelegt. Trimethylamin (2 mL, 5.6 M in Ethanol, 11 mmol) oder Pyridin (790 mg, 0.8 mL, 10 mmol) in DMF (2 mL) wurde über 5 mins. per Spritze über ein Septum zugetropft und der Ansatz über Nacht unter Feuchtigkeitsausschluss im Dunklen bei 50°C gerührt.

Der Ansatz wurde in die fünffache Menge Diethylether eingegossen. Man lässt den Niederschlag absetzen und dekantiert die überstehende Lösung vorsichtig ab. Der Rückstand wurde mehrmals mit Diethylether gewaschen und anschließend in 15 mL Chloroform/Diethylether 1:1 suspendiert. Man lässt vollständig absetzen, gießt die überstehende Lösung ab und trocknet den Niederschlag an der Hochvakuumpumpe. Das Produkt wurde durch HPLC gereinigt.

### lonenaustausch-Chromatografie

Eine Säule wurde mit Amberlite 954 beladen und der lonentauscher mit 0.1M HCl konditioniert. Nach waschen mit Wasser wurde auf Wasser/MeOH/MeCN 3:1:1 umkonditioniert. Anschließend wurde das TFA-Salz in wenig der Solvensmischung langsam über das Harz eluiert und mit wenig Solvens nachgewaschen. Nach Abziehen der Lösungsmittel unter vermindertem Druck, wurde die zurückbleibende wässrige Lösung gefriergetrocknet.

### C-28: 3,3'-(4,4'-((1E,6E)-3,5-dioxohepta-1,6-diene-1,7-diyl)bis(2-methoxy-4,1-phenylene))bis(oxy)bis(N,N,N-trimethylpropan-1-aminium) chlorid

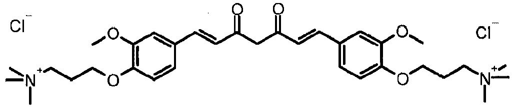

**Ausbeute:** 30 mg oranger Feststoff, 45 % der Theorie
**Molekülmasse**: 568.76 +2x 35.45 = 639.66 g/mol; **Summenforme:** C₃₃H₄₈N₂O₆Cl₂
¹H-NMR und MS siehe unten (SA-CUR-10a).

### C-27: 1,1'(3,3'-(4,4'-((1E,6E)-3,5-dioxohepta-1,6-diene-1,7-diyl)bis(2-methoxy-4,1-phenylene))bis(oxy)bis(propane-3,1-diyl))dipyridiniumchlorid

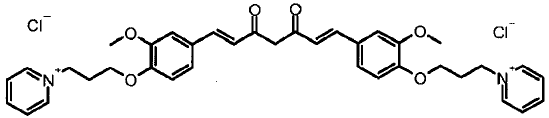

**Ausbeute:** 36 mg oranger Feststoff, 78 % der Theorie
**Molekülmasse:** 608.74+2x 35.45 = 679.64 g/mol; **Summenformel:** C₃₇H₄₀N₂O₆Cl₂
¹H-NMR und MS siehe unten (SA-CUR-10c).

### 2.5 Curcumine mit Phosphonium Gruppen

Das Bis-(3-bromo-propoxy)curcumin **C-25** (122 mg, 0.2 mmol) wurde in trockenem Dichlormethan (DCM) (10 mL) vorgelegt und unter Stickstoff gerührt. Das verwendete Phosphin in Toluen (2 mL, 1 M, 2 mmol) wurde über 5 Minuten per Spritze über ein Septum zugetropft. Der Ansatz wurde über Nacht im Schlenckrohr unter Feuchtigkeitsausschluss, im Dunklen und in Schutzgasatmosphäre bei 50°C gerührt. Alle flüchtigen Komponenten wurden bei vermindertem Druck abgezogen und der Rückstand mit Hilfe eines Ultraschallbades in 30 mL Diethylether suspendiert. Man lässt den Niederschlag absetzen und dekantiert die überstehende Lösung vorsichtig ab. Der Rückstand wurde mehrmals mit Diethylether gewaschen. Nach dem Ambsetzen des Niederschlages wurde die überstehende Lösung abgegossen und der Niederschlag an der Hochvakuumpumpe getrocknet.

### lonenaustausch-Chromatografie

Eine kurze Säule wurde mit Amberlite 954 beladen und der lonentauscher mit 0.1M HCl konditioniert. Nach waschen mit Wasser wurde auf Wasser/MeOH/MeCN 3:1:1 umkonditioniert. Anschließend wurde das TFA-Salz in wenig der Solvensmischung langsam über das Harz eluiert und mit wenig Solvens nachgewaschen. Nach Abziehen der Lösungsmittel unter vermindertem Druck, wurde die zurückbleibende wässrige Lösung gefriergetrocknet. Quantative Ausbeute.

### C-29: 3,3'-(4,4'-((1E,6E)-3,5-dioxohepta-1,6-diene-1,7-diyl)bis(2-methoxy-4,1-phenylene))bis(oxy)bis(trimethylpropan-1-phosphonium) chlorid

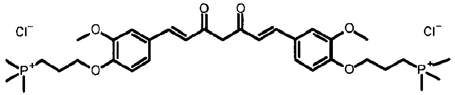

verwendetes Phosphin: Trimethylphosphin
**Ausbeute:** 58 mg oranger Feststoff, 21 % der Theorie
**Molekülmasse:** 602.69 +2x 35.45 = 673.59 g/mol; **Summenformel:** C₃₃H₄₈P₂O₆Cl₂
¹H-NMR und MS siehe unten SA-CUR-15a.

### C-30: 3,3'-(4,4'-((1E,6E)-3,5-dioxohepta-1,6-diene-1,7-diyl)bis(2-methoxy-4,1-phenylene))bis(oxy)bis(triphenylpropan-1-phosphonium) chlorid

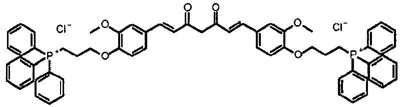

verwendetes Phosphin: Triphenylphosphin
**Ausbeute:** 107 mg oranger Feststoff, 56 % der Theorie
**Molekülmasse:** 975.12 +2x 35.45 = 1046.02 g/mol; **Summenformel:** C₆₃H₆₀P₂O₆Cl₂
¹H-NMR und MS siehe unten SA-CUR-15b.

### 2.6 Synthese substituierter Curcumine durch Alkylierung

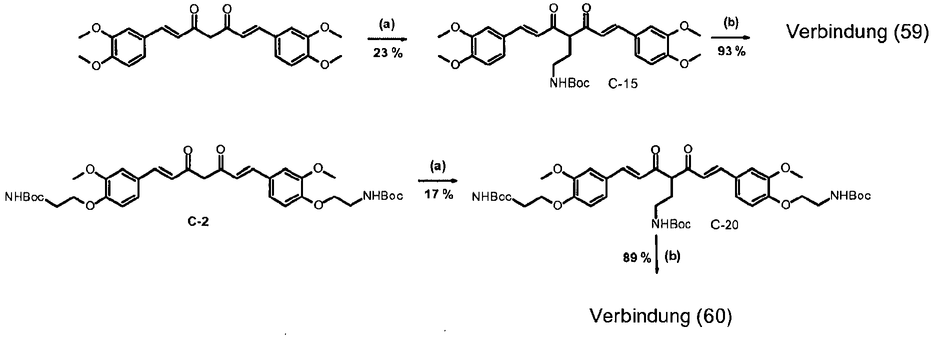

**Übersicht 7**: Synthese substituierter Curcumine durch Alkylierung; Bedingungen: (a) 2-N-tert-Butyloxycarbonyl-aminobromid, DBU, Toluol oder DCM oder THF, 0°C → RT → 60°C; (b) DCM, TFA, RT, 5h; dann lonentauscher Amberlite IRA-958, Wasser

Tetramethoxycurcumin (0,4 g, 1 mmol) oder Curcumin **C-2** (0,6 g, 1 mmol) und 2-N-tert-butoxycarbonylamonoethylbromid (0.34 g, 1,5 mmol) wurden in Toluen (4 mL) vorgelegt. 1,8-Diazabicyclo[5.4.0]undec-7-en (DBU) (0.15 g, 1 mmol) wurde zugegeben und die Mischung 15h bei Raumtemperatur gerührt. Die Lösung wurde mit Ethylacetat verdünnt (30 mL), mit Kochsalzlösung (30 mL), Kaliumhydrogensulfatlösung (5%, 30 mL) und Wasser (30 mL) gewaschen. After Nach Trocknen über MgSO₄ wurde einrotiert und der Rückstand durch Säulenchromatographie an Kieselgel mit Ethylacetate / Petroleum ether und anschließend durch präparative Dünnschichtchromatographie gereinigt.

### C-15: 1,7-Bis-(3,4-dimethoxyphenyl)-hepta-4-(2-tert-butoxycarbonylamino-ethyl)-1,6-dien-3,5-dion

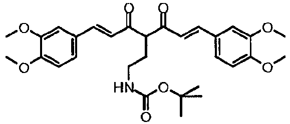

Säulenchromatographie an Kieselgel mit Aceton / PE = 1:1
**Ausbeute:** 14% der Theorie, oranger Feststoff bzw. orangegelbes Pulver (76 mg)
**Molekülmasse** = 539,63 g/mol; **Summenformel** = C₃₀H₃₇NO₈
**¹H NMR** (300 MHz, CDCl₃), δ = 7.72 - 7.53 (m, 2H), 7.22 - 7.04 (m, 4H), 6.86 (d, *J* = 8.1 Hz, 2H), 6.54 (d, *J* = 15.7 Hz, 2H), 5.11 (s, 1H), 3.88 - 3.72 (m, 2H), 3.93 (s, 12H), 2.81 - 2.70 (m, 2H), 1.43 (s, 9H). **MS** (ESI, CH₂Cl₂/MeOH + 10 mmol NH₄OAc): 562.3 (MNa⁺, 13%), 540.3 (MH⁺, 100%), 484.2 (2%, MH⁺-C₄H₉), 440.3 (61%, MH⁺-boc).

### C-20: [2-(4-{7-[4-(2-tert-Butoxycarbonylamino-ethoxy)-3-methoxy-phenyl]-3,5-dioxo-hepta-4-(2-tert-Butoxycarbonylamino-ethyl)-1,6-dienyl}-2-methoxy-phenoxy)-ethyl]-carbamic acid tert-butyl ester

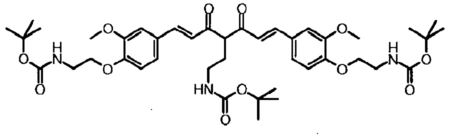

Säulenchromatographie an Kieselgel mit Aceton / PE = 1:3 → 1:1
**Ausbeute:** 12% der Theorie, oranger, orangegelbes Pulver (96 mg).
**Molekülmasse** = 797,95 g/mol; **Summenformel** = C₄₂H₅₉N₃O₁₂
**¹H NMR** (300 MHz, CDCl₃), δ = 7.64 (d, *J* = 15.7 Hz, 2H), 7.24 - 7.01 (m, 6H), 6.78 (d, *J* = 15.8 Hz, 2H), 5.16 (s, 2H), 4.12 (dd, *J* = 10.6, 5.4 Hz, 4H), 3.93 (s, 3H), 3.90 (s, 3H), 3.86 - 3.75 (m, 2H), 3.54 (m, 4H), 2.82 - 2.71 (m, 2H), 1.45 (s, 9H), 1.44 (s, 9H). **MS** (ESI, CH₂Cl₂/MeOH + 10 mmol NH₄OAc): 820.4 (MNa⁺, 69%), 798.4 (MH⁺, 100%), 742.4 (6%, MH⁺-C₄H₉), 698.3 (36%, MH⁺-boc).

### 2.7 Synthese unsymmetrisch substituierter Curcumine

### Allgemeine Vorschrift:

### Schritt 1:

Als beta-Diketon wurden Acetylaceton (1.5 g, 15 mmol) oder 3-Methyl-2,4-pentanedion (3.42 g, 30 mmol) verwendet. Das entsprechende beta-Diketon und Boroxid B₂O₃ (1.5 g, 21 mmol) wurden in Ethylacetat (20 mL) suspendiert und 60 Minuten bei 70°C gerührt. Das substituierte Benzaldehyd **E-3** (1.02 g, 3.5 mmol) in Ethylacetat (5 mL) und Tributylborate (1.68 g, 7 mmol) wurden zugegeben und der Ansatz für eine halbe Stunde bei 85°C gerührt. Nun wurde über 10 Minuten n-Butylamin (0.5 mL in 3 mL Ethylacetat) zugetropft. Nach weiteren drei Stunden Rühren bei 80°C wurde auf 50°C abgekühlt und zur Hydrolyse des Borkomplexes 100 mL 50%ige Essigsäure zugegeben.

Nach Rühren über Nacht bei Raumtemperatur wurde das Lösungsmittelgemisch unter Lichtschutz abgezogen und der Rückstand dreimal mit EE extrahiert (je 30 mL). Die vereinigten organischen Phasen wurden zweimal mit Wasser (je 50 mL) gewaschen, über MgSO₄ getrocknet und schließlich das Lösungsmittel bei vermindertem Druck abgezogen.

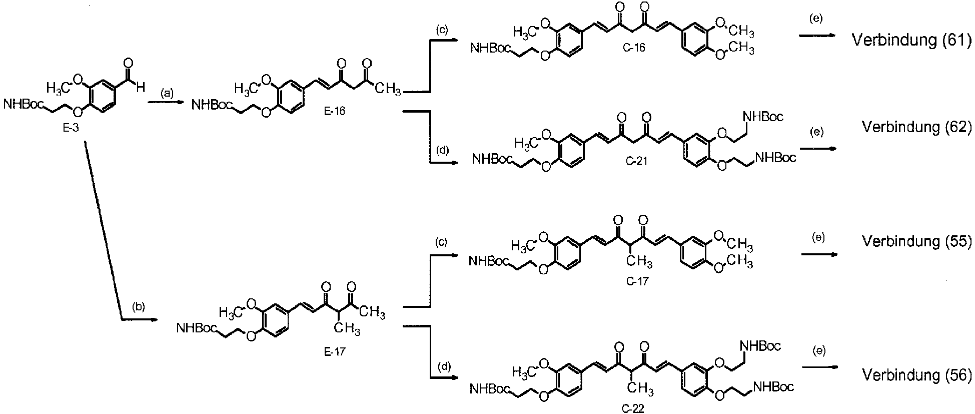

**Übersicht 7**: Synthese unsymmetrisch substituierter Curcumine über die entsprechenden Zwischenstufen (E-16 und E-17); Bedingungen: (a) Acetylaceton, B₂O₃, B(OBu)₃, n-Butylamin, DMF oder Ethylacetat, 60 - 80°C, 6h, dann Hydrolyse mit HOAc 40% über Nacht; (b) 3-Methyl-pentan-2,4-dion, B₂O₃, B(OBu)₃, n-Butylamin, Ethylacetat, 80°C, 6h, dann Hydrolyse mit HOAc 40% über Nacht; (c) 3,4-Dimethoxy-benzaldehyd, B₂O₃, B(OBu)₃, n-Butylamin, DMF oder Ethylacetat, 70°C, 6h, dann Hydrolyse mit HOAc 40% über Nacht; (d) E-11, B₂O₃, B(OBu)₃, n-Butylamin, DMF oder Ethylacetat, 80°C, 6h, dann Hydrolyse mit HOAc 40% über Nacht; (e) DCM, TFA, RT, 5h; dann Ionentauscher Amberlite IRA-958, Wasser

### E-16: tert-butyl 2-(4-(3,5-dioxohex-1-enyl)-2-methoxyphenoxy)ethylcarbamate

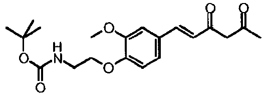

Als beta-Diketon wurde Acetylaceton verwendet. Die Aufreinigung erfolgt durch Säulenchromatographie an Kieselgel mit Aceton / PE = 2:5 → 1:2. Das entsprechend symetrisch substituierte Curcumin ist in EtOH schlechter löslich als das Produkt.
**Ausbeute:** 687 mg, 52% der Theorie, gelber Feststoff.
**Molekülmasse** = 377,44 g/mol; **Summenformel** = C₂₀H₂₇NO₆
**¹H NMR** (300 MHz, CDCl₃), δ = 7.53 (d, *J* = 15.8 Hz, 1H), 7.12 - 7.00 (m, 2H), 6.88 (d, *J* = 8.3 Hz, 1H), 6.34 (d, *J* = 15.8 Hz, 1H), 5.16 (s, 1H), 4.10 (t, *J* = 5.1 Hz, 2H), 3.90 (s, 3H), 3.56 (d, *J* = 5.3 Hz, 2H), 2.16 (s, 3H), 1.44 (s, 9H). **MS** (ESI, CH₂Cl₂/MeOH + 10 mmol NH₄OAc): 777.4 (46%, 2MNa⁺), 400.2 (38%, MNa⁺), 378.2 (12%, MH⁺), 322.1 (100%, MH⁺-C₄H₉), 278.1 (47%, MH⁺ - boc).

### E-17: tert-butyl 2-(4-(3,5-dioxo-4-methyl-hex-1-enyl)-2-methoxyphenoxy)-ethylcarbamate

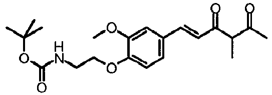

Als beta-Diketon wurde 3-Methyl-2,4-pentanedion verwendet. Die Aufreinigung erfolgt durch Säulenchromatographie an Kieselgel mit Aceton / PE = 1:3 und präparativem DC mit Aceton / PE = 1:2.
**Ausbeute**: 644 mg, 47% der Theorie, gelber Feststoff.
**Molekülmasse** = 391.47 g/mol; **Summenformel** = C₂₁H₂₉NO₆
**¹H NMR** (300 MHz, CDCl₃), δ = 7.58 (dd, *J* = 15.7, 6.5 Hz, 1H), 7.13 (d, *J* = 8.3 Hz, 1H), 7.05 (dd, *J* = 4.9, 1.8 Hz, 1H), 6.89 (d, *J* = 8.3 Hz, 1H), 6.74 (dd, *J* = 37.9, 15.7 Hz, 1H), 5.11 (s, 1H), 4.10 (t, *J* = 5.1 Hz, 2H), 3.91 (s, 3H), 3.56 (m, 2H), 2.25 + 2.18 (s, 3H), 2.02 (s, 3H), 1.44 (s, 10H). **MS** (ESI, CH₂Cl₂/MeOH + 10 mmol NH₄OAc): 805.5 (24%, 2MNa⁺), 414.3 (53%, MNa⁺), 392.3 (19%, MH⁺), 336.2 (100%, MH⁺-C₄H₉), 292.2 (34%, MH⁺ - boc).

### Schritt 2:

tert-Butyl-2-(4-(3,5-dioxohex-1-enyl)-2-methoxyphenoxy)ethylcarbamat (185 mg, 0.5 mmol) oder tert-Butyl-2-(4-(3,5-dioxo-4-methyl-hex-1-enyl)-2-methoxyphenoxy)ethylcarbamat (191 mg, 0.5 mmol) und Boroxid B₂O₃ (0.07 g, 1 mmol) wurden in Ethylacetat (3 mL) suspendiert und 60 Minuten bei 80°C gerührt. Das substituierte Benzaldehyd (0.6 mmol) in Ethylacetat (3 mL) sowie Tributylborat (0.24 g, 1 mmol) wurden nacheinander zugegeben und der Ansatz für eine halbe Stunde gerührt bei 80°C. Nun wurde über 5 Minuten n-Butylamin (0.1 mL in 1 mL EE) zugetropft. Nach weiteren drei Stunden Rühren bei 80°C wurde die leicht abgekühlte, noch ca. 50°C warme Lösung in 40 mL 50%ige Essigsäure eingegossen. Nach Rühren über Nacht bei Raumtemperatur unter Lichtschutz wurde das Lösungsmittelgemisch bei vermindertem Druck abgezogen und der Rückstand dreimal mit EE extrahiert (je 20 mL). Die vereinigten organischen Phasen wurden zweimal mit Wasser (je 20 mL) gewaschen, über MgSO₄ getrocknet und schließlich das Lösungsmittel bei vermindertem Druck abgezogen. Die Aufreinigung erfolgt durch Säulenchromatographie an Kieselgel.

### C-16: tert-butyl 2-(4-((1E,6E)-7-(3,4-dimethoxyphenyl)-3,5-dioxohepta-1,6-dienyl)-2-methoxyphenoxy)ethylcarbamat

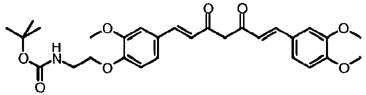

Vom entsprechenden Aldehyd **E-16** wurden eingesetzt: 100 mg = 0,6 mmol Säulenchromatographie mit Aceton / PE = 1:2 → 2:3
**Ausbeute:** 121 mg, 46% der Theorie, oranger, zäher Feststoff bzw. orangegelbes Pulver.
**Molekülmasse** = 525,60 g/mol; **Summenformel** = C₂₉H₃₅NO₈
**¹H NMR** (300 MHz, CDCl₃), δ = 7.60 (dd, *J* = 15.7, 4.5 Hz, 2H), 7.15 - 7.06 (m, 4H), 6.89 (dd, *J* = 8.3, 3.3 Hz, 2H), 6.50 (d, *J* = 15.8 Hz, 2H), 5.13 (s, 1H), 4.11 (t, *J* = 5.0 Hz, 2H), 3.94 (s, 3H), 3.93 (s, 3H), 3.92 (s, 3H), 3.62 - 3.52 (m, 2H), 1.45 (s, 9H).
**MS** (ESI, CH₂Cl₂/MeOH + 10 mmol NH₄OAc): 1073.5 (19%, 2M+Na⁺), 526.2 (100%, MH⁺), 470.2 (21%, MH⁺-C₄H₉).

### C-21: 2-(4-((1E,6E)-7-(3,4-bis(2-(tert-Butoxycarbonylamino)ethoxy)phenyl)-3,5-dioxohepta-1,6-dienyl)-2-methoxyphenoxy)ethylcarbamat

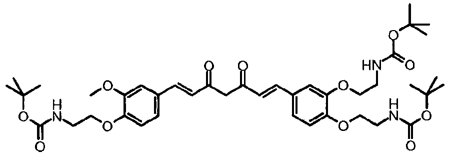

Vom entsprechenden Aldehyd **E-16** wurden eingesetzt: 255 mg = 0.6 mmol Säulenchromatographie mit Aceton / PE = 2:5 → 1:2
**Ausbeute**: 172 mg, 44% der Theorie, oranger, sehr zäher Feststoff.
**Molekülmasse** = 783,92 g/mol; **Summenformel** = C₄₁H₅₇N₃O₁₂
**¹H NMR** (300 MHz, CDCl₃), δ = 7.57 (dd, *J* = 15.7, 9.3 Hz, 2H), 7.17 - 7.05 (m, 4H), 6.90 (dd, *J* = 8.2, 6.6 Hz, 2H), 6.49 (d, *J* = 15.7 Hz, 2H), 5.27 (s, 2H), 5.14 (s, 1H), 4.10 (m, 6H), 3.91 (s, 3H), 3.55 (m, 6H), 1.46 (s, 9H), 1.44 (s, 18H). **MS** (ESI, CH₂Cl₂/MeOH + 10 mmol NH₄OAc): 804.6 (73%, MNa⁺), 784.4 (100%, MH⁺), 684.3 (71%, MH⁺-boc).

### C-17: tert-Butyl-2-(4-((1E,6E)-7-(3,4-dimethoxyphenyl)-3,5-dioxo-4-methyl-hepta-1,6-dienyl)-2-methoxyphenoxy)ethylcarbamat

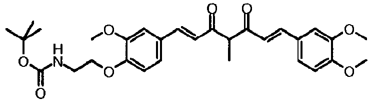

Vom entsprechenden Aldehyd **E-17** wurden eingesetzt: 100 mg = 0,6 mmol Säulenchromatographie mit Aceton / PE = 1:2 → 2:3
**Ausbeute**: 113 mg, 42% der Theorie, oranger, zäher Feststoff.
**Molekülmasse** = 539.63 g/mol; **Summenformel** = C₃₀H₃₇NO₈
**¹H NMR** (300 MHz, CDCl₃), δ = 7.75 - 7.56 (m, 2H), 7.20 - 6.82 (m, 7H), 6.70 (d, J = 15.9 Hz, 1H), 5.15 (s, 1H), 4.15 - 4.06 (m, 2H), 3.95 (s, 3H), 3.91 (s, 3H), 3.89 (s, 3H), 3.57 (m, 2H), 2.18 (s, 3H), 1.44 (s, 9H). **MS** (ESI, CH₂Cl₂/MeOH + 10 mmol NH₄OAc): 562.2 (MNa⁺, 41%), 540.3 (MH⁺, 100%), 484.2 (MH⁺ -C₄H₉, 46%), 440.3 (MH⁺ -boc, 3%).

### C-22: 2-(4-((1E,6E)-7-(3,4-bis(2-(tert-butoxycarbonylamino)ethoxy)phenyl)-3,5-dioxo-4-methyl-hepta-1,6-dienyl)-2-methoxyphenoxy)ethylcarbaminsäure

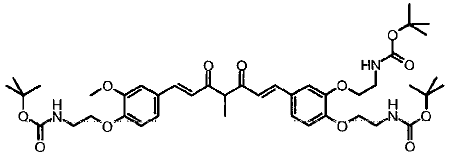

Vom entsprechenden Aldehyd **E-17** wurden eingesetzt: 255 mg = 0.6 mmol Säulenchromatographie mit Aceton / PE = 2:5 → 1:2
**Ausbeute:** 160 mg, 40% der Theorie, oranger, sehr zäher Feststoff.
**Molekülmasse** = 797,95 g/mol; **Summenformel** = C₄₂H₅₉N₃O₁₂
**¹H NMR** (300 MHz, CDCl₃), δ = 7.74 - 7.52 (m, 2H), 7.21 - 6.88 (m, 7H), 6.70 (d, *J* = 15.9 Hz, 1H), 5.29 (s, 2H), 5.16 (s, 1H), 4.10 (t, *J* = 4.8 Hz, 6H), 3.90 (m, 3H), 3.56 (m, 6H), 2.17 (m, 3H), 1.46 (s, 9H), 1.45 (s, 18H). **MS** (ESI, CH₂Cl₂/MeOH + 10 mmol NH₄OAc): 820.4 (100%, MNa⁺), 798.3 (43%, MH⁺), 698.4 (67%, MH⁺ -boc).

### 3. Boc-Entschützung der Curcumine und lonenaustausch-Chromatographie

Das entsprechende Boc-geschützte Curcumin (0.2 mmol, 120 - 160 mg) wurde in DCM (6 mL) gelöst. Unter Rühren wurden 4 mL einer 10%ige Lösung von TFA in DCM (enth. TIS 6%) langsam zugetropft. Nach 5 h Rühren bei RT unter Lichtschutz, wurde das Produkt durch Zugabe von Diethylether (10 mL) gefällt. Der Niederschlag wurde abzentrifugiert und anschließend der Überstand verworfen. Der Feststoff wurde in Diethylether suspendiert (30 mL) und erneut abzentrifugiert. Der Überstand wurde ebenfalls verworfen. Dieser Waschschritt wurde nochmal wiederholt und anschließend das Produkt an der Luft im Dunklen getrocknet.

### lonenaustausch-Chromatographie

Eine Säule wurde mit Amberlite 954 beladen und der lonentauscher mit 0.1M HCl konditioniert. Nach waschen mit Wasser bis zur schwach sauren Reaktion wurde bei Bedarf auf Wasser/MeOH/MeCN Gemisch umkonditioniert. Anschließend wurde das TFA-Salz in möglichst wenig der Solvensmischung langsam über das Harz eluiert und mehrmals mit wenig Solvens nachgewaschen. Nach Abziehen der Lösungsmittel unter vermindertem Druck, wurde die zurückbleibende wässrige Lösung gefriergetrocknet. Quant. Ausbeute.

**Eluent:**

| | |
|---|---|
| Curcumine 12a und 12b: | Wasser/MeOH/MeCN 1:1:1 |
| Curcumine 04, 07, 09b, 11b/c und 14b: | Wasser/MeOH/MeCN 9:4:2 |
| Alle anderen Curcumine: | reines Wasser |

### 4. Curcuminkomplexe

| | | | |
|---|---|---|---|
| *Curcumin 01a BF-Komplex **(BF-SA-CUR-1a)*** | 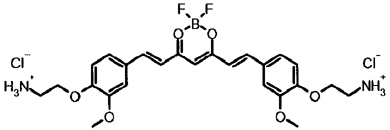 | (1E,6E)-1,7-Bis(4-(2-aminoethoxy)-3-methoxyphenyl)hepta-1,6-diene-3,5-dion Hydrochlorid BF₂-Komplex | Molekülmasse: 504.34 +2x 35.45 = 527.44 g/mol |
| | | | Summenformel: C₂₅H₃₁ N₂O₆BF₂Cl₂ |
| *Roseo-Curcumin 01a Hydrochlorid **(RO-SA-CUR-1a)*** | 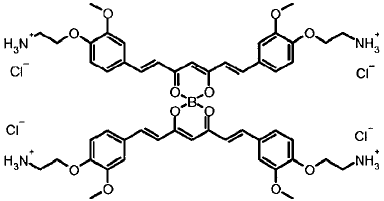 | Bis[(1E,6E)-1,7-bis(4-(2-aminoethoxy)-3-methoxyphenyl)hepta-1,6-diene-3,5-dion Hydrochlorid] Borkomplex | Molekülmasse: 921.88 +4x 35.45 = 1063.68 g/mol |
| | | | Summenformel: C₅₀H₆₂BN₄O₁₂Cl₄ |
| *Curcumin 01a Zink-Komplex **(Zn-SA-CUR-1a)*** | 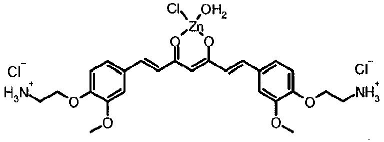 | (1E,6E)-1,7-Bis(4-(2-aminoethoxy)-3-methoxyphenyl)hepta-1,6-diene-3,5-dion Hydrochlorid-Zink Komplex | Molekülmasse: 575.36 +2x 35.45 = 527.44 g/mol |
| | | | Summenformel: C₂₅H₃₁N₂O₇ZnCl₃ |

4.a) SA-CUR-01a TFA-Salz (68 mg, 0.1 mmol) wurde in trockenem DCM (10 mL) vorgelegt. Bortrifluorid Etherat (20 µL, 0.12 mmol) wurde zugetropft und der Ansatz über Nacht gerührt. Die Lösung wurde mit Diethylether (20 mL) verdünnt, auf zwei Blue Caps verteilt und zentrifugiert. Der Niederschlag wurde mehrmals mit Diethylether gewaschen und an der Luft getrocknet. Rotes Pulver (TFA-Salz), Ausbeute quant.

### Ionenaustausch-Chromatographie

Eine kurze Säule wurde mit Amberlite 954 beladen und der lonentauscher mit 0.1 M HCl konditioniert. Nach waschen mit Wasser bis zur schwach sauren Reaktion wurde das TFA-Salz in möglichst wenig Wasser gelöst, langsam über das Harz eluiert und mehrmals mit wenig Solvens nachgewaschen. Die wässrige Lösung wurde gefriergetrocknet. Ausbeute quantitativ.
4.b) SA-CUR-01a Chlorid (108 mg, 0.2 mmol) und Bortrioxid (0.05 mmol) wurden in wässriger HCl (1 M, 2 mL) über Nacht bei Raumtemperatur gerührt. Das Lösungsmittel wurde im Stickstoffstrom abgetrieben und der Rückstand getrocknet. Rotes Pulver, Ausbeute quantitativ.
4.c) SA-CUR-01a Chlorid (54 mg, 0.1 mmol) und Zinkacetat Dihydrat (0.05 mmol) wurden in Ethanol / Essigsäure / Wasser 3:2:1 (3 mL) für 2 Tage refluxiert. Das Lösungsmittel wurde im Stickstoffstrom abgetrieben und der Rückstand getrocknet. Oranges Pulver, Ausbeute quantitativ.

Eine Zusammenfassung der hergestellten Verbindungen ist in Übersicht 8 gezeigt.

| **Bezeichnung / laufende Nr.** | **Struktur** | **Analytik** |
|---|---|---|
| *Curcumin 0 Hydrochlorid (**SA-CUR-0**)* Verbindung (40) | 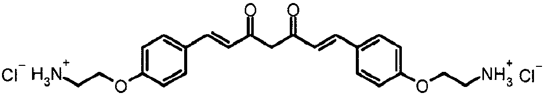 | **¹H NMR** (300 MHz, MeOD), δ = 7.60 (m, 6H), 7.04 (d, *J* = 6.9 Hz, 4H), 6.67 (d, *J* = 13.8 Hz, 2H), 4.32 - 4.17 (m, 4H), 3.46 - 3.34 (m, 4H). |
| | | **MS** (ESI, MeCN/H₂O + 0.06 % TFA): 198.1 (100%, (M+2H⁺)²⁺), 352.2 (9%, MH⁺-C₂H₅N), 395.2 (2%, MH⁺) |
| *Curcumin 01a Hydrochlorid (**SA-CUR-1a**)* Verbindung (41) | 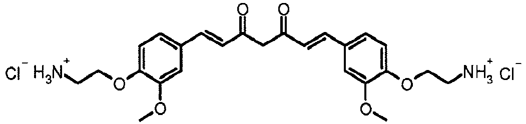 | **¹H NMR** (300 MHz, MeOD), δ = 7.61 (d, *J* = 15.7 Hz, 2H), 7.33 (s, 2H), 7.23 (d, *J* = 8.2 Hz, 2H), 7.06 (d, *J* = 8.2 Hz, 2H), 6.74 (d, *J* = 15.7 Hz, 2H), 6.03 (s, 1H), 4.33 - 4.22 (m, 4H), 3.95 (s, 6H), 3.45 - 3.34 (m, 4H). |
| | | **MS** (ESI, MeCN/H₂O + 0.06 % TFA): 228.1 (100%, (M+2H⁺)²⁺), 412.2 (5%, MH⁺-C₂H₅N), 455.2 (3%, MH⁺) |
| *Curcumin 01d Hydrochlorid (**SA-CUR-1d**), HO-SA-CUR-1* Verbindung (71) | 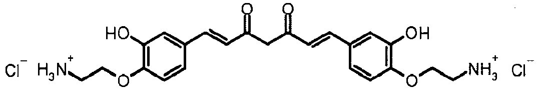 | **¹H NMR** (300 MHz, MeOD), δ = 7.65 - 6.70 (m, 10H), 4.35 - 4.24 (m, 4H), 3.46 - 3.34 (m, 4H). |
| | | **MS** (ESI, MeCN/H₂O + 0.06 % TFA): 214.1 (100%, (M+2H⁺)²⁺), 384.2 (7%, MH⁺-C₂H₅N), 427.2 (5%, MH⁺) |
| *Curcumin 01b Hydrochlorid (**SA-CUR-1b**), Iso-SA-CUR-1* Verbindung (42) | 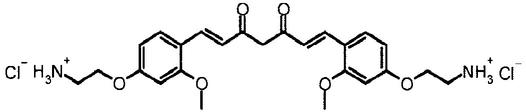 | **¹H NMR** (300 MHz, MeOD), δ = 7.63 (d, *J* = 15.8 Hz, 2H), 7.52 -7.02 (m, 6H), 6.77 (d, *J* = 15.7 Hz, 2H), 4.35 - 4.25 (m, 4H), 3.87 (s, 6H), 3.42 - 3.32 (m, 4H). |
| | | **MS** (ESI, MeCN/H₂O + 0.06 % TFA): 228.1 (100%, (M+2H⁺)²⁺), 412.2 (5%, MH⁺-C₂H₅N), 455.2 (3%, MH⁺) |
| *Curcumin 01e Hydrochlorid (**SA-CUR-1e**), Me-SA-CUR-1* Verbindung (43) | 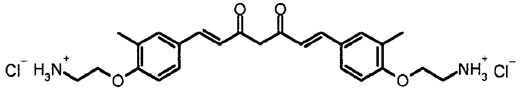 | **¹H NMR** (300 MHz, MeOD), δ = 7.60 (d, 14.3 Hz, 2H), 7.55 - 7.37 (m, 4H), 6.99 (d, *J* = 6.8 Hz, 2H), 6.68 (d, *J* = 14.3 Hz, 2H), 4.28 (m, 4H), 3.48 - 3.39 (m, 4H), 2.31 (s, 6H). |
| | | **MS** (ESI, MeCN/H₂O + 0.06 % TFA): 212.1 (100%, (M+2H⁺)²⁺), 380.2 (6%, MH⁺ -C₂H₅N), 423.2 (2%, MH⁺) |
| *Curcumin 01c Hydrochlorid (**SA-CUR-1c**), Iodo-SA-CUR-1* Verbindung (44) | 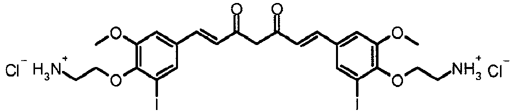 | **¹H NMR** (300 MHz, MeOD), δ = 7.68 (s, 2H), 7.56 (d, 14.5 Hz, 2H), 7.35 (s, 2H), 6.81 (d, *J* = 13.0 Hz, 2H), 6.07 (s, 1H), 4.27 - 4.15 (m, 4H), 3.95 (s, 6H), 3.42 - 3.32 (m, 4H). |
| | | MS (ESI, MeCN/H₂O + 0.06 % TFA): 354.0 (100%, (M+2H⁺)²⁺), 664.0 (4%, MH⁺-C₂H₅N), 707.1 (1%, MH⁺) |
| *Curcumin 02 Hydrochlorid (**SA-CUR-2**)* Verbindung (45) | 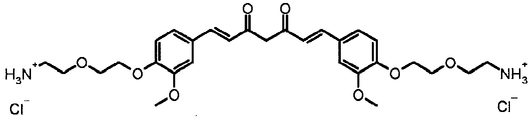 | **¹H NMR** (300 MHz, MeOD), δ = 7.60 (d, *J* = 15.7 Hz, 2H), 7.28 (s, 2H), 7.20 (d, *J* = 8.2 Hz, 2H), 7.01 (d, *J* = 8.3 Hz, 2H), 6.70 (d, *J* = 15.8 Hz, 2H), 4.22 (dd, *J* = 5.2, 3.3 Hz, 4H), 3.91 (s, 6H), 3.94 - 3.88 (m, 4H), 3.83 - 3.76 (m, 4H), 3.16 (t, *J* = 3.2 Hz, 4H). |
| | | **MS** (ESI, MeCN/H₂O + 0.06 % TFA): 272.1 (100%, (M+2H⁺)²⁺), 500.2 (3%, MH⁺-C₂H₅N), 543.3 (3%, MH⁺) |
| *Curcumin 08 Hydrochlorid (**SA-CUR-8**)* Verbindung (46) | 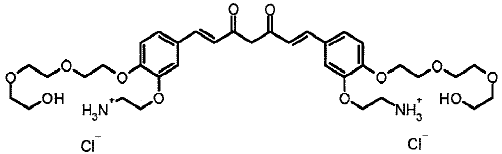 | **¹H NMR** (600 MHz, MeOD), δ = 7.66 - 6.78 (m, 10H), 4.34 - 4.17 (m, 6H), 3.89 (m, 4H), 3.81 - 3.38 (m, 20H), 3.27 (m, 2H). |
| | | **MS** (ESI, MeCN/H₂O + 0.06 % TFA): 346.2 (100%, (M+2H⁺)²⁺) |
| *Curcumin 04 Hydrochlorid (**SA-CUR-4**)* Verbindung (51) | 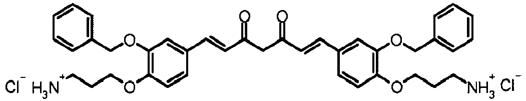 | **¹H NMR** (300 MHz, MeOD), δ = 7.57 (m, 2H), 7.48 - 7.33 (m, 12H), 7.28 (m, 2H), 7.08 (m, 2H), 6.68 (m, 2H), 5.13 (s, 4H), 4.23 (m, 4H), 3.16 (t, *J* = 6.4 Hz, 4H), 2.16 (m, 4H). |
| | | **MS** (ESI, MeCN/H₂O + 0.06 % TFA): 286.1 (100%, (M+2H⁺)²⁺), 545.3 (4%, MH⁺-C₂H₅N), 635.3 (1%, MH⁺) |
| *Curcumin 07 Hydrochlorid (**SA-CUR-07**)* Verbindung (48) | 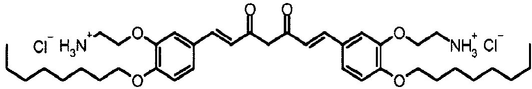 | **¹H** NMR (300 MHz, DMSO), δ = 8.06 (s, 6H), 7.59 (d, *J* = 15.8 Hz, 2H), 7.41 (s, 2H), 7.28 (d, *J* = 8.4 Hz, 2H), 7.09 (d, *J* = 8.4 Hz, 2H), 6.87 (d, *J* = 15.9 Hz, 2H), 6.13 (s, 1H), 4.22 (t, *J* = 5.3 Hz, 4H), 4.06 (t, *J* = 6.7 Hz, 4H), 3.22 (t, *J* = 5.2 Hz, 4H), 1.86 - 1.68 (m, 4H), 1.49 - 1.22 (m, 20H), 0.87 (t, *J* = 8.7, 6H). |
| | | MS (ESI, MeCN/H₂O + 0.06 % TFA): 326.2 (100%, (M+2H⁺)²⁺), 651.4 (2%, MH⁺) |
| *Curcumin 03 Hydrochlorid (**SA-CUR-3**)* Verbindung (49) | 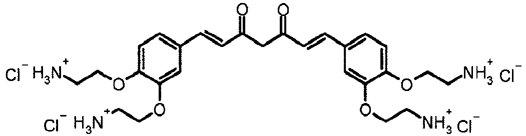 | **¹H NMR** (300 MHz, MeOD), δ = 7.61 (d, *J* = 15.8 Hz, 2H), 7.37 (s, 2H), 7.35 - 7.28 (m, 2H), 7.10 (d, *J* = 8.4 Hz, 2H), 6.74 (d, *J* = 15.8 Hz, 2H), 6.02 (s, 1H), 4.32 (dd, *J* = 9.8, 4.9 Hz, 8H), 3.43 (t, *J* = 4.8 Hz, 8H). MS (ESI, MeCN/H₂O + 0.06 % TFA): 171.8 (100%, (M+3H⁺)³⁺), 235.6 (43%, (M+2H⁺)²⁺ -C₂H₅N), 257.1 (44%, (M+2H⁺)²⁺), 513.3 (15%, MH⁺) |
| *Curcumin 05 Hydrochlorid (**SA-CUR-5**)* Verbindung (50) | 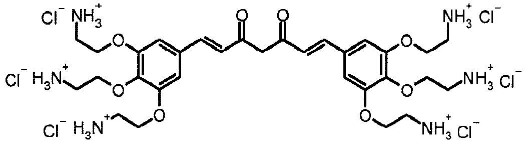 | **¹H NMR** (300 MHz, MeOD), δ = 7.62 (d, *J* = 15.8 Hz, 2H), 7.12 (s, 4H), 6.84 (d, *J* = 15.9 Hz, 2H), 4.41 - 4.29 (m, 8H), 4.27 - 4.20 (m, 4H), 3.50 - 3.40 (m, 8H), 3.39 - 3.33 (m, 4H). |
| | | **MS** (ESI, MeCN/H₂O + 0.06 % TFA): 158.6 (47%, (M+4H⁺)⁴⁺), 211.1 (100%, (M+3H⁺)³⁺), 273.1 (6%, (M+2H⁺)²⁺ -C₂H₅N), 316.2 (24%, (M+2H⁺)²⁺), 631.3 (12%, MH⁺) |
| *Curcumin 01a BF-Komplex (**BF-SA-CUR-1a**)* Verbindung (68) | 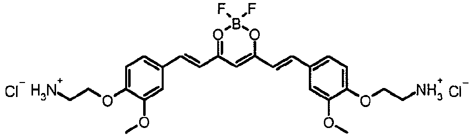 | **¹H NMR** (300 MHz, D₂O), δ = 7.32 (m, 2H), 6.83 (s, 2H), 7.72 (m, 4H), 6.31 (m, 2H), 5.84 (s, 1H), 4.11 - 3.96 (m, 4H), 3.58 (s, 6H), 3.36 - 3.21 (m, 4H). |
| | | **MS** (ESI, MeCN/H₂O + 0.06 % TFA): 228.6 (100%, (M+2H⁺)²⁺ -BF₂), 252.1 (3%, (M+2H⁺)²⁺), 503.2 (7%, MH+) |
| *Roseo-Curcumin 01a Hydrochlorid (**RO-SA-CUR-1a**)* Verbindung (70) | 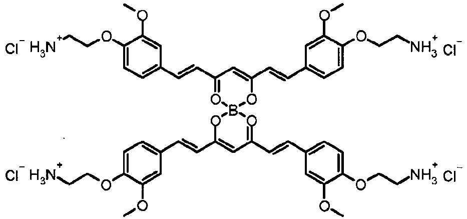 409.8 | **MS** (ESI, MeCN/H₂O + 0.06 % TFA): 228.6 (100%, (M+2H⁺)²⁺-B-ligand), 409.8 (3%, (M+2H⁺)²⁺), 918.5 (1%, MH+) |
| *Curcumin 01a Zink-Komplex (**Zn-SA-CUR-1a**)* Verbindung (69a) | 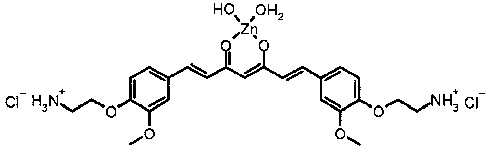 | **MS** (ESI, MeCN/H₂O + 0.06 % TFA): 228.6 (100%, (M+2H⁺)²⁺-Zn), 553.2 (1%, MH+) |
| *Curcumin 09a Hydrochlorid (**Me-SA-CUR-9a**)* Verbindung (47) | 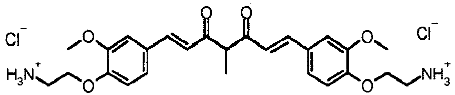 | **¹H NMR** (300 MHz, MeOD), δ = 7.66 (d, *J* = 15.5 Hz, 2H), 7.35 (s, 2H), 7.29 - 7.20 (m, 4H), 7.06 (d, *J* = 8.3 Hz, 2H), 4.32 - 4.22 (m, 4H), 3.96 (s, 6H), 3.44 - 3.35 (m, 4H), 2.22 (s, 3H). |
| | | **MS** (ESI, MeCN/H₂O + 0.06 % TFA): 235.1 (100%, (M+2H⁺)²⁺), 426.2 (2%, MH⁺-C₂H₅N), 469.2 (1%, MH⁺) |
| *Curcumin 09bHydrochlorid (**cyclo-SA-CUR-9b**)* Verbindung (54) | 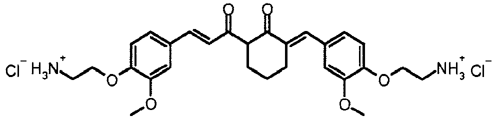 | **¹H NMR** (400 MHz, MeOD), δ = 7.73 - 7.56 (m, 2H), 7.34 - 7.21 (m, 2H), 7.19 - 6.98 (m, 6H), 4.26 (m, 4H), 3.95 (s, 3H), 3.91 (s, 3H), 3.37 (m, 4H), 2.81 - 2.62 (m, 4H), 1.79 (m, 2H). |
| | | **MS** (ESI, MeCN/H₂O + 0.06 % TFA): 248.1 (100%, (M+2H⁺)²⁺), 452.2 (4%, MH⁺-C₂H₅N), 495.2 (1%, MH⁺) |
| *Curcumin 10a Hydrochlorid (**SA-CUR-10a**)* Verbindung (66) | 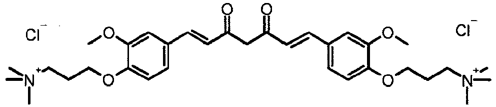 | **¹H NMR** (300 MHz, MeOD), δ = 7.60 (d, *J* = 15.6 Hz, 2H), 7.28 (s, 2H), 7.20 (d, *J* = 7.6 Hz, 2H), 7.01 (d, *J* = 8.1 Hz, 2H), 6.71 (d, *J* = 15.7 Hz, 2H), 4.17 (t, *J* = 5.4 Hz, 4H), 3.91 (s, 6H), 3.66 - 3.53 (m, 4H), 3.20 (s, 18H), 2.38 - 2.24 (m, 4H). |
| | | **MS** (ESI, MeCN/H₂O + 0.06 % TFA): 284.2 (100%, M²⁺) |
| *Curcumin 10b Hydrochlorid (**GUA-SA-CUR-10b**)* Verbindung (63) | 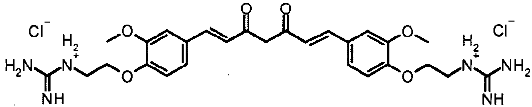 | **¹H NMR** (400 MHz, MeOD), δ = 7.61 (d, *J* = 13.1 Hz, 2H), 7.34 - 7.16 (m, 4H), 7.01 (d, *J* = 7.5 Hz, 2H), 6.74 (m, 4H), 4.18 (t, *J* = 4.7 Hz, 4H), 3.92 (d, *J* = 8.1 Hz, 6H), 3.64 (t, *J* = 4.3 Hz, 4H). |
| | | MS (ESI, MeCN/H₂O + 0.06 % TFA): 270.1 (100%, (M+2H⁺)²⁺), 539.3 (2%, MH⁺) |
| *Curcumin 10c Hydrochlorid (**SA-CUR-10c**)* Verbindung (67) | 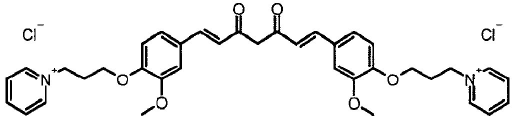 | **¹H NMR** (300 MHz, MeOD), δ = 9.06 (d, *J* = 5.6 Hz, 4H), 8.61 (t, *J* = 7.8 Hz, 2H), 8.17 - 8.02 (m, 4H), 7.58 (d, *J* = 15.9 Hz, 2H), 7.22 (s, 2H), 7.17 (d, *J* = 7.6 Hz, 2H), 6.96 (d, *J* = 8.3 Hz, 2H), 6.69 (d, *J* = 15.8 Hz, 2H), 4.92 - 4.83 (m, 4H), 4.19 (t, *J* = 5.4 Hz, 4H), 3.83 (s, 6H), 2.67 - 2.48 (m, 4H). |
| | | **MS** (ESI, MeCN/H₂O + 0.06 % TFA): 304.1 (100%, M²⁺) |
| *Curcumin 11a Hydrochlorid (**SA-CUR-11a**)* Verbindung (59) | 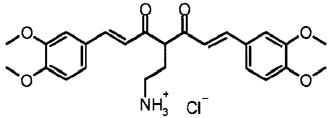 | **¹H NMR** (300 MHz, MeOD), δ = 7.78 - 6.65 (m, 10H), 4.03 - 3.85 (m, 2H), 3.90 (s, 12H), 2.97 - 2.80 (m, 2H). |
| | | **MS** (ESI, MeCN/H₂O + 0.06 % TFA): 440.2 (MH⁺, 100%) |
| *Curcumin 11b Hydrochlorid (**SA-CUR-11b**)* Verbindung (61) | 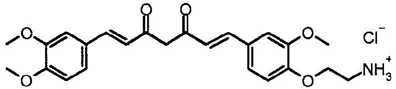 | **¹H NMR** (300 MHz, MeOD), δ = 7.72 - 6.40 (m, 10H), 4.25 (m, 2H), 3.94 (s, 3H), 3.84 (s, 6H), 3.37 (m, 2H). |
| | | **MS** (ESI, MeCN/H₂O + 0.06 % TFA): 426.2 (100%, (MH⁺) |
| *Curcumin 11c Hydrochlorid (**SA-CUR-11c**)* Verbindung (55) | 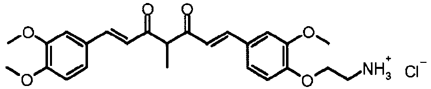 | **¹H NMR** (300 MHz, MeOD), δ = 7.75 - 6.50 (m, 10H), 4.27 (m, 2H), 3.92 (s, 3H), 3.82 (s, 6H), 3.36 (m, 2H), 2.02 (m, 3H). |
| | | **MS** (ESI, MeCN/H₂O + 0.06 % TFA): 442.2 (MH⁺, 100%) |
| *Curcumin 12a Hydrochlorid (**SA-CUR-12a**)* Verbindung (52) | 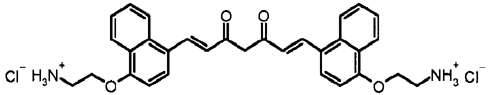 | **¹H NMR** (300 MHz, DMF), δ = 9.12 (s, 6H), 8.79 - 8.66 (m, 4H), 8.57 (d, *J* = 8.5 Hz, 2H), 8.29 (d, *J* = 8.3 Hz, 2H), 7.93 (t, *J* = 7.4 Hz, 2H), 7.84 (t, *J* = 7.5 Hz, 2H), 7.39 (d, *J* = 8.3 Hz, 2H), 7.23 (d, *J* = 15.6 Hz, 2H), 4. 89 - 4.78 (m, 4H), 4.01 - 3.90 (m, 4H). |
| | | **MS** (ESI, MeCN/H₂O + 0.06 % TFA): 248.1 (100%, (M+2H⁺)²⁺), 452.2 (5%, MH⁺-C₂H₅N), 495.2 (3%, MH⁺) |
| *Curcumin 12b Hydrochlorid (**SA-CUR-12b**)* Verbindung (53) | 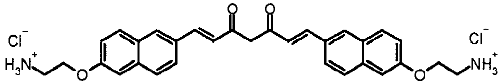 | **¹H NMR** (300 MHz, DMSO), δ = 8.18 (s, 2H), 8.10 (s, 6H), 7.98 - 7.86 (m, 6H), 7.80 (d, *J* = 15.8 Hz, 2H), 7.44 (d, *J* = 2.0 Hz, 2H), 7.27 (dd, *J* = 8.9, 2.3 Hz, 2H), 7.06 (d, *J* = 15.9 Hz, 2H), 4.32 (m, 4H), 3.46 (m, 4H). |
| | | **MS** (ESI, MeCN/H₂O + 0.06 % TFA): 248.1 (100%, (M+2H⁺)²⁺), 452.2 (3%, MH⁺-C₂H₅N), 495.2 (2%, MH⁺) |
| *Curcumin 13a Hydrochlorid (**SA-CUR-13a**)* Verbindung (60) | 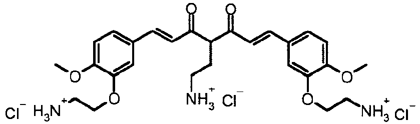 | **¹H NMR** (300 MHz, MeOD), δ = 7.78 - 6.65 (m, 10H), 4.34 - 4.22 (m, 4H), 4.04 - 3.82 (m, 8H), 3.46 - 3.34 (m, 4H), 2.98 - 2.82 (m, 2H). |
| | | **MS** (ESI, MeCN/H₂O + 0.06 % TFA): 167.1 (100%, (M+3H⁺)³⁺), 229.2 (17%, (M+2H⁺)²⁺ -C₂H₅N), 250.6 (40%, (M+2H⁺)²⁺), 500.3 (3%, MH⁺) |
| *Curcumin 13b Hydrochlorid (**SA-CUR-13b**)* Verbindung (62) | 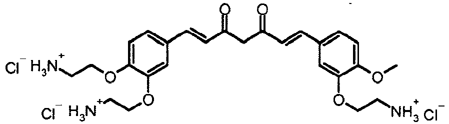 | **¹H NMR** (300 MHz, MeOD), δ = 7.60 (d, *J* = 13.2 Hz, 2H), 7.42 - 7.17 (m, 4H), 7.12 - 6.93 (m, 2H), 6.73 (d, *J* = 12.8 Hz, 2H), 4.40 - 4.22 (m, 6H), 3.95 (s, 3H), 3.46 - 3.36 (m, 6H). |
| | | **MS** (ESI, MeCN/H₂O + 0.06 % TFA): 162.1 (100%, (M+3H⁺)³⁺), 221.1 (44%, (M+2H⁺)²⁺ -C₂H₅N), 242.6 (52%, (M+2H⁺)²⁺), 484.2 (7%, MH⁺) |
| *Curcumin 13c Hydrochlorid (**SA-CUR-13c**)* Verbindung (56) | 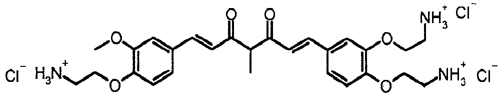 | **¹H NMR** (300 MHz, MeOD), δ = 7.80-6.66 (m, 10H), 4.43 - 4.10 (m, 6H), 3.93 (s, 3H), 3.49 - 3.24 (m, 6H), 2.11 (m, 3H). |
| | | **MS** (ESI, MeCN/H₂O + 0.06 % TFA): 166.8 (87%, (M+3H⁺)³⁺), 249.6 (100%, (M+2H⁺)²⁺), 498.3 (16%, MH⁺) |
| *Curcumin 14a Hydrochlorid (**SA-CUR-14a**)* Verbindung (57) | 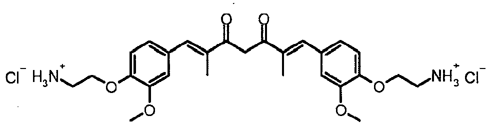 | **¹H NMR** (400 MHz, MeOD), δ = 7.69 - 7.58 (m, 2H), 7.23 - 7.02 (m, 6H), 4.32 - 4.21 (m, 4H), 3.93 (s, 6H), 3.44 - 3.35 (m, 4H), 2.20 - 2.06 (m, 6H). |
| | | **MS** (ESI, MeCN/H₂O + 0.06 % TFA): 242.1 (100%, (M+2H⁺)²⁺), 440.2 (7%, MW⁺-C₂H₅N), 483.2 (3%, MH⁺) |
| *Curcumin 14b Hydrochlorid (**SA-CUR-14b**)* Verbindung (58) | 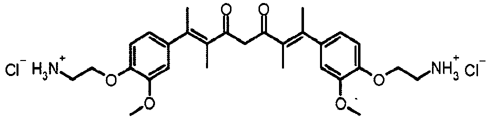 | **MS** (ESI, MeCN/H₂O + 0.06 % TFA): 256.1 (100%, (M+2H⁺)²⁺), 469.3 (7%, MH⁺-C₂H₅N), 512.3 (4%, MH⁺) |
| *Curcumin 15a Hydrochlorid (**SA-CUR-15a**)* Verbindung (64) | 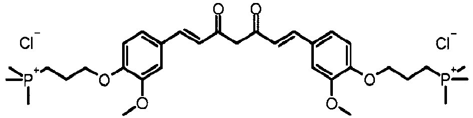 | **¹H NMR** (300 MHz, MeOD), δ = 7.58 (d, *J* = 15.7, 2H), 7.27 (s, 2H), 7.16 (d, *J* = 8.8 Hz, 2H), 6.98 (d, *J* = 8.2 Hz, 2H), 6.70 (d, *J* = 15.8 Hz, 2H), 4.23 (m, 4H), 3.92 (s, 6H), 3.61 (m, 4H), 2.20 (m, 4H), 1.87 (d, *J* = 14.3, 18H). |
| | | **MS** (ESI, MeCN/H₂O + 0.06 % TFA): 301.1 (100%, M²⁺) |
| *Curcumin 15b Hydrochlorid (**SA-CUR-15b**)* Verbindung (65) | 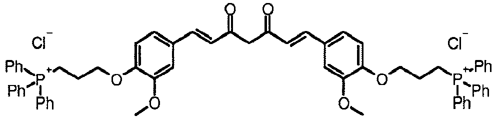 | **¹H NMR** (300 MHz, MeOD), δ = 7.96 - 7.51 (m, 32H), 7.28 (s, 2H), 7.19 (d, *J* = 8.8 Hz, 2H), 6.96 (d, *J* = 8.2 Hz, 2H), 6.72 (d, *J* = 15.8 Hz, 2H), 4.21 (m, 4H), 3.91 (s, 6H), 3.63 (m, 4H), 2.18 (m, 4H). |
| | | **MS** (ESI, MeCN/H₂O + 0.06 % TFA): 487.2 (100%, M²⁺) |

### Beispiel 2) Phototoxizitätsexperimente

### a) Herstellung wachsender Kulturen der Bakterienstämme

Alle Experimente wurden unter sterilen Bedingungen in einer Sicherheitswerkbank (Biosafe 4-130, Ehret, Emmedingen, Deutschland) durchgeführt. Nach Zugabe der photoaktiven Substanzen wurde ausschließlich im Dunkeln gearbeitet.

Eine Probe des Bakterienstammes *Staphylococcus. aureus* (ATCC-Nummer: 25923) oder *Escherichia. coli* (ATCC-Nummer: 25922) wurde aus einer Cryo-Gefrierkultur entnommen und unter aeroben Bedingungen bei 37°C und 175 rpm in einer Übernachtkultur im Inkubationsschüttler (MAXQ4000, Thermo Scientific, Dubuque, lowa, USA) kultiviert. Dabei erfolgte das Wachstum in 20 mL Todd-Hewitt Bouillon (Carl Roth, Karlsruhe, Deutschland) ergänzt mit 0.3 % Hefeextrakt (AppliChem, Darmstadt, Deutschland).

Alternativ wurde zur Kultivierung Müller-Hinton-Medien verwendet:
Müller-Hinton-Flüssigmedium (Merck KGaA, Darmstadt, Deutschland) 2,0 g/L Fleischextrakt, 17,5 g/L Caseinhydrolysat, 1,5 g/L Stärke, pH: 7,4 + 0,2.

Müller Hinton-Agar (Merck KGaA, Darmstadt, Deutschland) 2,0 g/L Fleischextrakt, 17,5 g/L Caseinhydrolysat, 1,5 g/L Stärke, 15 g/L Agar, pH: 7,4 + 0,2.

### b) Herstellung einer Kultur in der exponentiellen Wachstumsphase

Nachfolgend wurden Verdünnungen (0, 5, 10, 25 und 20 % v/v der Übernachtkultur) hergestellt und deren Absorption bei 600 nm in Triplikaten zu 100 µL gemessen (Infinite 200 M Pro, Tecan, Männedorf, Schweiz). Aus einer Kalibriergerade (Microsoft Excel) wurde jenes Volumen errechnet, welches für die Erzeugung von 20 mL einer Kultur mit 0.05 Absorption bei 600 nm benötigt wird. Das somit errechnete Volumen der Übernachtkultur wurde mit Todd-Hewitt Bouillon (Zusatz wie oben) auf 20 mL ergänzt und für zwei Stunden bei 37 °C unter konstanter Bewegung (175 rpm, MAXQ4000) inkubiert. Die Kulturen befanden sich damit in der exponentiellen Wachstumsphase, die Absorption bei 600 nm lag zwischen 0.3 und 0.45.

Die nachfolgende Inkubation mit den photoaktiven Substanzen sowie Bestrahlung mit elektromagnetischer Strahlung und Bestimmung der Phototoxizität wurde mit zwei verschiedenen Methoden durchgeführt.

### c.1) Inkubation, Bestrahlung und Bestimmung der Phototoxizität

Eine 2-Stunden Kultur wurde in Aliquots zu 1800 µL geteilt. Nach einer Zentrifugation bei 20 °C, 830 rcf, 5 min (5417R Zentrifuge, Eppendorf, Hamburg, Deutschland) wurden die Pellets in Photphatpuffer (Dulbeccos' Modified Phosphate Saline, DPBS, Sigma-Aldrich) mit entweder 10 oder 50 µM des entsprechenden Photosensibilisators resuspendiert, wobei das Endvolumen immer 1800 µL betrug. Die erhaltenen Lösungen wurden umgehend am Inkubationsschüttler (Parameter siehe oben) für 5 oder 25 Minuten inkubiert.

Für jeden Photosensibilisator wurden drei Kontrollen mitgeführt. Die "light only"-Kontrolle (LO) enthielt DPBS ohne Photosensibilisator. Die "photosensitizer only"-Kontrolle (PS only) wurde inkubiert wie die PDI-Proben, jedoch nicht bestrahlt und strikt im Dunkeln gehalten. Eine weitere Kontrolle (Doppelt Negativ, Co -/-) erhielt weder Licht noch Photosensibilisator.

Nach der Inkubation wurden Duplikate der Proben zu je 500 µL in eine 24-well Mikrotiterplatte (Cellstar, Greiner Bio-One, Frickenhausen, Deutschland) transferiert. Die Proben PS only und Co -/- kamen in eine eigene Mikrotiterplatte, welche mit Aluminumfolie lichtdicht verpackt wurde.

Die Bestrahlung erfolge unter ständigem Schütteln (MTS4, IKA, Staufen, Deutschland, ∼ 175 prm) von unten auf einem LED array mit höchster Homogenität der Belichtungsfläche. Alle Kontrollen wurden gleichfalls geschüttelt. Die technischen Daten der Lichtquelle finden sich in Tabelle 2, die gesamt applizierte Lichtdosis betrug 33,8 J/cm².

**Tabelle 2:Technische Daten des LED Arrays:**

| Hersteller der Dioden | Roithner Lasertechnik, Wien, Österreich |
|---|---|
| Bezeichnung der Dioden | LED 435-12-30 |
| Dominante Wellenlänge | 430 nm - 435 nm |
| Anzahl der Dioden im Array | 432 |
| Intsnsität | 9,4 mW/cm² |

Die Bestimmung der koloniebildenden Einheiten (CFU) wurde gemäß der von Miles und Misra publizierten Methode durchgeführt (Miles, AA; Misra, SS, Irwin, JO (1938 Nov). "The estimation of the bactericidal power of the blood" The Journal of hygiene 38 (6): 732-49). Dazu wurden serielle Verdünnungen (1:10) der entsprechenden Bakteriensuspension in DPBS hergestellt. Je 5x 10 µL der Bakterienverdünnungen wurden dann auf Todd-Hewitt Platten (wie Bouillon, zusätzlich 1.5 % Agar (Agar-Agar, Kobe I, Roth, Karlsruhe, Deutschland) aufgetropft und bei 37°C für 24 h inkubiert. Danach wurde die Anzahl der überlebenden koloniebildenden Einheiten bestimmt. Alle Versuche wurden viermal wiederholt.

### c.2) Inkubation, Bestrahlung und Bestimmung der Phototoxizität

In einem zweiten Experiment wurden die verwendeten Photosensibilisatoren (PS) in Millipore Wasser gelöst und auf verschiedene Konzentrationen eingestellt. 25 µL einer über Nacht gewachsenen Bakteriensuspension (∼ 108 /mL) wurden mit 25 µL Photosensibilisatorlösung verschiedener Konzentrationen bei Raumtemperatur für 10 Sekunden im Dunklen in einer 96 well Platte inkubiert.

Anschließend wurde die Suspension für 5 - 20 Minuten bestrahlt. Zur Bestrahlung wurde die Lichtquelle BlueV der Firma Waldmann (Villingen-Schwenningen, Deutschland) genutzt, welche Licht von 380 bis 480 nm emittiert (Emissionsmaximum bei ca. 420 nm). Die applizierte Leistung betrug 17,5 mW/cm².

Bei jedem Experiment wurden drei Kontrollen mitgeführt, um Nebeneffekte der Bestrahlung / des Photosensibilisators (PS) auf das Überleben der Bakterien auszuschließen: (i) kein PS, nur Licht (= Lichtkontrolle), (ii) kein Licht, nur PS (= Dunkelkontrolle) und (iii) weder Licht, noch PS (= Referenzkontrolle). Die Bestimmung der koloniebildenden Einheiten (KbE) pro ml wurde ebenfalls gemäß der von Miles, Misra und Irwin publizierten Methode wie oben unter c.1) beschrieben durchgeführt. Alle Versuche wurden viermal wiederholt.

### d) Ergebnis der Phototoxizitätsexperimente

Die Ergebnisse der oben unter c.1) beschriebenen Phototoxizitätsexperimente sind in den Figuren 1 bis 20 dargestellt. Die Photosensibilisatoren wurden gegen den Bakterienstamm *E. coli ATCC* 25922 getestet. Dargestellt ist in den jeweiligen Figuren 1 - 20 die gemessenen überlebenden koloniebildenden Einheiten (CFU).

Die Ergebnisse der oben unter c.2) beschriebenen Phototoxizitätsexperimente sind in den Figuren 21 bis 33 dargestellt. Die Photosensibilisatoren (PS) SACUR-01a, SACUR-03 und SACUR-07 wurden gegen die Bakterienstämme *S. aureus* ATCC 25923 und *E. coli* ATCC 25922 getestet (Fig. 21-23). Alle übrigen PS wurden gegen S. *aureus* ATCC 25923 getestet (Fig. 24 - 33). Dargestellt ist in den jeweiligen Figuren 21 - 33 die logarithmische Abnahme nach Belichtung in Bezug auf die Referenzkontrolle.

**Figur 21** zeigt das Ergebnis des Phototoxizitätstests von SACUR-01a gegen *E. coli* ATCC 25922 (links) und gegen *S. aureus* ATCC 25923 (rechts). Die Bestrahlungszeit für *E. coli* betrug 15 Minuten, für *S. aureus* 5 Minuten. Die gemittelte Referenzkontrolle (arithmethisches Mittel mit Standardabweichung) (kein Licht, kein PS) betrug für *E. coli* 3,6 x 10⁸/mL und für *S. aureus* 3,9 x 10⁸/mL.

**Figur 22** zeigt das Ergebnis des Phototoxizitätstests von SACUR-03 gegen *E*. *coli* ATCC 25922 (links) und gegen *S. aureus* ATCC 25923 (rechts). Die Bestrahlungszeit für *E*. *coli* betrug 15 Minuten, für *S. aureus* 5 Minuten. Die gemittelte Referenzkontrolle (arithmethisches Mittel mit Standardabweichung) (kein Licht, kein PS) betrug für *E*. *coli* 3,3 x 10⁸/mL und für *S. aureus* 4,3 x 10⁸/mL.

**Figur 23** zeigt das Ergebnis des Phototoxizitätstests von SACUR-07 gegen *E*. *coli* ATCC 25922 (links) und gegen *S. aureus* ATCC 25923 (rechts). Die Bestrahlungszeit für *E. coli* betrug 45 Minuten, für *S. aureus* 5 Minuten. Die gemittelte Referenzkontrolle(arithmethisches Mittel mit Standardabweichung) (kein Licht, kein PS) betrug für *E. coli* 2,6 x 10⁸/mL und für *S. aureus* 3,6 x 10⁸/mL.

**Figur 24** zeigt das Ergebnis des Phototoxizitätstests von SACUR-01a BF2 gegen *S. aureus* ATCC 25923. Die Bestrahlungszeit für *S. aureus* betrug 5 Minuten, die gemittelte Referenzkontrolle (arithmethisches Mittel mit Standardabweichung) (kein Licht, kein PS) entsprach ∼ 3,6 x 10⁸ Bakterien pro Milliliter.

**Figur 25** zeigt das Ergebnis des Phototoxizitätstests von SACUR-09a gegen *S. aureus* ATCC 25923. Die Bestrahlungszeit für *S. aureus* betrug 5 Minuten, die gemittelte Referenzkontrolle(arithmethisches Mittel mit Standardabweichung) (kein Licht, kein PS) entsprach ∼ 3,3 x 10⁸ Bakterien pro Milliliter.

**Figur 26** zeigt das Ergebnis des Phototoxizitätstests von SACUR-11a gegen *S. aureus* ATCC 25923. Die Bestrahlungszeit für *S. aureus* betrug 5 Minuten, die gemittelte Referenzkontrolle(arithmethisches Mittel mit Standardabweichung) (kein Licht, kein PS) entsprach ∼ 6,3 x 10⁸ Bakterien pro Milliliter.

**Figur 27** zeigt das Ergebnis des Phototoxizitätstests von SACUR-11c gegen *S. aureus* ATCC 25923. Die Bestrahlungszeit für *S. aureus* betrug 5 Minuten, die gemittelte Referenzkontrolle(arithmethisches Mittel mit Standardabweichung) (kein Licht, kein PS) entsprach ∼ 5,1 x 10⁸ Bakterien pro Milliliter.

**Figur 28** zeigt das Ergebnis des Phototoxizitätstests von SACUR-12b gegen *S. aureus* ATCC 25923. Die Bestrahlungszeit für *S. aureus* betrug 30 Minuten, die gemittelte Referenzkontrolle(arithmethisches Mittel mit Standardabweichung) (kein Licht, kein PS) entsprach ∼ 6,2 x 10⁸ Bakterien pro Milliliter.

**Figur 29** zeigt das Ergebnis des Phototoxizitätstests von SACUR-13a gegen *S. aureus* ATCC 25923. Die Bestrahlungszeit für *S. aureus* betrug 5 Minuten, die gemittelte Referenzkontrolle (arithmethisches Mittel mit Standardabweichung) (kein Licht, kein PS) entsprach ∼ 7,5 x 10⁸ Bakterien pro Milliliter.

**Figur 30** zeigt das Ergebnis des Phototoxizitätstests von SACUR-13c gegen *S. aureus* ATCC 25923. Die Bestrahlungszeit für *S. aureus* betrug 5 Minuten, die gemittelte Referenzkontrolle (arithmethisches Mittel mit Standardabweichung) (kein Licht, kein PS) entsprach ∼ 5,5 x 10⁸ Bakterien pro Milliliter.

**Figur 31** zeigt das Ergebnis des Phototoxizitätstests von SACUR-14a gegen S. *aureus* ATCC 25923. Die Bestrahlungszeit für *S. aureus* betrug 10 Minuten, die gemittelte Referenzkontrolle (arithmethisches Mittel mit Standardabweichung) (kein Licht, kein PS) entsprach ∼ 4,2 x 10⁸ Bakterien pro Milliliter.

**Figur 32** zeigt das Ergebnis des Phototoxizitätstests von SACUR-15a gegen *S. aureus* ATCC 25923. Die Bestrahlungszeit für *S. aureus* betrug 5 Minuten, die gemittelte Referenzkontrolle (arithmethisches Mittel mit Standardabweichung) (kein Licht, kein PS) entsprach ∼ 3,6 x 10⁸ Bakterien pro Milliliter.

**Figur 33** zeigt das Ergebnis des Phototoxizitätstests von SACUR-15b gegen *S. aureus* ATCC 25923. Die Bestrahlungszeit für *S. aureus* betrug 5 Minuten, die gemittelte Referenzkontrolle (arithmethisches Mittel mit Standardabweichung) (kein Licht, kein PS) entsprach ∼ 6,3 x 10⁸ Bakterien pro Milliliter.

Wie aus den Figuren 1 - 33 ersichtlich, hat eine Bestrahlung der verwendeten Mikroorganismen *Staphylococcus aureus* (*S. aureus*) und *Escherichia coli* (*E. coli*) mit der oben beschrieben Lichtdosis mit blauem Licht (390nm - 500nm) in Abwesenheit eines Photosensibilisators (0 µM des jeweiligen Curcumins) keinen Einfluss auf die Anzahl der überlebenden Mikroorganismen im Vergleich zur unbelichteten Kontrolle.

Tabelle 3 zeigt die Wirkung der getesteten Substanzen gegen *E. coli ATCC* 25922 bei einer applizierten Lichtdosis von 33,8 J/cm² bei ca. 435 nm Emissionsmaximum (60 Minuten Belichtung mit einer Intensität von 9,4 mW/cm²). Die gemittelte Referenzkontrolle (arithmethisches Mittel mit Standardabweichung) (kein Licht, kein PS) betrug 3,6 x 10⁸/mL. Dargestellt ist die logarithmische Abnahme nach Belichtung in Bezug auf die Referenzkontrolle. Der jeweils obere, mit* gekennzeichnete Wert bezieht sich auf eine Inkubationszeit von 5 min, der jeweils untere, mit^{#} gekennzeichnete Wert auf eine Inkubationszeit von 25 min.

**Tabelle 3: Phototoxizitätstest der Substanzen gegen E. coli ATCC 25922.**

| **Bezeichnung** | **Wirksamkeit gegen *E.Coli* Reduktion CFU in log₁₀ bei Konzentration** | | | |
|---|---|---|---|---|
| | **10 µM** | **50 µM** | **100 µM** | **250 µM** |
| *Curcumin 0 Hydrochlorid* | > 4* | > 6* | n.a. | n.a. |
| *(**SA-CUR-0**)* | > 5^{#} | > 6^{#} | | |
| *Curcumin 01a Hydrochlorid* | > 1* | > 5* | n.a. | n.a. |
| ***(SA-CUR-1a)*** | >2^{#} | > 5^{#} | | |
| *Curcumin 01b Hydrochlorid* | > 2* | > 5* | n.a. | n.a. |
| *(**SA-CUR-1b**),* | > 3^{#} | > 7^{#} | | |
| *Curcumin 01e Hydrochlorid* | > 5* | > 4* | n.a. | n.a. |
| *(**SA-CUR-1e**),* | > 5^{#} | > 7^{#} | | |
| *Curcumin 01c Hydrochlorid* | > 2* | > 3* | n.a. | n.a. |
| *(**SA-CUR-1c*** | > 2^{#} | > 4^{#} | | |
| *Curcumin 02 Hydrochlorid* | > 2* | > 6* | n.a. | n.a. |
| ***(SA-CUR-2)*** | > 4^{#} | > 7^{#} | | |
| *Curcumin 08 Hydrochlorid* | ---* | ∼ 1* | n.a. | n.a. |
| *(**SA-CUR-8**)* | ---^{#} | > 1^{#} | | |
| *Curcumin 04 Hydrochlorid* | > 1* | > 3* | n.a. | n.a. |
| *(**SA-CUR-4**)* | > 3^{#} | > 4^{#} | | |
| *Curcumin 03 Hydrochlorid* | > 7* | > 7* | n.a. | n.a. |
| *(**SA-CUR-3**)* | > 7^{#} | > 7^{#} | | |
| *Curcumin 05 Hydrochlorid* | > 4* | > 7* | n.a. | n.a. |
| *(**SA-CUR-5**)* | > 4^{#} | > 6^{#} | | |
| *Roseo-Curcumin 01a Hydrochlorid* | > 5* | > 7* | n.a. | n.a. |
| ***(RO-SA-CUR-1a)*** | > 5^{#} | > 6^{#} | | |
| *Curcumin 01a Zink-Komplex* | > 3* | > 5* | n.a. | n.a. |
| *(**Zn-SA-CUR-1a**)* | > 2^{#} | > 5^{#} | | |
| *Curcumin 09b Hydrochlorid* | ---* | > 1* | n.a. | n.a. |
| *(**cyclo-SA-CUR-9b**)* | ---^{#} | > 2^{#} | | |
| *Curcumin 10a Hydrochlorid* | > 1* | > 5* | n.a. | n.a. |
| *(**SA-CUR-10a**)* | > 1^{#} | > 5^{#} | | |
| *Curcumin 10b Hydrochlorid* | > 5* | > 7* | n.a. | n.a. |
| *(**GUA-SA-CUR-10b**)* | > 4^{#} | > 6^{#} | | |
| *Curcumin 10cHydrochlorid* | > 4* | > 7* | n.a. | n.a. |
| *(**SA-CUR-10c**)* | > 5^{#} | > 7^{#} | | |
| *Curcumin 11b Hydrochlorid* | ∼ 1* | ∼ 3* | n.a. | n.a. |
| *(**SA-CUR-11b**)* | > 1^{#} | > 3^{#} | | |
| *Curcumin 12a Hydrochlorid* | > 1* | > 6* | n.a. | n.a. |
| *(**SA-CUR-12a**)* | > 4^{#} | > 6^{#} | | |
| *Curcumin 13b Hydrochlorid* | > 7* | > 7* | n.a. | n.a. |
| *(**SA-CUR-13b**)* | > 7^{#} | > 7^{#} | | |
| *Curcumin 14bHydrochlorid* | ---* | ---* | n.a. | n.a. |
| *(**SA-CUR-14b**)* | ---^{#} | > 1^{#} | | |

Tabelle 4 zeigt die Wirkung der getesteten Substanzen gegen *E. coli* ATCC 25922 bei einer applizierten Lichtdosis von 15,7 J/cm² bei ca. 420 nm Emissionsmaximum (15 min Belichtung mit einer Intensität von 17,5 mW/cm²). Die gemittelte Referenzkontrolle (arithmethisches Mittel mit Standardabweichung) (kein Licht, kein PS) betrug für *E. coli* 3,6 x 10⁸/mL. Dargestellt ist die logarithmische Abnahme nach Belichtung in Bezug auf die Referenzkontrolle.

**Tabelle 4: Phototoxizitätstest der Substanzen gegen E. coli ATCC 25922**

| **Bezeichnung / laufende Nr.** | **Wirksamkeit gegen *E.Coli* Reduktion CFU in log₁₀ bei Konzentration** | | | |
|---|---|---|---|---|
| | **10 µM** | **50 µM** | **100 µM** | **250 µM** |
| *Curcumin 01a Hydrochlorid (**SA-CUR-1a**)* | > 4 | > 5 | n.a. | n.a. |
| *Curcumin 07 Hydrochlorid (**SA-CUR-07**)* | > 1 | > 4 | n.a. | n.a. |
| *Curcumin 03 Hydrochlorid (**SA-CUR-3**)* | > 4 | > 5 | n.a. | n.a. |

Tabelle 5 zeigt die Wirkung der getesteten Substanzen gegen *S. aureus* ATCC 25923. Die Bestrahlungszeit betrug 5 Minuten (Emissionsmaximum bei ca. 420 nm) bei einer applizierten Intensität von 17,5 mW/cm², d.h. applizierte Lichtenergie (Dosis) von 5,3 J/cm². Die gemittelte Referenzkontrolle (arithmethisches Mittel mit Standardabweichung) (kein Licht, kein PS) betrug 3,9 x 10⁸/mL. Dargestellt ist die logarithmische Abnahme nach Belichtung in Bezug auf die Referenzkontrolle.

**Tabelle 5: Phototoxizitätstest gegen S.aureus ATCC 25923.**

| **Bezeichnung / laufende Nr.** | **Wirksamkeit gegen *S.aureus* Reduktion CFU in log₁₀ bei Konzentration** | | | |
|---|---|---|---|---|
| | **10 µM** | **50 µM** | **100 µM** | **259 µM** |
| *Curcumin 01a Hydrochlorid (**SA-CUR-1a**)* | > 3 | > 5 | n.a. | n.a. |
| *Curcumin 07 Hydrochlorid (**SA-CUR-07**)* | > 2 | > 5 | n.a. | n.a. |
| *Curcumin 03 Hydrochlorid (**SA-CUR-3**)* | > 4 | > 5 | n.a. | n.a. |
| *Curcumin 01a BF-Komplex (BF-SA-CUR-1a)* | > 5 | > 5 | n.a. | n.a. |
| *Curcumin 09a Hydrochlorid (Me-SA-CUR-9a)* | > 2 | > 5 | n.a. | n.a. |
| *Curcumin 11a Hydrochlorid (SA-CUR-11a)* | - | > 1 | > 2 | > 5 |
| *Curcumin 11c Hydrochlorid (SA-CUR-11c)* | - | > 1 | > 2 | > 5 |
| *Curcumin 12b Hydrochlorid (SA-CUR-12b)* | - | > 1 | ∼ 2 | > 3 |
| *Curcumin 13a Hydrochlorid (SA-CUR-13a)* | > 1 | > 5 | n.a. | n.a. |
| *Curcumin 13c Hydrochlorid (SA-CUR-13c)* | > 1 | > 5 | n.a. | n.a. |
| *Curcumin 14a Hydrochlorid (SA-CUR-14a)* | - | > 1 | > 2 | > 3 |
| *Curcumin 15a Hydrochlorid (SA-CUR-15a)* | ∼ 1 | > 5 | n.a. | n.a. |
| *Curcumin 15bHydrochlorid (SA-CUR-15b)* | > 4 | >5 | n.a. | n.a. |

Wie aus den Figuren 1 - 33 ersichtlich erfolgt nach Inkubation der Mikroorganismen in Abhängigkeit der verwendeten Konzentration der jeweiligen Photosensibilisatoren und nachfolgender Bestrahlung mit der oben angegebenen Lichtdosis eine Abnahme der KBE/mL und somit eine Inaktivierung von *E. coli* und S. *aureus.*

### Vergleichsbeispiel 3

In einem weiteren Versuch wurde die Stabiliät und Phototoxizität der folgenden Verbindungen getestet.

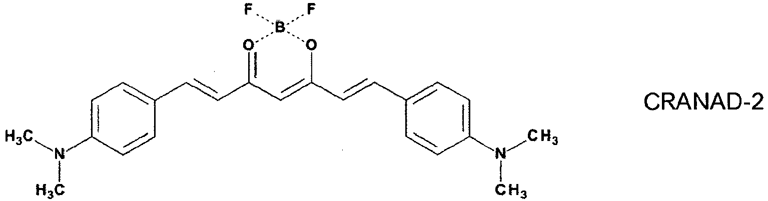

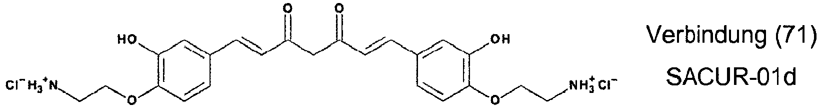

Die Verbindung CRANAD-2 ist ein guter Fluorophor, die eine mit Rhodamin oder Cy5-Farbstoffen vergleichbare Fluoreszenzquantenausbeute aufweist.
Die Inkubation mit CRANAD-2 und den verwendeten Bakterienstämme *S. aureus* ATCC 25923 und *E. coli ATCC* 25922 sowie Bestrahlung mit elektromagnetischer Strahlung und Bestimmung der Phototoxizität erfolgte jeweils wie oben unter c.1) und c.2) beschrieben. Es konnte mit beiden Methoden keine Inaktivierung der getesteten Bakterienstämme *S. aureus* ATCC 25923 und *E*. *coli* ATCC 25922 bei Belichtung in Gegenwart von CRANAD-2 festgestellt werden.

Durch die Aminsubtituenten direkt am Aromaten, die als Elektronenpaar-Donoren wirken können, wird die Photophysik zum Singulett-Prozess hin verschoben und es steht deutlich weniger Energie für Triplettprozesse zur Verfügung. Ein guter Energieübertrag ins Triplettniveau wäre die Vorraussetzung für eine photodynamische Wirkung, die jedoch für die Verbindung CRANAD-2 nicht feststgestellt wurde.

Die Inkubation mit der Verbindung (71) (SACUR-01d) und den verwendeten Bakterienstämme *S. aureus* ATCC 25923 und *E*. *coli ATCC* 25922 sowie Bestrahlung mit elektromagnetischer Strahlung und Bestimmung der Phototoxizität erfolgte ebenfalls jeweils wie oben unter c.1) und c.2) beschrieben. Es konnte mit beiden Methoden kaum eine Inaktivierung der getesteten Bakterienstämme *S. aureus* ATCC 25923 und *E. coli ATCC* 25922 bei Belichtung in Gegenwart von Verbindung (71) (SACUR-01d) festgestellt werden, da durch den Zerfall der Verbindung in der Messlösung keine verlässlichen Werte erhalten wurden. Verbindung (71) (SACUR-01d) zeigte eine vergleichbar niedrige Stabilität in wässriger Lösung wie die natürliche Grundsubstanz Curcumin. Die freien OH-Gruppen tragen wesentlich zu dieser Photoinstabilität bei, da dadurch die Verbindung leicht in das Chinoide Mesomer übergehen kann und somit die Abspaltung einer Molekülhälfte unter Bildung von Ferulasäuren bzw. subst. Vanilinen erleichtert wird.

Der molare Extinktionskoeffizient der Verbindung (71) (SACUR-01d) bei 420 nm in den unten angegenbenen wässrigen Lösungen A - C schwankte zwischen 8000 und 16000 M⁻¹cm⁻¹ und ist somit signifikant geringer als der für Curcumin bekannte Wert (ε_{420, H2O}= 23800 M⁻¹cm⁻¹) (siehe Arnaut LG, Formosinho SJ. J. Photochem. Photobiol. A: Chem. 75, 1993, Seiten 1 bis 20). Diese Ergebnisse zeigen, dass die Verbindung (71) keine ausreichende Stabilität in wässriger Lösung aufweist, um eine photodynamische Wirkung nach Bestrahlung sicherzustellen.
wässrige Lösung A: destiliertes Wasser.
wässrige Lösung B: isotonische Kochsalzlösung (0.9 Gew.-% NaCl).
wässrige Lösung C: PBS -Puffer, pH 7.4 (Zusammensetzung siehe Sambrook, J.; Maniatis, T.; Russel, D.W.: Molecular cloning: a laboratory manual. Cold Spring Harbor Laboratory Press; 3rd edition (2001)).

## Patentansprüche

1. Nicht-therapeutisches
Verfahren zur Inaktivierung von Mikroorganismen, wobei das Verfahren folgende Schritte umfasst:
(A) in Kontakt bringen der Mikroorganismen mit wenigstens einem Photosensibilisator, wobei der Photosensibilisator wenigstens ein 1,7-Diaryl-1,6-heptadien-3,5-dion-Derivat der Formel (100): 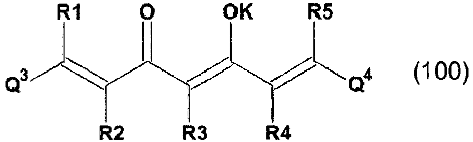 und/oder wenigstens ein 1,7-Diaryl-1,6-heptadien-3,5-dion-Derivat der Formel (101): 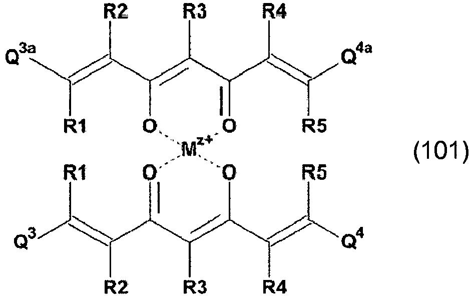 oder jeweils ein pharmakologisch verträgliches Salz und/oder Ester und/oder Komplex davon ist,
wobei die Reste Q³, Q^{3a}, Q⁴ und Q^{4a} jeweils unabhängig voneinander 1 substituierter oder unsubstituierter monozyklischer oder polyzyklischer, aromatischer Rest oder 1 substituierter oder unsubstituierter monozyklischer oder polyzyklischer, heteroaromatischer Rest bedeuten,
wobei die Verbindung mit der Formel (100) keine OH-Gruppe, die direkt an den organischen Rest Q³ oder Q⁴ gebunden ist, enthält, und
wobei die Verbindung mit der Formel (101) keine OH-Gruppe, die direkt an den organischen Rest Q³, Q^{3a}, Q⁴, oder Q^{4a} gebunden ist, enthält, und
wobei K Wasserstoff oder ein Kation bedeutet, und
wobei M^{z+} ein Kation eines Metalls bedeutet, wobei z die formale Oxidationszahl des Metalls M ist und z eine ganze Zahl von 1 bis 7, vorzugsweise von 2 bis 5, bedeutet, und wobei
(a1) wenigstens einer der Reste Q³, Q^{3a}, Q⁴ und Q^{4a} jeweils unabhängig voneinander ein unsubstituierter monozyklischer oder polyzyklischer, heteroaromatischer Rest ist, der wenigstens 5 Ringatome aufweist, wobei die Ringatome mindestens 1 Kohlenstoffatom und wenigstens 1, vorzugsweise protonierbares, Stickstoffatom enthalten, oder
(a2) wenigstens einer der Reste Q³, Q^{3a}, Q⁴ und Q^{4a} jeweils unabhängig voneinander mit mindestens einem organischen Rest W1 substituiert ist, wobei der mindestens eine organische Rest W1 die allgemeine Formel (4), (5), (6), (7), (8), oder (9) aufweist:
-(C(D)(E))ₕ-X, (4)
-A-(C(D)(E))ₕ-X, (5)
-(C(D)(E))ₖ-Aryl-(C(D)(E))ₗ-X, (6)
-A-(C(D)(E))ₖ-Aryl-(C(D)(E))ₗ-X, (7)
-((C(D)(E))ₘ-A)ₚ-(C(D)(E))ₙ-X, (8)
-A-((C(D)(E))ₘ-A)ₚ-(C(D)(E))ₙ-X, (9)
wobei h eine ganze Zahl von 1 bis 20 bedeutet, wobei k eine ganze Zahl von 0 bis 10 bedeutet, wobei l eine ganze Zahl von 0 bis 10 bedeutet, und wobei m, n und p jeweils unabhängig voneinander eine ganze Zahl von 1 bis 6 bedeuten, und
wobei A jeweils unabhängig voneinander Sauerstoff oder Schwefel bedeutet,
wobei D und E jeweils unabhängig voneinander Wasserstoff, Halogen, G-R^{(I)}, oder G-C(=G)-R(^{II}), bedeuten, wobei G jeweils unabhängig voneinander Sauerstoff oder Schwefel bedeutet, und wobei die Reste R^{(I)} und R^{(II)} jeweils unabhängig voneinander Wasserstoff, Methyl, Ethyl, n-Propyl, n-Butyl, n-Pentyl, Phenyl oder Benzyl bedeutet, wobei Phenyl und Benzyl unsubstituiert oder substituiert sein können,
wobei Aryl einen substituierten oder unsubstituierten Aromaten oder einen substituierten oder unsubstituierten Heteroaromaten, der kein Stickstoffatom enthält, bedeutet,
wobei X jeweils unabhängig voneinander ein organischer Rest ist, der (i) wenigstens ein neutrales, protonierbares Stickstoffatom, oder (ii) wenigstens ein positiv geladenes, vorzugsweise quartäres, Stickstoffatom oder (iii) wenigstens ein positiv geladenes, vorzugsweise quartäres, Phosphoratom enthält, und
wobei die Reste R1, R2, R3, R4 und R5 jeweils unabhängig voneinander Wasserstoff, Halogen, Alkyl mit 1 bis 12 C-Atomen, Cycloalkyl mit 1 bis 12 C-Atomen, Alkylaryl mit 1 bis 12 C-Atomen, Aryl mit 5 bis 20 C-Atomen, Ether mit 2 bis 12 C-Atomen oder Glykol mit 2 bis 12 C-Atomen bedeuten,
oder wobei
(b) der Rest R3 ein organischer Rest W2 ist, wobei der eine organische Rest W2 die allgemeine Formel (4), (5), (6), (7), (8), (9), oder (10):
-(C(D)(E))ₕ-X, (4)
-A-(C(D)(E))ₕ-X, (5)
-(C(D)(E))ₖ-Aryl-(C(D)(E))ₗ-X, (6)
-A-(C(D)(E))ₖ-Aryl-(C(D)(E))ₗ-X, (7)
-[(C(D)(E))ₘ-A]ₚ-(C(D)(E))ₙ-X, (8)
-A-[(C(D)(E))ₘ-A]ₚ-(C(D)(E))ₙ-X, (9)
-(-C(D)=C(E)-)ᵣ-X, (10)
aufweist, und
wobei, optional, wenigstens einer der Reste Q³, Q^{3a}, Q⁴ und Q^{4a} jeweils unabhängig voneinander mit mindestens einem organischen Rest W1, der die allgemeine Formel (4), (5), (6), (7), (8), oder (9) aufweist, substituiert sind,
wobei h eine ganze Zahl von 1 bis 20 bedeutet, wobei k eine ganze Zahl von 0 bis 10 bedeuten, wobei l eine ganze Zahl von 0 bis 10 bedeuten, und wobei m, n, p, und r jeweils unabhängig voneinander eine ganze Zahl von 1 bis 6 bedeuten, und
wobei A jeweils unabhängig voneinander Sauerstoff oder Schwefel bedeutet,
wobei D und E jeweils unabhängig voneinander Wasserstoff, Halogen, G-R(^{I}), oder G-C(=G)-R^{(II)}, bedeuten, wobei G jeweils unabhängig voneinander Sauerstoff oder Schwefel bedeutet, und wobei die Reste R^{(I)} und R^{(II)} jeweils unabhängig voneinander Wasserstoff, Methyl, Ethyl, n-Propyl, n-Butyl, n-Pentyl, Phenyl oder Benzyl bedeutet, wobei Phenyl und Benzyl unsubstituiert oder substituiert sein können,
wobei Aryl einen substituierten oder unsubstituierten Aromaten oder einen substituierten oder unsubstituierten Heteroaromaten, der kein Stickstoffatom enthält, bedeutet,
wobei X jeweils unabhängig voneinander ein organischer Rest ist, der (i) wenigstens ein neutrales, protonierbares Stickstoffatom, (ii) wenigstens ein positiv geladenes, vorzugsweise quartäres, Stickstoffatom oder (iii) wenigstens ein positiv geladenes, vorzugsweise quartäres, Phosphoratom enthält, und
wobei die Reste R1, R2, R4 und R5 jeweils unabhängig voneinander Wasserstoff, Halogen, Alkyl mit 1 bis 12 C-Atomen, Alkylaryl mit 1 bis 12 C-Atomen, Aryl mit 5 bis 20 C-Atomen, Ether mit 2 bis 12 C-Atomen oder Glykol mit 2 bis 12 C-Atomen bedeuten,
und
(B) Bestrahlen der Mikroorganismen und des wenigstens einen Photosensibilisators mit elektromagnetischer Strahlung geeigneter Wellenlänge und Energiedichte.

2. Verfahren nach Anspruch 1 wobei in der Verbindung mit der Formel (100) K ein Kation M^{z+} eines Metalls M, wobei z die formale Oxidationszahl des Metalls M ist und wobei z eine ganze Zahl von 1 bis 7, vorzugsweise von 2 bis 3, bedeutet, und wobei die Verbindung die Formel (102) aufweist: 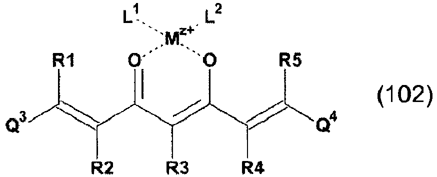 wobei L¹ und L² jeweils unabhängig voneinander Wasser, Fluorid, Chlorid, Bromid, lodid, Phosphat, Hydrogenphosphat, Dihydrogenphosphat, Sulfat, Hydrogensulfat, Tosylat, Mesylat oder wenigstens ein Carboxylation einer Carbonsäure mit 1 bis 15 Kohlenstoffatomen und/oder Mischungen davon bedeuten.

3. Verfahren nach einem der Ansprüche 1 oder 2 wobei die Bestrahlung der Mikroorganismen und des wenigstens einen Photosensibilisators mit elektromagnetischer Strahlung geeigneter Wellenlänge und Energiedichte in Gegenwart wenigstens einer Sauerstoff-abgebenden Verbindung, vorzugsweise Peroxid, und/oder wenigstens eines Sauerstoff-haltigen Gases, vorzugsweise Sauerstoff, erfolgt.

4. Verbindung mit der Formel (1): 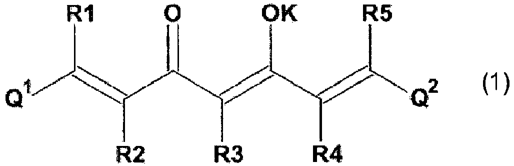 wobei die Reste Q¹ und Q² jeweils unabhängig voneinander 1 substituierter oder unsubstituierter monozyklischer oder polyzyklischer, aromatischer Rest bedeuten,
wobei die Verbindung mit der Formel (1) keine OH-Gruppe, die direkt an den organischen Rest Q¹ oder Q² gebunden ist, enthält, und
wobei K Wasserstoff oder ein Kation bedeutet, und wobei
(a) wenigstens einer der Reste Q¹ und Q² jeweils unabhängig voneinander mit mindestens einem organischen Rest W1a substituiert ist, wobei
der mindestens eine organische Rest W1a die allgemeine Formel (5a), (6a), (7a), (8a), oder (9a) aufweist:
-A-(C(D)(E))ₕ-X^{a}, (5a)
-(C(D)(E))ₖ-Aryl-(C(D)(E))ₗ-X^{a}, (6a)
-A-(C(D)(E))ₖ-Aryl-(C(D)(E))ₗ-X^{a}, (7a)
-((C(D)(E))ₘ-A)ₚ-(C(D)(E))ₙ-X^{a}, (8a)
-A-((C(D)(E))ₘ-A)ₚ-(C(D)(E))ₙ-X^{a}, (9a)
wobei h eine ganze Zahl von 1 bis 20 bedeutet, wobei k eine ganze Zahl von 0 bis 10 bedeutet, wobei l eine ganze Zahl von 0 bis 10 bedeutet, und wobei m, n und p jeweils unabhängig voneinander eine ganze Zahl von 1 bis 6 bedeuten, und
wobei A jeweils unabhängig voneinander Sauerstoff oder Schwefel bedeutet,
wobei D und E jeweils unabhängig voneinander Wasserstoff, Halogen, G-R^{(I)}, oder G-C(=G)-R(^{II}), bedeuten, wobei G jeweils unabhängig voneinander Sauerstoff oder Schwefel bedeutet, und wobei die Reste R^{(I)} und R^{(II)} jeweils unabhängig voneinander Wasserstoff, Methyl, Ethyl, n-Propyl, n-Butyl, n-Pentyl, Phenyl oder Benzyl bedeutet, wobei Phenyl und Benzyl unsubstituiert oder substituiert sein können,
wobei Aryl einen substituierten oder unsubstituierten Aromaten oder einen substituierten oder unsubstituierten Heteroaromaten, der kein Stickstoffatom enthält, bedeutet,
wobei X^{a} jeweils unabhängig voneinander ein Rest der Formel (20c), (20d) oder (21) bedeutet: 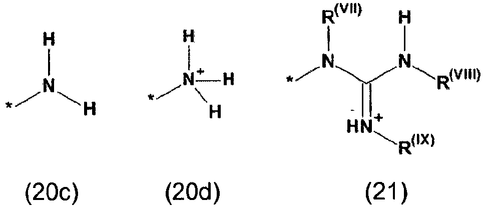 wobei jeder der Reste R^{(VII)}, R^{(VIII)}, und R^{(IX)} jeweils unabhängig voneinander Wasserstoff, ein Arylrest mit 5 bis 12 C-Atomen, ein Alkylarylrest mit 5 bis 12 C-Atomen, ein Alkylrest, der geradkettig oder verzweigt sein kann, mit 1 bis 8 C-Atomen, oder ein Etherrest, der geradkettig oder verzweigt sein kann, mit 1 bis 8 C-Atomen bedeutet, und
wobei die Reste R1, R2, R3, R4 und R5 jeweils unabhängig voneinander Wasserstoff, Halogen, Alkyl mit 1 bis 12 C-Atomen, Cycloalkyl mit 1 bis 12 C-Atomen, Alkylaryl mit 1 bis 12 C-Atomen, Aryl mit 5 bis 20 C-Atomen, Ether mit 2 bis 12 C-Atomen oder Glykol mit 2 bis 12 C-Atomen bedeuten.

5. Verbindung nach Anspruch 4,
wobei K ein Kation M^{z+} eines Metalls M bedeutet, wobei z die formale Oxidationszahl des Metalls M ist und eine ganze Zahl von 1 bis 7, vorzugsweise von 2 bis 5, bedeutet, und
wobei die Verbindung die Formel (2) aufweist: 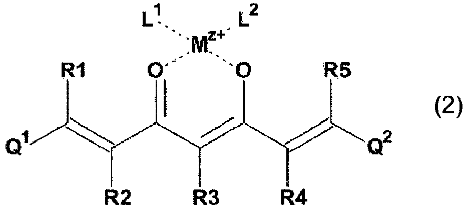 wobei L¹ und L² jeweils unabhängig voneinander Wasser, Halogenid, Cyanid, Thiocyanat, Phosphat, Hydrogenphosphat, oder ein Carboxylation einer Carbonsäure mit 1 bis 10 Kohlenstoffatomen, vorzugsweise Formiat, Acetat, n-Propionat, Lactat, Oxalat, Fumarat, Maleinat, Tartrat, Succinylat, Benzoat, Salicylat, oder Citrat, bedeuten,

6. Verbindung mit der Formel (3): 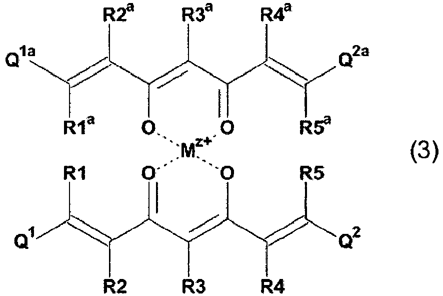 wobei M^{z+} ein Kation eines Metalls bedeutet, wobei z die formale Oxidationszahl des Metalls M ist und eine ganze Zahl von 1 bis 7, vorzugsweise von 2 bis 5, bedeutet, und
wobei Q¹ und Q² jeweils unabhängig voneinander 1 substituierter oder unsubstituierter monozyklischer oder polyzyklischer, aromatischer Rest bedeuten, und
wobei die Reste Q^{1a} und Q^{2a} jeweils unabhängig voneinander 1 substituierter oder unsubstituierter monozyklischer oder polyzyklischer, aromatischer Rest oder 1 substituierter oder unsubstituierter monozyklischer oder polyzyklischer, heteroaromatischer Rest bedeuten,
wobei die Verbindung mit der Formel (3) keine OH-Gruppe, die direkt an den organischen Rest Q¹, Q^{1a}, Q², oder Q^{2a} gebunden ist, enthält,
und wobei
(a) wenigstens einer der Reste Q¹ und Q² jeweils unabhängig voneinander mit mindestens einem organischen Rest W1a substituiert ist, der die allgemeine Formel (5a), (6a), (7a), (8a), oder (9a) aufweist:
-A-(C(D)(E))ₕ-X^{a}, (5a)
-(C(D)(E))ₖ-Aryl-(C(D)(E))ₗ-X^{a}, (6a)
-A-(C(D)(E))ₖ-Aryl-(C(D)(E))ₗ-X^{a}, (7a)
-((C(D)(E))ₘ-A)ₚ-(C(D)(E))ₙ-X^{a}, (8a)
-A-((C(D)(E))ₘ-A)ₚ-(C(D)(E))ₙ-X^{a}, (9a)
wobei wenigstens einer der Reste Q^{1a} und Q^{2a} jeweils unabhängig voneinander mit mindestens einem organischen Rest W1c substituiert ist, der die allgemeine Formel (5c), (6c), (7c), (8c), oder (9c) aufweist:
-A-(C(D)(E))ₕ-X^{c}, (5c)
-(C(D)(E))ₖ-Aryl-(C(D)(E))ₗ-X^{c}, (6c)
-A-(C(D)(E))ₖ-Aryl-(C(D)(E))ₗ-X^{c}, (7c)
-((C(D)(E))ₘ-A)ₚ-(C(D)(E))ₙ-X^{c}, (8c)
-A-((C(D)(E))ₘ-A)ₚ-(C(D)(E))ₙ-X^{c}, (9c)
wobei h eine ganze Zahl von 1 bis 20 bedeutet, wobei k eine ganze Zahl von 0 bis 10 bedeutet, wobei l eine ganze Zahl von 0 bis 10 bedeutet, und wobei m, n und p jeweils unabhängig voneinander eine ganze Zahl von 1 bis 6 bedeuten, und
wobei A jeweils unabhängig voneinander Sauerstoff oder Schwefel bedeutet,
wobei D und E jeweils unabhängig voneinander Wasserstoff, Halogen, G-R^{(I)}, oder G-C(=G)-R^{(II)}, bedeuten, wobei G jeweils unabhängig voneinander Sauerstoff oder Schwefel bedeutet, und wobei die Reste R^{(I)} und R^{(II)} jeweils unabhängig voneinander Wasserstoff, Methyl, Ethyl, n-Propyl, n-Butyl, n-Pentyl, Phenyl oder Benzyl bedeutet, wobei Phenyl und Benzyl unsubstituiert oder substituiert sein können,
wobei Aryl einen substituierten oder unsubstituierten Aromaten oder einen substituierten oder unsubstituierten Heteroaromaten, der kein Stickstoffatom enthält, bedeutet, und
wobei X^{a} jeweils unabhängig voneinander ein Rest der Formel (20c), (20d) oder (21) bedeutet: 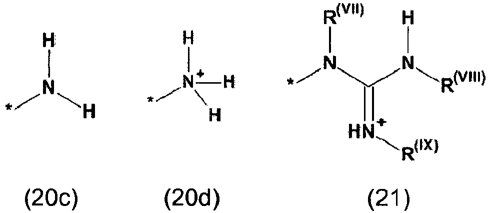 wobei jeder der Reste R^{(VII)}, R^{(VIII)}, und R^{(IX)} jeweils unabhängig voneinander Wasserstoff, ein Arylrest mit 5 bis 12 C-Atomen, ein Alkylarylrest mit 5 bis 12 C-Atomen, ein Alkylrest, der geradkettig oder verzweigt sein kann, mit 1 bis 8 C-Atomen, oder ein Etherrest, der geradkettig oder verzweigt sein kann, mit 1 bis 8 C-Atomen, bedeutet, und
wobei X^{c} jeweils unabhängig voneinander ein organischer Rest ist, der (i) wenigstens ein neutrales, protonierbares Stickstoffatom, oder (ii) wenigstens ein positiv geladenes, vorzugsweise quartäres, Stickstoffatom, oder (iii) wenigstens ein positiv geladenes, vorzugsweise quartäres, Phosphoratom enthält, und
wobei die Reste R1, R1^{a}, R2, R2^{a}, R3, R3^{a}, R4, R4^{a}, R5 und R5^{a} jeweils unabhängig voneinander Wasserstoff, Halogen, Alkyl mit 1 bis 12 C-Atomen, Cycloalkyl mit 1 bis 12 C-Atomen, Alkylaryl mit 1 bis 12 C-Atomen, Aryl mit 5 bis 20 C-Atomen, Ether mit 2 bis 12 C-Atomen oder Glykol mit 2 bis 12 C-Atomen bedeuten.

7. Verbindung nach Anspruch 6, wobei die Verbindung mit der Formel (3) die Formel (3a) aufweist: 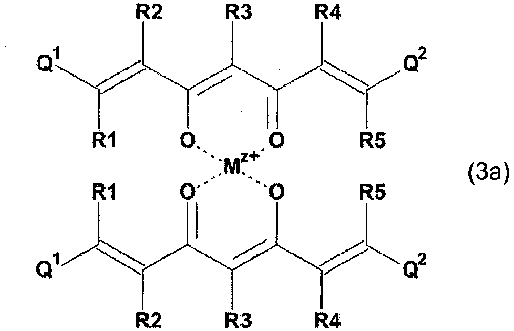 wobei M^{z+} ein Kation eines Metalls bedeutet, wobei z die formale Oxidationszahl des Metalls M ist und eine ganze Zahl von 1 bis 7, vorzugsweise von 2 bis 5, bedeutet, und
wobei Q¹ und Q² jeweils unabhängig voneinander 1 substituierter oder unsubstituierter monozyklischer oder polyzyklischer, aromatischer Rest bedeuten, und
wobei die Verbindung mit der Formel (3a) keine OH-Gruppe, die direkt an den organischen Rest Q¹ oder Q² gebunden ist, enthält,
und wobei
(a) wenigstens einer der Reste Q¹ und Q² jeweils unabhängig voneinander mit mindestens einem organischen Rest W1a substituiert ist, der die allgemeine Formel (5a), (6a), (7a), (8a), oder (9a) aufweist:
-A-(C(D)(E))ₕ-X^{a}, (5a)
-(C(D)(E))ₖ-Aryl-(C(D)(E))ₗ-X^{a}, (6a)
-A-(C(D)(E))ₖ-Aryl-(C(D)(E))ₗ-X^{a}, (7a)
-((C(D)(E))ₘ-A)ₚ-(C(D)(E))ₙ-X^{a}, (8a)
-A-((C(D)(E))ₘ-A)ₚ-(C(D)(E))ₙ-X^{a}, (9a)
wobei h eine ganze Zahl von 1 bis 20 bedeutet, wobei k eine ganze Zahl von 0 bis 10 bedeutet, wobei l eine ganze Zahl von 0 bis 10 bedeutet, und wobei m, n und p jeweils unabhängig voneinander eine ganze Zahl von 1 bis 6 bedeuten, und
wobei A jeweils unabhängig voneinander Sauerstoff oder Schwefel bedeutet,
wobei D und E jeweils unabhängig voneinander Wasserstoff, Halogen, G-R^{(I)}, oder G-C(=G)-R(^{II}), bedeuten, wobei G jeweils unabhängig voneinander Sauerstoff oder Schwefel bedeutet, und wobei die Reste R^{(I)} und R^{(II)} jeweils unabhängig voneinander Wasserstoff, Methyl, Ethyl, n-Propyl, n-Butyl, n-Pentyl, Phenyl oder Benzyl bedeutet, wobei Phenyl und Benzyl unsubstituiert oder substituiert sein können,
wobei Aryl einen substituierten oder unsubstituierten Aromaten oder einen substituierten oder unsubstituierten Heteroaromaten, der kein Stickstoffatom enthält, bedeutet, und
wobei X^{a} jeweils unabhängig voneinander ein Rest der Formel (20c), (20d) oder (21) bedeutet: 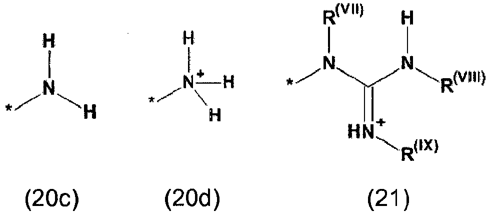 wobei jeder der Reste R^{(VII)}, R^{(VIII)}, und R^{(IX)} jeweils unabhängig voneinander Wasserstoff, ein Arylrest mit 5 bis 12 C-Atomen, ein Alkylarylrest mit 5 bis 12 C-Atomen, ein Alkylrest, der geradkettig oder verzweigt sein kann, mit 1 bis 8 C-Atomen, oder ein Etherrest, der geradkettig oder verzweigt sein kann, mit 1 bis 8 C-Atomen, bedeutet, und
wobei die Reste R1, R2, R3, R4, und R5 jeweils unabhängig voneinander Wasserstoff, Halogen, Alkyl mit 1 bis 12 C-Atomen, Cycloalkyl mit 1 bis 12 C-Atomen, Alkylaryl mit 1 bis 12 C-Atomen, Aryl mit 5 bis 20 C-Atomen, Ether mit 2 bis 12 C-Atomen oder Glykol mit 2 bis 12 C-Atomen bedeuten.

8. Verbindung nach einem der Ansprüche 5, 6 oder 7,
wobei M aus der Gruppe ausgewählt wird, die aus B, Al, Zn, Cu, Mg, Ca, Fe, Si, Ga, Sn, Rh, Co, Ti, Zr, V, Cr, Mo, Mn, Ru, Pd, Ir, Ni, und Kombinationen davon besteht.

9. Verbindung nach einem der Ansprüche 4 bis 8,
wobei die Reste Q¹ und Q^{1a} jeweils unabhängig von einander ein aromatischer Rest der allgemeinen Formel (11a), (12a), (13a), (14a), (15a), (16a), (17a), (18a), oder (19a): 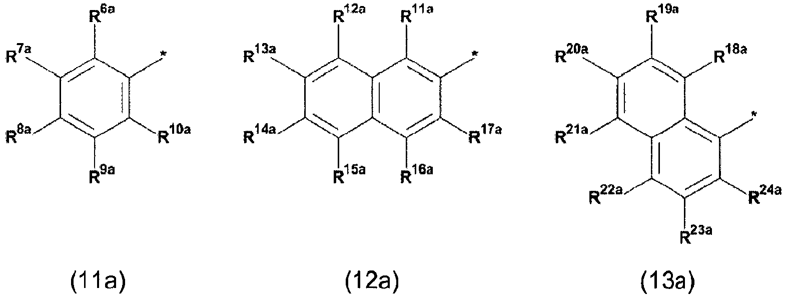 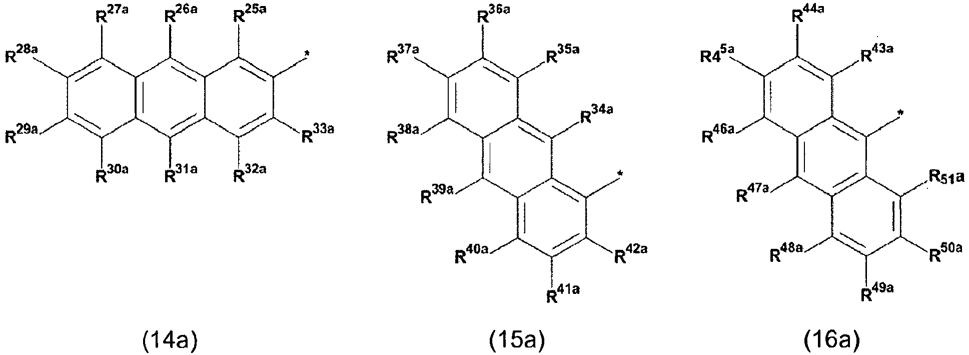 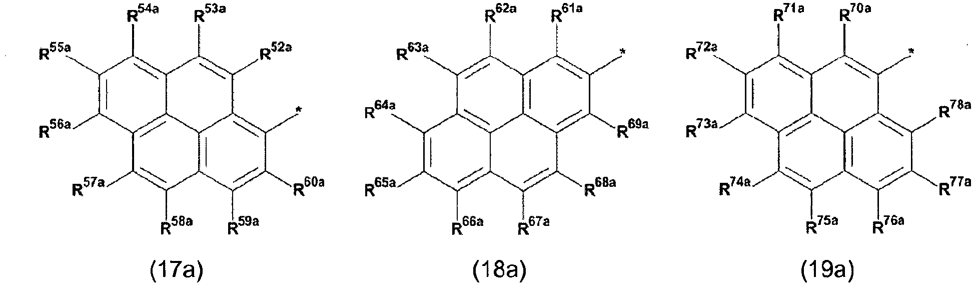 ist, und
wobei die Reste Q² und Q^{2a} jeweils unabhängig von einander ein aromatischer Rest der allgemeinen Formel (11b), (12b), (13b), (14b), (15b), (16b), (17b), (18b), oder (19b): 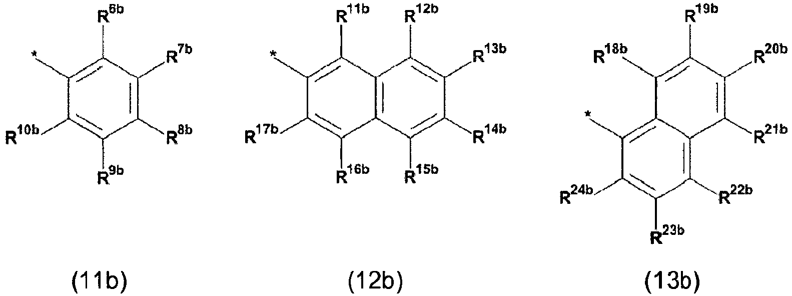 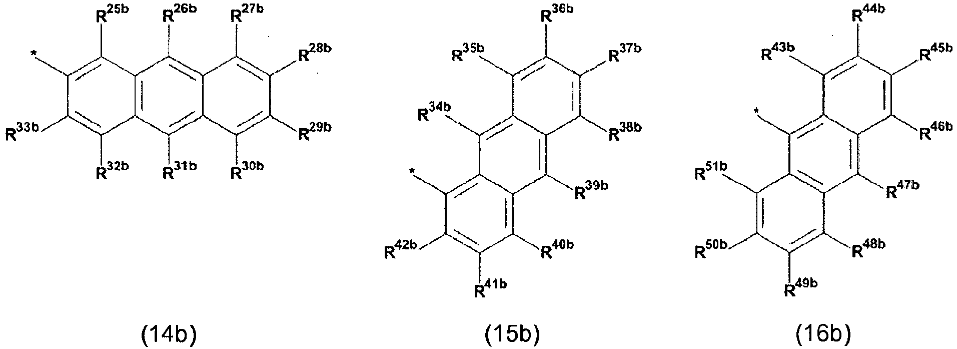 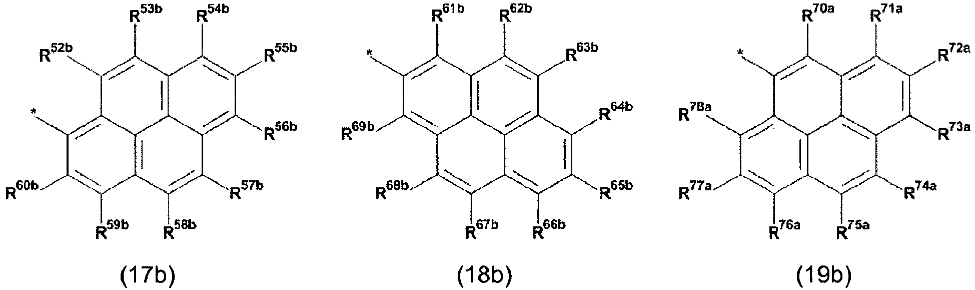 bedeuten, und
wobei jeweils mindestens 1 Rest R^{6a} bis R^{10a}, R^{11a} bis R^{17a}, R^{18a} bis R^{24a}, R^{25a} bis R^{33a}, R^{34a} bis R^{42a}, R^{43a} bis R^{51a}, R^{52a} bis R^{60a}, R^{61a} bis R^{69a}, R^{70a} bis R^{78a}, R^{6b} bis R^{10b}, R^{11b} bis R^{17b}, R^{18b} bis R^{24b}, R^{25b} bis R^{33b}, R^{34b} bis R^{42b}, R^{43b} bis R^{51b}, R^{52b} bis R^{60b}, R^{61b} bis R^{69b}, oder R^{70b} bis R^{78b} jeweils unabhängig voneinander ein organischer Rest W1a oder ein organischer Rest W1b oder ein organischer Rest W1c ist, und
wobei die Reste R^{6a} bis R^{10a}, R^{11a} bis R^{17a}, R^{18a} bis R^{24a}, R^{25a} bis R^{33a}, R^{34a} bis R^{42a}, R^{43a} bis R^{51a}, R^{52a} bis R^{60a}, R^{61a} bis R^{69a}, R^{70a} bis R^{78a}, R^{6b} bis R^{10b}, R^{11b} bis R^{17b}, R^{18b} bis R^{24b}, R^{25b} bis R^{33b}, R^{34b} bis R^{42b}, R^{43b} bis R^{51b}, R^{52b} bis R^{60b}, R^{61b} bis R^{69b}, oder R^{70b} bis R^{78b}, die nicht ein organischer Rest W1a oder ein organischer Rest W1b oder ein organischer Rest W1c sind, jeweils unabhängig voneinander gleich oder verschieden sind und Wasserstoff, Halogen, Hydroxyl, Thiol, Nitro, Carboxylat, Aldehyd mit 1 bis 8 C-Atomen, Keton mit 2 bis 8 C-Atomen, O-Alkyl mit 1 bis 12 C-Atomen, S-Alkyl mit 1 bis 12 C-Atomen, O-Alkenyl mit 2 bis 12 C-Atomen, S-Alkenyl mit 2 bis 12 C-Atomen, O-Aryl mit 5 bis 20 C-Atomen, S-Aryl mit 5 bis 20 C-Atomen, Ether mit 2 bis 12 C-Atomen, Thioether mit 2 bis 12 C-Atomen, Carbonsäureester mit 1 bis 12 C-Atomen, Carbonsäureamid mit 1 bis 12 C-Atomen, Thioester mit 1 bis 12 C-Atomen, Alkyl mit 1 bis 12 C-Atomen, Alkenyl mit 2 bis 12 C-Atomen, Cycloalkyl mit 3 bis 12 C-Atomen, Cycloalkenyl mit 3 bis 12 C-Atomen, Alkylaryl mit 1 bis 12 C-Atomen, Aryl mit 5 bis 20 C-Atomen oder Heteroaryl, das kein Stickstoffatom enthält, mit 4 bis 20 C-Atomen bedeuten.

10. Verbindung nach Anspruch 9,
wobei die Rest Q¹ und Q^{1a} jeweils unabhängig voneinander ein aromatischer Rest der allgemeinen Formel (11a), (12a) oder (13a) und wobei die Reste Q² und Q^{2a} jeweils unabhängig von einander ein aromatischer Rest der allgemeinen Formel (11b), (12b) oder (13b) ist, und
wobei jeweils mindestens 1 Rest R^{6a} bis R^{10a}, R^{11a} bis R^{17a}, R^{18a} bis R^{24a}, R^{6b} bis R^{10b}, R^{11b} bis R^{17b} oder R^{18b} bis R^{24b} jeweils unabhängig voneinander ein organischer Rest W1a oder ein organischer Rest W1b oder ein organischer Rest W1c ist, wobei
wobei die Reste R^{10a}, R^{11a} bis R^{17a}, R^{18a} bis R^{24a}, R^{6b} bis R^{10b}, R^{11b} bis R^{17b} und R^{18b} bis R^{24b}, die nicht ein organischer Rest W1a oder ein organischer Rest W1b oder ein organischer Rest W1c sind, jeweils unabhängig voneinander gleich oder verschieden sind und Wasserstoff, Halogen, Hydroxyl, Thiol, Nitro, Carboxylat, Aldehyd mit 1 bis 8 C-Atomen, Keton mit 2 bis 8 C-Atomen, O-Alkyl mit 1 bis 12 C-Atomen, S-Alkyl mit 1 bis 12 C-Atomen, O-Alkenyl mit 2 bis 12 C-Atomen, S-Alkenyl mit 2 bis 12 C-Atomen, O-Aryl mit 5 bis 20 C-Atomen, S-Aryl mit 5 bis 20 C-Atomen, Ether mit 2 bis 12 C-Atomen, Thioether mit 2 bis 12 C-Atomen, Carbonsäureester mit 1 bis 12 C-Atomen, Carbonsäureamid mit 1 bis 12 C-Atomen, Thioester mit 1 bis 12 C-Atomen, Alkyl mit 1 bis 12 C-Atomen, Alkenyl mit 2 bis 12 C-Atomen, Cycloalkyl mit 3 bis 12 C-Atomen, Cycloalkenyl mit 3 bis 12 C-Atomen, Alkylaryl mit 1 bis 12 C-Atomen, Aryl mit 5 bis 20 C-Atomen oder Heteroaryl, das kein Stickstoffatom enthält, mit 4 bis 20 C-Atomen bedeuten.

11. Verbindung nach Anspruch 6,
wobei der organische Rest X^{c} jeweils unabhängig voneinander ein Rest der Formel (20a), (20b), (21), (22a), (22b), (23a), (24b), oder (24): 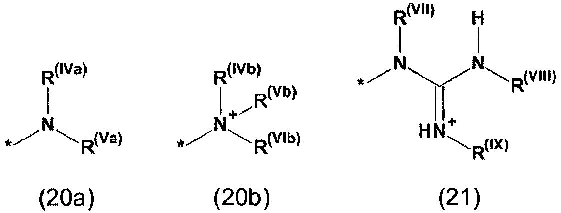 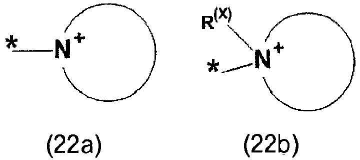 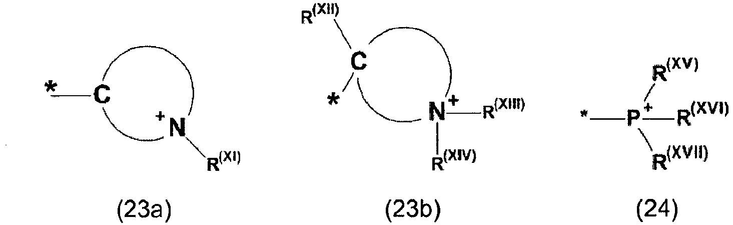 bedeute,
wobei jeder der Reste R^{(IVa)}, R^{(Va)}, R^{(IVb)}, R^{(Vb)}, R^{(VIb)}, R^{(VII)}, R^{(VIII)}, R^{(X)}, R^{(XI)}, R^{(XII)}, R^{(XIII)}, R^{(XIV)}, R^{(XV)}, R^{(XVI)}, und R^{(XVII)} jeweils unabhängig voneinander Wasserstoff, ein Arylrest mit 5 bis 12 C-Atomen, ein Alkylrest, der geradkettig oder verzweigt sein kann, mit 1 bis 8 C-Atomen, oder ein Etherrest, der geradkettig oder verzweigt sein kann, mit 1 bis 8 C-Atomen bedeutet, und
wobei der Rest mit der Formel (22a) und der Rest mit der Formel (23a): 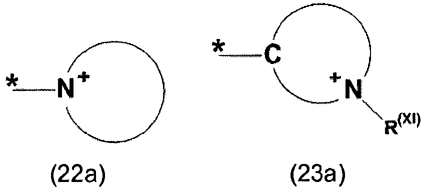 einen substituierten oder unsubstituierten heterocyclischen Rest mit 5 bis 7 Ringatomen, die mindestens 1 Kohlenstoffatom und mindestens 1 Stickstoffatom sowie optional 1 oder 2 Sauerstoffatome umfassen, wobei 1 Stickstoffatom eine Doppelbindung ausbildet, darstellt, und
wobei der Rest mit der Formel (22b) und der Rest mit der Formel (23b): 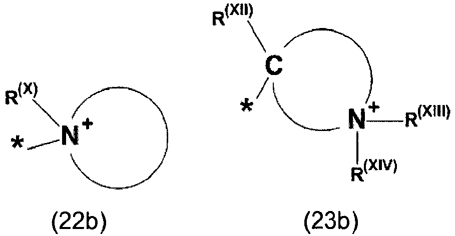 einen substituierten oder unsubstituierten heterocyclischen Rest mit 5 bis 7 Ringatomen, die mindestens 1 Kohlenstoffatom und mindestens 1 Stickstoffatom sowie optional 1 oder 2 Sauerstoffatome umfassen, wobei 1 Stickstoffatom eine Einfachbindung ausbildet, darstellt.

12. Verbindung nach Anspruch 6,
wobei der organische Rest W1c jeweils unabhängig voneinander ein organischer Rest der allgemeinen Formel (31a), (31b), (32), (34), (35), (37a) oder (37b) bedeutet: 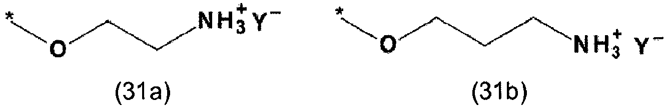 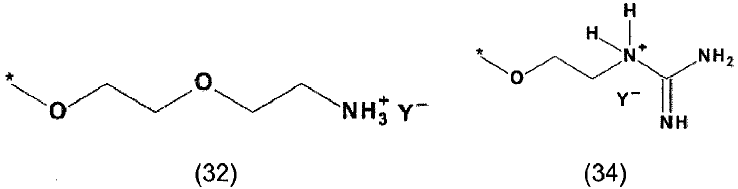 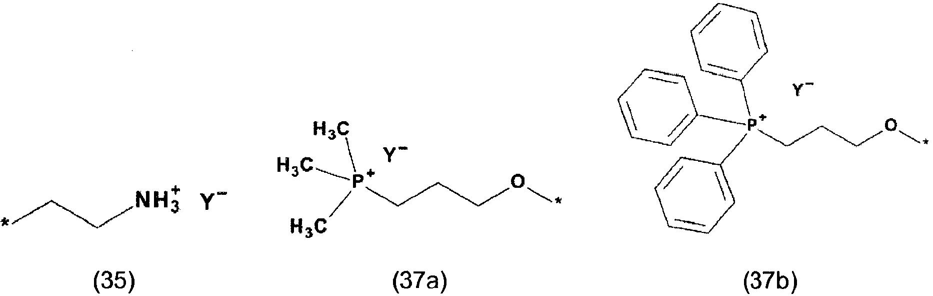 wobei Y⁻ ein Anion ist, das jeweils unabhängig voneinander Fluorid, Chlorid, Bromid, lodid, Sulfat, Hydrogensulfat, Phosphat, Hydrogenphosphat, Dihydrogenphosphat, Tosylat, Mesylat, oder wenigstens ein Carboxylation einer Carbonsäure mit 1 bis 15 Kohlenstoffatomen bedeutet.

13. Verbindung nach einem der Ansprüche 4 bis 10,
wobei der organische Rest W1a jeweils unabhängig voneinander ein organischer Rest der allgemeinen Formel (31a), (31b), (32) oder (34): 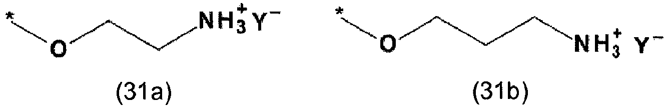 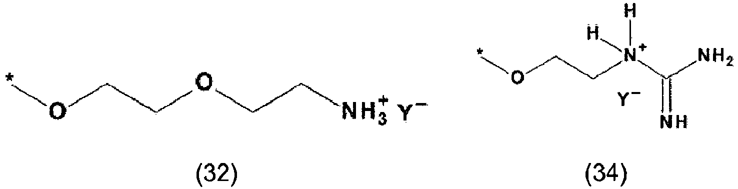 bedeutet, und
wobei Y⁻ ein Anion ist, das jeweils unabhängig voneinander Fluorid, Chlorid, Bromid, lodid, Sulfat, Hydrogensulfat, Phosphat, Hydrogenphosphat, Dihydrogenphosphat, Tosylat, Mesylat, oder wenigstens ein Carboxylation einer Carbonsäure mit 1 bis 15 Kohlenstoffatomen bedeutet.

14. Verbindung nach einem der Ansprüche 4 bis 13,
wobei die Verbindung wenigstens eine Verbindung der Formel (40) bis (63), (68), (69a), (69b) oder (70) ist:

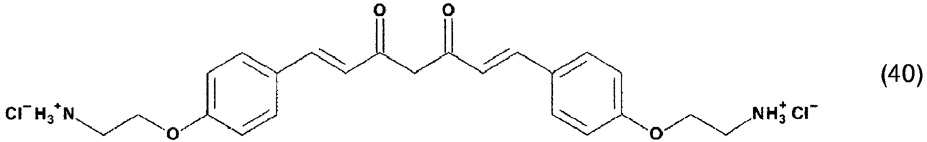

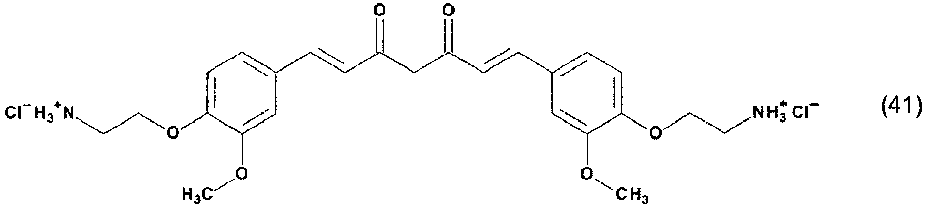

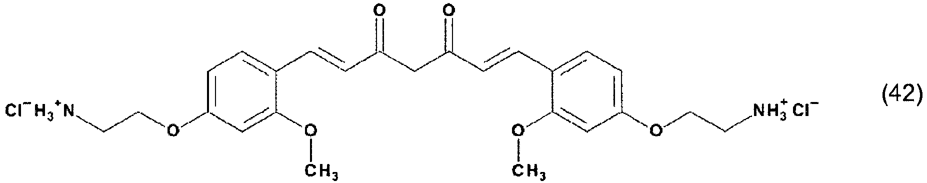

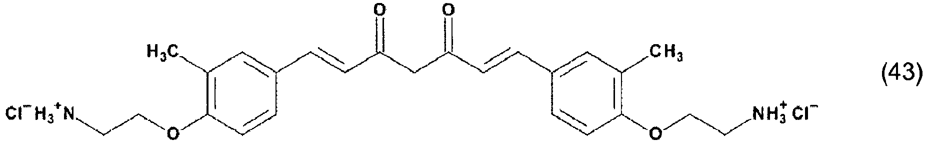

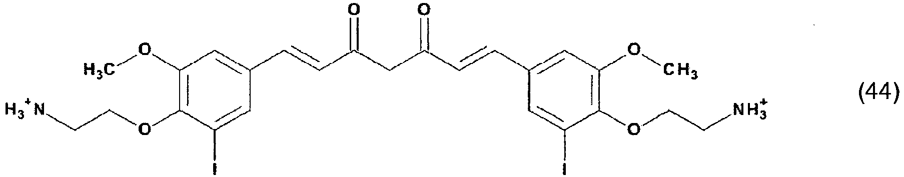

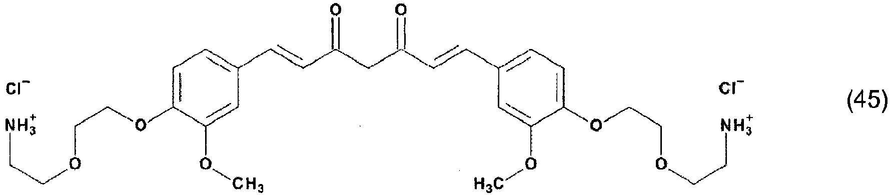

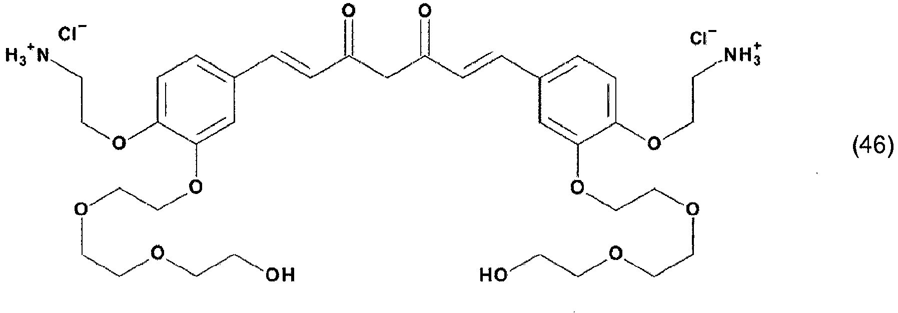

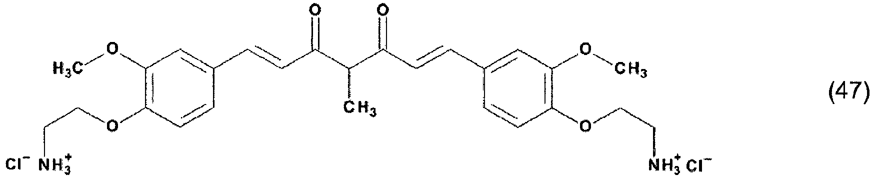

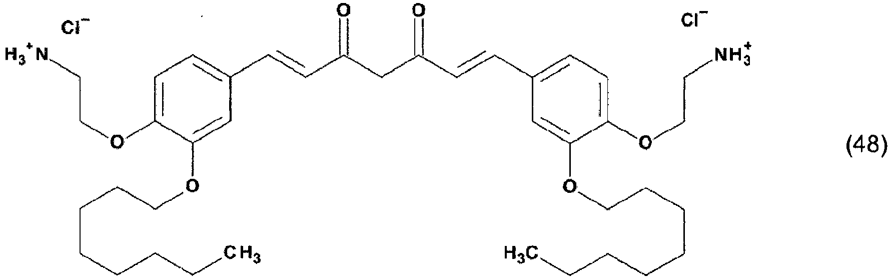

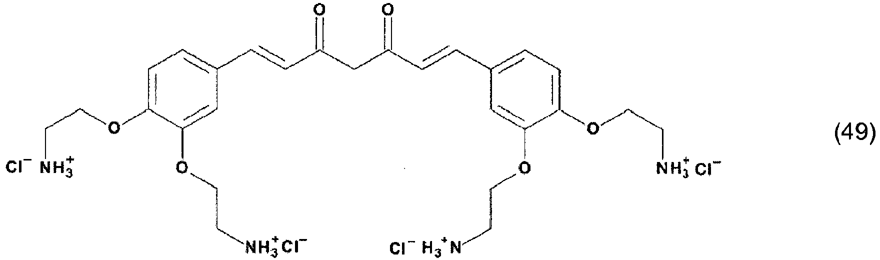

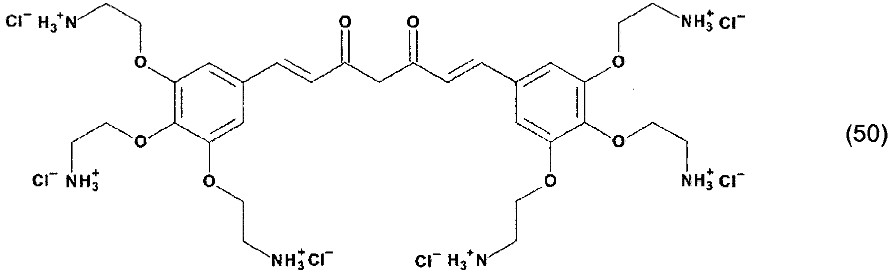

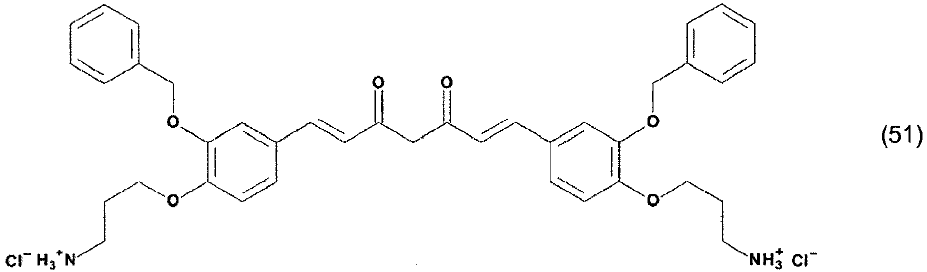

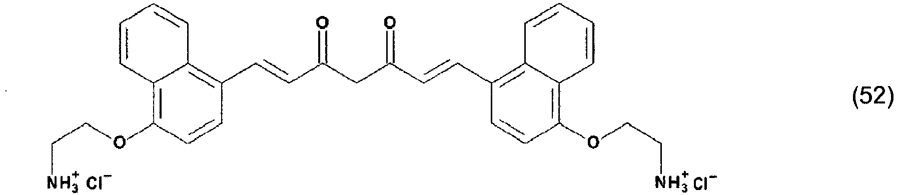

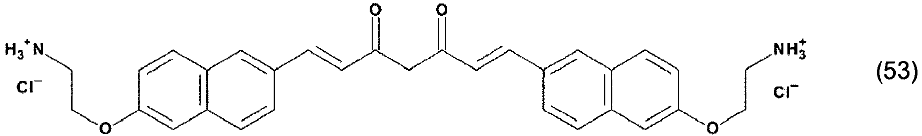

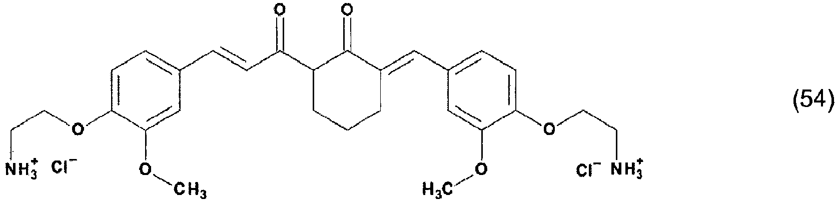

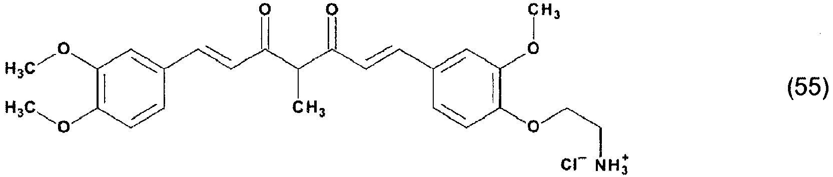

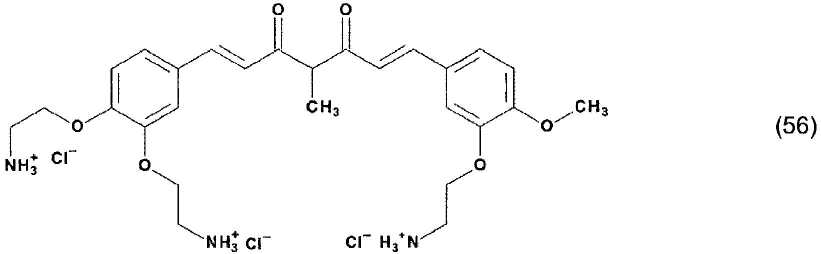

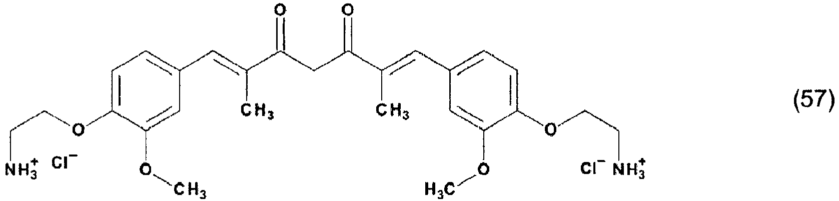

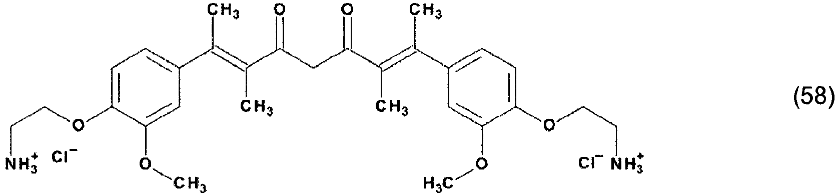

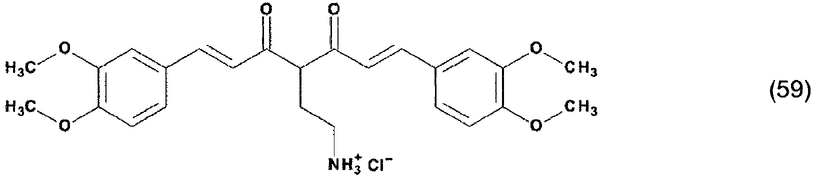

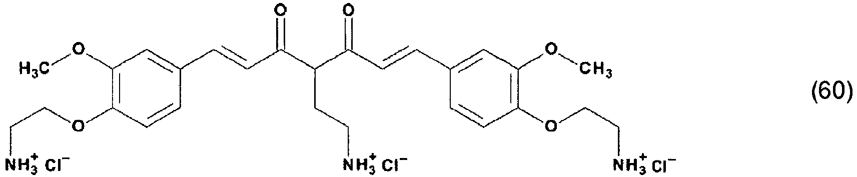

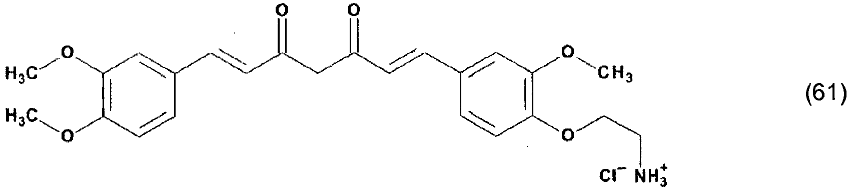

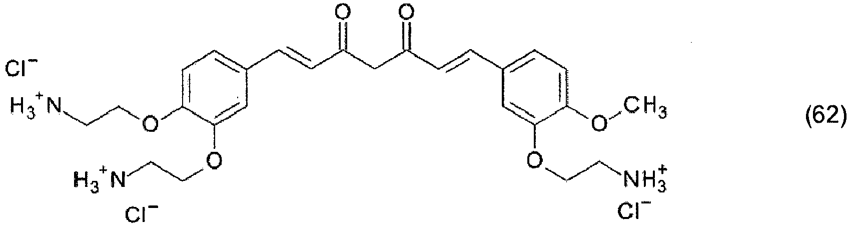

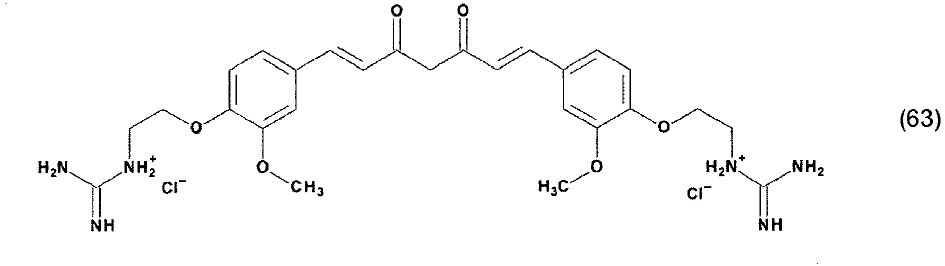

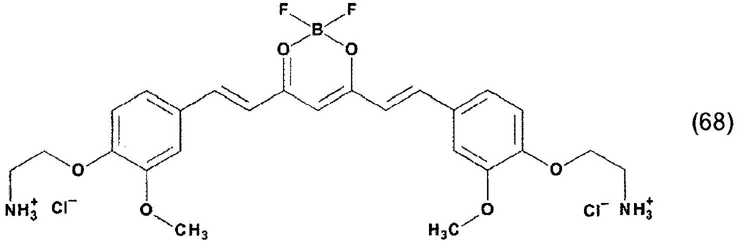

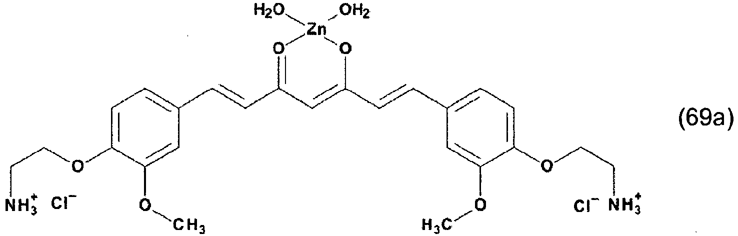

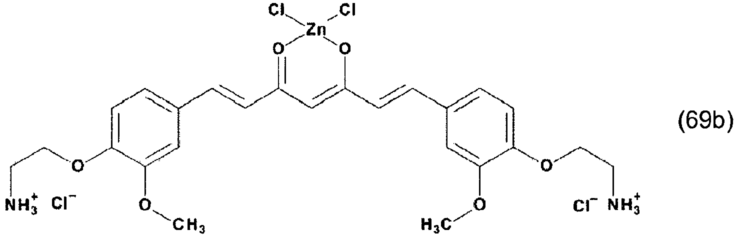

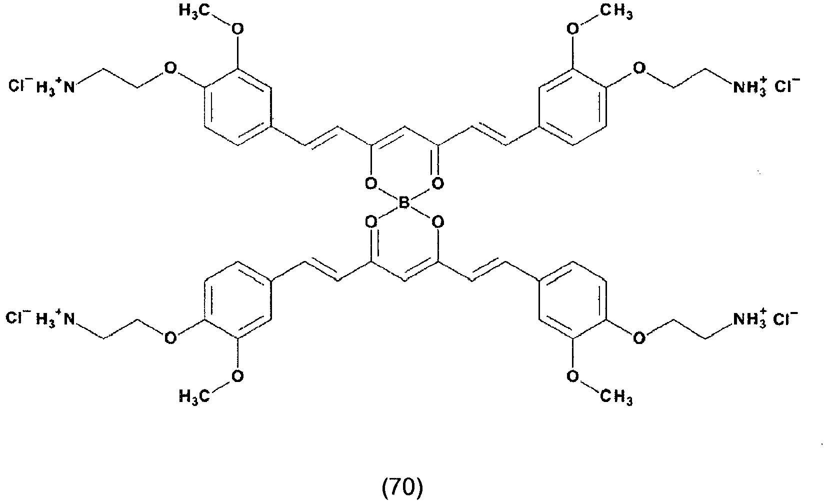

15. Verfahren nach einem der Ansprüche 1 bis 3,
wobei die Reste Q³ und Q^{3a} jeweils unabhängig von einander ein aromatischer Rest der allgemeinen Formel (11a), (12a), (13a), (14a), (15a), (16a), (17a), (18a), oder (19a): 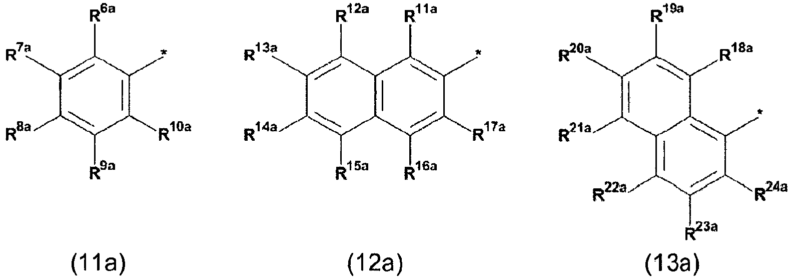 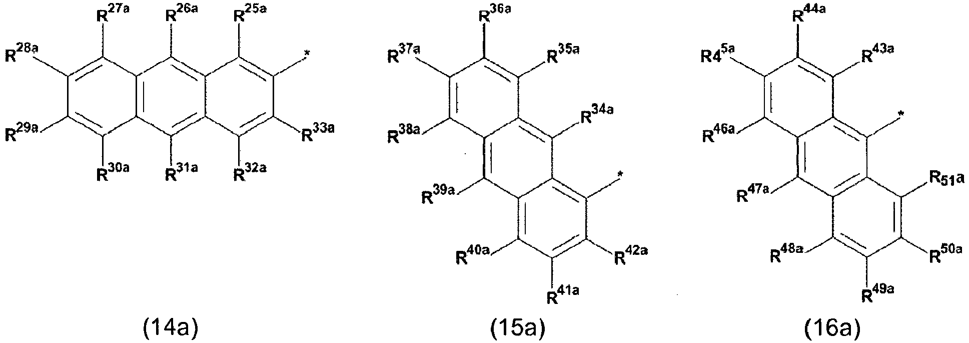 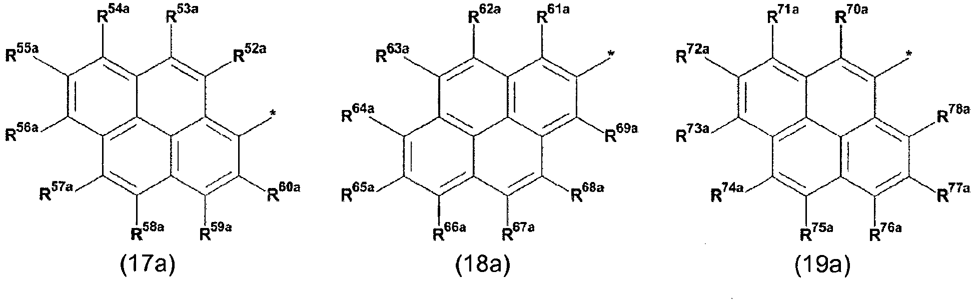 ist, und
wobei die Reste Q⁴ und Q^{4a} jeweils unabhängig von einander ein aromatischer Rest der allgemeinen Formel (11b), (12b), (13b), (14b), (15b), (16b), (17b), (18b), oder (19b): 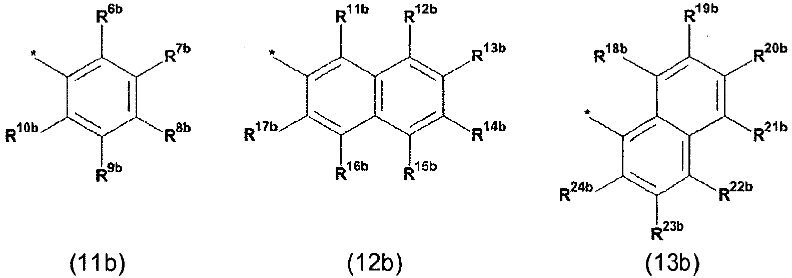 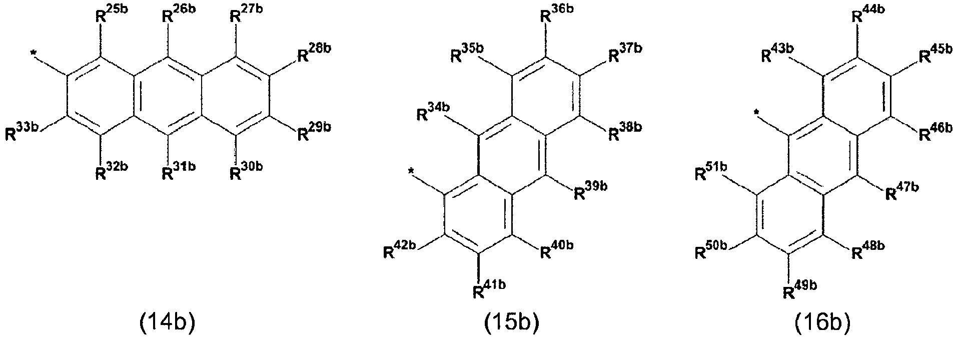 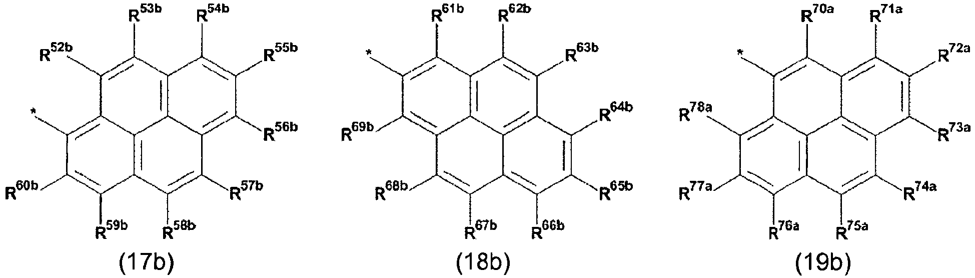 bedeuten, und
wobei jeweils mindestens 1 Rest R^{6a} bis R^{10a}, R^{11a} bis R^{17a}, R^{18a} bis R^{24a}, R^{25a} bis R^{33a}, R^{34a} bis R^{42a}, R^{43a} bis R^{51a}, R^{52a} bis R^{60a}, R^{61a} bis R^{69a}, R^{70a} bis R^{78a}, R^{6b} bis R^{10b}, R^{11b} bis R^{17b}, R^{18b} bis R^{24b}, R^{25b} bis R^{33b}, R^{34b} bis R^{42b}, R^{43b} bis R^{51b}, R^{52b} bis R^{60b} R^{61b} bis R^{69b}, oder R^{70b} bis R^{78b} jeweils unabhängig voneinander ein organischer Rest W1a oder ein organischer Rest W1b oder ein organischer Rest W1c ist, und
wobei die Reste R^{6a} bis R^{10a}, R^{11a} bis R^{17a}, R^{18a} bis R^{24a}, R^{25a} bis R^{33a}, R^{34a} bis R^{42a}, R^{43a} bis R^{51a}, R^{52a} bis R^{60a}, R^{61a} bis R^{69a}, R^{70a} bis R^{78a}, R^{6b} bis R^{10b}, R^{11b} bis R^{17b}, R^{18b} bis R^{24b}, R^{25b} bis R^{33b}, R^{34b} bis R^{42b}, R^{43b} bis R^{51b}, R^{52b} bis R^{60b}, R^{61b} bis R^{69b}, oder R^{70b} bis R^{78b}, die nicht ein organischer Rest W1a oder ein organischer Rest W1b oder ein organischer Rest W1c sind, jeweils unabhängig voneinander gleich oder verschieden sind und Wasserstoff, Halogen, Hydroxyl, Thiol, Nitro, Carboxylat, Aldehyd mit 1 bis 8 C-Atomen, Keton mit 2 bis 8 C-Atomen, O-Alkyl mit 1 bis 12 C-Atomen, S-Alkyl mit 1 bis 12 C-Atomen, O-Alkenyl mit 2 bis 12 C-Atomen, S-Alkenyl mit 2 bis 12 C-Atomen, O-Aryl mit 5 bis 20 C-Atomen, S-Aryl mit 5 bis 20 C-Atomen, Ether mit 2 bis 12 C-Atomen, Thioether mit 2 bis 12 C-Atomen, Carbonsäureester mit 1 bis 12 C-Atomen, Carbonsäureamid mit 1 bis 12 C-Atomen, Thioester mit 1 bis 12 C-Atomen, Alkyl mit 1 bis 12 C-Atomen, Alkenyl mit 2 bis 12 C-Atomen, Cycloalkyl mit 3 bis 12 C-Atomen, Cycloalkenyl mit 3 bis 12 C-Atomen, Alkylaryl mit 1 bis 12 C-Atomen, Aryl mit 5 bis 20 C-Atomen oder Heteroaryl, das kein Stickstoffatom enthält, mit 4 bis 20 C-Atomen bedeuten.

16. Verfahren nach Anspruch 15,
wobei die Rest Q³ und Q^{3a} jeweils unabhängig voneinander ein aromatischer Rest der allgemeinen Formel (11a), (12a) oder (13a) und wobei die Reste Q⁴ und Q^{4a} jeweils unabhängig von einander ein aromatischer Rest der allgemeinen Formel (11b), (12b) oder (13b) ist, und
wobei jeweils mindestens 1 Rest R^{6a} bis R^{10a}, R^{11a} bis R^{17a}, R^{18a} bis R^{24a}, R^{6b} bis R^{10b}, R^{11b} bis R^{17b} oder R^{18b} bis R^{24b} jeweils unabhängig voneinander ein organischer Rest W1a oder ein organischer Rest W1b oder ein organischer Rest W1c ist, wobei
wobei die Reste R^{10a}, R^{11a} bis R^{17a}, R^{18a} bis R^{24a}, R^{6b} bis R^{10b}, R^{11b} bis R^{17b} und R^{18b} bis R^{24b}, die nicht ein organischer Rest W1a oder ein organischer Rest W1b oder ein organischer Rest W1c sind, jeweils unabhängig voneinander gleich oder verschieden sind und Wasserstoff, Halogen, Hydroxyl, Thiol, Nitro, Carboxylat, Aldehyd mit 1 bis 8 C-Atomen, Keton mit 2 bis 8 C-Atomen, O-Alkyl mit 1 bis 12 C-Atomen, S-Alkyl mit 1 bis 12 C-Atomen, O-Alkenyl mit 2 bis 12 C-Atomen, S-Alkenyl mit 2 bis 12 C-Atomen, O-Aryl mit 5 bis 20 C-Atomen, S-Aryl mit 5 bis 20 C-Atomen, Ether mit 2 bis 12 C-Atomen, Thioether mit 2 bis 12 C-Atomen, Carbonsäureester mit 1 bis 12 C-Atomen, Carbonsäureamid mit 1 bis 12 C-Atomen, Thioester mit 1 bis 12 C-Atomen, Alkyl mit 1 bis 12 C-Atomen, Alkenyl mit 2 bis 12 C-Atomen, Cycloalkyl mit 3 bis 12 C-Atomen, Cycloalkenyl mit 3 bis 12 C-Atomen, Alkylaryl mit 1 bis 12 C-Atomen, Aryl mit 5 bis 20 C-Atomen oder Heteroaryl, das kein Stickstoffatom enthält, mit 4 bis 20 C-Atomen bedeuten.

17. Verfahren nach einem der Ansprüche 1 bis 3, wobei der wenigstens eine Photosensibilisator, wenigstens eine Verbindung nach einem der Ansprüche 4 bis 14 und/oder einem pharmakologisch verträglichen Salz und/oder Ester und/oder Komplex davon ist.

18. Nichtmedizinische Verwendung einer Verbindung nach einem der Ansprüche 4 bis 14 und/oder einem pharmakologisch verträglichen Salz und/oder Ester und/oder Komplex davon als Photosensibilisator zur Inaktivierung von Mikroorganismen, die vorzugsweise aus der Gruppe, die aus Viren, Archäeen, Bakterien, Bakteriensporen, Pilzen, Pilzsporen, Protozoen, Algen und blutübertragbaren Parasiten besteht, ausgewählt werden.

19. Verwendung nach Anspruch 18 zur Oberflächenreinigung und/oder - beschichtung eines Gegenstandes.

20. Verwendung nach einem der Ansprüche 18 oder 19 zur Oberflächenreinigung und/oder -beschichtung von Medizinprodukten, Lebensmittelverpackungen, Textilien; Baustoffen, elektronische Geräten, Möbeln oder Hygieneartikeln.

21. Verwendung nach einem der Ansprüche 18 oder 19 zur Entkeimung von Flüssigkeiten.

22. Verwendung nach einem der Ansprüche 18 oder 19 zur Entkeimung von Lebensmitteln.

23. Beschichteter Gegenstand, wobei die Oberfläche des Gegenstandes wenigstens eine Verbindung nach einem der Ansprüche 4 bis 14 aufweist.

24. Verbindung mit der Formel (100): 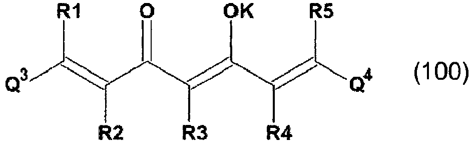 und/oder der Formel (101): 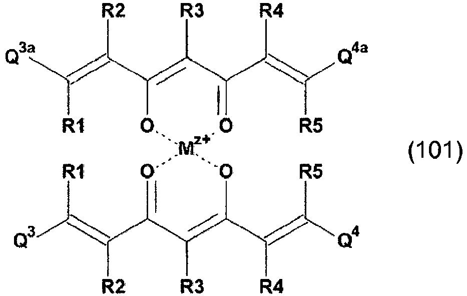 oder jeweils ein pharmakologisch verträgliches Salz und/oder Ester und/oder Komplex davon zur Verwendung als Photosensibilisator bei der medizinischen Behandlung zur Inaktivierung von Mikroorganismen,
wobei Q³, Q^{3a}, Q⁴ und Q^{4a} jeweils unabhängig voneinander 1 substituierter oder unsubstituierter monozyklischer oder polyzyklischer, aromatischer Rest oder 1 substituierter oder unsubstituierter monozyklischer oder polyzyklischer, heteroaromatischer Rest bedeuten,
wobei die Verbindung mit der Formel (100) keine OH-Gruppe, die direkt an den organischen Rest Q³ oder Q⁴ gebunden ist, enthält, und
wobei die Verbindung mit der Formel (101) keine OH-Gruppe, die direkt an den organischen Rest Q³, Q^{3a}, Q⁴, oder Q^{4a} gebunden ist, enthält, und
wobei K Wasserstoff oder ein Kation bedeutet, und
wobei M^{z+} ein Kation eines Metalls bedeutet, wobei z die formale Oxidationszahl des Metalls M ist und z eine ganze Zahl von 1 bis 7, vorzugsweise von 2 bis 5, bedeutet, und wobei
(a1) wenigstens einer der Reste Q³, Q^{3a}, Q⁴ und Q^{4a} jeweils unabhängig voneinander ein unsubstituierter monozyklischer oder polyzyklischer, heteroaromatischer Rest ist, der wenigstens 5 Ringatome aufweist, wobei die Ringatome mindestens 1 Kohlenstoffatom und wenigstens 1, vorzugsweise protonierbares, Stickstoffatom enthalten, oder
(a2) wenigstens einer der Reste Q³, Q^{3a}, Q⁴ und Q^{4a} jeweils unabhängig voneinander mit mindestens einem organischen Rest W1 substituiert ist, wobei der mindestens eine organische Rest W1 die allgemeine Formel (4), (5), (6), (7), (8) aufweist:
-(C(D)(E))ₕ-X, (4)
-A-(C(D)(E))ₕ-X, (5)
-(C(D)(E))ₖ-Aryl-(C(D)(E))ₗ-X, (6)
-A-(C(D)(E))ₖ-Aryl-(C(D)(E))ₗ-X, (7)
-((C(D)(E))ₘ-A)ₚ-(C(D)(E))ₙ-X, (8)
-A-((C(D)(E))ₘ-A)ₚ-(C(D)(E))ₙ-X, (9)
wobei h eine ganze Zahl von 1 bis 20 bedeutet, wobei k und I jeweils unabhängig voneinander eine ganze Zahl eine ganze Zahl von 0 bis 10 bedeuten, und wobei m, n und p jeweils unabhängig voneinander eine ganze Zahl von 1 bis 6 und
wobei A jeweils unabhängig voneinander Sauerstoff oder Schwefel bedeutet,
wobei D und E jeweils unabhängig voneinander Wasserstoff, Halogen, G-R^{(I)}, oder G-C(=G)-R^{(II)}, bedeuten, wobei G jeweils unabhängig voneinander Sauerstoff oder Schwefel bedeutet, und wobei die Reste R^{(I)} und R^{(II)} jeweils unabhängig voneinander Wasserstoff, Methyl, Ethyl, n-Propyl, n-Butyl, n-Pentyl, Phenyl oder Benzyl bedeutet, wobei Phenyl und Benzyl unsubstituiert oder substituiert sein können,
wobei Aryl einen substituierten oder unsubstituierten Aromaten oder einen substituierten oder unsubstituierten Heteroaromaten, der kein Stickstoffatom enthält, bedeutet,
wobei X jeweils unabhängig voneinander ein organischer Rest ist, der (i) wenigstens ein neutrales, protonierbares Stickstoffatom, oder (ii) wenigstens ein positiv geladenes Stickstoffatom oder (iii) wenigstens ein positiv geladenes Phosphoratom enthält, und
wobei die Reste R1, R2, R3, R4 und R5 jeweils unabhängig voneinander Wasserstoff, Halogen, Alkyl mit 1 bis 12 C-Atomen, Cycloalkyl mit 1 bis 12 C-Atomen, Alkylaryl mit 1 bis 12 C-Atomen, Aryl mit 5 bis 20 C-Atomen, Ether mit 2 bis 12 C-Atomen oder Glykol mit 2 bis 12 C-Atomen bedeuten,
oder wobei
(b) der Rest R3 ein organischer Rest W2 ist, wobei der eine organische Rest W2 die allgemeine Formel (4), (5), (6), (7), (8), (9), oder (10):
-(C(D)(E))ₕ-X, (4)
-A-(C(D)(E))ₕ-X, (5)
-(C(D)(E))ₖ-Aryl-(C(D)(E))ₗ-X, (6)
-A-(C(D)(E))ₖ-Aryl-(C(D)(E))ₗ-X, (7)
-[(C(D)(E))ₘ-A]ₚ-(C(D)(E))ₙ-X, (8)
-A-[(C(D)(E))ₘ-A]ₚ-(C(D)(E))ₙ-X, (9)
-(-C(D)=C(E)-)ᵣ-X, (10)
aufweist, und
wobei, optional, wenigstens einer der Reste Q³, Q^{3a}, Q⁴ und Q^{4a} jeweils unabhängig voneinander mit mindestens einem organischen Rest W1, der die allgemeine Formel (4), (5), (6), (7), (8), oder (9) aufweist, substituiert sind,
wobei h eine ganze Zahl von 1 bis 20 bedeutet, wobei k und I jeweils unabhängig voneinander eine ganze Zahl eine ganze Zahl von 0 bis 10 bedeuten und wobei m, n, p, und r jeweils unabhängig voneinander eine ganze Zahl von 1 bis 6, vorzugsweise von 2 bis 4, bedeuten, und
wobei A jeweils unabhängig voneinander Sauerstoff oder Schwefel bedeutet, wobei D und E jeweils unabhängig voneinander Wasserstoff, Halogen, G-R^{(I)}, oder G-C(=G)-R^{(II)}, bedeuten, wobei G jeweils unabhängig voneinander Sauerstoff oder Schwefel bedeutet, und wobei die Reste R^{(I)} und R^{(II)} jeweils unabhängig voneinander Wasserstoff, Methyl, Ethyl, n-Propyl, n-Butyl, n-Pentyl, Phenyl oder Benzyl bedeutet, wobei Phenyl und Benzyl unsubstituiert oder substituiert sein können,
wobei Aryl einen substituierten oder unsubstituierten Aromaten oder einen substituierten oder unsubstituierten Heteroaromaten, der kein Stickstoffatom enthält, bedeutet,
wobei X jeweils unabhängig voneinander ein organischer Rest ist, der (i) wenigstens ein neutrales, protonierbares Stickstoffatom, (ii) wenigstens ein positiv geladenes Stickstoffatom oder (iii) wenigstens ein positiv geladenes Phosphoratom enthält, und
wobei die Reste R1, R2, R4 und R5 jeweils unabhängig voneinander Wasserstoff, Halogen, Alkyl mit 1 bis 12 C-Atomen, Alkylaryl mit 1 bis 12 C-Atomen, Aryl mit 5 bis 20 C-Atomen, Ether mit 2 bis 12 C-Atomen oder Glykol mit 2 bis 12 C-Atomen bedeuten.

## Claims

1. Non-therapeutic method for the inactivation of microorganisms, wherein the method comprises the following steps:
(A) bringing the microorganisms into contact with at least one photosensitizer, wherein the photosensitizer is at least one 1,7-diaryl-1,6-heptadiene-3,5-dione derivative with formula (100): 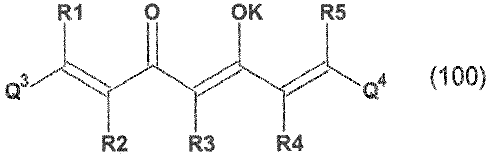 and/or at least one 1,7-diaryl-1,6-heptadiene-3,5-dione derivative with formula (101): 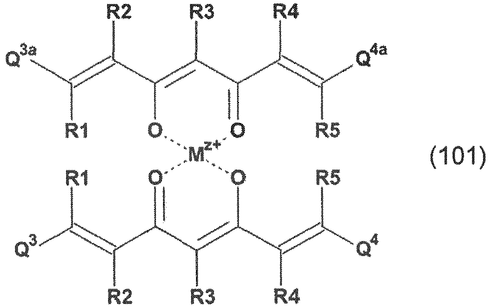 or respectively a pharmacologically acceptable salt and/or ester and/or complex thereof,
wherein the residues Q³, Q^{3a}, Q⁴ and Q^{4a}, respectively independently of each other, represent one substituted or unsubstituted, monocyclic or polycyclic aromatic residue or one substituted or unsubstituted, monocyclic or polycyclic heteroaromatic residue,
wherein the compound with formula (100) does not contain an OH group directly bonded to the organic residue Q³ or Q⁴, and
wherein the compound with formula (101) does not contain an OH group directly bonded to the organic residue Q³, Q³⁸, Q⁴ or Q^{4a}, and
wherein K represents hydrogen or a cation, and
wherein M^{z+} represents a cation of a metal, wherein z is the formal oxidation number of the metal M and z represents a whole number from 1 to 7, preferably from 2 to 5, and wherein
(a1) at least one of the residues Q³, Q^{3a}, Q⁴ and Q^{4a}, respectively independently of each other, is an unsubstituted, monocyclic or polycyclic heteroaromatic residue, which has at least 5 ring atoms, wherein the ring atoms contain at least one carbon atom and at least one nitrogen atom which preferably can be protonated, or
(a2) at least one of the residues Q³, Q^{3a}, Q⁴ and Q^{4a}, respectively independently of each other, is substituted with at least one organic residue W1, wherein the at least one organic residue W1 has the general formula (4), (5), (6), (7), (8), or (9):
-(C(D)(E))ₕ-X, (4)
-A-(C(D)(E))ₕ-X, (5)
-(C(D)(E))ₖ-aryl-(C(D)(E))ₗ-X, (6)
-A-(C(D)(E))ₖ-aryl-(C(D)(E))ₗ-X, (7)
-((C(D)(E))ₘ-A)ₚ-(C(D)(E))ₙ-X, (8)
-A-((C(D)(E))ₘ-A)ₚ-(C(D)(E))ₙ-X, (9)
wherein h represents a whole number from 1 to 20, wherein k represents a whole number from 0 to 10, wherein I represents a whole number from 0 to 10, and wherein m, n and p, respectively independently of each other, represent a whole number from 1 to 6, and
wherein A, respectively independently of each other, represents oxygen or sulphur,
wherein D and E, respectively independently of each other, represent hydrogen, halogen, G-R^{(I)}, or G-C(=G)-R^{(II)}, wherein G, respectively independently of each other, represents oxygen or sulphur, and wherein the residues R^{(I)} and R^{(II)}, respectively independently of each other, represent hydrogen, methyl, ethyl, n-propyl, n-butyl, n-pentyl, phenyl or benzyl, wherein phenyl and benzyl may be unsubstituted or substituted,
wherein aryl represents a substituted or unsubstituted aromatic group or a substituted or unsubstituted heteroaromatic group which does not contain a nitrogen atom,
wherein X, respectively independently of each other, is an organic residue which (i) contains at least one neutral nitrogen atom which can be protonated, or (ii) contains at least one positively charged, preferably quaternary, nitrogen atom, or (iii) contains at least one positively charged, preferably quaternary, phosphorus atom, and
wherein the residues R1, R2, R3, R4 and R5, respectively independently of each other, represent hydrogen, halogen, alkyl containing 1 to 12 C atoms, cycloalkyl containing 1 to 12 C atoms, alkylaryl containing 1 to 12 C atoms, aryl containing 5 to 20 C atoms, ether containing 2 to 12 C atoms or glycol containing 2 to 12 C atoms,
or wherein
(b) the residue R3 is an organic residue W2, wherein the one organic residue W2 has the general formula (4), (5), (6), (7), (8), (9), or (10):
-(C(D)(E))ₕ-X, (4)
-A-(C(D)(E))ₕ-X, (5)
-(C(D)(E))ₖ-aryl-(C(D)(E))ₗ-X, (6)
-A-(C(D)(E))ₖ-aryl-(C(D)(E))ₗ-X, (7)
-[(C(D)(E))ₘ-A]ₚ-(C(D)(E))ₙ-X, (8)
-A-[(C(D)(E))ₘ-A]ₚ-(C(D)(E))ₙ-X, (9)
-(-C(D)=C(E)-)ᵣ-X, (10)
and
wherein, optionally, at least one of the residues Q³, Q^{3a}, Q⁴ and Q^{4a}, respectively independently of each other, is substituted with at least one organic residue W1 which has the general formula (4), (5), (6), (7), (8), or (9),
wherein h represents a whole number from 1 to 20, wherein k represents a whole number from 0 to 10, wherein I represents a whole number from 0 to 10, and wherein m, n, p, and r, respectively independently of each other, represent a whole number from 1 to 6, and
wherein A, respectively independently of each other, represents oxygen or sulphur,
wherein D and E, respectively independently of each other, represent hydrogen, halogen, G-R^{(I)}, or G-C(=G)-R^{(II)}, wherein G, respectively independently of each other, represents oxygen or sulphur, and wherein the residues R^{(I)} and R^{(II)}, respectively independently of each other, represent hydrogen, methyl, ethyl, n-propyl, n-butyl, n-pentyl, phenyl or benzyl, wherein phenyl and benzyl may be unsubstituted or substituted,
wherein aryl represents a substituted or unsubstituted aromatic group or a substituted or unsubstituted heteroaromatic group which does not contain a nitrogen atom,
wherein X, respectively independently of each other, is an organic residue which (i) contains at least one neutral nitrogen atom which can be protonated, (ii) contains at least one positively charged, preferably quaternary, nitrogen atom, or (iii) contains at least one positively charged, preferably quaternary, phosphorus atom, and
wherein the residues R1, R2, R4 and R5, respectively independently of each other, represent hydrogen, halogen, alkyl containing 1 to 12 C atoms, alkylaryl containing 1 to 12 C atoms, aryl containing 5 to 20 C atoms, ether containing 2 to 12 C atoms or glycol containing 2 to 12 C atoms,
and
(B) irradiating the microorganisms and the at least one photosensitizer with electromagnetic radiation of a suitable wavelength and energy density.

2. Method according to claim 1 wherein, in the compound with formula (100), K is a cation M^{z+} of a metal M, wherein z is the formal oxidation number of the metal M and wherein z represents a whole number from 1 to 7, preferably from 2 to 3, and wherein the compound has the formula (102): 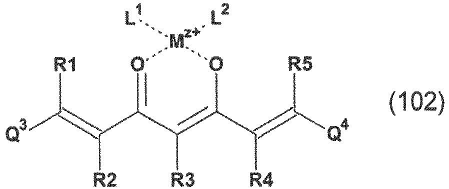 wherein L¹ and L², respectively independently of each other, represent water, fluoride, chloride, bromide, iodide, phosphate, hydrogen phosphate, dihydrogen phosphate, sulphate, hydrogen sulphate, tosylate, mesylate or at least one carboxylation of a carboxylic acid containing 1 to 15 carbon atoms and/or mixtures thereof.

3. Method according to claim 1 or claim 2, wherein the irradiation of the microorganisms and of the at least one photosensitizer with electromagnetic radiation of a suitable wavelength and energy density is carried out in the presence of at least one oxygen-donating compound, preferably peroxide, and/or at least one oxygen-containing gas, preferably oxygen.

4. Compound with formula (1): 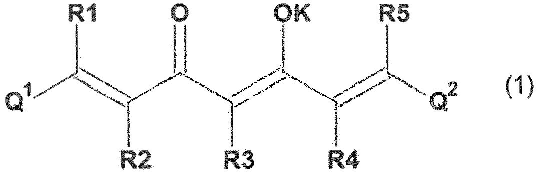 wherein the residues Q¹ and Q², respectively independently of each other, represent one substituted or unsubstituted, monocyclic or polycyclic aromatic residue,
wherein the compound with formula (1) does not contain an OH group directly bonded to the organic residue Q¹ or Q², and
wherein K represents hydrogen or a cation, and wherein
(a) at least one of the residues Q¹ and Q², respectively independently of each other, is substituted with at least one organic residue W1a, wherein
the at least one organic residue W1a has the general formula (5a), (6a), (7a), (8a), or (9a):
-A-(C(D)(E))ₕ-X^{a}, (5a)
-(C(D)(E))ₖ-aryl-(C(D)(E))ₗ-X^{a}, (6a)
-A-(C(D)(E))ₖ-aryl-(C(D)(E))ₗ-X^{a}, (7a)
-((C(D)(E))ₘ-A)ₚ-(C(D)(E))ₙ-X^{a}, (8a)
-A-((C(D)(E))ₘ-A)ₚ-(C(D)(E))ₙ-X^{a}, (9a)
wherein h represents a whole number from 1 to 20, wherein k represents a whole number from 0 to 10, wherein I represents a whole number from 0 to 10, and wherein m, n and p, respectively independently of each other, represent a whole number from 1 to 6, and
wherein A, respectively independently of each other, represents oxygen or sulphur,
wherein D and E, respectively independently of each other, represent hydrogen, halogen, G-R^{(I)}, or G-C(=G)-R^{(II)}, wherein G, respectively independently of each other, represents oxygen or sulphur, and wherein the residues R^{(I)} and R^{(II)}, respectively independently of each other, represent hydrogen, methyl, ethyl, n-propyl, n-butyl, n-pentyl, phenyl or benzyl, wherein phenyl and benzyl may be unsubstituted or substituted,
wherein aryl represents a substituted or unsubstituted aromatic group or a substituted or unsubstituted heteroaromatic group which does not contain a nitrogen atom,
wherein X^{a}, respectively independently of each other, represents a residue with formula (20c), (20d) or (21): 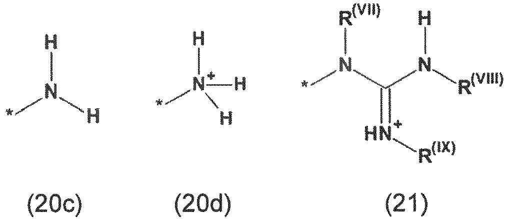 wherein each of the residues R^{(VII)}, R^{(VIII)}, and R^{(IX)}, respectively independently of each other, represents hydrogen, an aryl residue containing 5 to 12 C atoms, an alkylaryl residue containing 5 to 12 C atoms, an alkyl residue, which may be linear or branched, containing 1 to 8 C atoms, or an ether residue, which may be linear or branched, containing 1 to 8 C atoms, and
wherein the residues R1, R2, R3, R4 and R5, respectively independently of each other, represent hydrogen, halogen, alkyl containing 1 to 12 C atoms, cycloalkyl containing 1 to 12 C atoms, alkylaryl containing 1 to 12 C atoms, aryl containing 5 to 20 C atoms, ether containing 2 to 12 C atoms or glycol containing 2 to 12 C atoms.

5. Compound according to claim 4,
wherein K represents a cation M^{z+} of a metal M, wherein z is the formal oxidation number of the metal M and is a whole number from 1 to 7, preferably from 2 to 5, and
wherein the compound has the formula (2): 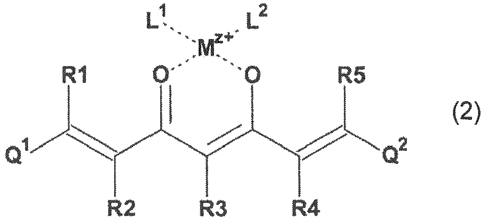 wherein L¹ and L², respectively independently of each other, represent water, halide, cyanide, thiocyanate, phosphate, hydrogen phosphate, or a carboxylation of a carboxylic acid containing 1 to 10 carbon atoms, preferably formate, acetate, n-propionate, lactate, oxalate, fumarate, maleinate, tartrate, succinylate, benzoate, salicylate, or citrate.

6. Compound with formula (3): 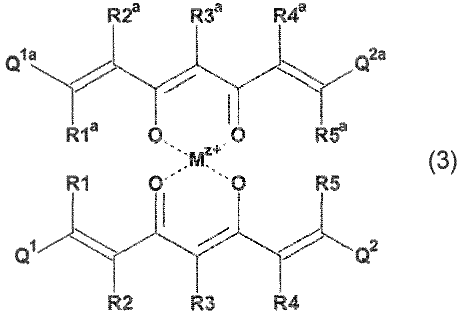 wherein M^{z+} represents a cation of a metal, wherein z is the formal oxidation number of the metal M and is a whole number from 1 to 7, preferably from 2 to 5, and
wherein Q¹ and Q², respectively independently of each other, represent one substituted or unsubstituted, monocyclic or polycyclic aromatic residue, and
wherein the residues Q^{1a} and Q^{2a}, respectively independently of each other, represent one substituted or unsubstituted, monocyclic or polycyclic aromatic residue or one substituted or unsubstituted, monocyclic or polycyclic heteroaromatic residue,
wherein the compound with formula (3) does not contain an OH group directly bonded to the organic residue Q¹, Q^{1a}, Q² or Q^{2a},
and wherein
(a) at least one of the residues Q¹ and Q², respectively independently of each other, is substituted with at least one organic residue W1a which has the general formula (5a), (6a), (7a), (8a), or (9a):
-A-(C(D)(E))ₕ-X^{a}, (5a)
-(C(D)(E))ₖ-aryl-(C(D)(E))ₗ-X^{a}, (6a)
-A-(C(D)(E))ₖ-aryl-(C(D)(E))ₗ-X^{a}, (7a)
-((C(D)(E))ₘ-A)ₚ-(C(D)(E))ₙ-X^{a}, (8a)
-A-((C(D)(E))ₘ-A)ₚ-(C(D)(E))ₙ-X^{a}, (9a)
wherein at least one of the residues Q^{1a} and Q^{2a}, respectively independently of each other, is substituted with at least one organic residue W1c which has the general formula (5c), (6c), (7c), (8c), or (9c):
-A-(C(D)(E))ₕ-X^{c}, (5c)
-(C(D)(E))ₖ-aryl-(C(D)(E))ₗ-X^{c}, (6c)
-A-(C(D)(E))ₖ-aryl-(C(D)(E))ₗ-X^{c}, (7c)
-((C(D)(E))ₘ-A)ₚ-(C(D)(E))ₙ-X^{c}, (8c)
-A-((C(D)(E))ₘ-A)ₚ-(C(D)(E))ₙ-X^{c}, (9c)
wherein h represents a whole number from 1 to 20, wherein k represents a whole number from 0 to 10, wherein I represents a whole number from 0 to 10, and wherein m, n and p, respectively independently of each other, represent a whole number from 1 to 6, and
wherein A, respectively independently of each other, represents oxygen or sulphur,
wherein D and E, respectively independently of each other, represent hydrogen, halogen, G-R^{(I)}, or G-C(=G)-R^{(II)}, wherein G, respectively independently of each other, represents oxygen or sulphur, and wherein the residues R^{(I)} and R^{(II)}, respectively independently of each other, represent hydrogen, methyl, ethyl, n-propyl, n-butyl, n-pentyl, phenyl or benzyl, wherein phenyl and benzyl may be unsubstituted or substituted,
wherein aryl represents a substituted or unsubstituted aromatic group or a substituted or unsubstituted heteroaromatic group which does not contain a nitrogen atom, and
wherein X^{a}, respectively independently of each other, represents a residue with formula (20c), (20d) or (21): 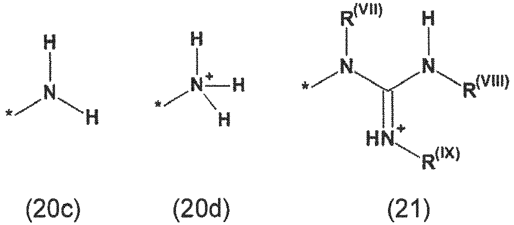 wherein each of the residues R^{(VII)}, R^{(VIII)}, and R^{(IX)}, respectively independently of each other, represents hydrogen, an aryl residue containing 5 to 12 C atoms, an alkylaryl residue containing 5 to 12 C atoms, an alkyl residue, which may be linear or branched, containing 1 to 8 C atoms, or an ether residue, which may be linear or branched, containing 1 to 8 C atoms, and
wherein X^{c}, respectively independently of each other, is an organic residue which (i) contains at least one neutral nitrogen atom which can be protonated, or (ii) contains at least one positively charged, preferably quaternary, nitrogen atom, or (iii) contains at least one positively charged, preferably quaternary, phosphorus atom, and
wherein the residues R1, R1^{a}, R2, R2^{a}, R3, R3^{a}, R4, R4^{a}, R5 and R5^{a}, respectively independently of each other, represent hydrogen, halogen, alkyl containing 1 to 12 C atoms, cycloalkyl containing 1 to 12 C atoms, alkylaryl containing 1 to 12 C atoms, aryl containing 5 to 20 C atoms, ether containing 2 to 12 C atoms or glycol containing 2 to 12 C atoms.

7. Compound according to claim 6, wherein the compound with formula (3) has the formula (3a): 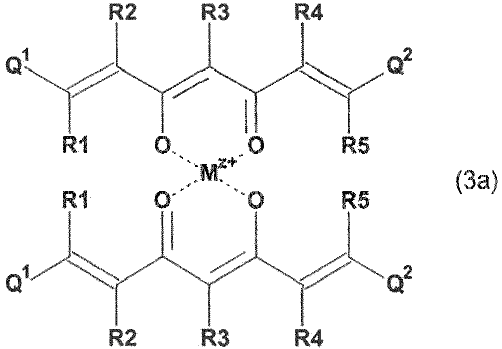 wherein M^{z+} represents a cation of a metal, wherein z is the formal oxidation number of the metal M and represents a whole number from 1 to 7, preferably from 2 to 5, and
wherein Q¹ and Q², respectively independently of each other, represent one substituted or unsubstituted, monocyclic or polycyclic aromatic residue, and
wherein the compound with formula (3a) does not contain an OH group directly bonded to the organic residue Q¹ or Q²,
and wherein
(a) at least one of the residues Q¹ and Q², respectively independently of each other, is substituted with at least one organic residue W1a which has the general formula (5a), (6a), (7a), (8a), or (9a):
-A-(C(D)(E))ₕ-X^{a}, (5a)
-(C(D)(E))ₖ-aryl-(C(D)(E))ₗ-X^{a}, (6a)
-A-(C(D)(E))ₖ-aryl-(C(D)(E))ₗ-X^{a}, (7a)
-((C(D)(E))ₘ-A)ₚ-(C(D)(E))ₙ-X^{a}, (8a)
-A-((C(D)(E))ₘ-A)ₚ-(C(D)(E))ₙ-X^{a}, (9a)
wherein h represents a whole number from 1 to 20, wherein k represents a whole number from 0 to 10, wherein I represents a whole number from 0 to 10, and wherein m, n and p, respectively independently of each other, represent a whole number from 1 to 6, and
wherein A, respectively independently of each other, represents oxygen or sulphur,
wherein D and E, respectively independently of each other, represent hydrogen, halogen, G-R^{(I)}, or G-C(=G)-R^{(II)}, wherein G, respectively independently of each other, represents oxygen or sulphur, and wherein the residues R^{(I)} and R^{(II)}, respectively independently of each other, represent hydrogen, methyl, ethyl, n-propyl, n-butyl, n-pentyl, phenyl or benzyl, wherein phenyl and benzyl may be unsubstituted or substituted,
wherein aryl represents a substituted or unsubstituted aromatic group or a substituted or unsubstituted heteroaromatic group which does not contain a nitrogen atom, and
wherein X^{a}, respectively independently of each other, represents a residue with formula (20c), (20d) or (21): 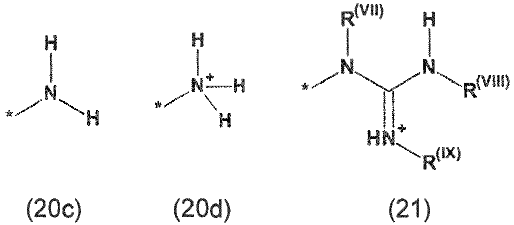 wherein each of the residues R^{(VII)}, R^{(VIII)}, and R^{(IX)}, respectively independently of each other, represents hydrogen, an aryl residue containing 5 to 12 C atoms, an alkylaryl residue containing 5 to 12 C atoms, an alkyl residue, which may be linear or branched, containing 1 to 8 C atoms, or an ether residue, which may be linear or branched, containing 1 to 8 C atoms, and
wherein the residues R1, R2, R3, R4, and R5, respectively independently of each other, represent hydrogen, halogen, alkyl containing 1 to 12 C atoms, cycloalkyl containing 1 to 12 C atoms, alkylaryl containing 1 to 12 C atoms, aryl containing 5 to 20 C atoms, ether containing 2 to 12 C atoms or glycol containing 2 to 12 C atoms.

8. Compound according to one of claims 5, 6 or 7,
wherein M is selected from the group which consists of B, Al, Zn, Cu, Mg, Ca, Fe, Si, Ga, Sn, Rh, Co, Ti, Zr, V, Cr, Mo, Mn, Ru, Pd, Ir, Ni, and combinations thereof.

9. Compound according to one of claims 4 to 8,
wherein the residues Q¹ and Q^{1a}, respectively independently of each other, are an aromatic residue with general formula (11a), (12a), (13a), (14a), (15a), (16a), (17a), (18a), or (19a): 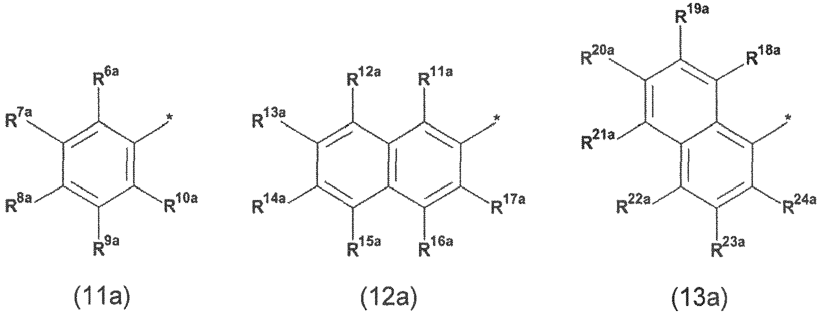 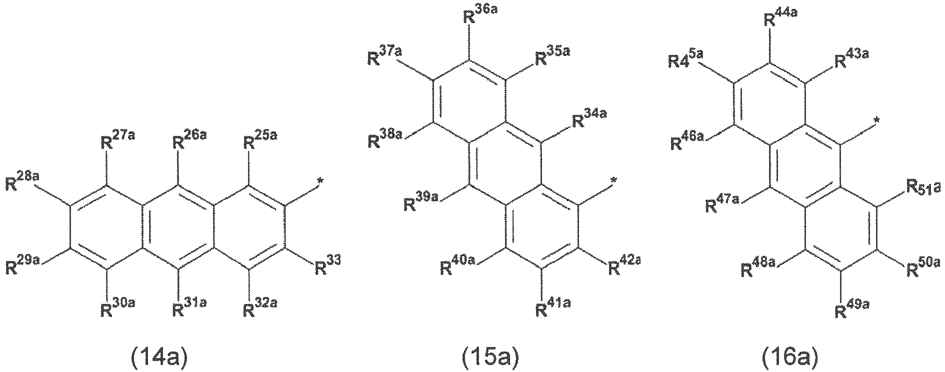 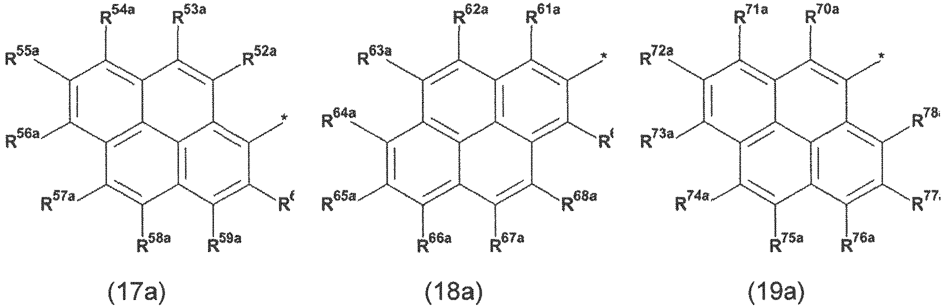 and
wherein the residues Q² and Q^{2a}, respectively independently of each other, represent an aromatic residue with general formula (11b), (12b), (13b), (14b), (15b), (16b), (17b), (18b), or (19b): 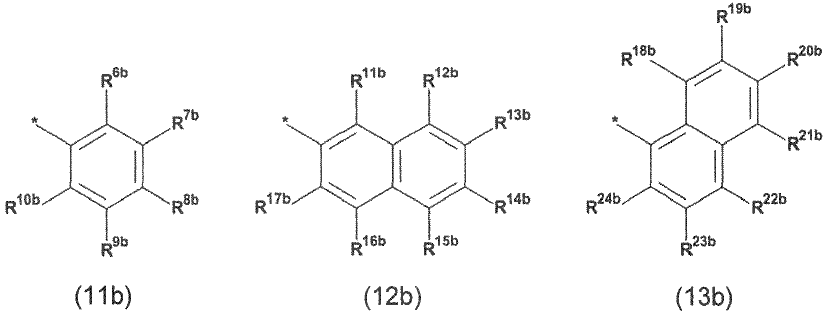 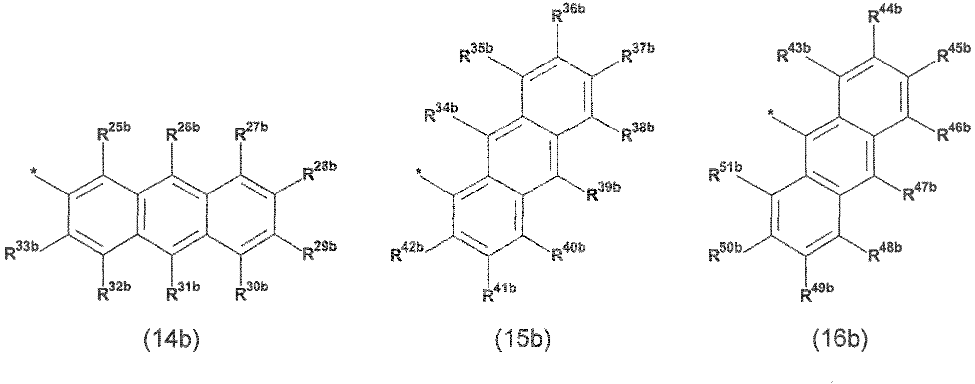 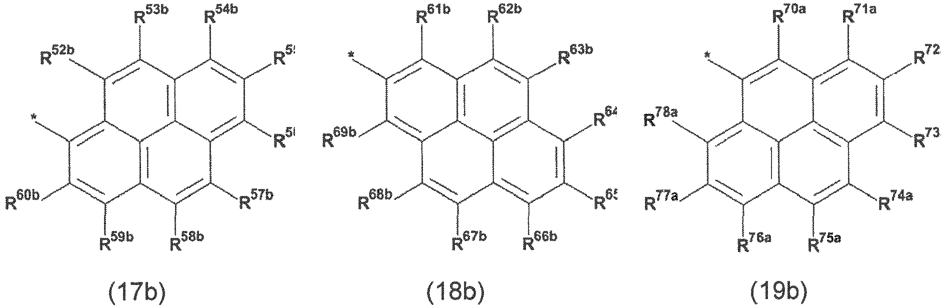 and
wherein respectively, at least one residue R^{6a} to R^{10a}, R^{11a} to R^{17a}, R^{18a} to R^{24a}, R^{25a} to R^{33a}, R^{34a} to R^{42a}, R^{43a} to R^{51a}, R^{52a} to R^{60a}, R^{61a} to R^{69a}, R^{70a} to R^{78a}, R^{6b} to R^{10b}, R^{11b} to R^{17b}, R^{18b} to R^{24b}, R^{25b} to R^{33b}, R^{34b} to R^{42b}, R^{43b} to R^{51b}, R^{52b} to R^{60b}, R^{61b} to R^{69b}, or R^{70b} to R^{78b}, respectively independently of each other, is an organic residue W1a or an organic residue W1b or an organic residue W1c, and
wherein the residues R^{6a} to R^{10a}, R^{11a} to R^{17a}, R^{18a} to R^{24a}, R^{25a} to R^{33a}, R^{34a} to R^{42a}, R^{43a} to R^{51a}, R^{52a} to R^{60a}, R^{61a} to R^{69a}, R^{70a} to R^{78a}, R^{6b} to R^{10b}, R^{11b} to R^{17b} R^{18b} to R^{24b}, R^{25b} to R^{33b}, R^{34b} to R^{42b}, R^{43b} to R^{51b}, R^{52b} to R^{60b}, R^{61b} to R^{69b}, or R^{70b} to R^{78b}, which are not an organic residue W1a or an organic residue W1b or an organic residue W1c, respectively independently of each other, are identical or different and represent hydrogen, halogen, hydroxyl, thiol, nitro, carboxylate, aldehyde containing 1 to 8 C atoms, ketone containing 2 to 8 C atoms, O-alkyl containing 1 to 12 C atoms, S-alkyl containing 1 to 12 C atoms, O-alkenyl containing 2 to 12 C atoms, S-alkenyl containing 2 to 12 C atoms, O-aryl containing 5 to 20 C atoms, S-aryl containing 5 to 20 C atoms, ether containing 2 to 12 C atoms, thioether containing 2 to 12 C atoms, carboxylic acid ester containing 1 to 12 C atoms, carboxylic acid amide containing 1 to 12 C atoms, thioester containing 1 to 12 C atoms, alkyl containing 1 to 12 C atoms, alkenyl containing 2 to 12 C atoms, cycloalkyl containing 3 to 12 C atoms, cycloalkenyl containing 3 to 12 C atoms, alkylaryl containing 1 to 12 C atoms, aryl containing 5 to 20 C atoms or heteroaryl, which does not contain a nitrogen atom, containing 4 to 20 C atoms.

10. Compound according to claim 9,
wherein the residues Q¹ and Q^{1a}, respectively independently of each other, are an aromatic residue with general formula (11a), (12a) or (13a) and wherein the residues Q² and Q^{2a}, respectively independently of each other, are an aromatic residue with general formula (11b), (12b) or (13b), and
wherein respectively at least one residue R^{6a} to R^{10a}, R^{11a} to R^{17a}, R^{18a} to R^{24a}, R^{6b} to R^{10b}, R^{11b} to R^{17b} or R^{18b} to R^{24b}, respectively independently of each other, is an organic residue W1a or an organic residue W1b or an organic residue W1c,
wherein the residues R^{10a}, R^{11a} to R^{17a}, R^{18a} to R^{24a}, R^{6b} to R^{10b}, R^{11b} to R^{17b} and R^{18b} to R^{24b}, which are not an organic residue W1a or an organic residue W1b or an organic residue W1c, respectively independently of each other, are identical or different and represent hydrogen, halogen, hydroxyl, thiol, nitro, carboxylate, aldehyde containing 1 to 8 C atoms, ketone containing 2 to 8 C atoms, O-alkyl containing 1 to 12 C atoms, S-alkyl containing 1 to 12 C atoms, O-alkenyl containing 2 to 12 C atoms, S-alkenyl containing 2 to 12 C atoms, O-aryl containing 5 to 20 C atoms, S-aryl containing 5 to 20 C atoms, ether containing 2 to 12 C atoms, thioether containing 2 to 12 C atoms, carboxylic acid ester containing 1 to 12 C atoms, carboxylic acid amide containing 1 to 12 C atoms, thioester containing 1 to 12 C atoms, alkyl containing 1 to 12 C atoms, alkenyl containing 2 to 12 C atoms, cycloalkyl containing 3 to 12 C atoms, cycloalkenyl containing 3 to 12 C atoms, alkylaryl containing 1 to 12 C atoms, aryl containing 5 to 20 C atoms or heteroaryl, which does not contain a nitrogen atom, containing 4 to 20 C atoms.

11. Compound according to claim 6,
wherein the organic residue X^{c}, respectively independently of each other, represents a residue with formula (20a), (20b), (21), (22a), (22b), (23a), (24b), or (24): 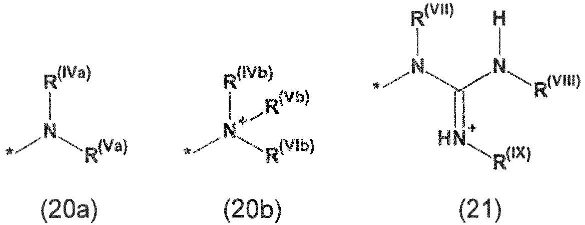 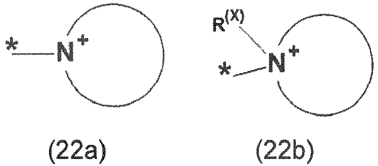 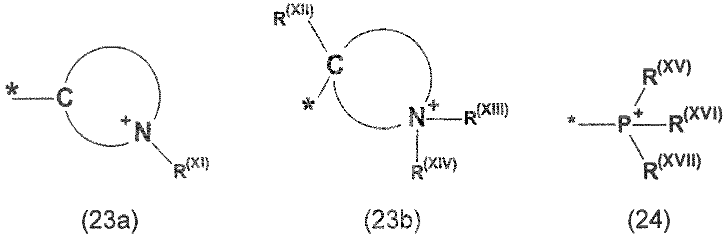 wherein each of the residues R^{(IVa)}, R^{(Va)}, R^{(IVb)}, R^{(Vb)}, R^{(VIb)}, R^{(VII)}, R^{(VIII)}, R^{(X)}, R^{(XI)}, R^{(XII)}, R^{(XIII)}, R^{(XIV)}, R^{(XV)}, R^{(XVI)}, and R^{(XVII)}, respectively independently of each other, represents hydrogen, an aryl residue containing 5 to 12 C atoms, an alkyl residue, which may be linear or branched, containing 1 to 8 C atoms, or an ether residue, which may be linear or branched, containing 1 to 8 C atoms, and
wherein the residue with formula (22a) and the residue with formula (23a): 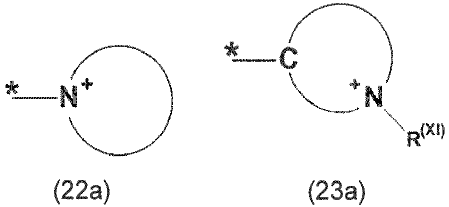 represent a substituted or unsubstituted heterocyclic residue with 5 to 7 ring atoms, which comprise at least one carbon atom and at least one nitrogen atom as well as, optionally, 1 or 2 oxygen atoms, wherein 1 nitrogen atom forms a double bond, and
wherein the residue with formula (22b) and the residue with formula (23b): 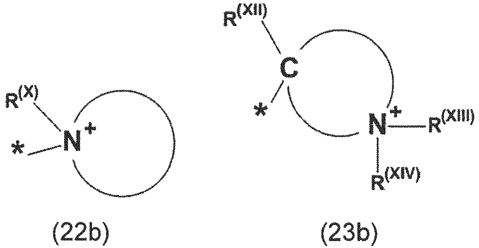 represent a substituted or unsubstituted heterocyclic residue with 5 to 7 ring atoms, which comprise at least one carbon atom and at least one nitrogen atom as well as, optionally, 1 or 2 oxygen atoms, wherein 1 nitrogen atom forms a single bond.

12. Compound according to claim 6,
wherein the organic residue W1c, respectively independently of each other, represents an organic residue with general formula (31a), (31b), (32), (34), (35), (37a) or (37b): 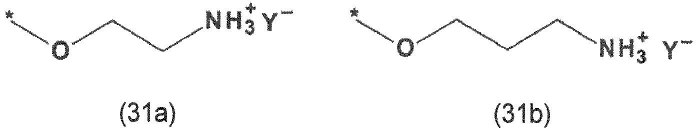 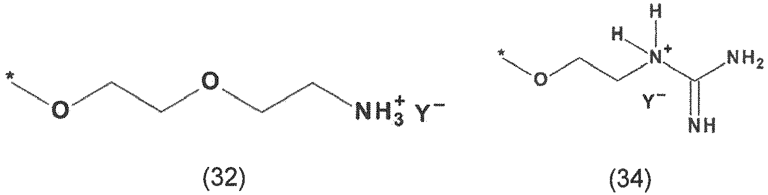 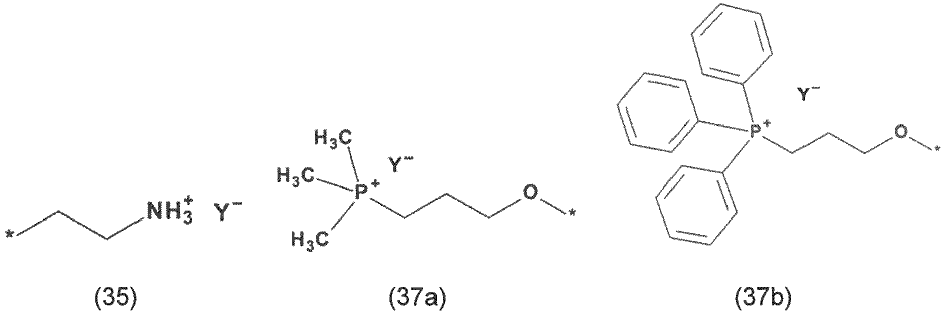 wherein Y⁻ is an anion which, respectively independently of each other, represents fluoride, chloride, bromide, iodide, sulphate, hydrogen sulphate, phosphate, hydrogen phosphate, dihydrogen phosphate, tosylate, mesylate, or at least one carboxylation of a carboxylic acid containing 1 to 15 carbon atoms.

13. Compound according to one of claims 4 to 10,
wherein the organic residue W1a, respectively independently of each other, represents an organic residue with general formula (31a), (31b), (32) or (34): 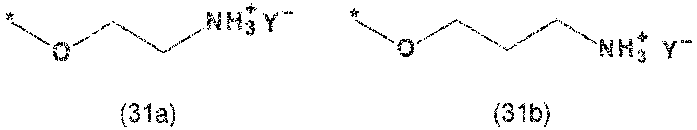 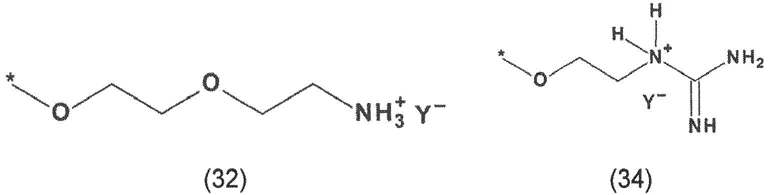 and
wherein Y⁻ is an anion which, respectively independently of each other, represents fluoride, chloride, bromide, iodide, sulphate, hydrogen sulphate, phosphate, hydrogen phosphate, dihydrogen phosphate, tosylate, mesylate, or at least one carboxylation of a carboxylic acid containing 1 to 15 carbon atoms.

14. Compound according to one of claims 4 to 13, wherein the compound is at least one compound with formula (40) to (63), (68), (69a), (69b) or (70):

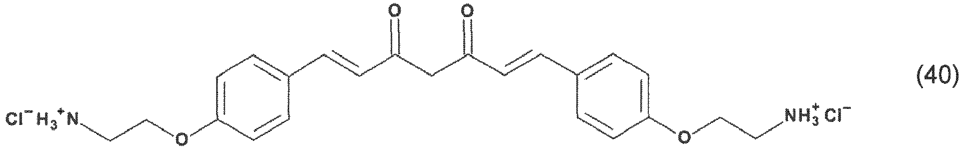

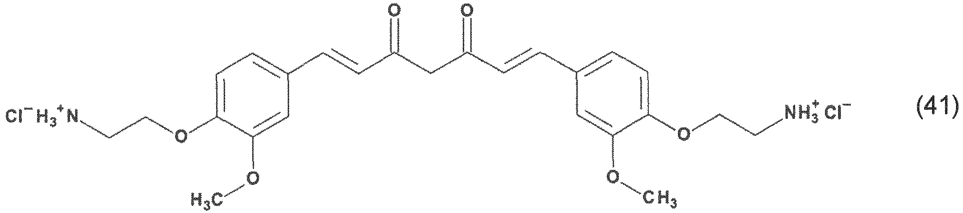

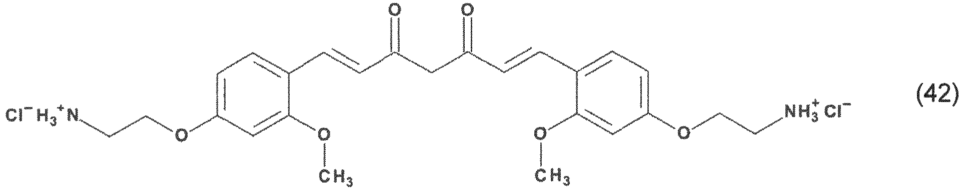

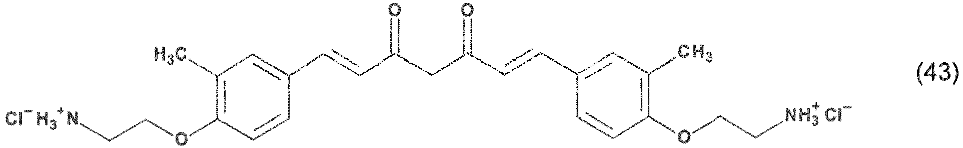

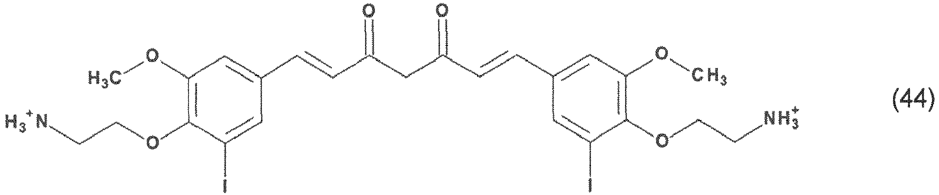

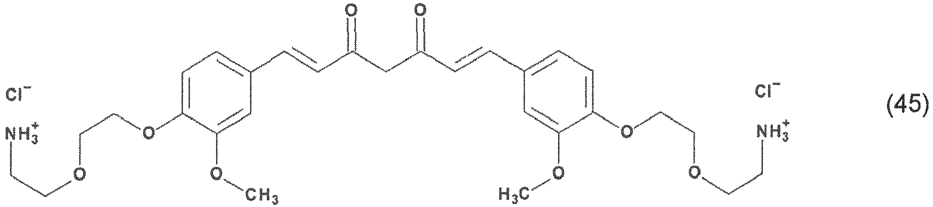

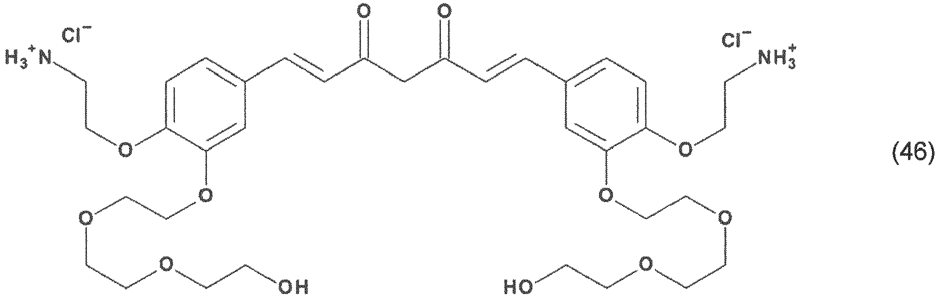

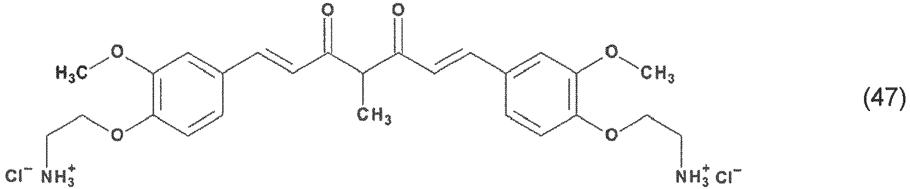

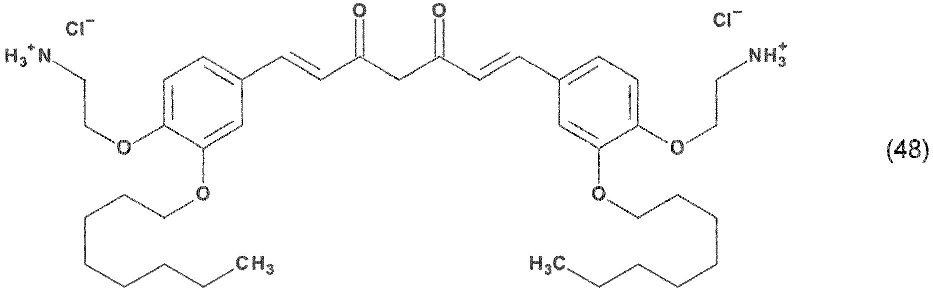

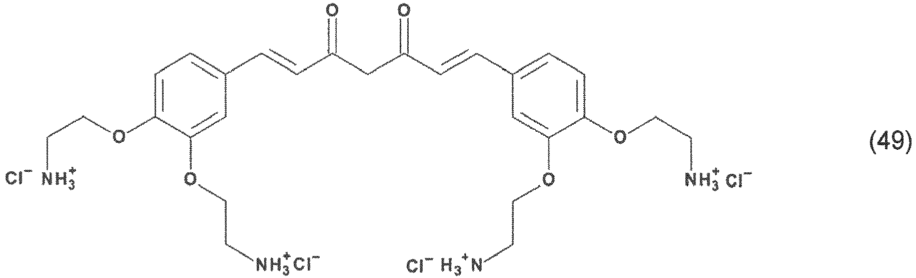

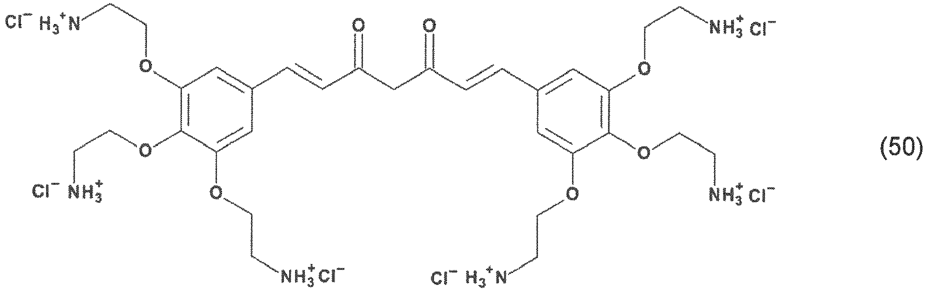

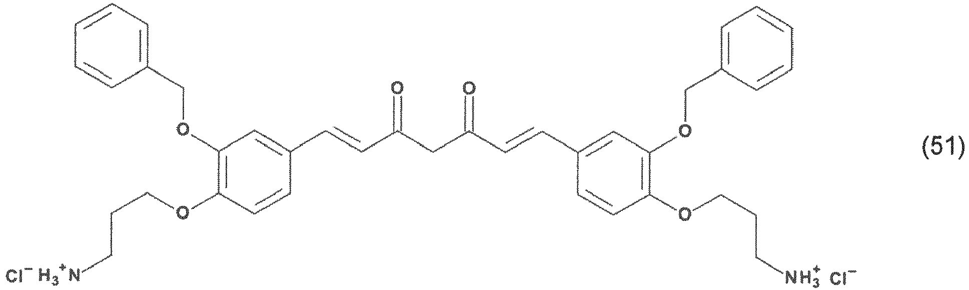

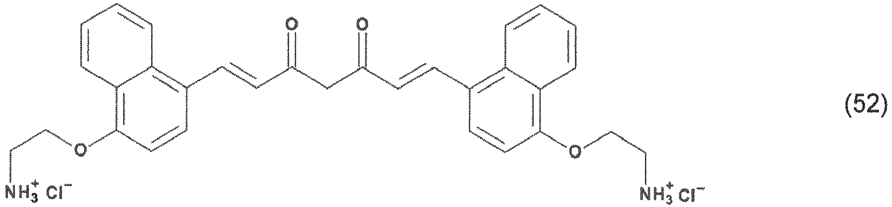

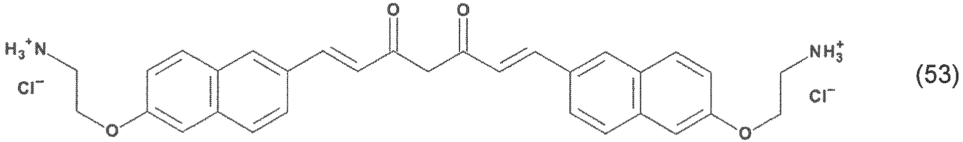

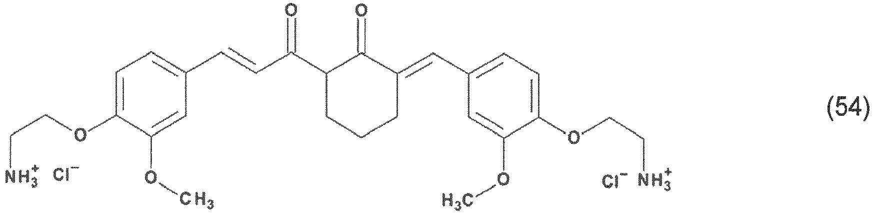

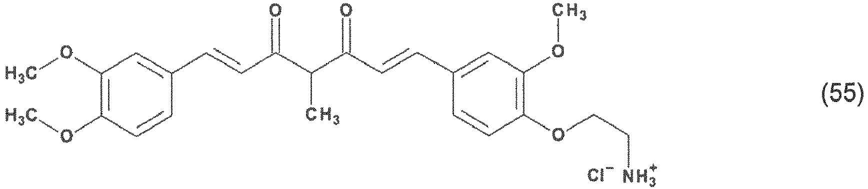

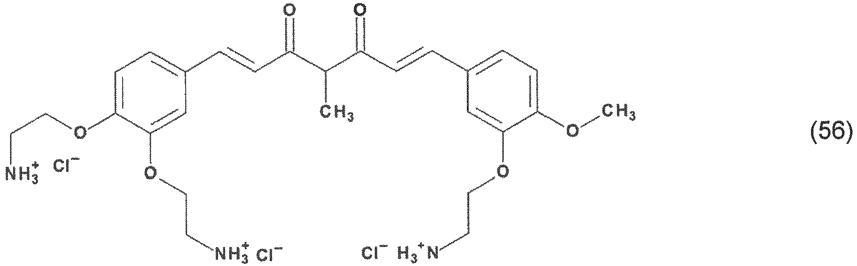

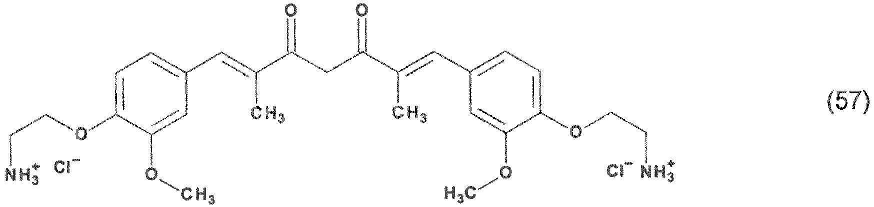

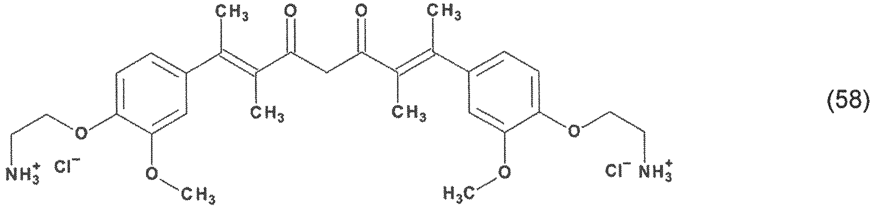

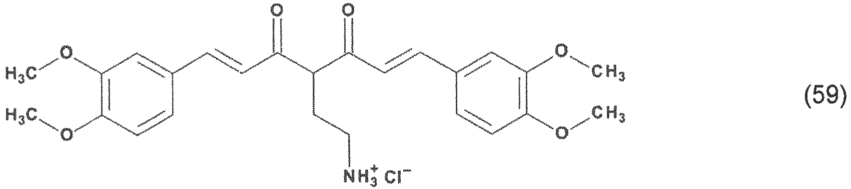

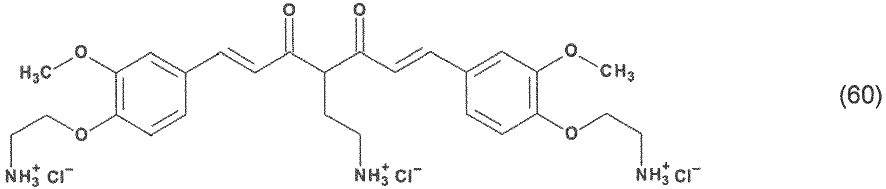

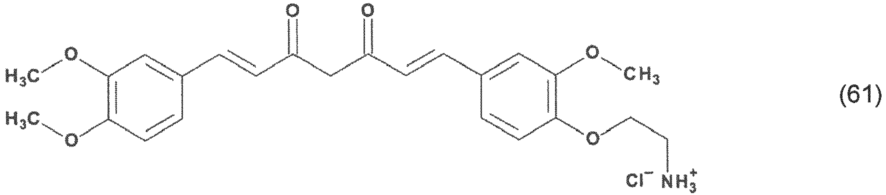

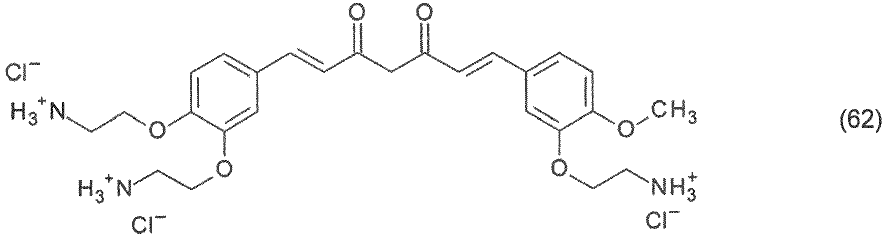

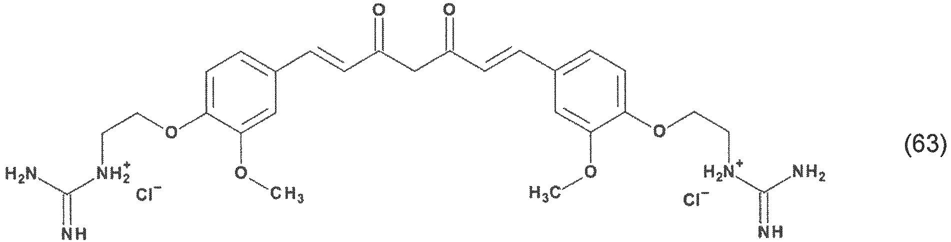

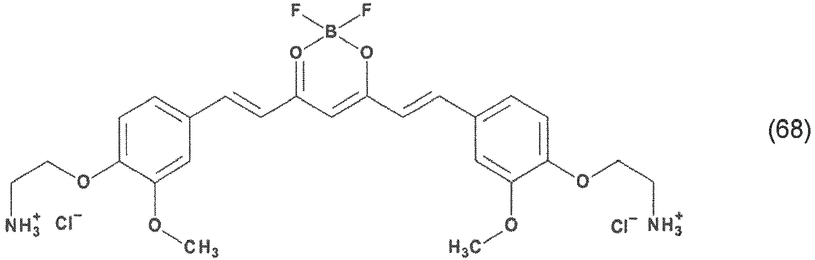

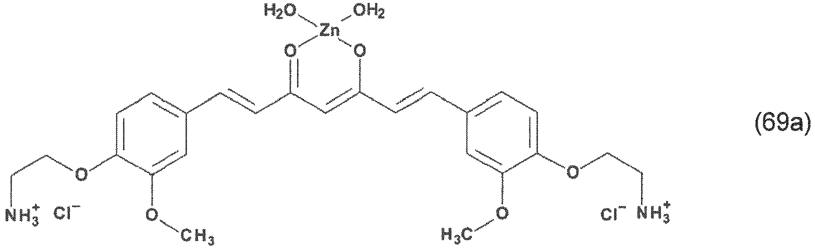

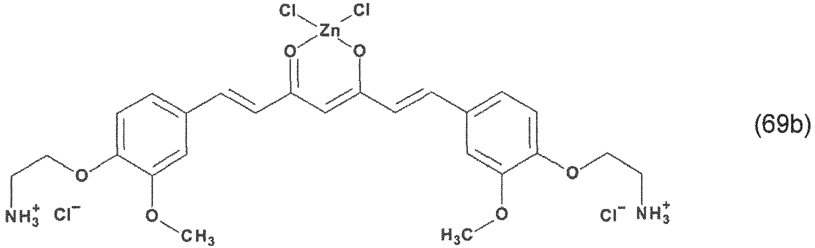

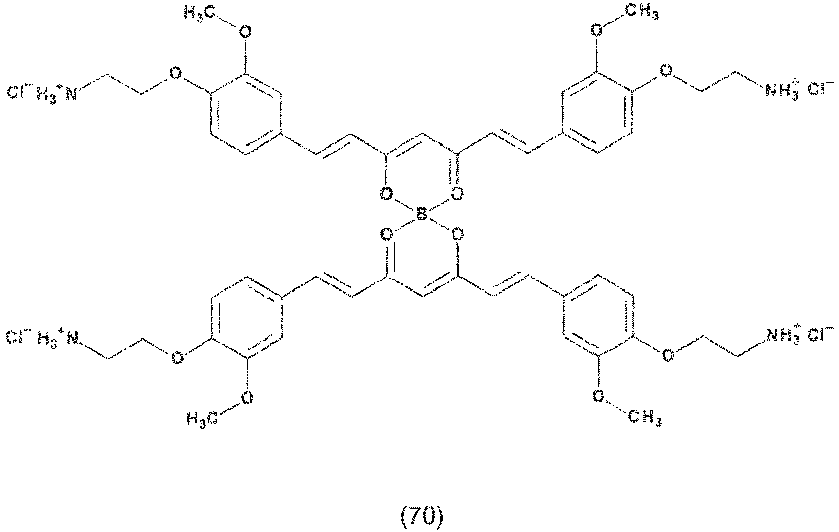

15. Method according to one of claims 1 to 3,
wherein the residues Q³ and Q^{3a}, respectively independently of one another, are an aromatic residue with general formula (11a), (12a), (13a), (14a), (15a), (16a), (17a), (18a), or (19a): 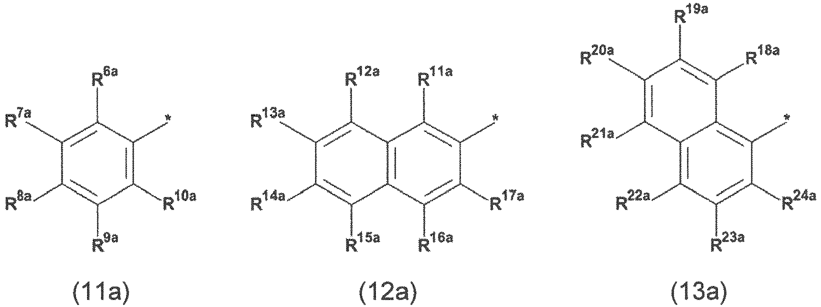 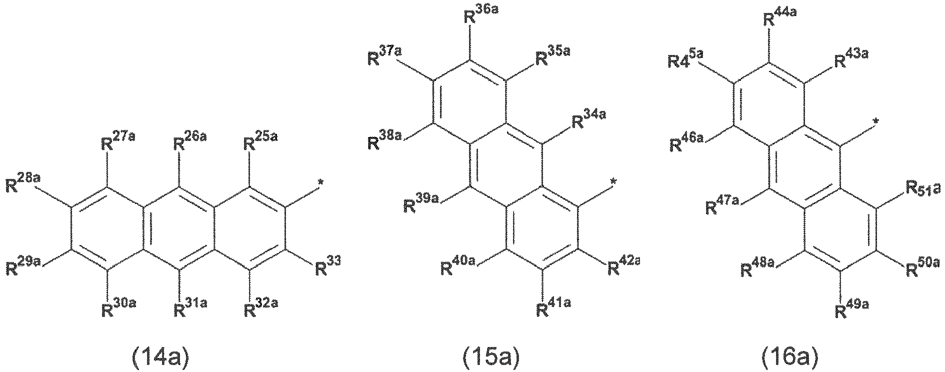 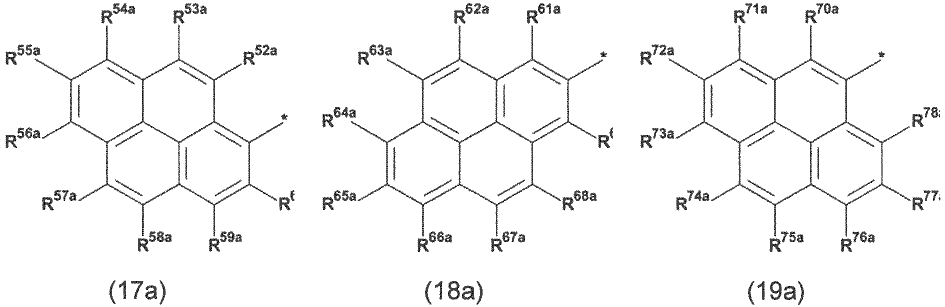 and
wherein the residues Q⁴ and Q^{4a}, respectively independently of each other, represent an aromatic residue with general formula (11b), (12b), (13b), (14b), (15b), (16b), (17b), (18b), or (19b): 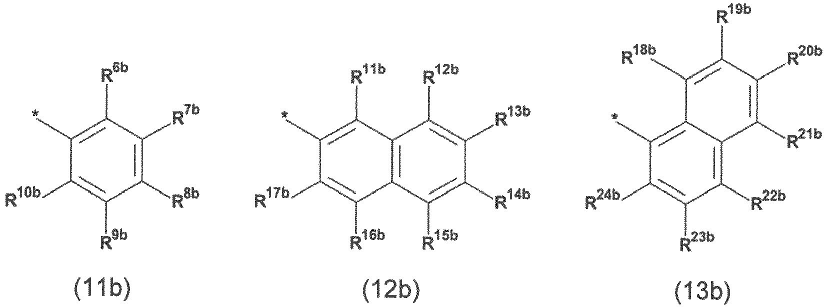 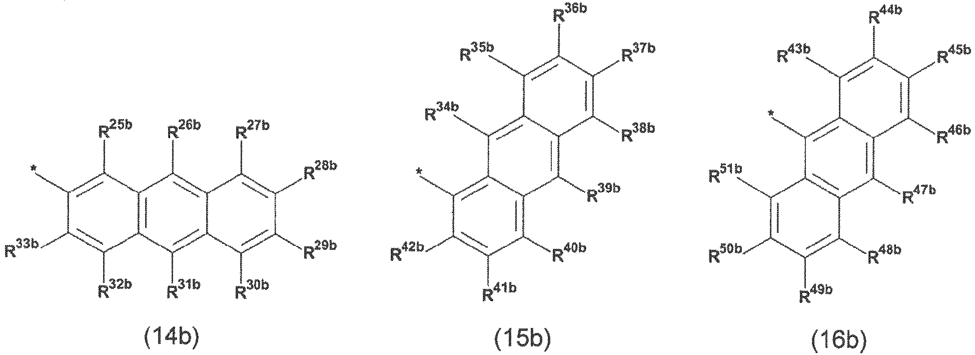 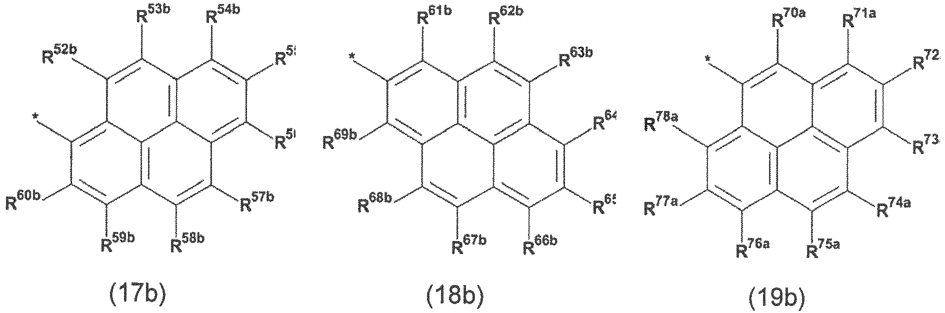 and
wherein, respectively, at least one residue R^{6a} to R^{10a}, R^{11a} to R^{17a}, R^{18a} to R^{24a}, R^{25a} to R^{33a}, R^{34a} to R^{42a}, R^{43a} to R^{51a}, R^{52a} to R^{60a}, R^{61a} to R^{69a}, R^{70a} to R^{78a}, R^{6b} to R^{10b}, R^{11b} to R^{17b}, R^{18b} to R^{24b}, R^{25b} to R^{33b}, R^{34b} to R^{42b}, R^{43b} to R^{51b}, R^{52b} to R^{60b}, R^{61b} to R^{69b}, or R^{70b} to R^{78b}, respectively independently of each other, is an organic residue W1a or an organic residue W1b or an organic residue W1c, and
wherein the residues R^{6a} to R^{10a}, R^{11a} to R^{17a}, R^{18a} to R^{24a}, R^{25a} to R^{33a}, R^{34a} to R^{42a}, R^{43a} to R^{51a}, R^{52a} to R^{60a}, R^{61a} to R^{69a}, R^{70a} to R^{78a}, R^{6b} to R^{10b}, R^{11b} to R^{17b}, R^{18b} to R^{24b}, R^{25b} to R^{33b}, R^{34b} to R^{42b}, R^{43b} to R^{51b}, R^{52b} to R^{60b}, R^{61b} to R^{69b}, or R^{70b} to R^{78b}, which are not an organic residue W1a or an organic residue W1b or an organic residue W1c, respectively independently of each other, are identical or different and represent hydrogen, halogen, hydroxyl, thiol, nitro, carboxylate, aldehyde containing 1 to 8 C atoms, ketone containing 2 to 8 C atoms, O-alkyl containing 1 to 12 C atoms, S-alkyl containing 1 to 12 C atoms, O-alkenyl containing 2 to 12 C atoms, S-alkenyl containing 2 to 12 C atoms, O-aryl containing 5 to 20 C atoms, S-aryl containing 5 to 20 C atoms, ether containing 2 to 12 C atoms, thioether containing 2 to 12 C atoms, carboxylic acid ester containing 1 to 12 C atoms, carboxylic acid amide containing 1 to 12 C atoms, thioester containing 1 to 12 C atoms, alkyl containing 1 to 12 C atoms, alkenyl containing 2 to 12 C atoms, cycloalkyl containing 3 to 12 C atoms, cycloalkenyl containing 3 to 12 C atoms, alkylaryl containing 1 to 12 C atoms, aryl containing 5 to 20 C atoms or heteroaryl, which does not contain a nitrogen atom, containing 4 to 20 C atoms.

16. Method according to claim 15,
wherein the residues Q³ and Q^{3a}, respectively independently of one another, are an aromatic residue with general formula (11a), (12a) or (13a) and wherein the residues Q⁴ and Q^{4a}, respectively independently of each other, are an aromatic residue with general formula (11b), (12b) or (13b), and
wherein respectively at least one residue R^{6a} to R^{10a}, R^{11a} to R^{17a}, R^{18a} to R^{24a}, R^{6b} to R^{10b}, R^{11b} to R^{17b} or R^{18b} to R^{24b}, respectively independently of each other, is an organic residue W1a or an organic residue W1b or an organic residue W1c,
wherein the residues R^{10a}, R^{11a} to R^{17a}, R^{18a} to R^{24a}, R^{6b} to R^{10b}, R^{11b} to R^{17b} and R^{18b} to R^{24b}, which are not an organic residue W1a or an organic residue W1b or an organic residue W1c, respectively independently of each other, are identical or different and represent hydrogen, halogen, hydroxyl, thiol, nitro, carboxylate, aldehyde containing 1 to 8 C atoms, ketone containing 2 to 8 C atoms, O-alkyl containing 1 to 12 C atoms, S-alkyl containing 1 to 12 C atoms, O-alkenyl containing 2 to 12 C atoms, S-alkenyl containing 2 to 12 C atoms, O-aryl containing 5 to 20 C atoms, S-aryl containing 5 to 20 C atoms, ether containing 2 to 12 C atoms, thioether containing 2 to 12 C atoms, carboxylic acid ester containing 1 to 12 C atoms, carboxylic acid amide containing 1 to 12 C atoms, thioester containing 1 to 12 C atoms, alkyl containing 1 to 12 C atoms, alkenyl containing 2 to 12 C atoms, cycloalkyl containing 3 to 12 C atoms, cycloalkenyl containing 3 to 12 C atoms, alkylaryl containing 1 to 12 C atoms, aryl containing 5 to 20 C atoms or heteroaryl, which does not contain a nitrogen atom, containing 4 to 20 C atoms.

17. Method according to one of claims 1 to 3, wherein the at least one photosensitizer is at least one compound according to one of claims 4 to 14 and/or a pharmacologically acceptable salt and/or ester and/or complex thereof.

18. Non-medical use of a compound according to one of claims 4 to 14 and/or a pharmacologically acceptable salt and/or ester and/or complex thereof, as a photosensitizer for the inactivation of microorganisms, which are preferably selected from the group formed by viruses, archaea, bacteria, bacterial spores, fungi, fungal spores, protozoa, algae and blood-borne parasites.

19. Use according to claim 18, for the surface cleaning and/or coating of an article.

20. Use according to claim 18 or claim 19, for the surface cleaning and/or coating of medical products, food packaging, textiles; building materials, electronic devices, furniture or hygiene articles.

21. Use according to claim 18 or claim 19, to sterilize fluids.

22. Use according to claim 18 or claim 19, to sterilize foodstuffs.

23. Coated article, wherein the surface of the article comprises at least one compound according to one of claims 4 to 14.

24. Compound with formula (100): 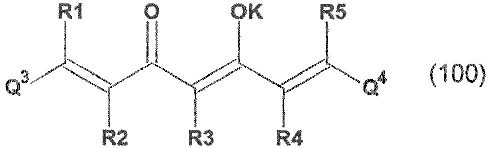 and/or with formula (101): 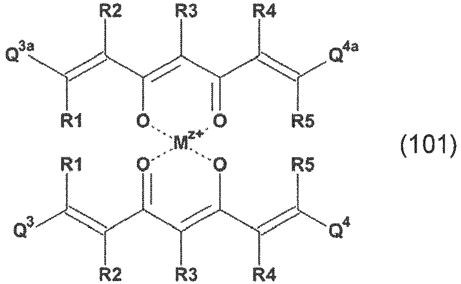 or respectively a pharmacologically acceptable salt and/or ester and/or complex thereof, for use as a photosensitizer in the medical treatment for the inactivation of microorganisms,
wherein Q³, Q^{3a}, Q⁴ and Q^{4a}, respectively independently of each other, represent one substituted or unsubstituted, monocyclic or polycyclic aromatic residue or one substituted or unsubstituted, monocyclic or polycyclic heteroaromatic residue,
wherein the compound with formula (100) does not contain an OH group directly bonded to the organic residue Q³ or Q⁴, and
wherein the compound with formula (101) does not contain an OH group directly bonded to the organic residue Q³, Q^{3a}, Q⁴ or Q^{4a}, and
wherein K represents hydrogen or a cation, and
wherein M^{z+} represents a cation of a metal, wherein z is the formal oxidation number of the metal M and z represents a whole number from 1 to 7, preferably from 2 to 5, and wherein
(a1) at least one of the residues Q³, Q^{3a}, Q⁴ and Q^{4a}, respectively independently of each other, is an unsubstituted, monocyclic or polycyclic heteroaromatic residue, which has at least 5 ring atoms, wherein the ring atoms contain at least one carbon atom and at least one nitrogen atom which preferably can be protonated, or
(a2) at least one of the residues Q³, Q^{3a}, Q⁴ and Q^{4a}, respectively independently of each other, is substituted with at least one organic residue W1, wherein the at least one organic residue W1 has the general formula (4), (5), (6), (7), (8):
-(C(D)(E))ₕ-X, (4)
-A-(C(D)(E))ₕ-X, (5)
-(C(D)(E))ₖ-aryl-(C(D)(E))ₗ-X, (6)
-A-(C(D)(E))ₖ-aryl-(C(D)(E))ₗ-X, (7)
-((C(D)(E))ₘ-A)ₚ-(C(D)(E))ₙ-X, (8)
-A-((C(D)(E))ₘ-A)ₚ-(C(D)(E))ₙ-X, (9)
wherein h represents a whole number from 1 to 20, wherein k and I, respectively independently of each other, represent a whole number from 0 to 10, and wherein m, n and p, respectively independently of each other, represent a whole number from 1 to 6 and
wherein A, respectively independently of each other, represents oxygen or sulphur,
wherein D and E, respectively independently of each other, represent hydrogen, halogen, G-R^{(I)}, or G-C(=G)-R^{(II)}, wherein G, respectively independently of each other, represents oxygen or sulphur, and wherein the residues R^{(I)} and R^{(II)}, respectively independently of each other, represent hydrogen, methyl, ethyl, n-propyl, n-butyl, n-pentyl, phenyl or benzyl, wherein phenyl and benzyl may be unsubstituted or substituted,
wherein aryl represents a substituted or unsubstituted aromatic group or a substituted or unsubstituted heteroaromatic group which does not contain a nitrogen atom,
wherein X, respectively independently of each other, is an organic residue which (i) contains at least one neutral nitrogen atom which can be protonated, or (ii) at least one positively charged nitrogen atom or (iii) at least one positively charged phosphorus atom, and
wherein the residues R1, R2, R3, R4 and R5, respectively independently of each other, represent hydrogen, halogen, alkyl containing 1 to 12 C atoms, cycloalkyl containing 1 to 12 C atoms, alkylaryl containing 1 to 12 C atoms, aryl containing 5 to 20 C atoms, ether containing 2 to 12 C atoms or glycol containing 2 to 12 C atoms, or wherein
(b) the residue R3 is an organic residue W2, wherein the one organic residue W2 has the general formula (4), (5), (6), (7), (8), (9), or (10):
-(C(D)(E))ₕ-X, (4)
-A-(C(D)(E))ₕ-X, (5)
-(C(D)(E))ₖ-aryl-(C(D)(E))ₗ-X, (6)
-A-(C(D)(E))ₖ-aryl-(C(D)(E))ₗ-X, (7)
-[(C(D)(E))ₘ-A]ₚ-(C(D)(E))ₙ-X, (8)
-A-[(C(D)(E))ₘ-A]ₚ-(C(D)(E))ₙ-X, (9)
-(-C(D)=C(E)-)ᵣ-X, (10)
and
wherein, optionally, at least one of the residues Q³, Q^{3a}, Q⁴ and Q^{4a}, respectively independently of each other, is substituted with at least one organic residue W1 which has the general formula (4), (5), (6), (7), (8), or (9),
wherein h represents a whole number from 1 to 20, wherein k and I, respectively independently of each other, represent a whole number from 0 to 10 and wherein m, n, p, and r, respectively independently of each other, represent a whole number from 1 to 6, preferably from 2 to 4, and
wherein A, respectively independently of each other, represents oxygen or sulphur,
wherein D and E, respectively independently of each other, represent hydrogen, halogen, G-R^{(I)}, or G-C(=G)-R^{(II)}, wherein G, respectively independently of each other, represents oxygen or sulphur, and wherein the residues R^{(I)} and R^{(II)}, respectively independently of each other, represent hydrogen, methyl, ethyl, n-propyl, n-butyl, n-pentyl, phenyl or benzyl, wherein phenyl and benzyl may be unsubstituted or substituted,
wherein aryl represents a substituted or unsubstituted aromatic group or a substituted or unsubstituted heteroaromatic group which does not contain a nitrogen atom,
wherein X, respectively independently of each other, is an organic residue which (i) contains at least one neutral nitrogen atom which can be protonated, (ii) contains at least one positively charged nitrogen atom or (iii) contains at least one positively charged phosphorus atom, and
wherein the residues R1, R2, R4 and R5, respectively independently of each other, represent hydrogen, halogen, alkyl containing 1 to 12 C atoms, alkylaryl containing 1 to 12 C atoms, aryl containing 5 to 20 C atoms, ether containing 2 to 12 C atoms or glycol containing 2 to 12 C atoms.

## Revendications

1. Procédé non-thérapeutique pour l'inactivation de microorganismes, dans lequel le procédé comprend les étapes suivantes :
(A) mise en contact des microorganismes avec au moins un photosensibilisant, le photosensibilisant étant au moins un dérivé de 1,7-diaryl-1,6-heptadiène-3,5-dione de formule (100) : 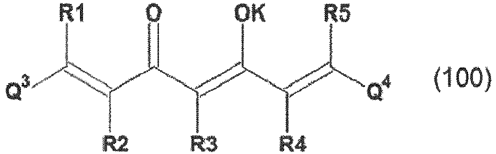 et/ou un dérivé de 1,7-diaryl-1,6-heptadiène-3,5-dione de formule (101) : 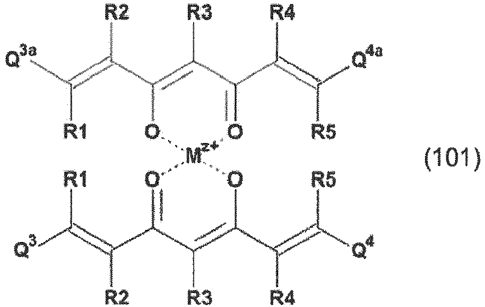 ou un sel et/ou un ester et/ou complexe pharmacologiquement acceptables de chacun d'eux,
dans lequel les radicaux Q³, Q^{3a}, Q⁴ et Q^{4a} représentent chacun indépendamment les uns des autres un radical aromatique monocyclique ou polycyclique substitué ou non substitué ou un radical hétéroaromatique monocyclique ou polycyclique substitué ou non substitué,
dans lequel le composé de formule (100) ne contient aucun groupe OH qui soit directement lié au radical organique Q³ ou Q⁴, et
dans lequel le composé de formule (101) ne contient aucun groupe OH qui soit directement lié au radical organique Q³, Q^{3a}, Q⁴ ou Q^{4a}, et
dans lequel K représente un atome d'hydrogène ou un cation, et
dans lequel M^{z+} représente un cation d'un métal, z étant le degré d'oxydation formel du métal M et z représentant un nombre entier de 1 à 7, de préférence de 2 à 5, et
(a1) au moins l'un des radicaux Q³, Q^{3a}, Q⁴ et Q^{4a} étant indépendamment des autres un radical hétéroaromatique monocyclique ou polycyclique non substitué, qui présente au moins 5 atomes nucléaires, les atomes nucléaires contenant au moins un atome de carbone et au moins un atome d'azote de préférence protonable, ou
(a2) au moins l'un des radicaux Q³, Q^{3a}, Q⁴ et Q^{4a} étant indépendamment des autres substitué par au moins un radical organique W1, l'au moins un radical organique W1 présentant la formule générale (4), (5), (6), (7), (8) ou (9) :
-(C(D)(E))ₕ-X, (4)
-A-(C(D)(E))ₕ-X, (5)
-(C(D)(E))ₖ-Aryl-(C(D)(E))ₗ-X, (6)
-A-(C(D)(E))ₖ-Aryl-(C(D)(E))ₗ-X, (7)
-((C(D)(E))ₘ-A)ₚ-(C(D)(E))ₙ-X, (8)
-A-((C(D)(E))ₘ-A)ₚ-(C(D)(E))ₙ-X, (9)
dans lequel h représente un nombre entier de 1 à 20, k représentant un nombre entier de 0 à 10, I représentant un nombre entier de 0 à 10, et m, n et p représentant chacun indépendamment des autres un nombre entier de 1 à 6, et
dans lequel chaque A représente indépendamment des autres un atome d'oxygène ou de soufre,
dans lequel D et E représentent chacun indépendamment de l'autre un atome d'hydrogène, un groupe halogéno, G-R^{(I)}, ou G-C(=G)-R^{(II)}, dans lequel chaque G représente indépendamment de l'autre un atome d'oxygène ou de soufre, et dans lequel les radicaux R^{(I)} et R^{(II)} représentent chacun indépendamment de l'autre un atome d'hydrogène, un groupe méthyle, éthyle, n-propyle, n-butyle, n-pentyle, phényle ou benzyle, les groupes phényle et benzyle pouvant être non substitués ou substitués,
dans lequel aryle représente un radical aromatique substitué ou non substitué ou un radical hétéroaromatique substitué ou non substitué, qui ne contient aucun atome d'azote,
dans lequel chaque X représente indépendamment des autres un radical organique, qui contient (i) au moins un atome d'azote neutre protonable, ou (ii) au moins un atome d'azote positivement chargé, de préférence quaternaire, ou (iii) au moins un atome de phosphore positivement chargé, de préférence quaternaire, et
dans lequel les radicaux R1, R2, R3, R4 et R5 représentent chacun indépendamment des autres un atome d'hydrogène, un groupe halogéno, alkyle ayant 1 à 12 atomes de carbone, cycloalkyle ayant 1 à 12 atomes de carbone, alkylaryle ayant 1 à 12 atomes de carbone, aryle ayant 5 à 20 atomes de carbone, éther ayant 2 à 12 atomes de carbone ou glycol ayant 2 à 12 atomes de carbone, ou dans lequel
(b) le radical R3 est un radical organique W2, dans lequel le radical organique W2 présente la formule générale (4), (5), (6), (7), (8), (9) ou (10) :
-(C(D)(E))ₕ-X, (4)
-A-(C(D)(E))ₕ-X. (5)
-(C(D)(E))ₖ-Aryl-(C(D)(E))ₗ-X, (6)
-A-(C(D)(E))ₖ-Aryl-(C(D)(E))ₗ-X, (7)
-[(C(D)(E))ₘ-A]ₚ-(C(D)(E))ₙ-X, (8)
-A-[(C(D)(E))ₘ-A]ₚ-(C(D)(E))ₙ-X, (9)
-(-C(D)=C(E)-)ᵣX, (10)
et
dans lequel, en option, au moins l'un des radicaux Q³, Q^{3a}, Q⁴ et Q^{4a} est, indépendamment des autres, substitué par au moins un radical organique W1 qui présente la formule générale (4), (5), (6), (7), (8) ou (9),
dans lequel h représente un nombre entier de 1 à 20, k représentant un nombre entier de 0 à 10, I représentant un nombre entier de 0 à 10, et m, n et p représentant chacun indépendamment des autres un nombre entier de 1 à 6, et
dans lequel chaque A représente indépendamment des autres un atome d'oxygène ou de soufre,
dans lequel D et E représentent chacun indépendamment de l'autre un atome d'hydrogène, un groupe halogéno, G-R^{(I)}, ou G-C(=G)-R^{(II)}, chaque G représentant indépendamment de l'autre un atome d'oxygène ou de soufre, et les radicaux R^{(I)} et R^{(II)} représentant chacun indépendamment de l'autre un atome d'hydrogène, un groupe méthyle, éthyle, n-propyle, n-butyle, n-pentyle, phényle ou benzyle, les groupes phényle et benzyle pouvant être non substitués ou substitués,
dans lequel aryle représente un radical aromatique substitué ou non substitué ou un radical hétéroaromatique substitué ou non substitué, qui ne contient aucun atome d'azote,
dans lequel chaque X est indépendamment des autres un radical organique qui contient (i) au moins un atome d'azote neutre protonable, (ii) au moins un atome d'azote positivement chargé, de préférence quaternaire, ou (iii) au moins un atome de phosphore positivement chargé, de préférence quaternaire, et
dans lequel les radicaux R1, R2, R4 et R5 représentent chacun indépendamment des autres un atome d'hydrogène, un groupe halogéno, alkyle ayant 1 à 12 atomes de carbone, alkylaryle ayant 1 à 12 atomes de carbone, aryle ayant 5 à 20 atomes de carbone, éther ayant 2 à 12 atomes de carbone, ou glycol ayant 2 à 12 atomes de carbone,
et
(B) irradiation des microorganismes et de l'au moins un photosensibilisant avec un rayonnement électromagnétique de longueur d'onde et de densité d'énergie appropriées.

2. Procédé selon la revendication 1, dans lequel, dans le composé de formule (100), K représente un cation M^{z+} d'un métal M, dans lequel z est le degré d'oxydation formel du métal M, et dans lequel z représente un nombre entier de 1 à 7, de préférence de 2 à 3, et dans lequel le composé présente la formule (102) 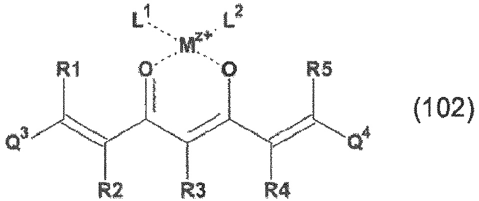 dans laquelle L¹ et L² représentent chacun indépendamment de l'autre un ion eau, fluorure, chlorure, bromure, iodure, phosphate, hydrogénophosphate, dihydrogénophosphate, sulfate, hydrogénosulfate, tosylate, mésylate ou au moins un ion carboxylate d'un acide carboxylique ayant 1 à 15 atomes de carbone, ou des mélanges de ceux-ci.

3. Procédé selon l'une des revendications 1 ou 2, dans lequel l'irradiation des microorganismes et de l'au moins un photosensibilisant par un rayonnement électromagnétique de longueur d'onde et de densité d'énergie appropriées a lieu en présence d'au moins un composé générateur d'oxygène, de préférence un peroxyde, et/ou d'au moins un gaz oxygéné, de préférence l'oxygène.

4. Composé de formule (1) 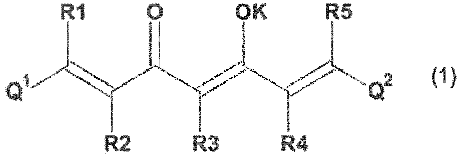 dans lequel les radicaux Q¹ et Q² représentent chacun indépendamment de l'autre un radical aromatique monocyclique ou polycyclique substitué ou non substitué,
dans lequel le composé de formule (1) ne contient aucun groupe OH qui soit directement lié au radical organique Q¹ ou Q², et
dans lequel K représente un atome d'hydrogène ou un cation, et
(a) au moins l'un des radicaux Q¹ et Q² étant indépendamment de l'autre substitué par au moins un radical organique W1a,
l'au moins un radical organique W1a présentant la formule générale (5a), (6a), (7a), (8a) ou (9a) :
-A-(C(D)(E))ₕ-X^{a}, (5a)
-(C(D)(E))ₖ-Aryl-(C(D)(E))ₗ-X^{a}, (6a)
-A-(C(D)(E))ₖ-Aryl-(C(D)(E))ₗ-X^{a}, (7a)
-((C(D)(E))ₘ-A)ₚ-(C(D)(E))ₙ-X^{a}, (8a)
-A-((C(D)(E))ₘ-A)ₚ-(C(D)(E))ₙ-X^{a}, (9a)
dans lequel h représente un nombre entier de 1 à 20, dans lequel k représente un nombre entier de 0 à 10, dans lequel I représente un nombre entier de 0 à 10, et dans lequel m, n et p représentent chacun indépendamment des autres un nombre entier de 1 à 6, et
dans lequel chaque A représente indépendamment des autres un atome d'oxygène ou de soufre,
dans lequel D et E représentent chacun indépendamment de l'autre un atome d'hydrogène, un groupe halogéno, G-R^{(I)}, ou G-C(=G)-R^{(II)}, dans lequel chaque G représente indépendamment de l'autre un atome d'oxygène ou de soufre, et dans lequel les radicaux R^{(I)} et R^{(II)} représentent chacun indépendamment de l'autre un atome d'hydrogène, un groupe méthyle, éthyle, n-propyle, n-butyle, n-pentyle, phényle ou benzyle, les groupes phényle et benzyle pouvant être non substitués ou substitués,
dans lequel aryle représente un radical aromatique substitué ou non substitué ou un radical hétéroaromatique substitué ou non substitué, qui ne contient aucun atome d'azote,
dans lequel chaque X^{a} représente indépendamment des autres un radical de formule (20c), (20d) ou (21) : 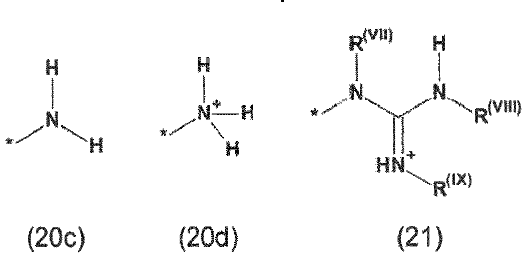 dans lequel chacun des radicaux R^{(VII)}, R^{(VIII)} et R^{(IX)} représente indépendamment des autres un radical alkyle ayant 5 à 12 atomes de carbone, un radical alkylaryle ayant 5 à 12 atomes de carbone, un radical alkyle, qui peut être linéaire ou ramifié, ayant 1 à 8 atomes de carbone, ou un radical éther, qui peut être linéaire ou ramifié, ayant 1 à 8 atomes de carbone, et
dans lequel les radicaux R1, R2, R3, R4 et R5 représentent chacun indépendamment des autres un atome d'hydrogène, un groupe halogéno, alkyle ayant 1 à 12 atomes de carbone, cycloalkyle ayant 1 à 12 atomes de carbone, alkylaryle ayant 1 à 12 atomes de carbone, aryle ayant 5 à 20 atomes de carbone, éther ayant 2 à 12 atomes de carbone ou glycol ayant 2 à 12 atomes de carbone.

5. Composé selon la revendication 4,
dans lequel K représente un cation M^{z+} d'un métal M, dans lequel z est le degré d'oxydation formel du métal M et représentant un nombre entier de 1 à 7, de préférence de 2 à 5, et
dans lequel le composé présente la formule (2) : 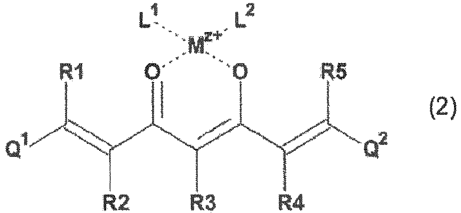 dans lequel L¹ et L² représentent chacun indépendamment de l'autre un ion eau, halogénure, cyanure, thiocyanate, phosphate, hydrogénophosphate, ou un ion carboxylate d'un acide carboxylique ayant 1 à 10 atomes de carbone, de préférence formiate, acétate, n-propionate, lactate, oxalate, fumarate, maléate, tartrate, succinate, benzoate, salicylate ou citrate.

6. Composé de formule (3) : 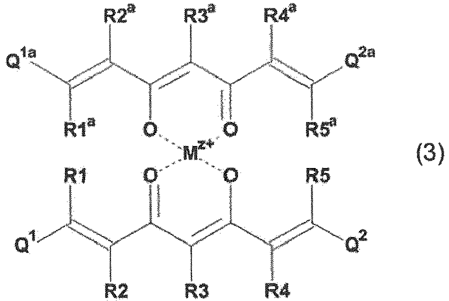 dans lequel M^{z+} est un cation d'un métal, dans lequel z est le degré d'oxydation formel du métal M et représentant un nombre entier de 1 à 7, de préférence de 2 à 5, et
dans lequel Q¹ et Q² représentent chacun indépendamment de l'autre un radical aromatique monocyclique ou polycyclique, substitué ou non substitué, et
dans lequel les radicaux Q^{1a} et Q^{2a} représentent chacun indépendamment de l'autre un radical aromatique monocyclique ou polycyclique, substitué ou non substitué, ou un radical hétéroaromatique monocyclique ou polycyclique, substitué ou non substitué,
dans lequel le composé de formule (3) ne contient aucun groupe OH qui soit directement lié au radical organique Q¹, Q^{1a}, Q² ou Q^{2a},
et
(a) au moins l'un des radicaux Q¹ et Q² étant indépendamment de l'autre substitué par au moins un radical organique W1a, qui présente la formule générale (5a), (6a), (7a), (8a) ou (9a) :
-A-(C(D)(E))ₕ-X^{a}, (5a)
-(C(D)(E))ₖ-Aryl-(C(D)(E))ₗ-X^{a}, (6a)
-A-(C(D)(E))ₖ-Aryl-(C(D)(E))ₗ-X^{a}, (7a)
-((C(D)(E))ₘ-A)ₚ-(C(D)(E))ₙ-X^{a}, (8a)
-A-((C(D)(E))ₘ-A)ₚ-(C(D)(E))ₙ-X^{a}, (9a)
dans lequel au moins l'un des radicaux Q^{1a} et Q^{2a} est indépendamment de l'autre substitué par au moins un radical organique W1c, qui présente la formule générale (5c), (6c), (7c), (8c) ou (9c) :
-A-(C(D)(E))ₕ-X^{c}, (5c)
-(C(D)(E))ₖ-Aryl-(C(D)(E))ₗ-X^{c}, (6c)
-A-(C(D)(E))ₖ-Aryl-(C(D)(E))ₗ-X^{c}, (7c)
-((C(D)(E))ₘ-A)ₚ-(C(D)(E))ₙ-X^{c}, (8c)
-A-((C(D)(E))ₘ-A)ₚ-(C(D)(E))ₙ-X^{c} (9c)
dans lequel h représente un nombre entier de 1 à 20, dans lequel k représente un nombre entier de 0 à 10, dans lequel l représente un nombre entier de 0 à 10, et dans lequel m, n et p représentent chacun indépendamment des autres un nombre entier de 1 à 6, et
dans lequel chaque A représente indépendamment des autres un atome d'oxygène ou de soufre,
dans lequel D et E représentent chacun indépendamment de l'autre un atome d'hydrogène, un groupe halogéno, G-R^{(I)}, ou G-C(=G)-R^{(II)}, dans lequel chaque G représente indépendamment de l'autre un atome d'oxygène ou de soufre, et dans lequel les radicaux R^{(I)} et R^{(II)} représentent chacun indépendamment de l'autre un atome d'hydrogène, un groupe méthyle, éthyle, n-propyle, n-butyle, n-pentyle, phényle ou benzyle, les groupes phényle et benzyle pouvant être non substitués ou substitués,
dans lequel aryle représente un radical aromatique substitué ou non substitué ou un radical hétéroaromatique substitué ou non substitué, qui ne contient aucun atome d'azote, et
dans lequel chaque X^{a} représente indépendamment des autres un radical de formule (20c), (20d) ou (21) : 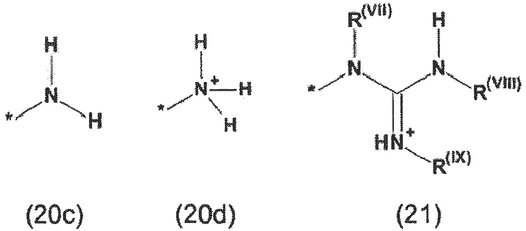 dans lequel chacun des radicaux R^{(VII)}, R^{(VIII)} et R^{(XI)} représentent indépendamment des autres un atome d'hydrogène, un radical aryle ayant 5 à 12 atomes de carbone, un radical alkylaryle ayant 5 à 12 atomes de carbone, un radical alkyle, qui peut être linéaire ou ramifié, ayant 1 à 8 atomes de carbone, ou un radical éther, qui peut être linéaire ou ramifié, ayant 1 à 8 atomes de carbone, et
dans lequel chaque X^{c} est indépendamment des autres un radical organique qui contient (i) au moins un atome d'azote neutre protonable, ou (ii) au moins un atome d'azote positivement chargé, de préférence quaternaire, ou (iii) au moins un atome de phosphore positivement chargé, de préférence quaternaire, et
dans lequel les radicaux R1, R1^{a}, R2, R2^{a}, R3, R3^{a}, R4, R4^{a}, R5 et R5^{a} représentent chacun indépendamment des autres un atome d'hydrogène, un groupe halogéno, alkyle ayant 1 à 12 atomes de carbone, cycloalkyle ayant 1 à 12 atomes de carbone, alkylaryle ayant 1 à 12 atomes de carbone, aryle ayant 5 à 20 atomes de carbone, éther ayant 2 à 12 atomes de carbone ou glycol ayant 2 à 12 atomes de carbone.

7. Composé selon la revendication 6, le composé de formule (3) présentant la formule (3a) : 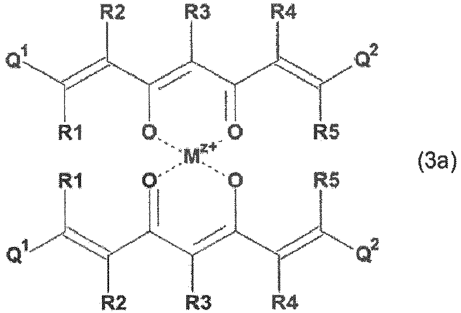 dans lequel M^{z+} représente un cation d'un métal, dans lequel z est le degré d'oxydation formel du métal M et représentant un nombre entier de 1 à 7, de préférence de 2 à 5, et
dans lequel Q¹ et Q² représentent chacun indépendamment de l'autre un radical aromatique monocyclique ou polycyclique, substitué ou non substitué, et
dans lequel le composé de formule (3a) ne contient aucun groupe OH qui soit directement lié au radical organique Q¹ ou Q²,
et
(a) au moins l'un des radicaux Q¹ et Q² étant, indépendamment de l'autre, substitué par au moins un radical organique W1a, qui présente la formule générale (5a), (6a), (7a), (8a) ou (9a) :
-A-(C(D)(E))ₕ-X^{a}, (5a)
-(C(D)(E))ₖ-Aryl-(C(D)(E))ₗ-X^{a}, (6a)
-A-(C(D)(E))ₖ-Aryl-(C(D)(E))ₗ-X^{a}, (7a)
-((C(D)(E))ₘ-A)ₚ-(C(D)(E))ₙ-X^{a}, (8a)
-A-((C(D)(E))ₘ-A)ₚ-(C(D)(E))ₙ-X^{a}, (9a)
dans lequel h représente un nombre entier de 1 à 20, dans lequel k représente un nombre entier de 0 à 10, dans lequel l représente un nombre entier de 0 à 10, et dans lequel m, n et p représentent chacun indépendamment des autres un nombre entier de 1 à 6, et
dans lequel chaque A représente indépendamment des autres un atome d'oxygène ou de soufre,
dans lequel D et E représentent chacun indépendamment de l'autre un atome d'hydrogène, un groupe halogéno, G-R^{(I)}, ou G-C(=G)-R^{(II)}, dans lequel chaque G représente indépendamment de l'autre un atome d'oxygène ou de soufre, et dans lequel les radicaux R^{(I)} et R^{(II)} représentent chacun indépendamment de l'autre un atome d'hydrogène, un groupe méthyle, éthyle, n-propyle, n-butyle, n-pentyle, phényle ou benzyle, les groupes phényle et benzyle pouvant être non substitués ou substitués,
dans lequel aryle représente un radical aromatique substitué ou non substitué ou un radical hétéroaromatique substitué ou non substitué, qui ne contient aucun atome d'azote, et
dans lequel les radicaux X^{a} représentent chacun indépendamment des autres un radical de formule (20c), (20d) ou (21) : 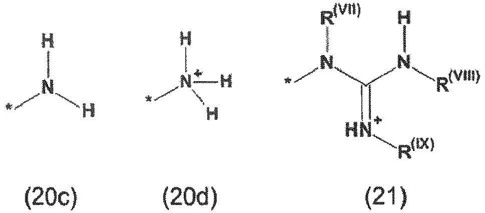 dans lequel chacun des radicaux R^{(VII)}, R^{(VIII)} et R^{(XI)} représentent indépendamment des autres un atome d'hydrogène, un radical aryle ayant 5 à 12 atomes de carbone, un radical alkylaryle ayant 5 à 12 atomes de carbone, un radical alkyle, qui peut être linéaire ou ramifié, ayant 1 à 8 atomes de carbone, ou un radical éther, qui peut être linéaire ou ramifié, ayant 1 à 8 atomes de carbone, et
dans lequel les radicaux R1, R2, R3, R4 et R5 représentent chacun indépendamment des autres un atome d'hydrogène, un groupe halogéno, alkyle ayant 1 à 12 atomes de carbone, cycloalkyle ayant 1 à 12 atomes de carbone, alkylaryle ayant 1 à 12 atomes de carbone, aryle ayant 5 à 20 atomes de carbone, éther ayant 2 à 12 atomes de carbone, ou glycol ayant 2 à 12 atomes de carbone.

8. Composé selon l'une des revendications 5, 6 ou 7, M étant choisi dans le groupe consistant en B, Al, Zn, Cu, Mg, Ca, Fe, Si, Ga, Sn, Rh, Co, Ti, Zr, V, Cr, Mo, Mn, Ru, Pd, Ir, Ni et les combinaisons de ceux-ci.

9. Composé selon l'une des revendications 4 à 8,
dans lequel les radicaux Q¹ et Q^{1a} sont chacun indépendamment de l'autre un radical aromatique de formule générale (11a), (12a), (13a), (14a), (15a), (16a), (17a), (18a) ou (19a) 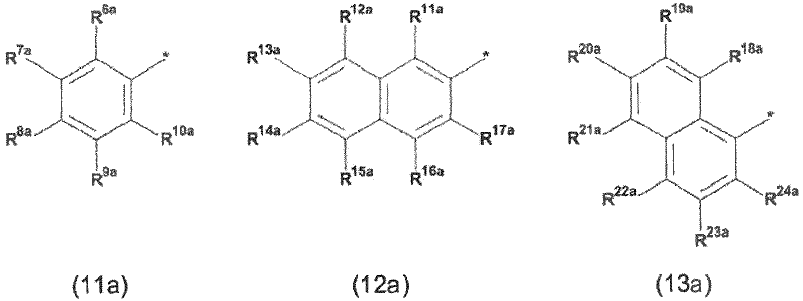 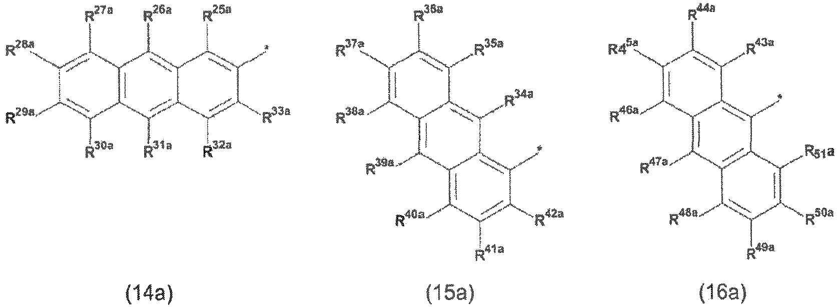 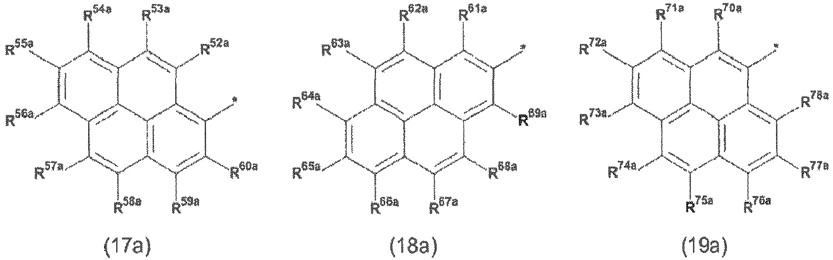 et
dans lequel les radicaux Q² et Q^{2a} représentent chacun indépendamment de l'autre un radical aromatique de formule générale (11b), (12b), (13b), (14b), (15b), (16b), (17b), (18b) ou (19b) : 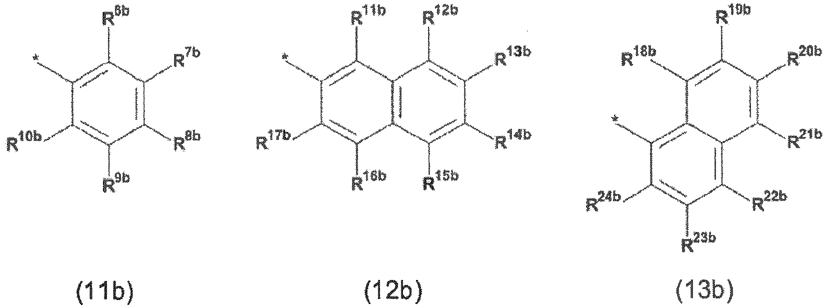 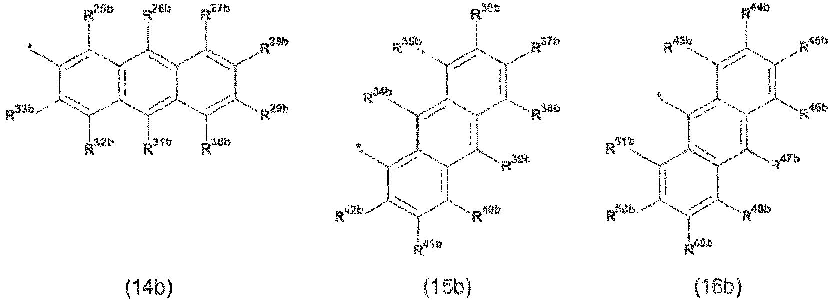 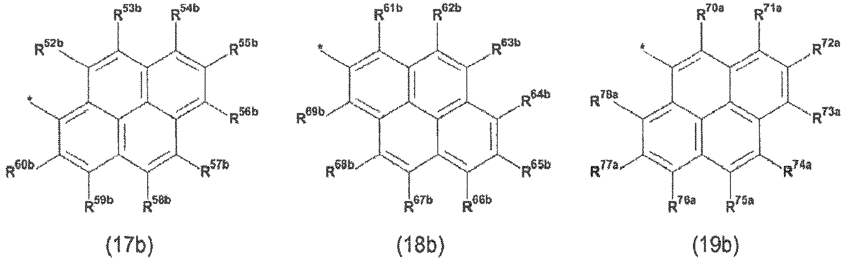 et
dans lequel au moins un radical R^{6a} à R^{10a}, R^{11a} à R^{17a}, R^{18a} à R^{24a}, R^{25a} à R^{33a}, R^{34a} à R^{42a}, R^{43a} à R^{51a}, R^{52a} à R^{60a}, R^{61a} à R^{69a}, R^{70a} à R^{78a}, R^{6b} à R^{10b}, R^{11b} à R^{17b}, R^{18b} à R^{24b}, R^{25b} à R^{33b}, R^{34b} à R^{42b}, R^{43b} à R^{51b}, R^{52b} à R^{60b}, R^{61b} à R^{69b}, ou R^{70b} à R^{78b}, est chacun indépendamment des autres un radical organique W1a ou un radical organique W1b ou un radical organique W1c, et
dans lequel les radicaux R^{6a} à R^{10a}, R^{11a} à R^{17a}, R^{18a} à R^{24a}, R^{25a} à R^{33a}, R^{34a} à R^{42a}, R^{43a} à R^{51a}, R^{52a} à R^{60a}, R^{61a} à R^{69a}, R^{70a} à R^{78a}, R^{6b} à R^{10b}, R^{11b} à R^{17b}, R^{18b} à R^{24b}, R^{25b} à R^{33b}, R^{34b} à R^{42b}, R^{43b} à R^{51b}, R^{52b} à R^{60b}, R^{61b} à R^{69b}, ou R^{70b} à R^{78b} qui ne sont pas un radical organique W1a ou un radical organique W1b ou un radical organique W1c, sont chacun indépendamment des autres identiques ou différents, et représentant chacun un atome d'hydrogène, un groupe halogéno, hydroxyle, thiol, nitro, carboxylate, aldéhyde ayant 1 à 8 atomes de carbone, cétone ayant 2 à 8 atomes de carbone, O-alkyle ayant 1 à 12 atomes de carbone, S-alkyle ayant 1 à 12 atomes de carbone, O-alcényle ayant 2 à 12 atomes de carbone, S-alcényle ayant 2 à 12 atomes de carbone, O-aryle ayant 5 à 20 atomes de carbone, S-aryle ayant 5 à 20 atomes de carbone, éther ayant 2 à 12 atomes de carbone, thioéther ayant 2 à 12 atomes de carbone, ester d'acide carboxylique ayant 1 à 12 atomes de carbone, carboxamide ayant 1 à 12 atomes de carbone, thioester ayant 1 à 12 atomes de carbone, alkyle ayant 1 à 12 atomes de carbone, alcényle ayant 2 à 12 atomes de carbone, cycloalkyle ayant 3 à 12 atomes de carbone, cycloalcényle ayant 3 à 12 atomes de carbone, alkylaryle ayant 1 à 12 atomes de carbone, aryle ayant 5 à 20 atomes de carbone, ou hétéroaryle, qui ne contient aucun atome d'azote, ayant 4 à 20 atomes de carbone.

10. Composé selon la revendication 9,
dans lequel les radicaux Q¹ et Q² sont chacun indépendamment de l'autre un radical aromatique de formule générale (11a), (12a) ou (13a), et les radicaux Q² et Q^{2a} étant chacun indépendamment de l'autre un radical aromatique de formule générale (11b), (12b) ou (13b),
dans lequel au moins un radical R^{6a} à R^{10a}, R^{11a} à R^{17a}, R^{18a} à R^{24a}, R^{6b} à R^{10b}, R^{11b} à R^{17b} ou R^{18b} à R^{24b} est chacun indépendamment des autres un radical organique W1a ou un radical organique W1b ou un radical organique W1c,
dans lequel les radicaux R^{10a}, R^{11a} à R^{17a}, R^{18a} à R^{24a}, R^{6b} à R^{10b}, R^{11b} à R^{17b} et R^{18b} à R^{24b} qui ne sont pas un radical organique W1a ou un radical organique W1b ou un radical organique W1c, sont chacun indépendamment des autres identiques ou différents, et représentant chacun un atome d'hydrogène, un groupe halogéno, hydroxyle, thiol, nitro, carboxylate, aldéhyde ayant 1 à 8 atomes de carbone, cétone ayant 2 à 8 atomes de carbone, O-alkyle ayant 1 à 12 atomes de carbone, S-alkyle ayant 1 à 12 atomes de carbone, O-alcényle ayant 2 à 12 atomes de carbone, S-alcényle ayant 2 à 12 atomes de carbone, O-aryle ayant 5 à 20 atomes de carbone, S-aryle ayant 5 à 20 atomes de carbone, éther ayant 2 à 12 atomes de carbone, thioéther ayant 2 à 12 atomes de carbone, ester d'acide carboxylique ayant 1 à 12 atomes de carbone, carboxamide ayant 1 à 12 atomes de carbone, thioester ayant 1 à 12 atomes de carbone, alkyle ayant 1 à 12 atomes de carbone, alcényle ayant 2 à 12 atomes de carbone, cycloalkyle ayant 3 à 12 atomes de carbone, cycloalcényle ayant 3 à 12 atomes de carbone, alkylaryle ayant 1 à 12 atomes de carbone, aryle ayant 5 à 20 atomes de carbone, ou hétéroaryle, qui ne contient aucun atome d'azote, ayant 4 à 20 atomes de carbone.

11. Composé selon la revendication 6,
dans lequel chacun des radicaux organiques X^{c} représente indépendamment des autres un radical de formule (20a), (20b), (21), (22a), (22b), (23a), (24b) ou (24) : 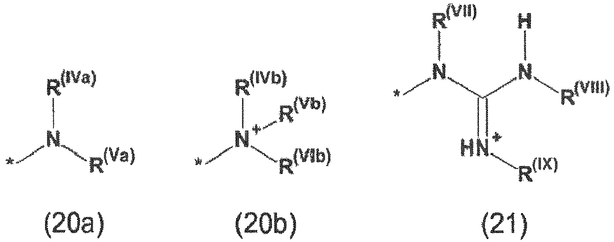 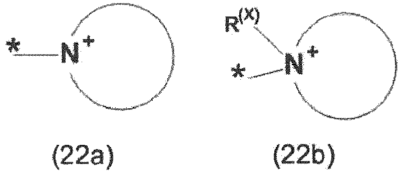 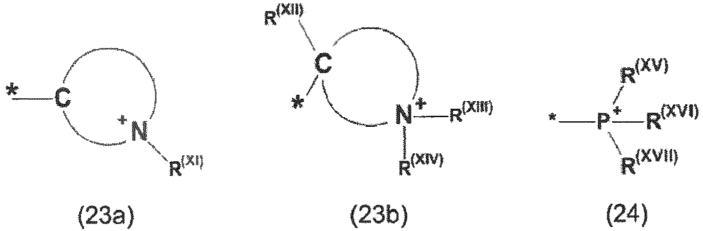
dans lequel chacun des radicaux R^{(IVa)}, R^{(Va)}, R^{(IVb)}, R^{(Vb)}, R^{(VIb)}, R^{(VII)}, R^{(VIII)}, R^{(X)}, R^{(XI)}, R^{(XII)}, R^{(XIII)}, R^{(XIV)}, R^{(XV)}, R^{(XVI)} et R^{(XVII)} représente indépendamment des autres un atome d'hydrogène, un radical aryle ayant 5 à 12 atomes de carbone, un radical alkyle, qui peut être linéaire ou ramifié, ayant 1 à 8 atomes de carbone, ou un radical éther, qui peut être linéaire ou ramifié, ayant 1 à 8 atomes de carbone, et
dans lequel le radical de formule (22a) et le radical de formule(23a) 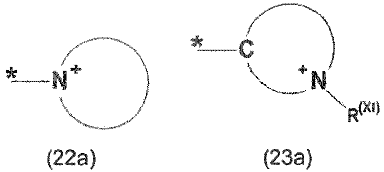 représente chacun un radical hétérocyclique substitué ou non substitué ayant 5 à 7 atomes nucléaires, qui comprennent au moins un atome de carbone et au moins 1 atome d'azote, et en option 1 ou 2 atomes d'oxygène, 1 atome d'azote formant une double liaison, et
dans lequel le radical de formule (22b) et le radical de formule (23b) : 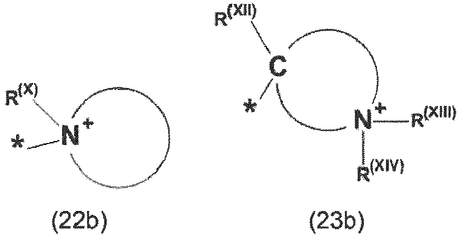 représente chacun un radical hétérocyclique substitué ou non substitué ayant 5 à 7 atomes nucléaires, qui comprennent au moins 1 atome de carbone et au moins 1 atome d'azote, et en option 1 ou 2 atomes d'oxygène, 1 atome d'azote formant une liaison simple.

12. Composé selon la revendication 6,
dans lequel chaque radical organique W1c représente indépendamment des autres un radical organique de formule générale (31a), (31b), (32), (34), (35), (37a) ou (37b) : 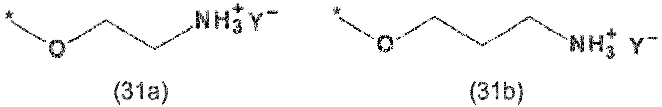 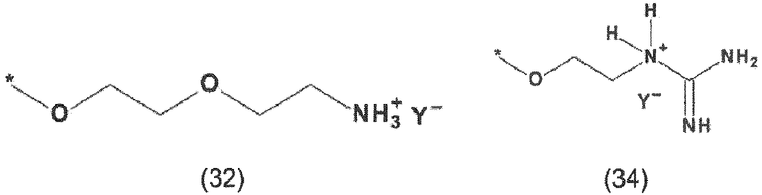 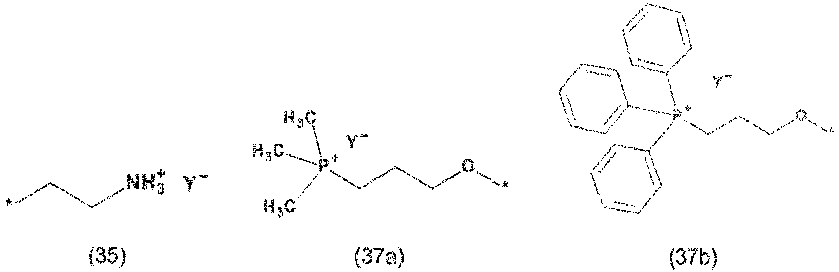
dans lequel Y- est un anion dont chacun représente indépendamment des autres un ion fluorure, chlorure, bromure, iodure, sulfate, hydrogénosulfate, phosphate, hydrogénophosphate, dihydrogénophosphate, tosylate, mésylate, ou au moins un ion carboxylate d'un acide carboxylique ayant 1 à 15 atomes de carbone.

13. Composé selon l'une des revendications 4 à 10,
dans lequel les radicaux organiques W1a représentent chacun indépendamment des autres un radical organique de formule générale (31a), (31b), (32) ou (34) : 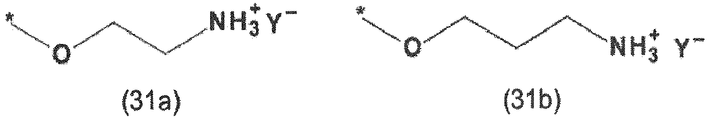 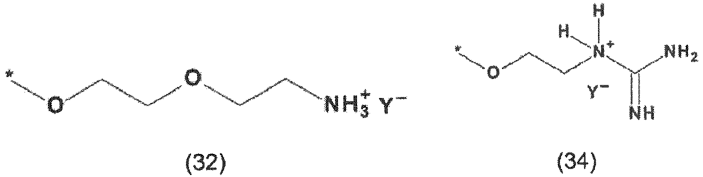 et
dans lequel Y⁻ est un anion dont chacun représente indépendamment des autres un ion fluorure, chlorure, bromure, iodure, sulfate, hydrogénosulfate, phosphate, hydrogénophosphate, dihydrogénophosphate, tosylate, mésylate, ou au moins un ion carboxylate d'un acide carboxylique ayant 1 à 15 atomes de carbone.

14. Composé selon l'une des revendications 4 à 13,
dans lequel le composé est au moins un composé de formule (40) à (63), (68), (69), (69b) ou (70) :

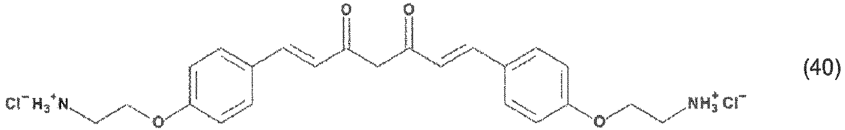

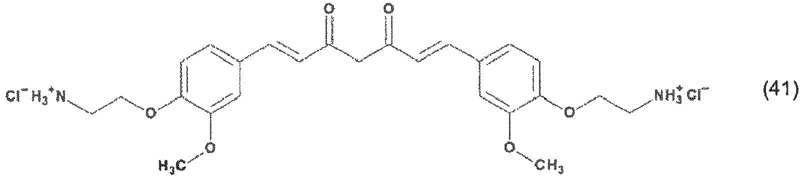

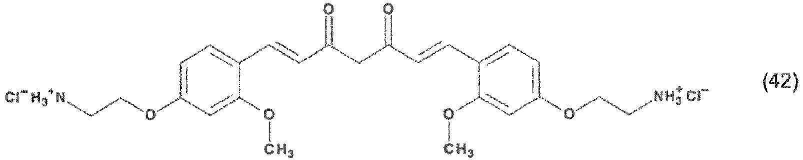

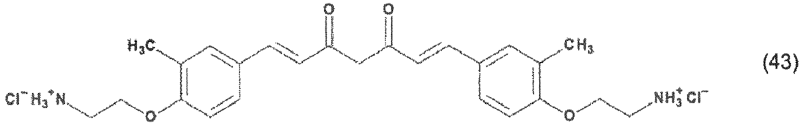

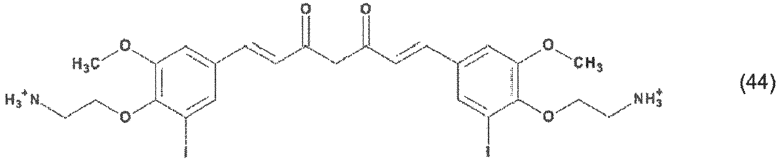

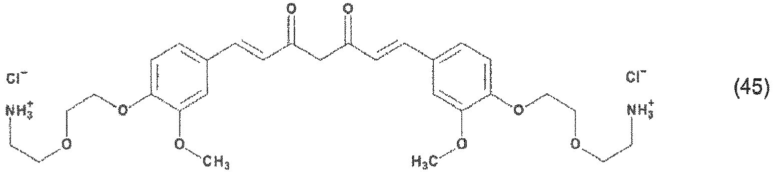

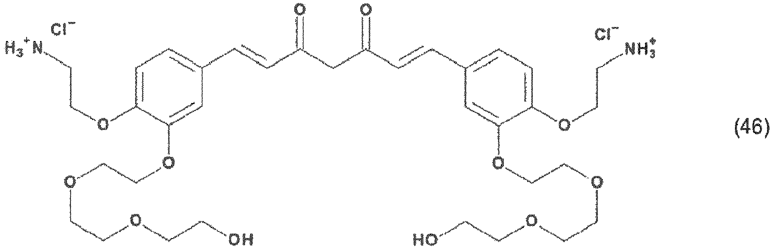

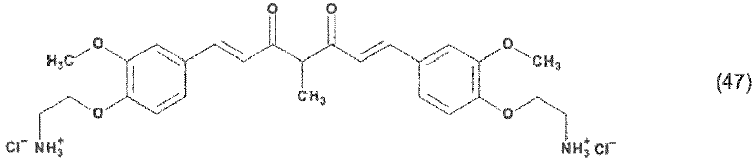

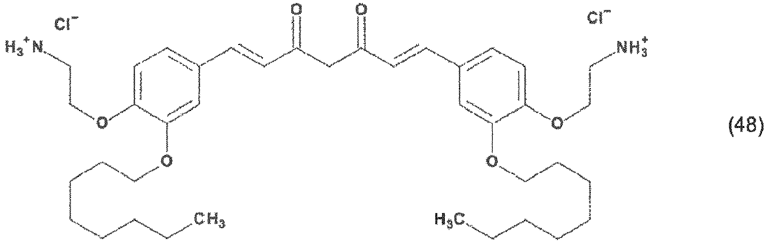

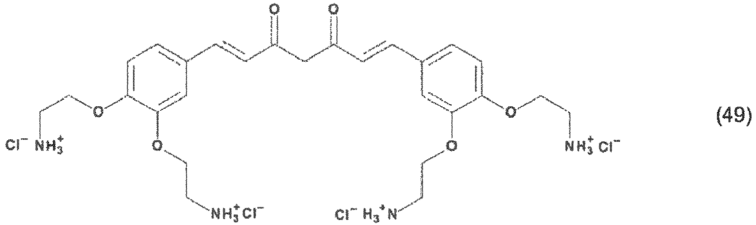

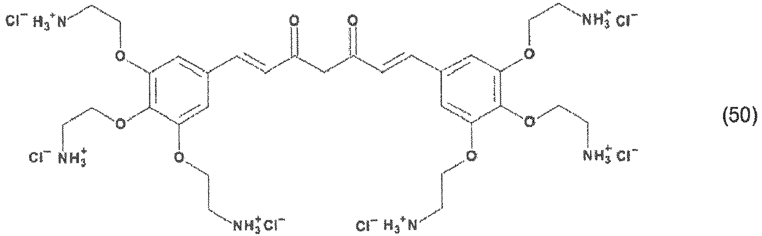

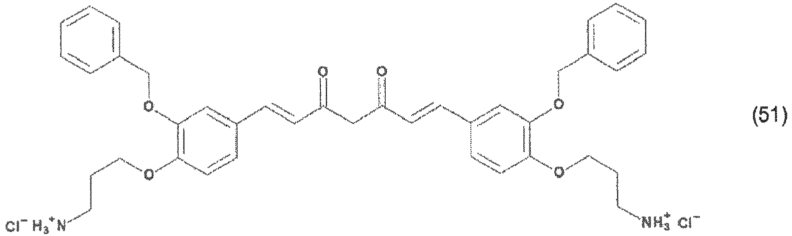

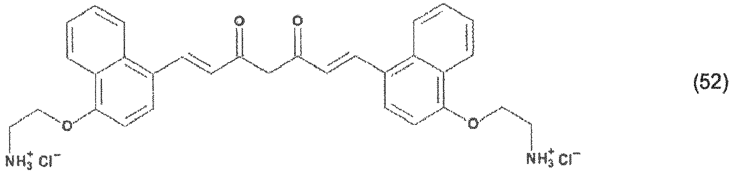

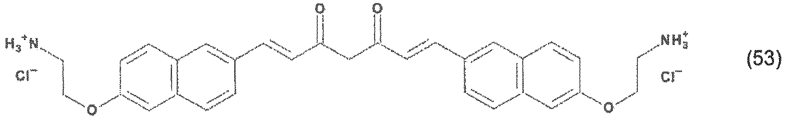

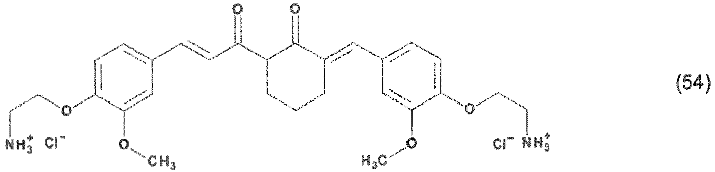

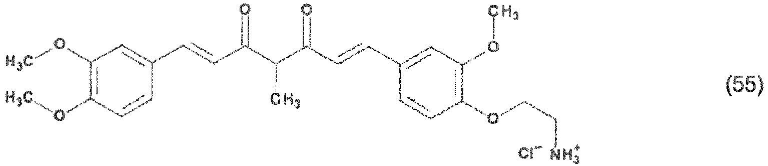

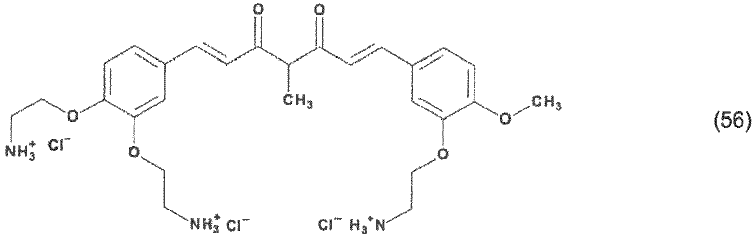

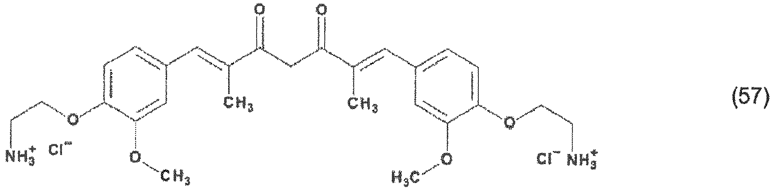

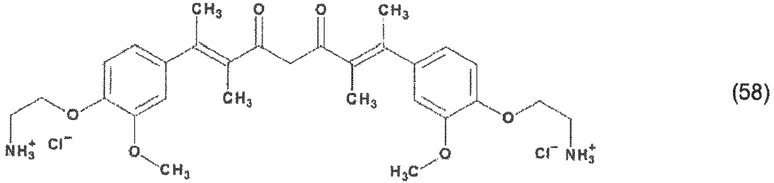

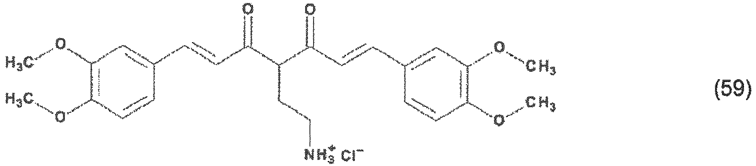

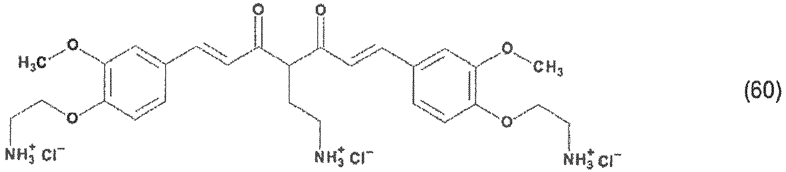

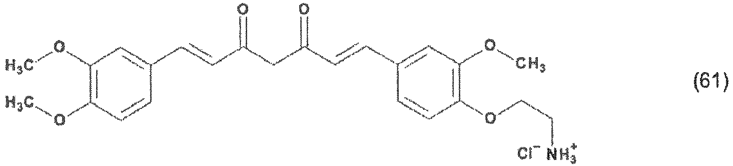

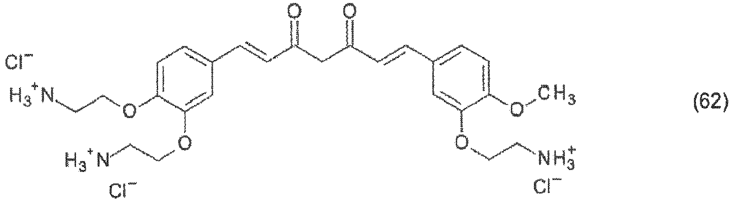

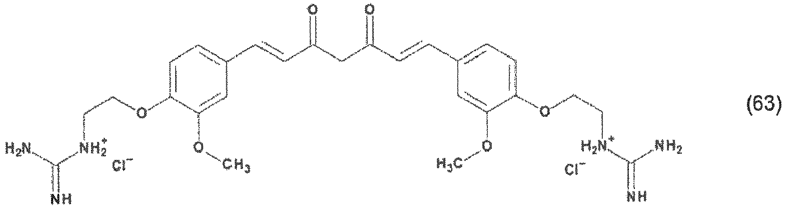

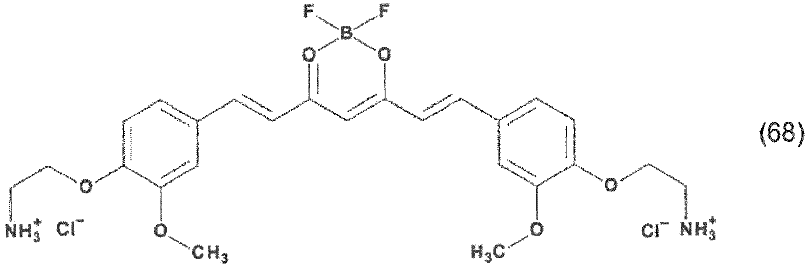

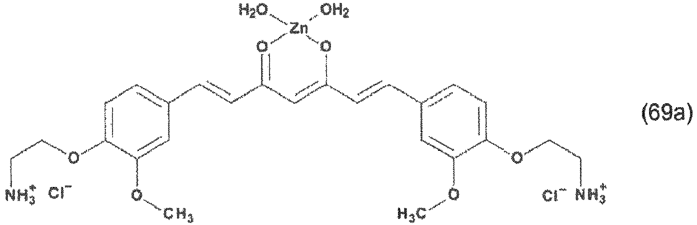

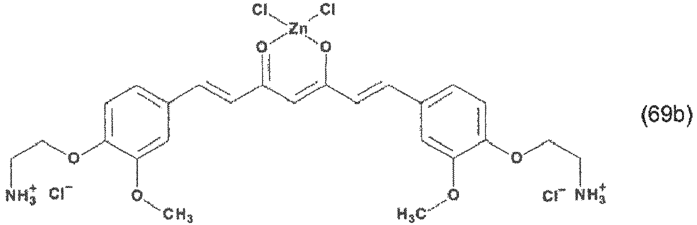

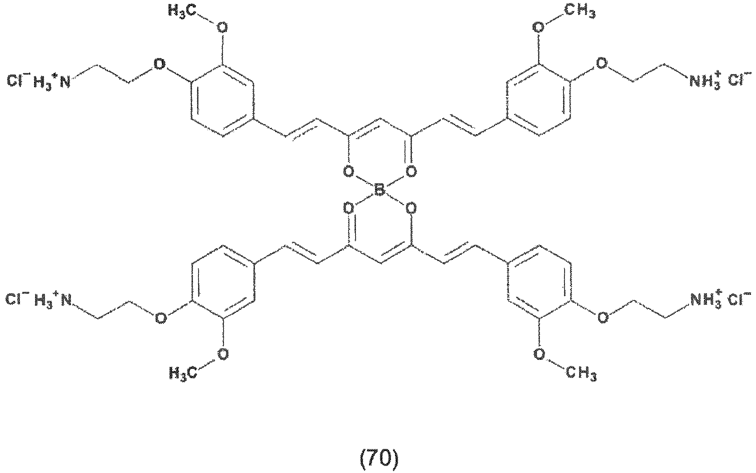

15. Procédé selon l'une des revendications 1 à 3,
dans lequel les radicaux Q³ et Q^{3a} sont chacun indépendamment de l'autre un radical aromatique de formule générale (11a), (12a), (13a), (14a), (15a), (16a), (17a), (18a) ou (19a) : 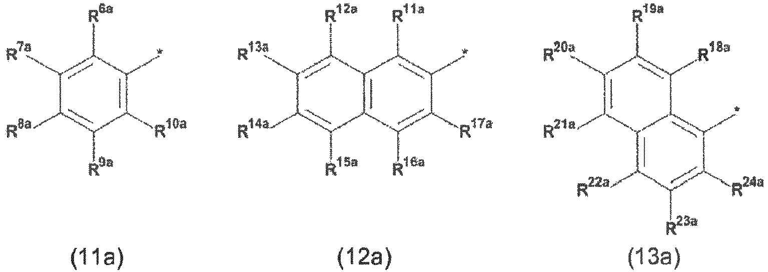 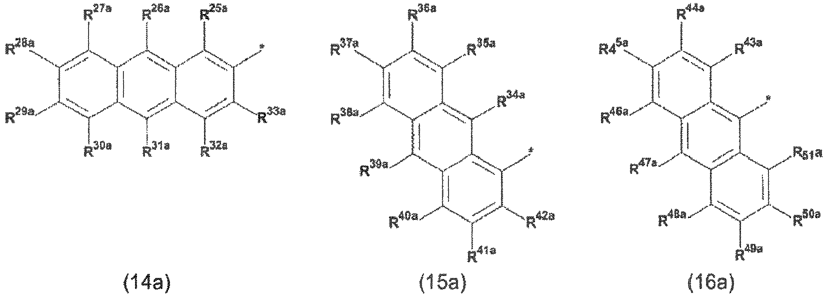 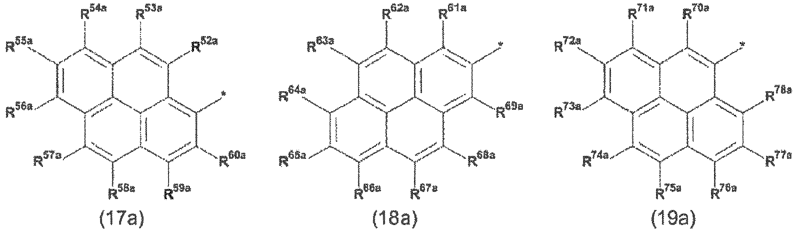 et
dans lequel les radicaux Q⁴ et Q^{4a} représentent chacun indépendamment des autres un radical aromatique de formule générale (11b), (12b), (13b), (14b), (15b), (16b), (17b), (18b) ou (19b) : 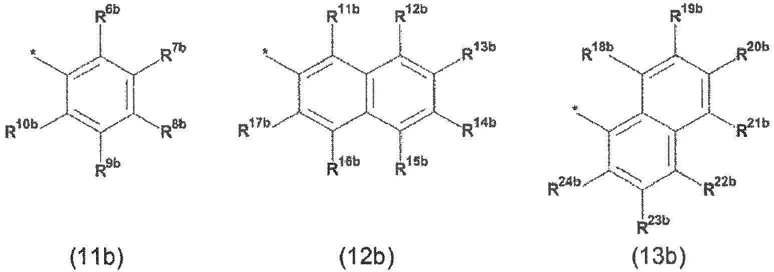 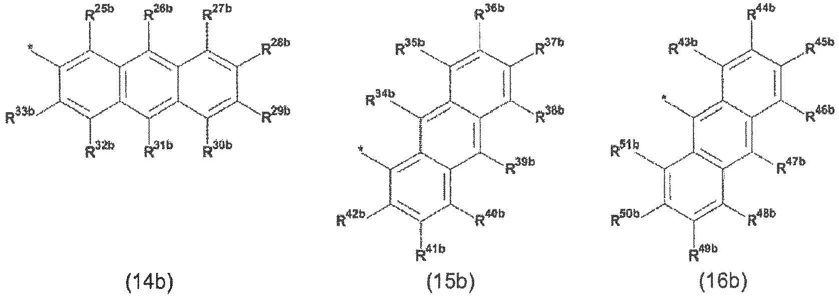 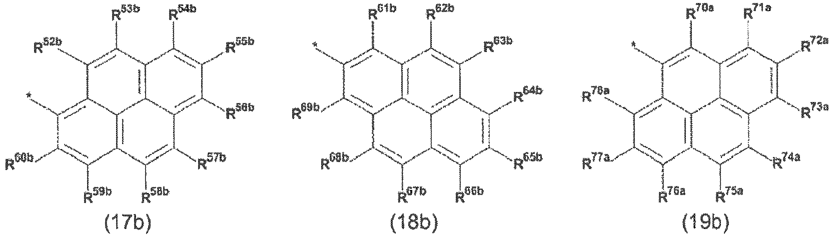 et
dans lequel au moins un radical R^{6a} à R^{10a}, R^{11a} à R^{17a}, R^{18a} à R^{24a}, R^{25a} à R^{33a}, R^{34a} à R^{42a}, R^{43a} à R^{51a}, R^{52a} à R^{60a}, R^{61a} à R^{69a}, R^{70a} à R^{78a}, R^{6b} à R^{10b}, R^{11b} à R^{17b}, R^{18b} à R^{24b}, R^{25b} à R^{33b}, R^{34b} à R^{42b}, R^{43b} à R^{51b}, R^{52b} à R^{60b}, R^{61b} à R^{69b}, ou R^{70b} à R^{78b}, est chacun indépendamment des autres un radical organique W1a ou un radical organique W1b ou un radical organique W1c, et
dans lequel les radicaux R^{6a} à R^{10a}, R^{11a} à R^{17a}, R^{18a} à R^{24a}, R^{25a} à R^{33a}, R^{34a} à R^{42a}, R^{43a} à R^{51a}, R^{52a} à R^{60a}, R^{61a} à R^{69a}, R^{70a} à R^{78a}, R^{6b} à R^{10b}, R^{11b} à R^{17b}, R^{18b} à R^{24b}, R^{25b} à R^{33b}, R^{34b} à R^{42b}, R^{43b} à R^{51b}, R^{52b} à R^{60b}, R^{61b} à R^{69b}, ou R^{70b} à R^{78b} qui ne sont pas un radical organique W1a ou un radical organique W1b ou un radical organique W1c, sont chacun indépendamment des autres identiques ou différents, et représentant chacun un atome d'hydrogène, un groupe halogéno, hydroxyle, thiol, nitro, carboxylate, aldéhyde ayant 1 à 8 atomes de carbone, cétone ayant 2 à 8 atomes de carbone, O-alkyle ayant 1 à 12 atomes de carbone, S-alkyle ayant 1 à 12 atomes de carbone, O-alcényle ayant 2 à 12 atomes de carbone, S-alcényle ayant 2 à 12 atomes de carbone, O-aryle ayant 5 à 20 atomes de carbone, S-aryle ayant 5 à 20 atomes de carbone, éther ayant 2 à 12 atomes de carbone, thioéther ayant 2 à 12 atomes de carbone, ester d'acide carboxylique ayant 1 à 12 atomes de carbone, carboxamide ayant 1 à 12 atomes de carbone, thioester ayant 1 à 12 atomes de carbone, alkyle ayant 1 à 12 atomes de carbone, alcényle ayant 2 à 12 atomes de carbone, cycloalkyle ayant 3 à 12 atomes de carbone, cycloalcényle ayant 3 à 12 atomes de carbone, alkylaryle ayant 1 à 12 atomes de carbone, aryle ayant 5 à 20 atomes de carbone, ou hétéroaryle, qui ne contient aucun atome d'azote, ayant 4 à 20 atomes de carbone.

16. Procédé selon la revendication 15,
dans lequel les radicaux Q³ et Q^{3a} sont chacun indépendamment de l'autre un radical aromatique de formule générale (11a), (12a) ou (13a), et les radicaux Q⁴ et Q^{4a} étant chacun indépendamment de l'autre un radical aromatique de formule générale (11b), (12b) ou (13b), et
dans lequel au moins un radical R^{6a} à R^{10a}, R^{11a} à R^{17a}, R^{18a} à R^{24a}, R^{6b} à R^{10b}, R^{11b} à R^{17b} ou R^{18b} à R^{24b} est chacun indépendamment des autres un radical organique W1a ou un radical organique W1b ou un radical organique W1c,
dans lequel les radicaux R^{10a}, R^{11a} à R^{17a}, R^{18a} à R^{24a}, R^{6b} à R^{10b}, R^{11b} à R^{17b} et R^{18b} à R^{24b} qui ne sont pas un radical organique W1a à ou un radical organique W1b ou un radical organique W1c sont chacun indépendamment des autres identiques ou différents, et représentent chacun un atome d'hydrogène, un groupe halogéno, hydroxyle, thiol, nitro, carboxylate, aldéhyde ayant 1 à 8 atomes de carbone, cétone ayant 2 à 8 atomes de carbone, O-alkyle ayant 1 à 12 atomes de carbone, S-alkyle ayant 1 à 12 atomes de carbone, O-alcényle ayant 2 à 12 atomes de carbone, S-alcényle ayant 2 à 12 atomes de carbone, O-aryle ayant 5 à 20 atomes de carbone, S-aryle ayant 5 à 20 atomes de carbone, éther ayant 2 à 12 atomes de carbone, thioéther ayant 2 à 12 atomes de carbone, ester d'acide carboxylique ayant 1 à 12 atomes de carbone, carboxamide ayant 1 à 12 atomes de carbone, thioester ayant 1 à 12 atomes de carbone, alkyle ayant 1 à 12 atomes de carbone, alcényle ayant 2 à 12 atomes de carbone, cycloalkyle ayant 3 à 12 atomes de carbone, cycloalcényle ayant 3 à 12 atomes de carbone, alkylaryle ayant 1 à 12 atomes de carbone, aryle ayant 5 à 20 atomes de carbone ou hétéroaryle, qui ne contient aucun atome d'azote, ayant 4 à 20 atomes de carbone.

17. Procédé selon l'une des revendications 1 à 3, dans lequel l'au moins un photosensibilisant est au moins un composé selon l'une des revendications 4 à 14 et/ou un sel et/ou un ester et/ou un complexe pharmacologiquement acceptables de chacun d'eux.

18. Utilisation non-médicale d'un composé selon l'une des revendications 4 à 14 et/ou d'un sel et/ou d'un ester et/ou d'un complexe pharmacologiquement acceptables de chacun d'eux, en tant que photosensibilisant pour l'inactivation de microorganismes qui de préférence sont choisis dans le groupe constitué de virus, des archées, des bactéries, des spores bactériennes, des champignons, des spores fongiques, des protozoaires, des algues et des parasites transmissibles par voie sanguine.

19. Utilisation selon la revendication 18 pour le nettoyage et/ou le revêtement de la surface d'un objet.

20. Utilisation selon l'une des revendications 18 ou 19 pour le nettoyage et/ou le revêtement de la surface de produits à usage médical, d'emballages alimentaires, de textiles ; de matériaux de construction, d'appareils électroniques, de meubles ou d'articles hygiéniques.

21. Utilisation selon l'une des revendications 18 ou 19 pour la stérilisation de liquides.

22. Utilisation selon l'une des revendications 18 ou 19 pour la stérilisation de produits alimentaires.

23. Objet revêtu, la surface de l'objet présentant au moins un composé selon l'une des revendications 4 à 14.

24. Composé de formule (100) : 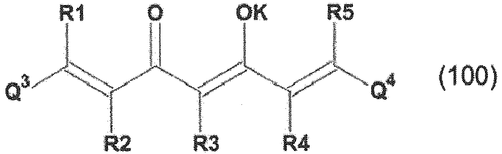 et/ou de formule (101) : 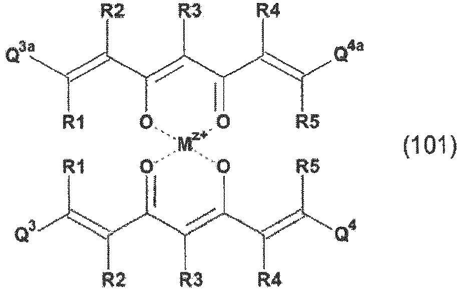 ou sel et/ou ester et/ou complexe pharmaceutiquement acceptables de chacun d'eux, pour une utilisation en tant que photosensibilisant lors du traitement médical destiné à l'inactivation de microorganismes,
dans lequel Q³, Q^{3a}, Q⁴ et Q^{4a} représentent chacun indépendamment les uns des autres un radical aromatique monocyclique ou polycyclique substitué ou non substitué ou un radical hétéroaromatique monocyclique ou polycyclique substitué ou non substitué,
dans lequel le composé de formule (100) ne contient aucun groupe OH qui soit directement lié au radical organique Q³ ou Q⁴, et
dans lequel le composé de formule (101) ne contient aucun groupe OH qui soit directement lié au radical organique Q³, Q^{3a}, Q⁴ ou Q^{4a}, et
dans lequel K représente un atome d'hydrogène ou un cation, et
dans lequel M^{z+} représente un cation d'un métal, z étant le degré d'oxydation formel du métal M et z représentant un nombre entier de 1 à 7, de préférence de 2 à 5, et
(a1) au moins l'un des radicaux Q³, Q^{3a}, Q⁴ et Q^{4a} étant indépendamment des autres un radical hétéroaromatique monocyclique ou polycyclique non substitué, qui présente au moins 5 atomes nucléaires, les atomes nucléaires contenant au moins un atome de carbone et au moins un atome d'azote de préférence protonable, ou
(a2) au moins l'un des radicaux Q³, Q^{3a}, Q⁴ et Q^{4a} étant indépendamment des autres substitué par au moins un radical organique W1, l'au moins un radical organique W1 présentant la formule générale (4), (5), (6), (7), (8) ou (9) :
-(C(D)(E)ₕ-X, (4)
-A-(C(D)(E)ₕ-X, (5)
-(C(D)(E))ₖ-Aryl-(C(D)(E)ₗ-X, (6)
-A-(C(D)(E))ₖ-Aryl-(C(D)(E))ₗ-X, (7)
-((C(D)(E))ₘ-A)ₚ-(C(D)(E))ₙ-X, (8)
-A-((C(D)(E))ₘ-A)ₚ-(C(D)(E))ₙ-X, (9)
dans lequel h représente un nombre entier de 1 à 20, dans lequel k et l représentent chacun indépendamment de l'autre un nombre entier de 0 à 10, et dans lequel m, n et p représentent chacun indépendamment des autres un nombre entier de 1 à 6, et
dans lequel chaque A représente indépendamment des autres un atome d'oxygène ou de soufre,
dans lequel D et E représentent chacun indépendamment de l'autre un atome d'hydrogène, un groupe halogéno, G-R^{(I)}, ou G-C(=G)-R^{(II)}, chaque G représentant indépendamment de l'autre un atome d'oxygène ou de soufre, et les radicaux R^{(I)} et R^{(II)} représentant chacun indépendamment de l'autre un atome d'hydrogène, un groupe méthyle, éthyle, n-propyle, n-butyle, n-pentyle, phényle ou benzyle, les groupes phényle et benzyle pouvant être non substitués ou substitués,
dans lequel aryle représente un radical aromatique substitué ou non substitué ou un radical hétéroaromatique substitué ou non substitué, qui ne contient aucun atome d'azote,
dans lequel chaque X représente indépendamment des autres un radical organique, qui contient (i) au moins un atome d'azote neutre protonable, ou (ii) au moins un atome d'azote positivement chargé, ou (iii) au moins un atome de phosphore positivement chargé, et
dans lequel les radicaux R1, R2, R3, R4 et R5 représentent chacun indépendamment des autres un atome d'hydrogène, un groupe halogéno, alkyle ayant 1 à 12 atomes de carbone, cycloalkyle ayant 1 à 12 atomes de carbone, alkylaryle ayant 1 à 12 atomes de carbone, aryle ayant 5 à 20 atomes de carbone, éther ayant 2 à 12 atomes de carbone ou glycol ayant 2 à 12 atomes de carbone, ou
(b) le radical R3 étant un radical organique W2, le radical organique W2 présentant la formule générale (4), (5), (6), (7), (8), (9) ou (10) :
-(C(D)(E))ₕ-X, (4)
-A-(C(D)(E))ₕ-X, (5)
-(C(D)(E))ₖ-Aryl-(C(D)(E))ₗ-X, (6)
-A-(C(D)(E))ₖ-Aryl-(C(D)(E))ₗ-X, (7)
-[(C(D)(E))ₘ-A]ₚ-(C(D)(E))ₙ-X, (8)
-A-[(C(D)(E))ₘ-A]ₚ-(C(D)(E))ₙ-X, (9)
-(-C(D)=C(E)-)ᵣ-X, (10)
et
dans lequel, en option, au moins l'un des radicaux Q³, Q^{3a}, Q⁴ et Q^{4a} sont, indépendamment des autres, substitué par au moins un radical organique W1 qui présente la formule générale (4), (5), (6), (7), (8) ou (9),
dans lequel h représente un nombre entier de 1 à 20, dans lequel k et l représentent chacun indépendamment de l'autre un nombre entier de 0 à 10, et dans lequel m, n et p représentent chacun indépendamment des autres un nombre entier de 1 à 6, de préférence 2 à 4, et
dans lequel chaque A représente indépendamment des autres un atome d'oxygène ou de soufre,
dans lequel D et E représentent chacun indépendamment de l'autre un atome d'hydrogène, un groupe halogéno, G-R^{(I)}, ou G-C(=G)-R^{(II)}, dans lequel chaque G représente indépendamment de l'autre un atome d'oxygène ou de soufre, et dans lequel les radicaux R^{(I)} et R^{(II)} représentent chacun indépendamment de l'autre un atome d'hydrogène, un groupe méthyle, éthyle, n-propyle, n-butyle, n-pentyle, phényle ou benzyle, les groupes phényle et benzyle pouvant être non substitués ou substitués,
dans lequel aryle représente un radical aromatique substitué ou non substitué ou un radical hétéroaromatique substitué ou non substitué, qui ne contient aucun atome d'azote,
dans lequel chaque X est indépendamment des autres un radical organique qui contient (i) au moins un atome d'azote neutre protonable, (ii) au moins un atome d'azote positivement chargé, ou (iii) au moins un atome de phosphore positivement chargé, et
dans lequel les radicaux R1, R2, R4 et R5 représentent chacun indépendamment des autres un atome d'hydrogène, un groupe halogéno, alkyle ayant 1 à 12 atomes de carbone, alkylaryle ayant 1 à 12 atomes de carbone, aryle ayant 5 à 20 atomes de carbone, éther ayant 2 à 12 atomes de carbone, ou glycol ayant 2 à 12 atomes de carbone.
